(19) Europäisches Patentamt
European Patent Office
Office européen des brevets

(11) EP 3 325 486 B1

(12) **FASCICULE DE BREVET EUROPEEN**

(45) Date de publication et mention de la délivrance du brevet:
**24.05.2023 Bulletin 2023/21**

(21) Numéro de dépôt: **16739523.5**

(22) Date de dépôt: **19.07.2016**

(51) Classification Internationale des Brevets (IPC):
***C07D 487/14*** (2006.01) ***C07D 471/14*** (2006.01)
***C07D 487/04*** (2006.01) ***C07F 5/02*** (2006.01)
***C07F 7/08*** (2006.01) ***H01L 51/00*** (2006.01)

(52) Classification Coopérative des Brevets (CPC):
**C09K 11/06; C07D 471/14; C07D 487/04; C07D 487/14; C07F 5/025; C07F 7/0812; H10K 85/6572**

(86) Numéro de dépôt international:
**PCT/EP2016/067215**

(87) Numéro de publication internationale:
**WO 2017/013135 (26.01.2017 Gazette 2017/04)**

(54) **COMPOSES POLYAZOTES ET LEURS UTILISATIONS COMME CHROMOPHORES FLUORESCENTS**

POLYSTICKSTOFFVERBINDUNGEN UND VERWENDUNGEN DAVON ALS FLUORESZIERENDE CHROMOPHOREN

POLYNITROGEN COMPOUNDS AND USES THEREOF AS FLUORESCENT CHROMOPHORES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.07.2015 FR 1556868**

(43) Date de publication de la demande:
**30.05.2018 Bulletin 2018/22**

(73) Titulaires:
- **Universite D'Orleans**
  **45067 Orleans Cedex 2 (FR)**
- **Centre National de la Recherche Scientifique**
  **75016 Paris (FR)**

(72) Inventeurs:
- **SUZENET, Franck**
  **45650 Saint Jean le Blanc (FR)**
- **SIRBU, Doina**
  **45160 Olivet (FR)**
- **GUILLAUMET, Gérald**
  **45650 Saint Jean Le Blanc (FR)**
- **BONNET, Pascal**
  **45160 Olivet (FR)**

(74) Mandataire: **Lavoix**
**2, place d'Estienne d'Orves**
**75441 Paris Cedex 09 (FR)**

(56) Documents cités:
**US-A- 3 166 567** **US-A- 3 262 942**
**US-B1- 8 273 877**

- **Q Lu ET AL: "Luminescent Nitro Derivatives of 5,11-Dehydro-5H, 11H-benzotriazolo(2,1-alpha)benzotriazole", Heteroatom Chemistry Volume, 7 août 1992 (1992-08-07), XP055230669, Extrait de l'Internet: URL:http://onlinelibrary.wiley.com/store/10.1002/hc.520040114/asset/520040114_ftp.pdf?v=1&t=ihd63j8b&s=c79f6eba0abfe496871eaa49cb9bcdcb199f35f4 [extrait le 2015-11-24]**
- **GALASSO V ET AL: "A study of the molecular structure and spectroscopic properties of benzo-and pyrido-tetraazapentalenes", CHEMICAL PHYSICS, NORTH-HOLLAND, NL, vol. 254, no. 2-3, 1 avril 2000 (2000-04-01), pages 375-384, XP027335257, ISSN: 0301-0104 [extrait le 2000-04-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



**(Cont. page suivante)**

- **DEVAN BALACHARI ET AL: "Synthesis, Thermal Stability and Impact Stability of Novel Tetranitro-Dipyridotetraazapentalene Derivatives", PROPELLANTS, EXPLOSIVES, PYROTECHNICS., vol. 25, no. 2, 1 avril 2000 (2000-04-01), pages 75-80, XP055230689, DE ISSN: 0721-3115, DOI: 10.1002/(SICI)1521-4087(200004)25:2<75::AI D-PREP75>3.0.CO;2-U**
- **MAQUESTIAU ET AL: "Nitration of Pyridinobenzotetraazapentalenes", BULLETIN DES SOCIÉTÉS CHIMIQUES BELGES : VLAAMSE CHEMISCHE VERENIGING, CENTERICK, BE, vol. 95, no. 12, 1 janvier 1986 (1986-01-01), pages 1117-1122, XP008073380, ISSN: 0037-9646**
- **DATABASE CA [Online] CHEMICAL ABSTRACTS SERVICE, COLUMBUS, OHIO, US; 1998, PIVINA, TATYANA S. ET AL: "Computer search for the structure of high-density energetic compounds among hydrogen-free heterocycles (tetraazapentalene set)", XP002751475, extrait de STN Database accession no. 1998:574371 & PIVINA, TATYANA S. ET AL: "Computer search for the structure of high-density energetic compounds among hydrogen-free heterocycles (tetraazapentalene set)", PROCEEDINGS OF THE INTERNATIONAL PYROTECHNICS SEMINAR , 24TH, 433-443 CODEN: PPYSD7; ISSN: 0270-1898, 1998,**
- **DATABASE CA [Online] CHEMICAL ABSTRACTS SERVICE, COLUMBUS, OHIO, US; 1996, POLITZER, PETER ET AL: "Computational determination of heats of formation of energetic compounds", XP002751476, extrait de STN Database accession no. 1996:176811 & POLITZER, PETER ET AL: "Computational determination of heats of formation of energetic compounds", MATERIALS RESEARCH SOCIETY SYMPOSIUM PROCEEDINGS , 418(DECOMPOSITION, COMBUSTION, AND DETONATION CHEMISTRY OF ENERGETIC MATERIALS), 55-66 CODEN: MRSPDH; ISSN: 0272-9172, 1996,**
- **NYFFENEGGER C ET AL: "An efficient route to polynitrogen-fused tricycles via a nitrene-mediated N-N bond formation under microwave irradiation", TETRAHEDRON, ELSEVIER SCIENCE PUBLISHERS, AMSTERDAM, NL, vol. 64, no. 40, 29 septembre 2008 (2008-09-29), pages 9567-9573, XP023976407, ISSN: 0040-4020, DOI: 10.1016/J.TET.2008.07.055 [extrait le 2008-07-19]**


Remarques:

Le dossier contient des informations techniques présentées postérieurement au dépôt de la demande et ne figurant pas dans le présent fascicule.

## Description

La présente invention a pour objet de nouveaux composés polyazotés, notamment des composés comprenant un motif 1,3a,6a-triazapentalène, et leurs utilisations comme chromophores fluorescents.

Elle a également pour objet l'utilisation de ces composés notamment dans le domaine de l'imagerie biologique ou de la microscopie par fluorescence.

Au cours des vingt dernières années, l'utilisation de la fluorescence dans la chimie du vivant a connu une expansion considérable. Elle rencontre actuellement des applications dans les biotechnologies, en cytométrie en flux, dans le diagnostic médical, dans le séquençage d'ADN, en imagerie cellulaire, etc. Dans de nombreuses applications, la haute sensibilité de cette technique a notamment permis de remplacer certains marqueurs basés sur des éléments radioactifs, dont l'application est hautement délicate. L'expansion de ces techniques a été consacrée en 2008, lors de la reconnaissance des applications de la GFP (Green Fluorescent Protein). Les technologies actuelles font de la fluorescence un domaine d'importance capitale dans la science du vivant.

Parmi les diverses classes de marqueurs fluorescents, les fluorophores organiques se démarquent grâce à leur propriétés photophysiques uniques. Ces derniers sont représentés principalement par les Cyanines, les Fluoresceines, les Rhodamines, les Alexa fluor et les Bodipy. Leurs emplois au sein de différents domaines sont divers et variés. Bien que largement utilisés, ces composés souffrent encore de défauts qui diminuent leur étendue d'action dans le domaine de la biologie. En particulier, ces composés présentent l'inconvénient d'avoir un faible déplacement de Stokes dû à la proximité énergétique des ondes d'émission et d'absorption (de l'ordre de 20 nm pour les Cyanines, 10-20 nm pour les Bodipy, 30 nm pour les Fluoresceines et 20 nm pour les Rhodamines). Il s'agit également de composés avec des structures chimiques complexes, obtenus par des voies de synthèses fastidieuses. Certains de ces composés présentent également une faible solubilité dans les milieux aqueux.

L'article de Qi Lu et al. (Heteroatom Chemistry, 1992) concerne des dérivés nitrés luminescents du benzotriazolo[2,1 a]benzotriazole, pouvant être utiles comme fluorophores.

Il existe donc à ce jour un besoin de fournir des composés fluorescents obtenus par des voies de synthèse plus simples, de préférence solubles en milieu aqueux, et présentant un déplacement de Stokes élevé.

Un but de la présente invention consiste à fournir de nouveaux composés fluorescents, notamment solubles dans l'eau.

L'invention a également pour but de fournir de nouveaux composés polycycliques fluorescents de faible poids moléculaire par rapport aux composés connus à ce jour.

L'invention a également pour but de fournir de nouveaux composés fluorescents présentant des déplacements de Stokes importants et des rendements quantiques élevés, en particulier supérieurs à 10%, notamment jusqu'à 55% dans le dichlorométhane, et des coefficients d'absorption élevés, de préférence jusqu'à 18 000.

L'invention a également pour but de fournir de nouveaux composés fluorescents stables en milieu aqueux à pH acide, neutre et basique.

Un but de la présente invention consiste à fournir de nouveaux composés fluorescents destinés à la préparation de bioconjugués pour la détection de molécules biologiques. L'invention est définie par les revendications annexées. La description qui suit est soumise à cette limitation. Tous les aspects et réalisations qui sont indiqués comme "aspect de l'invention" mais qui ne sont pas couverts par les revendications ne sont que des aspects de la présente divulgation et ne font pas partie de l'invention. Les composés qui ne relèvent pas de la portée des revendications sont inclus en tant qu'exemples de référence.

Ainsi, la présente demande décrit l'utilisation comme chromophore fluorescent d'un composé de formule (I) :

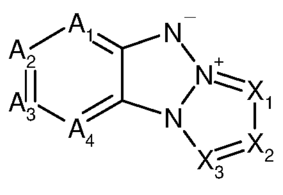

(I)

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, des groupes électrodonneurs et des groupes électroattracteurs,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$ sont choisis indépendamment les uns des autres dans le groupe constitué de H, des groupes électrodonneurs et des groupes électroattracteurs,

ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,

ledit composé de formule (I) pouvant être sous forme de sel ou de stéréoisomère pur ou sous forme de mélange d'énantiomères et/ou de diastéréoisomères, y compris de mélanges racémiques, ou sous forme de tautomères, à l'exception du composé de formule :

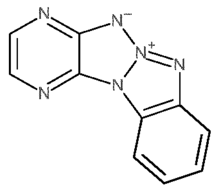

La présente demande décrit également en tant que tels les composés de formule (I) suivante : dans laquelle :

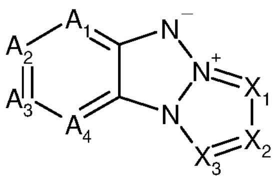

(I)

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, des groupes électrodonneurs et des groupes électroattracteurs,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$ sont choisis indépendamment les uns des autres dans le groupe constitué de H, des groupes électrodonneurs et des groupes électroattracteurs,

ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,

ledit composé de formule (I) pouvant être sous forme de sel ou de stéréoisomère pur ou sous forme de mélange d'énantiomères et/ou de diastéréoisomères, y compris de mélanges racémiques, ou sous forme de tautomères,

à l'exception des composés suivants :

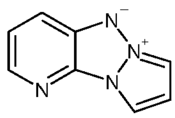
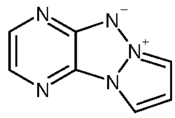
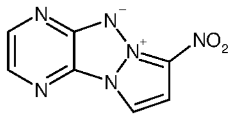
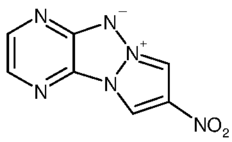
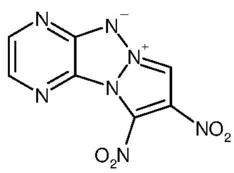
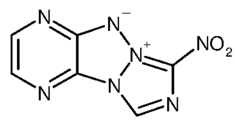
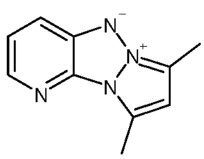
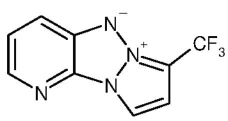
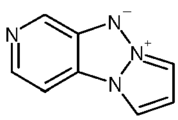
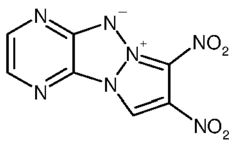
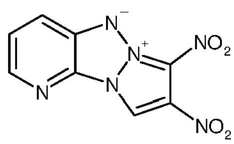
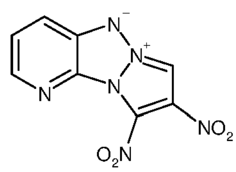
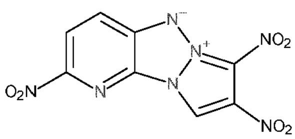
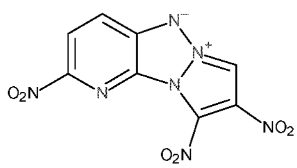
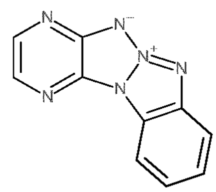

Selon l'invention, le composé de formule (I) peut exister sous différentes formes tautomères. Aussi, la présente invention a également pour objet les formes tautomères des composés de formule (I).

En particulier, les composés de formule (I) peuvent être aussi représentés par la formule suivante :

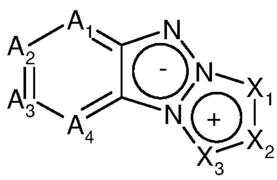

La présente invention concerne l'utilisation comme chromophore fluorescent d'un composé de formule (I) :

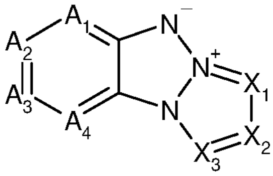

(I)

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, ($C_1$-$C_6$)alkyle, ($C_6$-$C_{10}$)aryle, hétéroaryle comprenant de 5 à 10 atomes, ($C_2$-$C_6$)alcényle, ($C_2$-$C_6$)alcynyle, CN, hydroxy, ($C_1$-$C_6$)alcoxy, thiol, ($C_1$-$C_6$)alkylthio, $(CH_2)_nSO_2$-$OR_a$ où n = 1-6, $CH_2SO_2$-$NR_aR_b$, $NO_2$, $SO_3R_a$, $NR_aR_b$, C(O)$OR_a$, C(O)$R_a$, et C(O)$NR_aR_b$, $R_a$ et $R_b$ représentant H ou un groupe ($C_1$-$C_6$)alkyle,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$, représentent, indépendamment les uns des autres, un atome d'hydrogène ou un atome d'halogène, ou sont choisis dans le groupe constitué de :

  . un groupe ($C_1$-$C_6$)alkyle, ($C_3$-$C_6$)cycloalkyle ou ($C_6$-$C_{10}$)aryle,
  ledit groupe ($C_6$-$C_{10}$)aryle étant éventuellement substitué par au moins un substituant choisi parmi : halogène, CN, ($C_1$-$C_6$)alkyle, OH, ($C_1$-$C_6$)alcoxy, C(O)$OR_a$ et $NR_aR_b$, $R_a$ et $R_b$ représentant H ou un groupe ($C_1$-$C_6$)alkyle,
  . un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, ($C_1$-$C_6$)alkyle, OH, ($C_1$-$C_6$)alcoxy, C(O)$OR_a$, C(O)$R_a$ et $NR_aR_b$, $R_a$ et $R_b$ représentant H ou un groupe ($C_1$-$C_6$)alkyle,
  . un groupe hétérocycloalkyle comprenant de 4 à 10 chaînons et contenant de un à trois hétéroatomes choisis parmi O, S ou N,
  . $NO_2$,
  . CHO,
  . un groupe C(O)$OR_a$, $R_a$ étant tel que défini ci-dessus,
  . un groupe -HC=CH-Ar, Ar représentant un groupe ($C_6$-$C_{10}$)aryle,
  . $SO_3H$,
  . un groupe $NR_cR_d$, $R_c$ et $R_d$ représentant H ou un groupe ($C_1$-$C_6$)alkyle, ou pouvant former un groupe hétéro($C_2$-$C_5$)cycloalkyle avec l'atome d'azote qui les porte ;
  . un groupe $OR_e$, $R_e$ représentant H, un groupe ($C_1$-$C_6$)alkyle ou un groupe ($C_6$-$C_{10}$)aryle ; et
  . un groupe de formule (A) suivante :

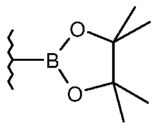

(A)

ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,

ledit composé de formule (I) pouvant être sous forme de sel ou de stéréoisomère pur ou sous forme de mélange d'énantiomères et/ou de diastéréoisomères, y compris de mélanges racémiques, ou sous forme de tautomères,

à l'exception du composé de formule :

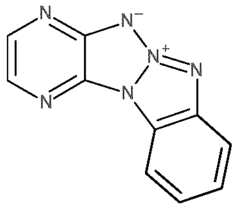

.

La présente invention concerne également un composé de formule (I) suivante :

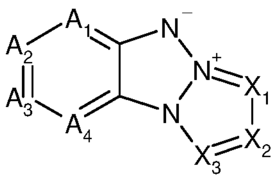

(I)

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, ($C_1$-$C_6$)alkyle, ($C_6$-$C_{10}$)aryle, hétéroaryle comprenant de 5 à 10 atomes, ($C_2$-$C_6$)alcényle, ($C_2$-$C_6$)alcynyle, CN, hydroxy, ($C_1$-$C_6$)alcoxy, thiol, ($C_1$-$C_6$)alkylthio, $(CH_2)_nSO_2$-$OR_a$ où n = 1-6, $CH_2SO_2$-$NR_aR_b$, $NO_2$, $SO_3R_a$, $NR_aR_b$, C(O)$OR_a$, C(O)$R_a$, et C(O)$NR_aR_b$, $R_a$ et $R_b$ représentant H ou un groupe ($C_1$-$C_6$)alkyle,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;

- $Y_5$, $Y_6$ et $Y_7$, représentent, indépendamment les uns des autres, un atome d'hydrogène ou un atome d'halogène, ou sont choisis dans le groupe constitué de :

  . un groupe $(C_1\text{-}C_6)$alkyle, $(C_3\text{-}C_6)$cycloalkyle ou $(C_6\text{-}C_{10})$aryle, ledit groupe $(C_6\text{-}C_{10})$aryle étant éventuellement substitué par au moins un substituant choisi parmi : halogène, CN, $(C_1\text{-}C_6)$alkyle, OH, $(C_1\text{-}C_6)$alcoxy, $C(O)OR_a$ et $NR_aR_b$, $R_a$ et $R_b$ représentant H ou un groupe $(C_1\text{-}C_6)$alkyle,
  . un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, $(C_1\text{-}C_6)$alkyle, OH, $(C_1\text{-}C_6)$alcoxy, $C(O)OR_a$, $C(O)R_a$ et $NR_aR_b$, $R_a$ et $R_b$ représentant H ou un groupe $(C_1\text{-}C_6)$alkyle,
  . un groupe hétérocycloalkyle comprenant de 4 à 10 chaînons et contenant de un à trois hétéroatomes choisis parmi O, S ou N,
  . $NO_2$,
  . CHO,
  . un groupe $C(O)OR_a$, $R_a$ étant tel que défini ci-dessus,
  . un groupe -HC=CH-Ar, Ar représentant un groupe $(C_6\text{-}C_{10})$aryle,
  . $SO_3H$,
  . un groupe $NR_cR_d$, $R_c$ et $R_d$ représentant H ou un groupe $(C_1\text{-}C_6)$alkyle, ou pouvant former un groupe hétéro$(C_2\text{-}C_5)$cycloalkyle avec l'atome d'azote qui les porte ;
  . un groupe $OR_e$, $R_e$ représentant H, un groupe $(C_1\text{-}C_6)$alkyle ou un groupe $(C_6\text{-}C_{10})$aryle ; et
  . un groupe de formule (A) suivante :

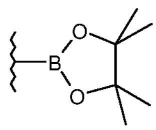

(A)

ledit composé de formule (I) pouvant être sous forme de sel ou de stéréoisomère pur ou sous forme de mélange d'énantiomères et/ou de diastéréoisomères, y compris de mélanges racémiques, ou sous forme de tautomères,

à l'exception des composés suivants :

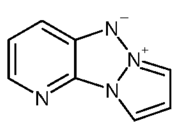
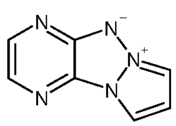
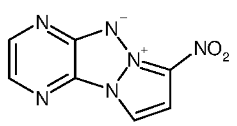
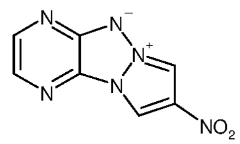
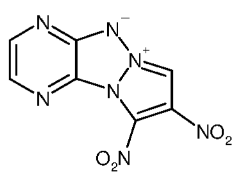
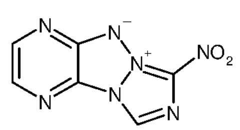
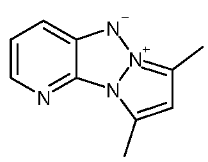
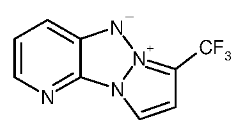
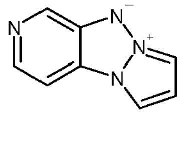
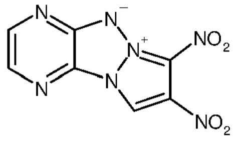
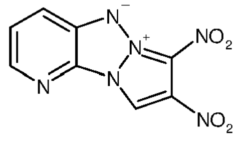
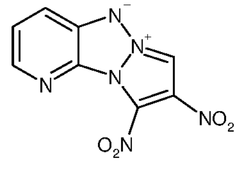

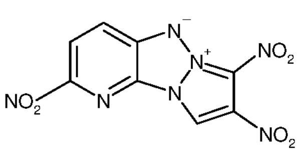 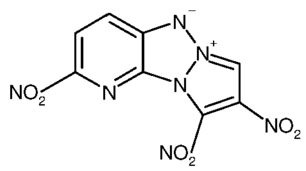

Selon l'invention, le terme "chromophore fluorescent" désigne une molécule pouvant réémettre de la lumière après excitation avec un rendement quantique supérieur à 0,001 (0,1%).

Selon l'invention, le terme "(hétéro)cycloalkyle" englobe à la fois les termes "cycloalkyle" et "hétérocycloalkyle", ces termes étant tels que définis ci-après.

Selon l'invention, le terme "(hétéro)aryle" englobe à la fois les termes "aryle" et "hétéroaryle", ces termes étant tels que définis ci-après.

Selon l'invention, lorsque des groupes $Y_i$ et $Y_{i+1}$ (i étant un entier allant de 1 à 6) forment ensemble, avec les atomes de carbone qui les portent, un groupe (hétéro)cycloalkyle, éventuellement substitué, ou un groupe (hétéro)aryle, éventuellement substitué, comprenant de 5 à 10 atomes, ces groupes cycliques peuvent être soit un monocycle soit des bicycles ou mêmes des (poly)cycles. Ils peuvent donc également former des cycles fusionnés eux-mêmes.

Selon l'invention, le terme "groupe électrodonneur" désigne un atome ou groupe d'atome qui partage une partie de sa densité électronique avec un système conjugué voisin (ici le motif de la formule (I)) par résonnance (effet mésomère) ou effet inductif.

Parmi les groupes électrodonneurs préférés, on peut citer les groupes hydroxy, alcoxy, thiol, alkylthio, alkyle, alcène, alcyne, amino, alkylamino et aryle, lui-même porteur d'un ou plusieurs groupes électrodonneurs.

Selon l'invention, le terme "groupe électroattracteur" désigne un atome ou groupe d'atome qui attire, généralement par résonnance ou effet inductif, une partie de la densité électronique d'un voisin (ici le motif de la formule (I)) sur lequel il est fixé.

Parmi les groupes électroattracteurs préférés, on peut citer les groupes halogénoalkyle, nitro, acyle, carboxyle, ester, formyle, sulfonyle, trifluorométhyle, cyano, halogène et aryle, lui-même porteur d'un ou plusieurs groupes électroattracteurs.

Selon l'invention, dans la formule (I) susmentionnée, $Y_1$, $Y_2$, $Y_3$ et $Y_4$ sont choisis indépendamment les uns des autres dans le groupe constitué de : H, halogène, $(C_1\text{-}C_6)$alkyle, $(C_6\text{-}C_{10})$aryle, hétéroaryle comprenant de 5 à 10 atomes, $(C_2\text{-}C_6)$alcényle, $(C_2\text{-}C_6)$alcynyle, CN, hydroxy, $(C_1\text{-}C_6)$alcoxy, thiol, $(C_1\text{-}C_6)$alkylthio, $(CH_2)_nSO_2\text{-}OR_a$ où n = 1-6, $CH_2SO_2\text{-}NR_aR_b$, $NO_2$, $SO_3R_a$, $NR_aR_b$, $C(O)OR_a$, $C(O)R_a$, et $C(O)NR_aR_b$, $R_a$ et $R_b$ représentant H ou un groupe $(C_1\text{-}C_6)$alkyle. Selon un mode de réalisation, dans la formule (I) selon l'invention, $Y_1$, $Y_2$, $Y_3$ et $Y_4$ peuvent représenter un groupe éthynyle, substitué par un groupe phényle, éventuellement substitué par un ou plusieurs substituants. Parmi les substituants éventuels de ce groupe phényle, on peut citer les groupes suivants : amino, hydroxy, thiol, oxo, halogène, alkyle, alcoxy, alkylthio, alkylamino, aryloxy, arylalcoxy, cyano, nitro, trifluorométhyle, sulfonate, carboxy ou carboxyalkyle.

Lorsque $Y_2$, $Y_3$ et $Y_4$ sont choisis parmi les groupes $(C_1\text{-}C_6)$alkyle, $(C_6\text{-}C_{10})$aryle, hétéroaryle, $(C_2\text{-}C_6)$alcényle ou $(C_2\text{-}C_6)$alcynyle, ceux-ci peuvent comprendre en outre au moins un substituant. Parmi ces substituants, on peut citer les groupes suivants : amino, hydroxy, thiol, oxo, halogène, alkyle, alcoxy, alkylthio, alkylamino, aryloxy, arylalcoxy, cyano, nitro, trifluorométhyle, sulfonate, carboxy ou carboxyalkyle.

Selon l'invention, dans la formule (I) susmentionnée, $Y_5$, $Y_6$ et $Y_7$ représentent, indépendamment les uns des autres, un atome d'hydrogène ou un atome d'halogène, ou sont choisis dans le groupe constitué de :

. un groupe $(C_1\text{-}C_6)$alkyle, $(C_3\text{-}C_6)$cycloalkyle ou $(C_6\text{-}C_{10})$aryle,
ledit groupe $(C_6\text{-}C_{10})$aryle étant éventuellement substitué par au moins un substituant choisi parmi : halogène, CN, $(C_1\text{-}C_6)$alkyle, OH, $(C_1\text{-}C_6)$alcoxy, $C(O)OR_a$ et $NR_aR_b$, $R_a$ et $R_b$ représentant H ou un groupe $(C_1\text{-}C_6)$alkyle,
. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, $(C_1\text{-}C_6)$alkyle, OH, $(C_1\text{-}C_6)$alcoxy, $C(O)OR_a$, $C(O)R_a$ et $NR_aR_b$, $R_a$ et $R_b$ représentant H ou un groupe $(C_1\text{-}C_6)$alkyle,
. un groupe hétérocycloalkyle comprenant de 4 à 10 chaînons et contenant de un à trois hétéroatomes choisis parmi O, S ou N,
. $NO_2$,
. CHO,
. un groupe $C(O)OR_a$, $R_a$ étant tel que défini ci-dessus,
. un groupe -HC=CH-Ar, Ar représentant un groupe $(C_6\text{-}C_{10})$aryle,
. $SO_3H$ (ou son sel),
. un groupe $NR_cR_d$, $R_c$ et $R_d$ représentant H ou un groupe $(C_1\text{-}C_6)$alkyle, ou pouvant former un groupe hé-

téro($C_2$-$C_5$)cycloalkyle avec l'atome d'azote qui les porte ;
. un groupe $OR_e$, $R_e$ représentant H, un groupe ($C_1$-$C_6$)alkyle ou un groupe ($C_6$-$C_{10}$)aryle ; et
. un groupe de formule (A) suivante :

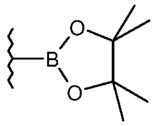

(A)

Selon un mode de réalisation, dans la formule (I) selon l'invention, $Y_5$, $Y_6$ et $Y_7$ peuvent représenter un groupe éthynyle, substitué par un groupe phényle, éventuellement substitué. Parmi les substituants éventuels de ce groupe phényle, on peut citer les groupes suivants : amino, hydroxy, thiol, oxo, halogène, alkyle, alcoxy, alkylthio, alkylamino, aryloxy, arylalcoxy, cyano, nitro, trifluorométhyle, sulfonate, carboxy ou carboxyalkyle.

Dans le cadre de la présente invention, on entend par groupe ($C_t$-$C_z$) un groupe comprenant une chaîne carbonée pouvant avoir de t à z atomes de carbone, par exemple $C_1$-$C_6$ une chaîne carbonée qui peut avoir de 1 à 6 atomes de carbone.

Dans le cadre de la présente invention, le terme "atome d'halogène" désigne les atomes fluor, chlore, brome ou iode.

Selon l'invention, le terme "alkyle" désigne des groupes aliphatiques hydrocarbonés, saturés, linéaires ou ramifiés comprenant, sauf mention contraire, de 1 à 6 atomes de carbone. A titre d'exemples, on peut citer les groupes méthyle, éthyle, n-propyle, isopropyle, butyle, isobutyle, tertbutyle ou pentyle.

Selon l'invention, le terme "cycloalkyle" désigne des groupes carbonés cycliques comprenant, sauf mention contraire, de 3 à 6 atomes de carbone. A titre d'exemples, on peut citer les groupes cyclopropyle, cyclobutyle, cyclopentyle ou cyclohexyle.

Selon l'invention, le terme "halogénoalkyle" désigne un groupe alkyle tel que défini ci-dessus, dans lequel un ou plusieurs des atomes d'hydrogène est(sont) remplacé(s) par un atome d'halogène. A titre d'exemple, on peut citer les fluoroalkyles, en particulier $CF_3$ ou $CHF_2$.

Selon l'invention, le terme "alcoxy" désigne un radical -O-alkyle où le groupe alkyle est tel que précédemment défini. A titre d'exemples, on peut citer les groupes -O-($C_1$-$C_4$)alkyle, et en particulier le groupe -O-méthyle, le groupe -O-éthyle, comme groupe -O-Csalkyle, le groupe -O-propyle, -O-isopropyle, et comme groupe -O$C_4$alkyle, le groupe -O-butyle, -O-isobutyle, -O-tertbutyle.

Selon l'invention, les groupes aryles sont des groupes aromatiques cycliques comprenant entre 6 et 10 atomes de carbone. A titre d'exemples de groupes aryles, on peut citer les groupes phényle ou naphtyle.

Selon l'invention, le terme "hétéroaryle" désigne un groupe monocyclique ou bicyclique aromatique de 5 à 10 chaînons contenant de 1 à 4 hétéroatomes choisis parmi O, S ou N. Selon l'invention, le terme hétéroaryle bicyclique inclue les bicycles aromatiques fusionnés.

A titre d'exemples, on peut citer les groupes imidazolyle, thiazolyle, oxazolyle, furanyle, thiophényle, pyrazolyle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle.

A titre d'hétéroaryle comprenant 5 à 6 atomes, dont 1 à 4 atomes d'azote, on peut notamment citer les groupes représentatifs suivants : pyrrolyle, pyrazolyle, 1,2,3-triazolyle, 1,2,4-triazolyle, tétrazolyle et 1,2,3-triazinyle.

On peut également citer, à titre d'hétéroaryle, le thiophényle, l'oxazolyle, le furazanyle, le 1,2,4-thiadiazolyle, le naphthyridinyle, le quinoxalinyle, le phtalazinyle, l'imidazo[1,2-a]pyridine, l'imidazo[2,1-b]thiazolyle, le cinnolinyle, le benzofurazanyle, l'azaindolyle, le benzimidazolyle, le benzothiophényle, le thiénopyridyle, le thiénopyrimidinyle, le pyrrolopyridyle, l'imidazopyridyle, le benzoazaindole, le 1,2,4-triazinyle, l'indolizinyle, l'isoxazolyle, l'isoquinolinyle, l'isothiazolyle, le purinyle, le quinazolinyle, le quinolinyle, l'isoquinolyle, le 1,3,4-thiadiazolyle, le thiazolyle, l'isothiazolyle, le carbazolyle, ainsi que les groupes correspondants issus de leur fusion ou de la fusion avec le noyau phényle.

Selon l'invention le terme "hétérocycloalkyle" désigne un groupe monocyclique ou bicyclique saturé ou partiellement insaturé de 4 à 10 chaînons, comprenant de un à trois hétéroatomes choisis parmi O, S ou N, le groupe hétérocycloalkyle pouvant être rattaché au reste de la molécule par un atome de carbone ou par un hétéroatome. Selon l'invention, le terme hétérocycloalkyle bicyclique inclue les bicycles fusionnés.

A titre d'hétérocycloalkyle saturé comprenant de 5 à 6 atomes, on peut citer l'oxétanyle, le tétrahydrofuranyle, le dioxolanyle, le pyrrolidinyle, l'azépinyle, l'oxazépinyle, le pyrazolidinyle, l'imidazolidinyle, le tétrahydrothiophényle, le dithiolanyle, le thiazolidinyle, le tétrahydropyranyle, le tétrahydropyridinyle, le dioxanyle, le morpholinyle, le pipéridinyle, le pipérazinyle, le tétrahydrothiopyranyle, le dithianyle, le thiomorpholinyle, ou l'isoxazolidinyle.

Selon l'invention, le terme "alkylthio" désigne un groupe -S-alkyle, le groupe alkyle étant tel que défini ci-dessus.

Selon l'invention, le terme "alkylamino" désigne un groupe -NH-alkyle ou N(alkyle)$_2$, le groupe alkyle étant tel que défini ci-dessus.

Selon l'invention, le terme "aryloxy" désigne un groupe -O-aryle, le groupe aryle étant tel que défini ci-dessus.

Selon l'invention, le terme "arylalcoxy" désigne un groupe aryl-alcoxy-, les groupes aryles et alcoxy étant tels que définis ci-dessus.

Selon l'invention, le terme "carboxyalkyle" désigne un groupe HOOC-alkyl-, le groupe alkyle étant tel que défini ci-dessus. Comme exemple de groupes carboxyalkyles, on peut citer notamment le carboxyméthyle ou le carboxyéthyle.

Selon l'invention, le terme "carboxyle" désigne un groupe COOH et le terme "oxo" désigne un groupe C(O).

Les radicaux "alkyle", "cycloalkyle", "aryle", "hétéroaryle" et "hétérocycloalkyle" susmentionnés peuvent être substitués par un ou plusieurs substituants. Parmi ces substituants, on peut citer les groupes suivants : amino, hydroxy, thiol, oxo, halogène, alkyle, alcoxy, alkylthio, alkylamino, aryloxy, arylalcoxy, cyano, trifluorométhyle, carboxy ou carboxyalkyle.

Lorsqu'un radical alkyle est substitué par un groupe aryle, on parle de radical "arylalkyle" ou "aralkyle". Les radicaux "arylalkyles" ou "aralkyles" sont des radicaux aryl-alkyl-, les groupes aryles et alkyles étant tels que définis ci-dessus. Parmi les radicaux arylalkyles, on peut notamment citer le radical benzyle ou phénéthyle.

Une famille particulière de composés selon l'invention, et utilisés comme chromophores fluorescents, est constituée des composés de formule (I) dans laquelle $A_1$ est -N-.

Une telle famille de composés répond à la formule (II) suivante :

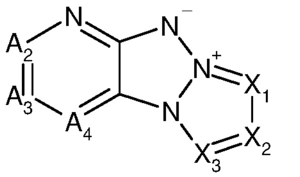

(II)

$A_2$, $A_3$, $A_4$, $X_1$, $X_2$ et $X_3$ étant tels que définis ci-dessus dans la formule (I).

Une autre famille particulière de composés selon l'invention, et utilisés comme chromophores fluorescents, est constituée des composés de formule (I) dans laquelle $A_1$ et $A_4$ sont -N-, $A_2$ est -C($Y_2$)- et $A_3$ est -C($Y_3$)-.

Cette famille de composés, dérivés de pyrazine, répond à la formule (III) suivante :

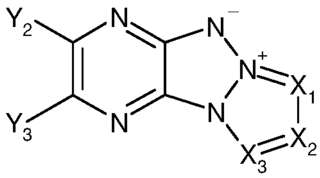

(III)

$Y_2$, $Y_3$, $X_1$, $X_2$ et $X_3$ étant tels que définis ci-dessus dans la formule (I).

De préférence, dans la formule (III), $Y_2$ et $Y_3$ sont H ou un groupe électroattracteur. Préférentiellement, dans la formule (III), $Y_2$ et $Y_3$ sont H.

Une autre famille particulière de composés selon l'invention, et utilisés comme chromophores fluorescents, est constituée des composés de formule (I) dans laquelle $A_1$ et $A_4$ sont -N-, $A_2$ est -CH- et $A_3$ est -C($Y_3$)-.

Cette famille de composés répond à la formule (III-1) suivante :

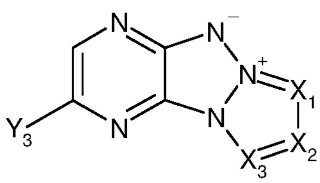

(III-1)

$Y_3$, $X_1$, $X_2$ et $X_3$ étant tels que définis ci-dessus dans la formule (I).

De préférence, dans la formule (III-1), $Y_3$ est un groupe électroattracteur. De préférence, $Y_3$ est un groupe (hétéro)aryle, éventuellement substitué, tel que défini ci-dessus.

Une autre famille particulière de composés selon l'invention, et utilisés comme chromophores fluorescents, est constituée des composés de formule (I) dans laquelle $Y_2$ et $Y_3$ forment ensemble, avec les atomes de carbone qui les portent, un groupe phényle, éventuellement substitué, ou un groupe hétérocycloalkyle, éventuellement substitué.

Parmi les éventuels substituants du groupe phényle, on peut par exemple citer les groupes amino, hydroxy, thiol, oxo, halogène, alkyle, alcoxy, alkylthio, alkylamino, aryloxy, arylalcoxy, cyano, trifluorométhyle, carboxy ou carboxyalkyle.

On peut par exemple citer les composés répondant à la formule (IV) suivante :

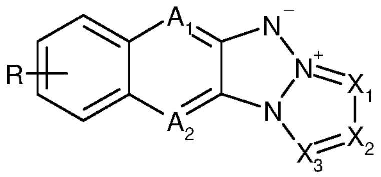

(IV)

$A_1$, $A_2$, $X_1$, $X_2$ et $X_3$ étant tels que définis ci-dessus dans la formule (I),

R représentant H ou au moins un substituant choisi parmi les substituants mentionnés ci-dessus.

De préférence, dans la formule (IV), $A_1$ et $A_2$ sont -N-.

De préférence, dans la formule (IV), R est H.

Une autre famille particulière de composés selon l'invention, et utilisés comme chromophores fluorescents, est constituée des composés de formule (I) dans laquelle $X_1$ est $-C(Y_5)-$, $X_2$ est $-C(Y_6)-$ et $X_3$ est $-C(Y_7)-$.

Une telle famille de composés répond à la formule (V) suivante :

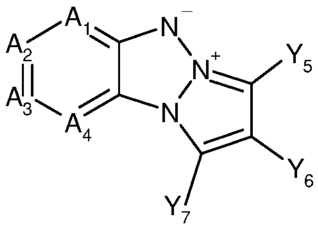

(V)

$A_1$, $A_2$, $A_3$, $A_4$, $Y_5$, $Y_6$ et $Y_7$ étant tels que définis ci-dessus dans la formule (I).

Selon un mode de réalisation, dans la formule (V), $A_1$ et $A_4$ sont -N- et $A_2$ et $A_3$ sont -CH-. Une telle sous-famille de composés répond à la formule (V-1) suivante :

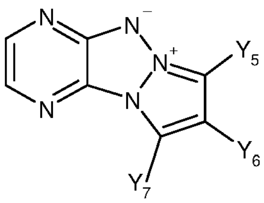

(V-1)

$Y_5$, $Y_6$ et $Y_7$ étant tels que définis ci-dessus dans la formule (I).

De préférence, dans la formule (V) ou (V-1), un seul des $Y_5$, $Y_6$ et $Y_7$ est différent de H.

Une sous-famille de composés de formule (V) est constituée par des composés de formule (V-2) ci-dessous :

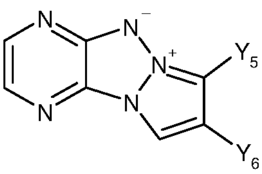

(V-2)

$Y_5$ et $Y_6$ étant tels que définis ci-dessus dans la formule (I).

Une sous-famille de composés de formule (V) est constituée par des composés de formule (V-3) ci-dessous :

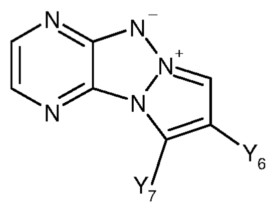

(V-3)

$Y_6$ et $Y_7$ étant tels que définis ci-dessus dans la formule (I).

Une autre famille particulière de composés selon l'invention, et utilisés comme chromophores fluorescents, est constituée des composés de formule (I) dans laquelle $X_1$ est -CH-, $X_2$ est -C($Y_6$)- et $X_3$ est -CH-, $Y_6$ étant différent de H.

Une telle famille de composés répond à la formule (VI) suivante :

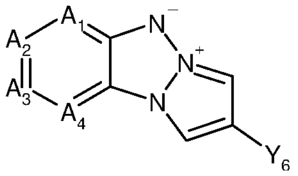

(VI)

$A_1$, $A_2$, $A_3$, $A_4$ et $Y_6$ étant tels que définis ci-dessus dans la formule (I).

Les composés de formule (VI) correspondent aux composés de formule (V) dans laquelle $Y_5$=$Y_7$=H.

Selon un mode de réalisation, dans la formule (VI) susmentionnée, $A_1$ et $A_4$ sont -N- et $A_2$ et $A_3$ sont -CH-. Cette famille de composés répond à la formule (VI-1) suivante :

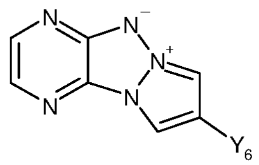

(VI-1)

$Y_6$ étant tel que défini ci-dessus dans la formule (I).

De préférence, dans les formules (VI) ou (VI-1), $Y_6$ représente un atome d'hydrogène ou un atome d'halogène, notamment Br ou I, ou est choisi dans le groupe constitué de :

. un groupe ($C_6$-$C_{10}$)aryle, de préférence phényle, éventuellement substitué par au moins un substituant, de préférence en para, choisi parmi : CN, ($C_1$-$C_6$)alcoxy, C(O)$OR_a$ et $NR_aR_b$, $R_a$ et $R_b$ représentant H ou un groupe ($C_1$-$C_6$)alkyle,
. un groupe hétéroaryle comprenant de 5 à 10 chaînons et contenant de 1 à 4 hétéroatomes choisis parmi O, S ou N, de préférence thiophényle, éventuellement substitué par C(O)$R_a$, $R_a$ étant tel que défini ci-dessus, ou pyrimidinyle,
. COOH,
. CH=CH-phényle,
. $NO_2$, et
. un groupe de formule (A) telle que définie ci-dessus.

Une autre famille particulière de composés selon l'invention, et utilisés comme chromophores fluorescents, est constituée des composés de formule (I) dans laquelle $X_1$ est -C($Y_5$)-, $X_2$ est -CH- et $X_3$ est -CH-, $Y_5$ étant différent de H.

Une telle famille de composés répond à la formule (VII) suivante :

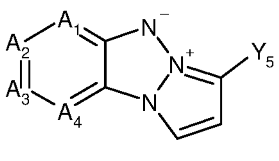

(VII)

$A_1$, $A_2$, $A_3$, $A_4$ et $Y_5$ étant tels que définis ci-dessus dans la formule (I).

Les composés de formule (VII) correspondent aux composés de formule (V) dans laquelle $Y_6$=$Y_7$=H.

Selon un mode de réalisation, dans la formule (VII) susmentionnée, $A_1$ et $A_4$ sont -N- et $A_2$ et $A_3$ sont -CH-. Cette famille de composés répond à la formule (VII-1) suivante :

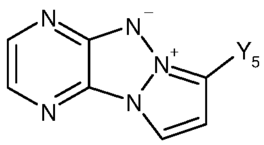

(VII-1)

$Y_5$ étant tel que défini ci-dessus dans la formule (I).
De préférence, dans les formules (VII) ou (VII-1), $Y_5$ représente $NO_2$, un halogène, notamment I, un groupe aryle, notamment phényle, ou un groupe $OR_e$, $R_e$ représentant H ou un groupe $(C_1-C_6)$alkyle.
Une autre famille particulière de composés selon l'invention, et utilisés comme chromophores fluorescents, est constituée des composés de formule (I) dans laquelle l'un au moins des $X_1$ et $X_2$ est -N- et $X_3$ est $-C(Y_7)-$.
Dans cette famille de composés, on peut mentionner les sous-familles suivantes :

- composés de formule (VIII) suivante :

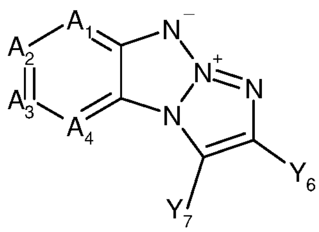

(VIII)

$A_1$, $A_2$, $A_3$, $A_4$, $Y_6$ et $Y_7$ étant tels que définis ci-dessus dans la formule (I) ; et

- composés de formule (IX) suivante :

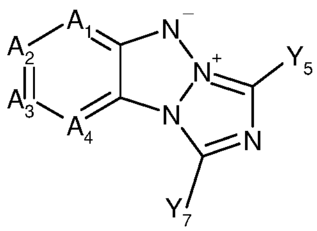

(IX)

$A_1$, $A_2$, $A_3$, $A_4$, $Y_5$ et $Y_7$ étant tels que définis ci-dessus dans la formule (I).

Selon un mode de réalisation, dans la formule (VIII), $Y_6$ et $Y_7$ sont H.
Selon un mode de réalisation, dans la formule (IX), $Y_5$ et $Y_7$ sont H.
Selon un mode de réalisation, dans la formule (IX), $Y_5$ est $NO_2$ et $Y_7$ est H.
Selon un mode de réalisation, dans la formule (IX), $Y_5$ est un groupe alkyle, notamment méthyle, et $Y_7$ est un hétérocycloalkyle, notamment morpholine.
Selon un mode de réalisation, dans les formules (VIII) et (IX), $A_1$ et $A_4$ sont -Net $A_2$ et $A_3$ sont -CH-.
Une autre famille particulière de composés selon l'invention, et utilisés comme chromophores fluorescents, est constituée des composés de formule (I) dans laquelle $Y_6$ et $Y_7$ forment ensemble, avec les atomes de carbone qui les portent, un groupe (hétéro)aryle, éventuellement substitué, de préférence un groupe phényle ou un groupe pyrazine.
De préférence, pour cette famille de composés, $X_1$ est -CH-.
Parmi les éventuels substituants du groupe phényle susmentionné, on peut par exemple citer les groupes amino, hydroxy, thiol, oxo, halogène, hétéroaryle, alkyle, alcoxy, alkylthio, alkylamino, aryloxy, arylalcoxy, cyano, trifluorométhyle, nitro, carboxy ou carboxyalkyle, ou encore un groupe $-C(O)-NH-CH_2-C\equiv C$, ou un groupe $SO_3H$ ou SOsNa.
Une autre famille particulière de composés selon l'invention, et utilisés comme chromophores fluorescents, est constituée des composés de formule (VIII) dans laquelle $Y_6$ et $Y_7$ forment ensemble, avec les atomes de carbone qui les portent, un groupe aryle, éventuellement substitué, de préférence un groupe phényle.

Parmi les éventuels substituants du groupe phényle susmentionné, on peut par exemple citer les groupes amino, hydroxy, thiol, oxo, halogène, hétéroaryle, alkyle, alcoxy, alkylthio, alkylamino, aryloxy, arylalcoxy, cyano, trifluorométhyle, nitro, carboxy ou carboxyalkyle, ou encore un groupe $-C(O)-NH-CH_2-C =C$, ou un groupe $SO_3H$ ou SOsNa.

Une autre famille particulière de composés selon l'invention, et utilisés comme chromophores fluorescents, est constituée des composés de formule (V-2) dans laquelle $Y_5$ et $Y_6$ forment ensemble, avec les atomes de carbone qui les portent, un groupe (hétéro)aryle, éventuellement substitué, de préférence un groupe phényle ou pyrazinyle.

Parmi les éventuels substituants du groupe phényle susmentionné, on peut par exemple citer les groupes amino, hydroxy, thiol, oxo, halogène, hétéroaryle, alkyle, alcoxy, alkylthio, alkylamino, aryloxy, arylalcoxy, cyano, trifluorométhyle, nitro, carboxy ou carboxyalkyle, ou encore un groupe $-C(O)-NH-CH_2-C \equiv C$, ou un groupe $SO_3H$ ou SOsNa.

Une autre famille particulière de composés selon l'invention, et utilisés comme chromophores fluorescents, est constituée des composés de formule (V-3) dans laquelle $Y_6$ et $Y_7$ forment ensemble, avec les atomes de carbone qui les portent, un groupe (hétéro)aryle, éventuellement substitué, de préférence un groupe phényle ou pyrazinyle.

Parmi les éventuels substituants du groupe phényle susmentionné, on peut par exemple citer les groupes amino, hydroxy, thiol, oxo, halogène, hétéroaryle, alkyle, alcoxy, alkylthio, alkylamino, aryloxy, arylalcoxy, cyano, trifluorométhyle, nitro, carboxy ou carboxyalkyle, ou encore un groupe $-C(O)-NH-CH_2-C\ C$, ou un groupe $SO_3H$ ou SOsNa.

La présente invention concerne également en tant que tels les composés de formule (II), (III), (III-1), (IV), (V), (V-1), (V-2), (V-3), (VI), (VI-1), (VII), (VII-1), (VIII) et (IX) telles que définies ci-dessus.

La présente invention concerne également une composition aqueuse comprenant au moins un composé de formule (I) telle que définie ci-dessus.

La présente invention concerne également un composé de formule (I') suivante :

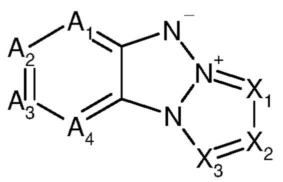

(I')

dans laquelle :

- $A_1$ est -N- ou $-C(Y_1)-$ ;
- $A_2$ est -N- ou $-C(Y_2)-$ ;
- $A_3$ est -N- ou $-C(Y_3)-$ ;
- $A_4$ est -N- ou $-C(Y_4)-$ ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, $(C_1-C_6)$alkyle, $(C_6-C_{10})$aryle, hétéroaryle comprenant de 5 à 10 atomes, $(C_2-C_6)$alcényle, $(C_2-C_6)$alcynyle, CN, hydroxy, $(C_1-C_6)$alcoxy, thiol, $(C_1-C_6)$alkylthio, $(CH_2)_nSO_2-OR_a$ où n = 1-6, $CH_2SO_2-NR_aR_b$, $NO_2$, $SO_3R_a$, $NR_aR_b$, $C(O)OR_a$, $C(O)R_a$, et $C(O)NR_aR_b$, $R_a$ et $R_b$ représentant H ou un groupe $(C_1-C_6)$alkyle, ou peuvent représenter un groupe de formule -L-Z, L représentant un bras espaceur comprenant de 1 à 10 atomes de carbone et Z représentant un groupe réactif susceptible de se lier à une molécule biologique, choisi dans le groupe constitué des halogènes, des acides carboxyliques, des esters de succinimide, des esters de tétrafluorophényle, des azotures d'acyle, des anhydrides, des halogénures d'acide, des acrylamides, des alcools, des amines, des alcynes, des aminooxyacétamides, des azotures, des imidoesters, des esters de sulfonate, des halogéno-acétamides, des halogénures d'alkyle, des halogénures de sulfonyle, des hydrazines, des hydrazides, des isocyanates, des isothiocyanates, des tétrazines et des maléimides,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou $-C(Y_5)-$ ;
- $X_2$ est -N- ou $-C(Y_6)-$ ;

- $X_3$ est -N- ou -C($Y_7$)- ;
- $Y_5$, $Y_6$ et $Y_7$ représentent, indépendamment les uns des autres, un atome d'hydrogène ou un atome d'halogène, ou sont choisis dans le groupe constitué de :

. un groupe ($C_1$-$C_6$)alkyle, ($C_3$-$C_6$)cycloalkyle ou ($C_6$-$C_{10}$)aryle, ledit groupe ($C_6$-$C_{10}$)aryle étant éventuellement substitué par au moins un substituant choisi parmi : halogène, CN, ($C_1$-$C_6$)alkyle, OH, ($C_1$-$C_6$)alcoxy, C(O)$OR_a$ et $NR_aR_b$, $R_a$ et $R_b$ représentant H ou un groupe ($C_1$-$C_6$)alkyle,
. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, ($C_1$-$C_6$)alkyle, OH, ($C_1$-$C_6$)alcoxy, C(O)$OR_a$, C(O)$R_a$ et $NR_aR_b$, $R_a$ et $R_b$ représentant H ou un groupe ($C_1$-$C_6$)alkyle,
. un groupe hétérocycloalkyle comprenant de 4 à 10 chaînons et contenant de un à trois hétéroatomes choisis parmi O, S ou N,
. $NO_2$,
. CHO,
. un groupe C(O)$OR_a$, $R_a$ étant tel que défini ci-dessus,
. un groupe -HC=CH-Ar, Ar représentant un groupe ($C_6$-$C_{10}$)aryle,
. $SO_3H$,
. un groupe $NR_cR_d$, $R_c$ et $R_d$ représentant H ou un groupe ($C_1$-$C_6$)alkyle, ou pouvant former un groupe hétéro($C_2$-$C_5$)cycloalkyle avec l'atome d'azote qui les porte ;
. un groupe $OR_e$, $R_e$ représentant H, un groupe ($C_1$-$C_6$)alkyle ou un groupe ($C_6$-$C_{10}$)aryle ; et
. un groupe de formule (A) suivante :

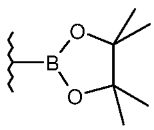

(A)

ou $Y_5$, $Y_6$ et $Y_7$ représentent un groupe de formule -L-Z, L et Z étant tels que définis ci-dessus,
ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,

ledit composé de formule (I') pouvant être sous forme de sel ou de stéréoisomère pur ou sous forme de mélange d'énantiomères et/ou de diastéréoisomères, y compris de mélanges racémiques, ou sous forme de tautomères,

dans laquelle l'un au moins des $Y_1$, $Y_2$, $Y_3$, $Y_4$, $Y_5$, $Y_6$ et $Y_7$ est un groupe de formule -L-Z,

à l'exception du composé de formule suivante :

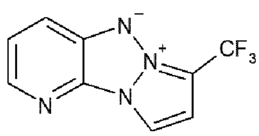

.

Les composés de formule (I') sont des composés dérivés des composés de formule (I). Ces composés comprennent au moins un groupe -L-Z comme défini plus haut et sont utilisés pour préparer des conjugués comme décrit plus loin. Ils sont donc destinés au couplage ultérieur avec des molécules biologiques telles que définies ci-après.
De préférence, dans la formule (I'), L est un radical ($C_1$-$C_{10}$)alkylène, un radical ($C_6$-$C_{10}$)arylène, un radical -($C_2$-$C_6$)alcynylène ou un radical -($C_2$-$C_6$)alcynyle-($C_1$-$C_6$)alkylène-, ou encore un radical -($C_2$-$C_6$)alcynyle-($C_6$-$C_{10}$)arylène.
Selon un mode de réalisation particulier, dans la formule (I'), le groupe -L-Z est choisi parmi les groupes suivants :

. un groupe de formule (B) suivante :

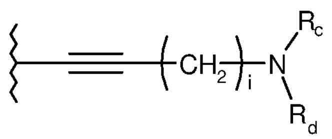

(B)

dans laquelle :

* i est un nombre entier allant de 1 à 10,
* $R_c$ représente H ou un groupe ($C_1$-$C_6$)alkyle, et
* $R_d$ représente H, un groupe ($C_1$-$C_6$)alkyle ou un groupe -COOAlk, Alk représentant un groupe ($C_1$-$C_6$)alkyle ; ou

. un groupe de formule (C) suivante :

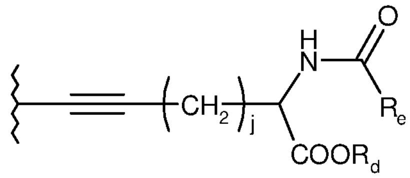

(C)

*j est un nombre entier allant de 1 à 10,
* $R_d$ représente un H, un groupe ($C_1$-$C_6$)alkyle, et
* $R_e$ représente un groupe ($C_6$-$C_{10}$)aryle ou un groupe ($C_1$-$C_6$)alcoxy ; ou

. un groupe de formule (D) suivante :

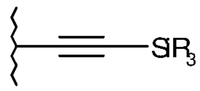

(D)

R représentant un groupe ($C_1$-$C_6$)alkyle.

Parmi les familles préférées de composés de formule (I'), on peut citer les familles constituées des composés suivants :

- ceux de formule (I'-1) suivante :

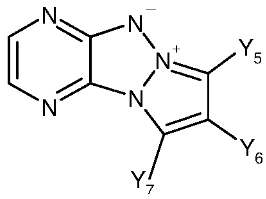

$Y_5$, $Y_6$ et $Y_7$ étant tels que définis ci-dessus dans la formule (I'), l'un des $Y_5$, $Y_6$ et $Y_7$ représentant un groupe de formule -L-Z telle que définie ci-dessus ; ou

- ceux de formule (I'-2) suivante :

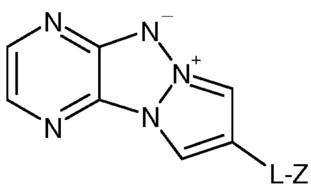

L et Z étant tels que définis ci-dessus ; ou

- ceux de formule (I'-3) suivante :

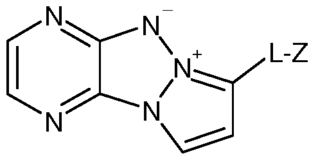

L et Z étant tels que définis ci-dessus.

Selon un mode de réalisation, dans les formules (I'), (I'-1), (I'-2) et (I'-3) susmentionnées, Z est choisi dans le groupe constitué des halogènes, des acides carboxyliques, des esters de succinimide, des esters de tétrafluorophényle, des azotures d'acyle, des anhydrides, des halogénures d'acide, des acrylamides, des alcools, des amines, des alcynes, des aminooxyacétamides, des azotures, des imidoesters, des esters de sulfonate, des halogéno-acétamides, des halogénures d'alkyle, des halogénures de sulfonyle, des hydrazines, des hydrazides, des isocyanates, des isothiocyanates, des tétrazines et des maléimides.
La présente invention concerne également un conjugué comprenant une molécule biologique et un composé de formule (I) telle que définie ci-dessus, dans lequel ledit composé de formule (I) est lié à la molécule biologique par l'intermédiaire d'un linker, ladite molécule biologique étant choisie dans le groupe constitué des anticorps, des protéines, des peptides, des glucides, des lipides, des polysaccharides, des acides gras, des acides aminés, des acides désoxyribonucléiques, des acides ribonucléiques, des oligonucléotides, des médicaments et des ligands (molécules ayant une affinité pour une cible biologique).
Selon l'invention, le linker utilisé pour la préparation des conjugués est dérivé du groupe -L-Z défini plus haut pour la formule (I').
Selon un mode de réalisation, les conjugués selon l'invention répondent à la formule (I") suivante :

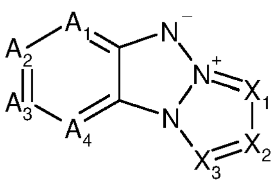

(I'')

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, ($C_1$-$C_6$)alkyle, ($C_6$-$C_{10}$)aryle, hétéroaryle comprenant de 5 à 10 atomes, ($C_2$-$C_6$)alcényle, ($C_2$-$C_6$)alcynyle, CN, hydroxy, ($C_1$-$C_6$)alcoxy, thiol, ($C_1$-$C_6$)alkylthio, $(CH_2)_nSO_2$-$OR_a$ où n = 1-6, $CH_2SO_2$-$NR_aR_b$, $NO_2$, $SO_3R_a$, $NR_aR_b$, C(O)$OR_a$, C(O)$R_a$, et C(O)$NR_aR_b$, $R_a$ et $R_b$ représentant H ou un groupe ($C_1$-$C_6$)alkyle,,

ou peuvent représenter un groupe de formule -L'-Z', L' représentant un linker et Z' représentant une molécule biologique choisie dans le groupe constitué des anticorps, des protéines, des peptides, des glucides, des lipides, des polysaccharides, des acides gras, des acides aminés, des acides désoxyribonucléiques, des acides ribonucléiques et des oligonucléotides,
ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,

et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$ représentent, indépendamment les uns des autres, un atome d'hydrogène ou un atome d'halogène, ou sont choisis dans le groupe constitué de :

. un groupe ($C_1$-$C_6$)alkyle, ($C_3$-$C_6$)cycloalkyle ou ($C_6$-$C_{10}$)aryle, ledit groupe ($C_6$-$C_{10}$)aryle étant éventuellement substitué par au moins un substituant choisi parmi : halogène, CN, ($C_1$-$C_6$)alkyle, OH, ($C_1$-$C_6$)alcoxy, C(O)$OR_a$ et $NR_aR_b$, $R_a$ et $R_b$ représentant H ou un groupe ($C_1$-$C_6$)alkyle,
. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, ($C_1$-$C_6$)alkyle, OH, ($C_1$-$C_6$)alcoxy, C(O)$OR_a$, C(O)$R_a$ et $NR_aR_b$, $R_a$ et $R_b$ représentant H ou un groupe ($C_1$-$C_6$)alkyle,
. un groupe hétérocycloalkyle comprenant de 4 à 10 chaînons et contenant de un à trois hétéroatomes choisis parmi O, S ou N,
. $NO_2$,
. CHO,
. un groupe C(O)$OR_a$, $R_a$ étant tel que défini ci-dessus,
. un groupe -HC=CH-Ar, Ar représentant un groupe ($C_6$-$C_{10}$)aryle,
. $SO_3H$,
. un groupe $NR_cR_d$, $R_c$ et $R_d$ représentant H ou un groupe ($C_1$-$C_6$)alkyle, ou pouvant former un groupe hétéro($C_2$-$C_5$)cycloalkyle avec l'atome d'azote qui les porte ;
. un groupe $OR_e$, $R_e$ représentant H, un groupe ($C_1$-$C_6$)alkyle ou un groupe ($C_6$-$C_{10}$)aryle ; et
. un groupe de formule (A) suivante :

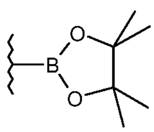

(A)

ou $Y_5$, $Y_6$ et $Y_7$ représentent un groupe de formule -L'-Z', L' et Z' étant tels que définis ci-dessus,
ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,

ledit composé de formule (I") pouvant être sous forme de sel ou de stéréoisomère pur ou sous forme de mélange d'énantiomères et/ou de diastéréoisomères, y compris de mélanges racémiques, ou sous forme de tautomères,

dans laquelle l'un au moins des $Y_1$, $Y_2$, $Y_3$, $Y_4$, $Y_5$, $Y_6$ et $Y_7$ est un groupe de formule -L'-Z'.

Selon l'invention, le linker L' est dérivé du groupe -L-Z défini plus haut pour la formule (I'). En effet, les composés de formule (I") sont obtenus par conjugaison entre les composés de formule (I') et une molécule biologique, la molécule biologique se liant via le groupe réactif de Z défini plus haut.

Les conjugués de l'invention sont donc des composés comprenant un marqueur fluorescent, ledit marqueur fluorescent étant dérivé d'un composé de formule (I) tel que défini ci-dessus.

La présente invention concerne également l'utilisation des conjugués susmentionnés pour la détection par fluorescence d'une molécule biologique.

La présente invention concerne également l'utilisation des chromophores fluorescents susmentionnés dans divers domaines d'application, et notamment dans le domaine de l'imagerie biologique, de la microscopie par fluorescence, du marquage de biomolécule, du criblage par spectroscopie de fluorescence. Ces composés peuvent également être utilisés comme sondes fluorescentes.

La présente invention concerne également un procédé de détection d'au moins une molécule biologique dans

un milieu biologique, comprenant :

- une étape d'introduction d'un conjugué selon l'invention dans ledit milieu biologique, ledit conjugué comprenant un marqueur fluorescent et une molécule biologique,
- une étape d'excitation dudit milieu, et
- une étape de détection d'au moins un signal de fluorescence dudit marqueur fluorescent.

Dans toute la demande, le libellé "comprenant un" ou "comportant un" signifie "comprenant au moins un" ou "comportant au moins un" sauf si le contraire est spécifié.

Dans toute la description ci-dessus, sauf mention contraire, le terme « compris(e) entre x et y » correspond à une gamme inclusive, c'est-à-dire que les valeurs x et y sont incluses dans la gamme.

## EXEMPLES

Les réactions devant être menées sous conditions anhydres sont effectuées dans une verrerie séchée à l'étuve (T>100°C) puis refroidie sous atmosphère inerte d'argon, avec des solvants également anhydres : l'acétonitrile, le toluène et le tétrahydrofurane sont séchés par passage sur la colonne d'une station, le dichlorométhane est distillé sur hydrure de calcium, la triéthylamine et le 1,4-dioxane sont distillés sur hydroxyde de potassium, et le DMF anhydre conservé sous tamis est disponible chez ALFA Aesar.

Le 1H-pyrazole, le 2-iodo-1-nitrobenzène, le 4-(3H)-pyrimidinone, le (1H)-1,2,3-triazole, le 4-nitro-1H-pyrazole, le n-BuLi, l'hydrazine monohydrate, le 4-pyrazoleboronique pinacol ester, le tert-butyl prop-2-ynylcarbamate, le $Pd(Ph_3)_2Cl_2$ , le $P(PPh_3)_4$, et le carbonate de césium sont disponibles chez ALDRICH.

Le 1,2,4(1H)-triazole, le D,L-propargylglycine, le 3-iodo-1H-pyrazole, le phényl acétylene et le 1-éthynyl-4-méthoxybenzène sont disponibles chez ACROS.

Le 2,3-dichloropyrazine, le 2,6-dichloropyrazine, le 4-iodo-1H-pyrazole, le 4-bromo-1H-pyrazole et le N-iodosuccinimide sont disponibles chez APOLLO Scientific UK.

Le 1,2-dichlorobenzène, le 3-nitro-1,2,4-(1H)-triazole, le 1,3-dithiane, 4-(N,N-diméthylamino)phénylboronique, et carbonate de potassium sont disponibles chez ALFA Aesar.

Les différents indazoles de départ utilisés sont disponibles chez APOLLO Scientific UK.

## I - SCHEMAS REACTIONNELS

***Synthèse du noyau [benzo]-1,3a,6a-triazapentalène***

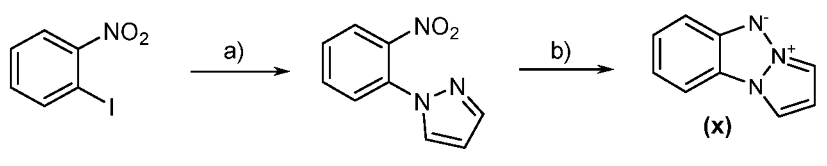


a) pyrazole (1éq), $Cu_2O$ (0.1éq), $Cs_2CO_3$ (2éq), DMF, 100°C ; b) $P(OEt)_3$ (22éq), 176°C, MW.

***Synthèse du noyau [pyrimidino]-1,3a,6a-triazapentalène***

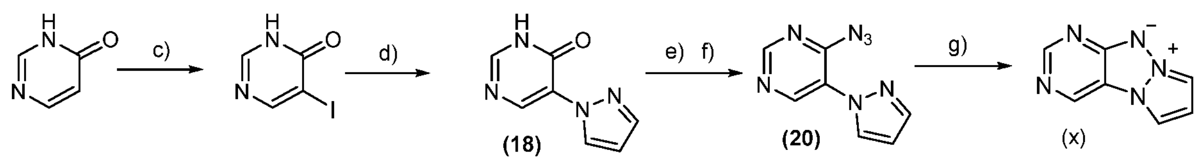


c) NIS (1.1éq), AcOH, 50°C. d) pyrazole (1.5éq), $Cu_2O$ (0.05éq), $K_2CO_3$ (2éq), salicylaldoxime (0.2éq), DMF, 170°C, MW ; e) $POCl_3$ (20éq), 110°C ; f) $NaN_3$ (2éq), DMF ; g) 1,2-dichlorobenzène, 165°C.

***Synthèse des structures de type [pyrazino]-1,3a,6a-triazapentalène***

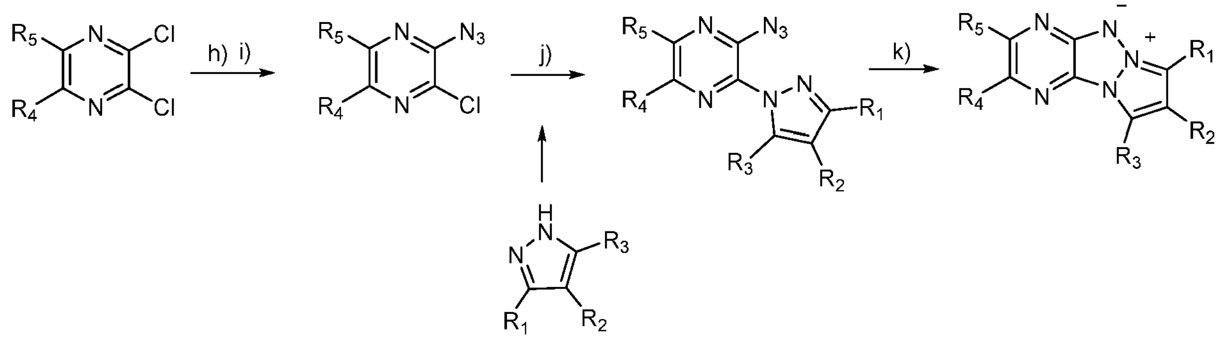


h) $N_2H_4.H_2O$ (2.1 éq); i) $NaNO_2$ (1.1éq), $AcOH/H_2O$ ; j) $K_2CO_3$ (2éq), ACN, 82°C ; k) 1,2-dichlorobenzène, 165°C.

***Synthèse des structures de type 4-amino-[pyrazino]-1,3a,6a-triazapentalène***

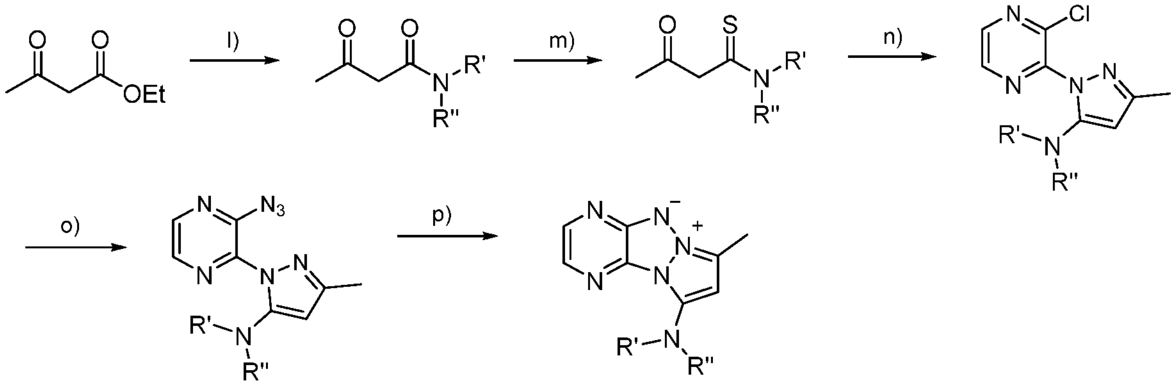


l) 4-DMAP(0.1éq), toluène ; m) Réactif de Lawesson (0.5éq) , toluène, MW, 140°C ; n) Pyridine (4.5éq) , toluène, MW, 140°C ;o) $NaN_3$ (2.5éq), DMF, 60°C ; p) 1,2-dichlorobenzène, 165°C.

***Synthèse des structures de type 4-amino-[pyrazino]-1,3a,6a-triazapentalène***

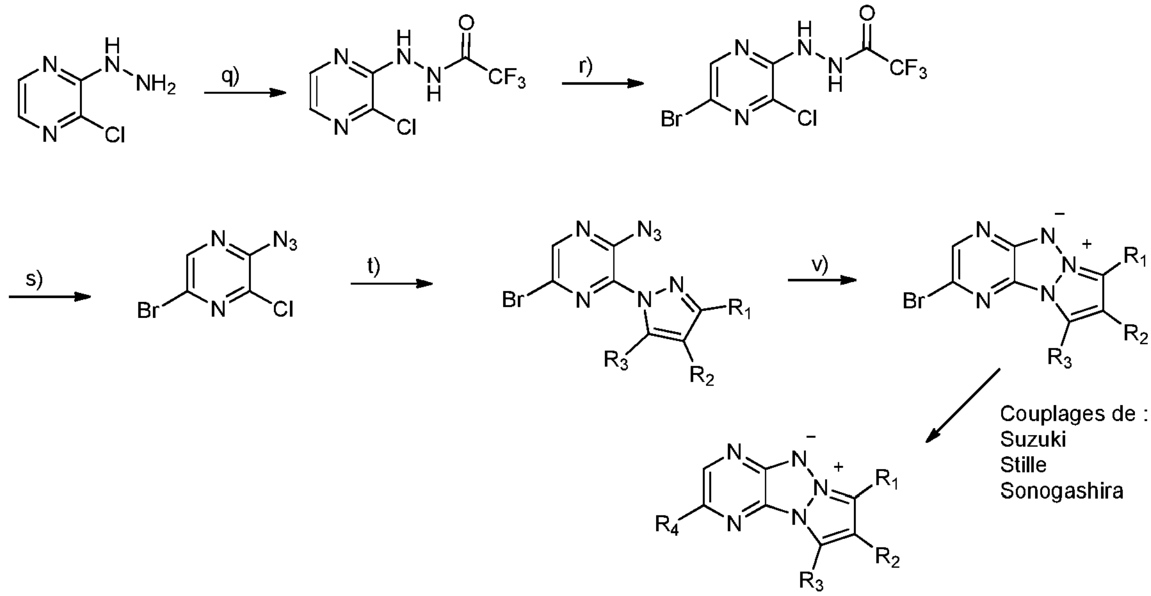


q) TFAA, THF. r) NBS, dichlorométhane; s) HCl/EtOH puis $NaNO_2/H_2O$; f) $K_2CO_3$, ACN, reflux; v) 1,2-dichlorobenzène, 165°C.

## II - SYNTHESE DES INTERMEDIAIRES

### *1-(2-nitrophenyl)-1H-pyrazole (1)*

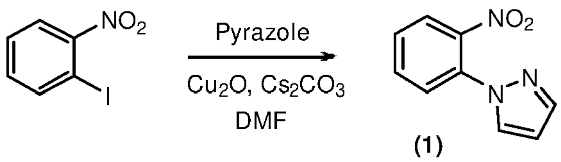


(1)

$C_9H_7N_3O_2$
MW: 189,17 g.mol$^{-1}$

Dans un ballon placé sous argon, le pyrazole (3g, 0.012mol, 1éq), le 2-iodo-1-nitrobenzène (1.2g, 0.018mol, 1.5éq), l'oxyde de cuivre(I) (0.17g, 0.001 mol, 0.1éq), le $Cs_2CO_3$ (7.9g, 0.024mol, 2éq) et le DMF anhydre (25mL) sont introduits. La suspension obtenue est laissée sous agitation à 100°C pendant 20h. Après retour à température ambiante, le milieu réactionnel est concentré à sec. Le résidu récupéré est repris à l'acétate d'éthyle et est lavé plusieurs fois avec une solution saturée de chlorure de sodium. La phase organique est séchée sur $MgSO_4$, filtrée et concentrée à sec. Le mélange brut est purifié par chromatographie sur colonne de gel de silice (éther de pétrole/acétate d'éthyle : 7/3 ; dépôt solide). Le produit (1) est obtenu avec un rendement de 75% (1.7g) sous forme d'une poudre jaune. RMN($^1$H, 250 MHz, chloroforme-d) $\delta$ 7.88 (dd, $J$ = 8.0, 1.5 Hz, 1H), 7.75 (d, $J$ = 1.9 Hz, 1H), 7.72 (d, $J$ = 2.5 Hz, 1H), 7.67 (dd, $J$ = 7.4, 1.4 Hz, 1H), 7.62 - 7.57 (m, 1H), 7.56 - 7.47 (m, 1H). RMN($^{13}$C, 400 MHz, chloroforme-d) $\delta$ 142.74, 133.43, 130.19, 128.80, 126.73, 125.48, 108.63. SM (IC+) *m/z*190 ($M+H^+$), 212 ($M+Na^+$).

### *1-Nitropyrazole (2)*

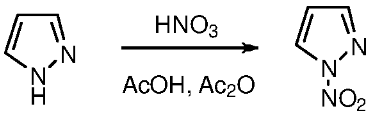


(2)

$C_3H_3N_3O_2$
MW: 113,08 g.mol$^{-1}$

A une suspension de pyrazole (2g, 0.03mol, 1éq) dans l'acide acétique (6mL) à 0°C, l'acide nitrique fumant (1.5mL, 0.036mol, 1.2éq) est ajouté goutte à goutte. L'anhydride acétique (4mL, 0.041mol, 1.4éq) est ajoutée en dernier. Le mélange réactionnel est laissé sous agitation pendant 30min à 0°C puis pendant 17h à température ambiante. Le suivi est réalisé par CCM dans éther de pétrole/acétate d'éthyle (7:3). Dans le milieu sont ajoutés 35mL d'eau froide, le précipité formé est récupéré par filtration et est lavé à l'eau glacée. Le produit attendu **(2)** est obtenu sous forme d'une poudre blanche avec un rendement de 86% (2.9g). RMN ($^1$H, 250 MHz, DMSO-d6) $\delta$ 7.97 (dd, $J$ = 3.0, 0.8 Hz, 1H), 7.06 (dd, $J$ = 1.7, 0.9 Hz, 1H), 5.88 (dd, $J$ = 3.0, 1.7 Hz, 1H).

***3-Nitropyrazole (3)***

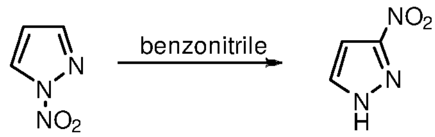


$C_3H_3N_3O_2$
MW: 113,08 g.mol$^{-1}$

**(2)** **(3)**

Une solution de 1-nitropyrazole **(2)** (2.9g, 0.026mol, 1éq) dans 36mL de benzonitrile est chauffée à 180°C pendant 5h. Après retour à température ambiante, l'heptane (100mL) est ajouté. Le précipité blanc formé est récupéré par filtration et est rincé à l'heptane pour donner le produit **(3)** sous forme d'une poudre blanche avec un rendement de 58% (1.7g). RMN ($^1$H, DMSO-d6, 250MHz): $\delta$ 13.14 (1H, s, H), 8.03 (1H, d, $J_1$=2.5Hz, $H_5$), 7.03 (1H, d, $J_1$=2.5Hz, $H_4$).

***4-(1H)-Nitropyrazole (4)***

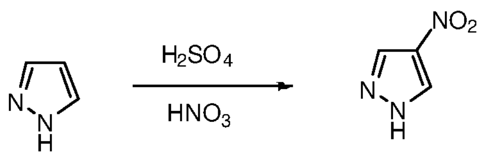


$C_3H_3N_3O_2$
MW: 113,08 g.mol$^{-1}$

**(4)**

Dans un ballon contenant l'acide sulfurique à 95% (22mL) refroidi à 0°C, le pyrazole (3g, 43.9mmol, 1éq) est introduit par des petites portions. Après 5 min d'agitation à froid, la température du milieu réactionnel est montée à 55°C. L'acide nitrique (3.2mL, 48.3mmol, 1.1éq) est ensuite ajouté goutte à goutte tout en maintenant la température constante de 55°C. Le milieu réactionnel est laissé sous agitation à cette température pendant 3h. Après retour à température ambiante, le mélange réactionnel est basifié avec du carbonate de sodium solide solide et est extrait 3 fois à l'acétate d'éthyle. Les phases organiques sont rassemblées, lavées avec une solution saturée en chlorure de sodium, séchées sur $MgSO_4$, filtrées et concentrées à sec. Le 4-nitro-(1H)-pyrazole **(4)** est obtenu sous forme d'une poudre jaune avec un rendement de 89% (4,4g). RMN ($^1$H, DMSO-d6, 250MHz): $\delta$ 13.91 (se, 1H), 8.54 (se, 2H).

***1H-Pyrazol-3(2H)-one (5)***

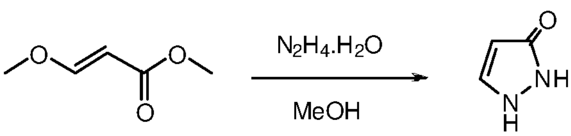


$C_3H_4N_2O$
MW: 84,08 g.mol$^{-1}$

**(5)**

L'hydrazine monohydrate (2.7mL, 55mmol, 1.1éq) est ajoutée goutte à goutte à une solution de (E)-méthoxyacrylate (5.5mL, 51mmol, 1éq) dans méthanol (5mL). Le mélange obtenu est laissé sous agitation à reflux du solvant pendant 1h30. Le suivi est réalisé par CCM dans dichlorométhane/méthanol (95:5). Après retour à température ambiante,

le milieu réactionnel est concentré à sec pour donner le produit désiré **(5)** sous la forme d'une poudre jaune avec un rendement quantitatif (4.68g). Le produit est engagé dans la réaction suivante sans purification. RMN ($^1$H, DMSO-d6, 250MHz) δ 10.30 (s, 1H), 7.34 (d, *J* = 2.2 Hz, 1H), 5.43 (d, *J* = 2.3 Hz, 1H).

***1-Acetyl-3-hydroxy-1H-pyrazole (6)***

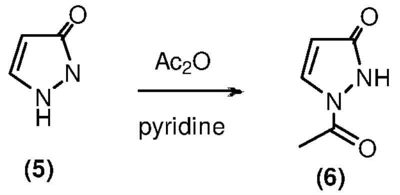


**(5)** **(6)**

$C_5H_6N_2O_2$
MW: 126,12 g.mol$^{-1}$

Dans une solution de 1H-pyrazol-3(2H)-one **(5)** (4.5g, 0.05mol, 1éq) dans la pyridine (22mL), préchauffée à 95°C, une mélange d'anhydride acétique (5.3mL, 0.06mol, 1.1éq) dans la pyridine (11mL) est ajoutée goutte à goutte pendant 15min. La solution obtenue est laissée sous agitation à 95°C pendant 1h (coloration du milieu du jaune claire vers marron). Le suivi est réalisé par CCM dans dichlorométhane /méthanol (95:5). Après retour à température ambiante, le milieu réactionnel est concentré à sec et est coévaporé au toluène afin d'éliminer la pyridine résiduelle. Le précipité obtenu est repris à l'éther et est laissé sous agitation à température ambiante pendant 1h. Le solide est récupéré par filtration, rincé puis séché pour donner le produit désiré **(6)** sous la forme d'une poudre jaune avec un rendement de 79% (5g). Le produit est engagé dans l'étape suivante sans purification sur colonne de silice. RMN ($^1$H, DMSO-d6, 250MHz) δ 10.94 (s, 1H), 8.12 (d, J = 3.0 Hz, 1H), 6.00 (d, J = 3.0 Hz, 1H), 2.48 (s, 3H).

***1-(3-methoxypyrazol-1-yl)ethanone (7)***

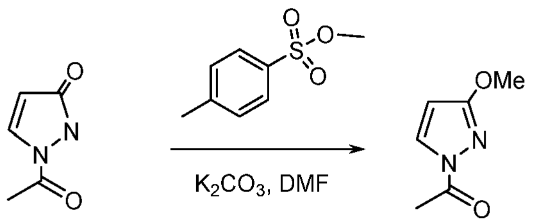


**(6)** **(7)**

**DS 83**

$C_6H_8N_2O_2$
MW: 140,14 g.mol$^{-1}$

Dans un ballon placé sous atmosphère d'argon, le 1-acétyl-3-hydroxy-1H-pyrazole **(6)** (300mg, 2.4mmol, 1éq), le carbonate de potassium (658mg, 4.8mmol, 2éq) et le N,N-diméthylformamide anhydre (6mL) sont introduits. Le p-toluènesulfonate de méthyle (444mg, 2.4mmol, 1éq) est ajouté au mélange en dernier. Le milieu réactionnel est laissé sous agitation à température ambiante et sous atmosphère d'argon pendant 15h. Le suivi est réalisé par CCM dans acétate d'éthyle /éther de pétrole (7:3). Le milieu réactionnel est repris à l'acétate d'éthyle et est lavé à l'eau, puis avec une solution saturée en chlorure de sodium. La phase organique est séchée sur $MgSO_4$, filtrée et concentrée à sec pour donner le produit désiré sous la forme d'une huile jaune. Le composé brut est ensuite purifié sur colonne de silice (acétate d'éthyle/éther de pétrole : 1/9 ; dépôt liquide) pour donner le composé **(7)** sous forme d'une huile incolore avec un rendement de 42% (140mg). RMN ($^1$H, 250MHz, Chloroform-d) δ 8.05 (d, *J* = 2.7 Hz, 1H), 5.95 (d, *J* = 2.8 Hz, 1H), 3.95 (s, 3H), 2.59 (s, 3H). RMN ($^{13}$C, 400 MHz, Chloroforme-d) δ 169.15, 166.29, 130.42, 99.67, 56.77, 21.90.

***3-Methoxy-1H-pyrazole (8)***

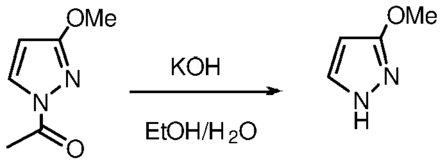


$C_4H_6N_2O$
MW: 98,11g. $mol^{-1}$

**(7)** **(8)**

L'hydroxyde de potassium solide (120mg, 2.14mmol, 3éq) est ajouté à une solution de 1-(3-methoxypyrazol-1-yl)éthanone **(7)** (100mg, 0.71mmol, 1éq) dans un mélange éthanol/eau (1mL/0.5mL). La solution est laissée sous agitation à température ambiante pendant 2h. Le suivi est réalisé par CCM dans l'éther de pétrole/ acétate d'éthyle (95:5). Le milieu réactionnel est repris à l'eau et est extrait à l'acétate d'éthyle. La phase aqueuse est extraite 3 fois avec le mélange DCM/iPrOH (95/5). Les phases organiques sont rassemblées, séchées sur $MgSO_4$, filtrées et concentrées à sec pour donner le produit **(8)** avec un rendement de 61% (43mg). RMN ($^1H$, 250MHz, chloroforme-d) $\delta$ 9.8 (se, 1H), 7.36 (d, J = 1.8 Hz, 1H), 5.73 (d, J = 1.8 Hz, 1H), 3.91 (s, 3H). RMN ($^{13}C$, 101 MHz, chloroforme-d) $\delta$ 164.10, 129.84, 89.63, 55.88.

***2-Chloro-3-[1,3]dithian-2-yl-pyrazine (9)***

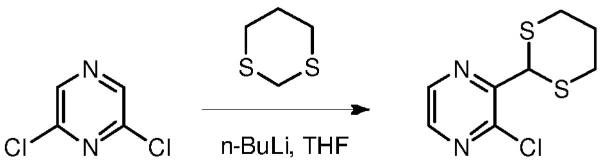


$C_8H_9ClN_2S_2$
MW: 232,74g.$mol^{-1}$

**(9)**

Dans un ballon sec placé sous atmosphère d'argon, le 1,3-dithiane (2.66g, 22.0mmol, 1éq) est solubilisé dans le THF anhydre (18mL). La solution est refroidie à -78°C et une solution de n-BuLi (9.6mL, 24.0mmol, 1.2éq) dans l'hexane à 2.5M est ajoutée lentement. Après 30 min d'agitation à -78°, une solution de 2,6-dichloropyrazine (3.0g, 20.1mmol, 1.1éq) dans le THF anhydre (12mL) est ajoutée goutte à goutte. Le milieu réactionnel est laissé sous agitation à -78°C pendant 1h. La réaction est stoppée en ajoutant de l'eau, suivie d'une extraction à l'acétate d'éthyle (3 fois). Les phases organiques sont rassemblées, lavées avec une solution de chlorure de sodium saturée, séchées sur $MgSO_4$ et concentrées à sec. Le résidu brut est purifié par chromatographie sur colonne de gel de silice (éther de pétrole/acétate d'éthyle: 10/0 et 9/1 ; dépôt solide). Le 2-chloro-3-[1,3]dithian-2-yl-pyrazine **(9)** est obtenu avec un rendement de 82% (3.84g) forme d'une poudre jaune claire. RMN ($^1H$, 400 MHz, chloroforme-d) $\delta$ 8.52 (d, *J* = 2.2 Hz, 1H), 8.32 (d, *J* = 2.1 Hz, 1H), 5.55 (s, 1H), 3.18 (ddd, *J* = 14.0, 6.6, 2.9 Hz, 2H), 3.00 (ddd, *J* = 13.3, 10.0, 2.5 Hz, 2H), 2.30 - 1.98 (m, 2H). RMN ($^{13}C$, 101 MHz, chloroforme-d) $\delta$ 153.20 (Cq), 147.15(Cq), 143.05($C_{Ar}$), 142.37($C_{Ar}$), 46.81, 30.07, 25.41.

***3-Chloro-pyrazine-2-carbaldehyde(10)***

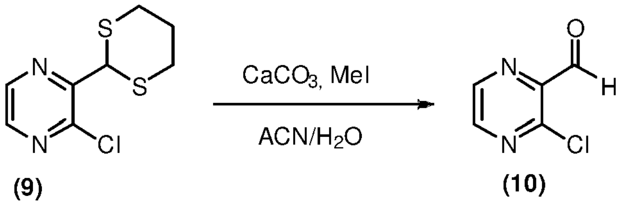


$C_5H_3ClN_2O$
MW: 142,54 g.$mol^{-1}$

**(9)** **(10)**

Dans un ballon contenant le 2-chloro-3-[1,3]dithian-2-yl-pyrazine **(9)** (2.1g, 9.05mmol, 1éq) dans un mélange d'ACN/$H_2O$ (135mL / 35mL), le carbonate de calcium (2.72g, 27.18mmol, 3éq) et l'iodure de méthyle (5.6mL, 27.18mmol, 10éq) sont introduits. La suspension obtenue est laissée sous agitation à 60°C pendant 24h. Après retour à température ambiante, le milieu réactionnel est filtré sur Milli-Pore; le filtrat est concentré à sec, repris à l'eau et extrait à l'acétate d'éthyle. Les phases organiques sont rassemblées, lavées avec une solution de chlorure de sodium saturée, séchées

sur $MgSO_4$, filtrées et concentrées à sec. Le 3-chloro-pyrazine-2-carbaldehyde **(10)** est obtenu avec un rendement quantitatif (1.5g) sous forme d'une huile rouge. Ce produit est engagé immédiatement dans la réaction suivante (dégradation ou polymérisation lors de sa conservation et lors de la purification sur silice). RMN ($^1$H, 400 MHz, acétonitrile-d3) δ 10.21 (s, 1H), 8.78 (d, *J* = 2.3 Hz, 1H), 8.61 (d, *J* = 2.3 Hz, 1H). RMN ($^{13}$C, 101 MHz, acétonitrile-d3) δ 190.53, 149.49, 148.38, 145.08, 144.64.

***1H-Pyrazolo[3,4-b]pyrazine (11)***

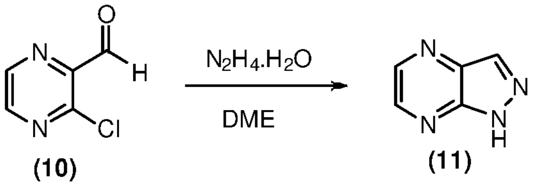


**(10)** **(11)**

$C_5H_4N_4$
MW: 120,12 g.mol$^{-1}$

Sous atmosphère d'argon, l'hydrazine monohydrate (0.55mL, 11.2 mmol, 10éq) est ajoutée à une solution de 3-chloro-pyrazine-2-carbaldehyde **(10)** (155mg, 1.12mmol, 1éq) dans le diméthoxyéthane (5mL). Le milieu réactionnel est laissé sous agitation à reflux du solvant pendant 14h. Après retour à température ambiante le solvant est évaporé sous pression réduite et le résidu brut est recristallisé dans le cyclohexane. Le produit **(11)** est obtenu avec un rendement de 97% (132mg) sous forme d'un solide marron. RMN ($^1$H, 400 MHz, Acétone-d6) δ 13.11 (se, 1H), 8.61 (d, *J* = 2.1 Hz, 1H), 8.54 (d, *J* = 2.1 Hz, 1H), 8.34 (s, 1H). RMN ($^{13}$C, 101 MHz, Acétone-d6) δ 143.85, 141.97, 134.86, 134.55. SM ($IC^+$) *m/z* 121($M+H^+$), 149 ($M+Na^+$).

***(3-chloropyrazin-2-yl)hydrazine (12)***

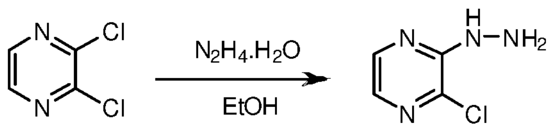


**(12)**

$C_4H_5ClN_4$
MW: 144,56g.mol$^{-1}$

Dans un ballon contenant une solution de 2,3-dichloropyrazine (10.0g, 65.8mmol, 1éq) dans l'éthanol (400mL), l'hydrazine monohydrate (6.84mL, 138.2mmol, 2.1éq) est introduite goutte à goutte. Le milieu réactionnel est laissé sous agitation à reflux du solvant pendant 6h. Afin de consommer la quantité restante du 2,3-dichloropyrazine une quantité supplémentaire d'hydrazine (1.71mL, 34.6mmol, 0.25éq) est ajoutée. Après 1h à reflux et retour à température ambiante, le mélange réactionnel est concentré sous pression réduite. Les solide cristallin obtenu est repris à l'éther diéthylique et est filtré sur Milli-Pore pour donner le produit désiré **(12)** sous forme d'un solide jaune pâle avec un rendement quantitatif (14.9 g). RMN ($^1$H, 250MHz, chloroforme-d) δ 8.03 (dd, *J* = 2.8, 0.4 Hz, 1H), 7.69 (dd, *J* = 2.8, 0.4 Hz, 1H), 6.45 (s, 1H), 3.97 (s, 2H). RMN ($^1$H, 400MHz, Méthanol-d3) δ 8.04 (d, *J* = 2.8Hz, 1H), 7.58 (dd, *J* = 2.8Hz, 1H). RMN ($^{13}$C, 400MHz, Méthanol-d3) δ 154.3, 141.9, 134.8, 131.9. SM ($IC^+$) *m/z* 145($MH^+$).

***2-azido-3-chloro-pyrazine (13)***

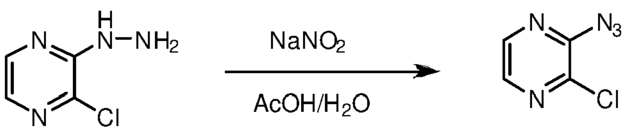


**(12)** **(13)**

$C_4H_2ClN_5$
MW: 155,55g.mol$^{-1}$

La (3-chloropyrazin-2-yl)hydrazine **(12)** (5g, 27.6mmol, 1éq) est solubilisée dans une solution aqueuse d'acide acétique à 10% (100mL). Le milieu réactionnel est refroidi à 0°C, puis une solution de nitrite de sodium (2.1g, 30mmol, 1.1éq) dans l'eau distillée (7mL) est ajoutée goutte à goutte. Après 30min d'agitation à 0°C, le milieu réactionnel est extrait à l'acétate d'éthyle (x2). Les phases organiques sont rassemblées et neutralisées avec une solution de $K_2CO_3$ à 5%, puis lavées avec une solution saturée en NaCl, séchées sur $MgSO_4$ et concentrées à sec. Le résidu brut est ensuite purifié par chromatographie sur colonne de gel de silice (dichlorométhane : 100% ; dépôt solide) pour obtenir le composé **(13)** sous forme d'une poudre jaune pâle avec un rendement de 73% (3.12g). RMN ($^1$H, 250MHz, chloroforme-d) $\delta$ 8.75 (d, *J* = 4.6 Hz, 1H, $H_5$), 8.13 (d, *J* = 4.6 Hz, 1H, $H_6$). RMN ($^{13}$C, 400MHz, chloroforme-d) $\delta$144, 13.4, 118.3. SM (IC$^+$) *m/z* 156 (MH$^+$), 128 (MH$^+$-$N_2$).

***2,3-dichloroquinoxaline (14)***

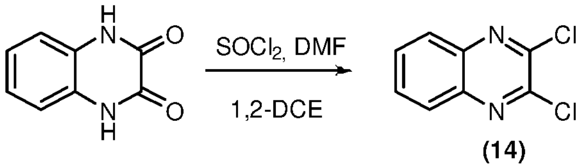


**(14)**

$C_8H_4Cl_2N_2$
MW: 199,03 g.mol$^{-1}$

Le N,N-diméthylformamide (50$\mu$L, 0.62mmol, 0.05éq) est ajouté à une suspension de quinoxaline-2,3(1H,4H)-dione (2g, 12.3mmoL, 1éq) et chlorure de thionyle (2.25mL, 31mmol, 2.5éq) dans 1,2-dichloroéthane (20mL). Le milieu réactionnel est laissé sous agitation à reflux du solvant pendant 2h. Le suivi est réalisé par CCM dans le mélange acétate d'éthyle/éther de pétrole (8/2). Après retour à température ambiante, le milieu réactionnel est concentré à sec. Le résidu brut obtenu est recristallisé dans un mélange acétonitrile/eau (9/1). Après le retour à température ambiante, le mélange est refroidi à 0°C et est laissé à cette température pendant 30min. Les cristaux formés sont récupérés par filtration et sont lavés à l'eau froide. Le produit attendu **(14)** est obtenu sous la forme d'un solide cristallin jaune pâle avec un rendement de 87% (2.1g). RMN ($^1$H, 250MHz, 250 MHz, DMSO-d6) $\delta$ 8.15 - 8.02 (m, 2H), 8.00 - 7.89 (m, 2H).

***2-chloro-3-hydrazinylquinoxaline (15)***

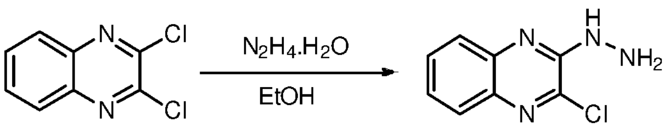


**(14)** **(15)**

$C_8H_7ClN_4$
MW: 194,62 g.mol$^{-1}$

Dans un ballon contenant une solution de 2,3-dichloroquinoxaline **(14)** (500mg, 2.5mmol, 1éq) dans l'éthanol (15mL), l'hydrazine monohydrate (0.25mL, 5.3mmol, 2.1éq) est introduite goutte à goutte. Le milieu réactionnel est laissé sous agitation au reflux du solvant pendant 4h. Arès retour à température ambiante, le précipité formé est récupéré par filtration, lavé avec de l'éthanol froid et séché sous pression réduite. Le composé brut est ensuite purifié par chromatographie sur colonne de gel de silice (dépôt solide, AcOEt/Ether de Pétrole : 5/5). Le produit attendu **(15)** est obtenu sous la forme d'une poudre orange avec un rendement de 38% (186mg). RMN ($^1$H, 250 MHz, Méthanol-d4) $\delta$ 7.77 - 7.67 (m, 1H), 7.58 (ddd, *J* = 8.2, 7.0, 1.5 Hz, 1H), 7.39 (ddd, *J* = 8.4, 7.0, 1.4 Hz, 1H). SM (IC+) *m/z* 165(MH$^+$-$N_2H_3$).

***2-azido-3-chloroquinoxaline (16)***

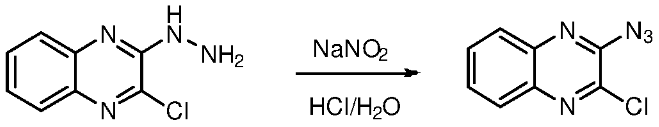


(15) (16)

$C_8H_4ClN_5$
MW: 205,61 g.mol$^{-1}$

L'acide chlorhydrique concentré à 37% (0.26mL) est ajouté lentement à une suspension de 2-chloro-3-hydrazinylquinoxaline **(15)** (139mg, 0.72mmol, 1éq) dans l'eau distillée (0.6mL). La solution obtenue est refroidie à 0°C, puis une solution de nitrite de sodium (730mg, 10.6mmol, 2.6éq) dans l'eau distillée (0.6mL) est introduite goutte à goutte. Après 35min d'agitation à 0°C et retour à température ambiante, le milieu réactionnel est repris à l'eau et est extrait à l'acétate d'éthyle (x3). Les phases organiques sont rassemblées, lavées avec une solution de bicarbonate de sodium et une solution saturée en NaCl, séchées sur $MgSO_4$, filtrées et concentrées à sec. Le composé brut est purifié par chromatographie sur colonne de gel de silice (dépôt solide, AcOEt/Ether de Pétrole : 2/8). Le produit **(16)** est obtenu sous forme d'une poudre jaune avec un rendement de 77% (115mg). RMN ($^1H$, 400 MHz, chloroforme-d) δ 8.63 (dd, *J* = 8.1, 1.2 Hz, 1H), 8.24 (dd, *J* = 8.1, 1.1 Hz, 1H), 7.95 (td, *J* = 7.8, 1.5 Hz, 1H), 7.89 (td, *J* = 7.8, 1.5 Hz, 1H). RMN ($^{13}C$, 101 MHz, chloroforme-d) δ 142.28, 140.46, 136.16, 131.84, 130.63, 130.02, 124.79, 116.71.

***5-Iodo-3H-pyrimidin-4-one (17)***

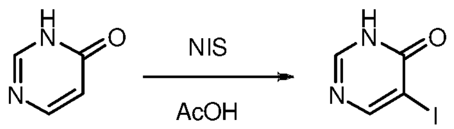


(17)

$C_4H_3IN_2O$
MW: 221,99 g.mol$^{-1}$

Dans un ballon sec placé sous atmosphère d'argon, contenant une solution de 4(3H)-pyrimidinone (750mg, 7.81mmol, 1 éq) dans l'acide acétique glacial (27mL), le *N*-iodosuccinimide (2.04g, 8.61mmol, 1.1éq) est introduit par petites portions. Le mélange réactionnel est laissé sous agitation à 50°C pendant 4h. Après retour à température ambiante, la solution est refroidie à 0°C à l'aide d'un bain de glace. Le précipité formé est filtré, lavé à l'eau froide et est rincé à l'éthanol. Le produit désiré **(17)** est obtenu sous forme d'une poudre jaune avec un rendement de 89% (1.74g). RMN ($^1H$, 400 MHz, DMSO-d6) δ 12.94(se,1H), 8.44 (s, 1H), 8.18 (s, 1H). RMN ($^{13}C$, 101 MHz, DMSO-d6) δ 206.76, 160.00, 159.02, 150.23, 90.99. SM (IC+) *m/z* 223 ($M+H^+$).

***5-Pyrazol-1-yl-3H-pyrimidin-4-one (18)***

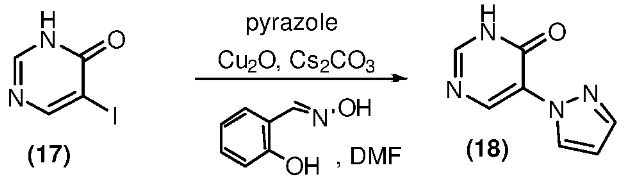


(17) (18)

$C_7H_6N_4O$
MW: 162,15 g.mol$^{-1}$

Dans un tube scellé sec placé sous argon, le $Cu_2O$ (11.2mg, 0.078mmol, 0.05éq), le 2-hydroxybenzaldehyde oxime (42.8mg, 0.312mmol, 0.2éq), le pyrazole (163.5mg, 2.35mmol, 1.5éq), le $Cs_2CO_3$ (1.02g, 3.12mmol, 2éq), le 5-Iodopyrimidin-4-ol **(17)** (350mg, 1.56mmol, 1éq) et le *N,N*-diméthylformamide anhydre (1.75mL) sont introduits. La suspension obtenue est chauffée au micro-ondes à 170°C pendant 1h10. Après retour à température ambiante, le milieu réactionnel est concentré à sec et est purifié par chromatographie sur colonne de gel de silice (dichlorométhane/ méthanol : 10/0, 97/3, 95/5 et 9/1; dépôt solide). Le produit attendu **(18)** est obtenu avec un rendement de 95% (239mg) sous forme d'une poudre jaune pâle. RMN ($^1H$, 250 MHz, DMSO-d6) δ 8.59 (d, *J* = 2.4 Hz, 1H), 8.48 (s, 1H), 8.22 (s, 1H), 7.74 (d, *J* = 1.2 Hz, 1H), 6.61 - 6.38 (m, 1H). RMN ($^{13}C$, 101 MHz, DMSO) δ 147.53, 140.65, 131.16, 106.89. SM (IC+) *m/z* 163 ($M+H^+$), 185 ($M+Na^+$).

***4-Chloro-5-pyrazol-1-yl-pyrimidine (19)***

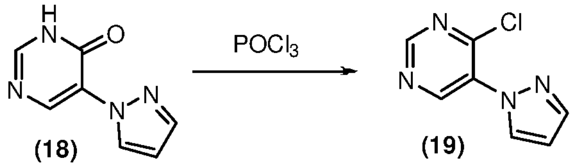


(18) (19)

$C_7H_5ClN_4$
MW: 180,60 g.mol$^{-1}$

Une solution de 5-yrazol-1-yl-3H-pyrimidin-4-one **(18)** (53mg, 0.33mmol, 1éq) dans le trichlorure de phosphoryle (0.61mL, 6.54mmol, 20éq) est chauffée à 110°C pendant 1h. Après retour à température ambiante, le mélange réactionnel est repris à l'eau et est extrait à l'acétate d'éthyle (x2). Les phases organiques sont rassemblées, lavées avec une solution de $K_2CO_3$ à 5% et avec une solution saturée de chlorure de sodium, séchées sur $MgSO_4$, filtrées et concentrées à sec. Le produit désiré **(19)** est obtenu avec un rendement de 93% (55mg) sous forme d'un solide marron. Ce produit est engagé directement dans l'étape suivante (produit peu stable). $R_f$ (éther de pétrole/acétate d'éthyle: 7/3): 0.53. RMN ($^1$H, 250 MHz, chloroforme-d) δ 9.05 (s, 1H), 8.98 (s, 1H), 8.08 (d, *J* = 2.6 Hz, 1H), 7.84 (d, *J* = 1.7 Hz, 1H), 6.59 (dd, *J* = 2.5, 1.9 Hz, 1H). RMN ($^{13}$C, 63 MHz, chloroforme-d) δ 156.31, 154.42, 142.69, 131.13, 108.52. SM (IC+) *m/z* 181(MH$^+$).

***4-azido-5-(1H-pyrazol-1-yl)pyrimidine (20)***

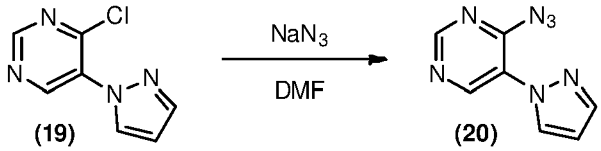


(19) (20)

$C_7H_5N_7$
MW: 187,17g. mol$^{-1}$

Dans un tube scellé sec sous atmosphère d'argon, contenant une solution de 4-chloro-5-pyrazol-1-yl-pyrimidine **(19)** (100mg, 0.55mmol, 1éq) dans le N,N-diméthylformamide anhydre (1.7mL), l'azoture de sodium (72mg, 1.11mmol, 2éq) est ajoutée. Le mélange réactionnel est agité pendant 15h à 50°C et 2h à 70°C. Après retour à température ambiante, le mélange brut est repris à l'eau et est extrait à l'acétate d'éthyle (x3). Les phases organiques sont rassemblées, lavées avec une solution saturée en chlorure de sodium, séchées sur $MgSO_4$, filtrées et concentrées à sec. Le résidu brut est purifié par chromatographie sur colonne de gel de silice (cyclohéxane / acétate d'éthyle: 8/2; dépôt solide). Le produit **(20)** est obtenu avec un rendement de 22% (23mg) sous forme d'une poudre blanche. $R_f$ (éther de pétrole/acétate d'éthyle:7/3): 0.70. RMN ($^1$H, 250 MHz, chloroforme-d) δ 9.58 (s, 1H), 9.21 (d, *J* = 2.7 Hz, 1H), 9.08 (s, 1H), 7.90 (d, *J* = 1.7 Hz, 1H), 6.66 (dd, *J* = 2.7, 1.8 Hz, 1H). SM ($IC_+$) *m/z* 160(MH$^+$-$N_2$), 188(MH$^+$).

***N'-(3-chloropyrazin-2-yl)-2,2,2-trifluoroacetohydrazide (75)***

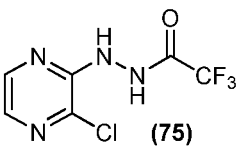

(75)

$C_6H_4ClF_3N_4O$
MW: 240,00g.mol$^{-1}$

A une suspension de (3-chloropyrazin-2-yl)hydrazine ***(12)*** (4.8g, 22mmol, 1éq) dans 58mL de THF anhydre, placée sous atmosphère d'argon et refroidie à 0°C, est ajoutée une solution d'anhydride trifluoroacétique (4mL, 29mmol, 1.3éq) dans 58 mL de THF anhydre. Après 2h d'agitation à 0°C, le milieu réactionnel est concentré. Le brut réactionnel est repris à l'eau et est extrait au DCM (x3). Les phases organiques sont rassemblées, séchés sur $MgSO_4$, et concentrées sous pression réduite. Le résidu brut est purifié par chromatographie sur colonne de gel de silice (Cyclohexane/AcOEt: 8/2 ; dépôt solide). Le produit désiré **(75)** est obtenu avec un rendement de 86% sous forme d'une poudre blanche. RMN ($^1$H, 400MHz, chloroforme-d) δ 8.79 (se, 1H), 8.10 (d, J = 2.7 Hz, 1H), 7.96 (d, J = 2.7 Hz, 1H). 7.37 (se, 1H). RMN ($^{13}$C, 101MHz, chloroforme-d) δ 148.47, 140.18, 135.88, 134.45, 117.27, 114.06. SM (IC$^+$) *m/z* 241 (MH$^+$).

***N'-(5-bromo-3-chloropyrazin-2-yl)-2,2,2-trifluoroacetohydrazide (76)***

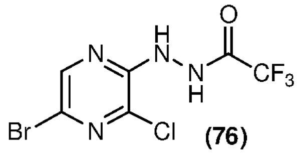

**(76)**

$C_6H_3BrClF_3N_4O$
MW: 319,47g.mol$^{-1}$

A une suspension de N'-(3-chloropyrazin-2-yl)-2,2,2-trifluoroacetohydrazide (2.9g, 11.9 mmol, 1éq) dans 100mL de chloroforme, à 0°C et sous atmosphère d'argon, le N-bromosuccinimide (3.2g, 17.9mmol, 1.5éq) est ajouté par petites portions. La suspension obtenue est laissée sous agitation à température ambiante pendant 24h. Le solvant est ensuite évaporé et le résidu brut est purifié par chromatographie sur colonne de gel de silice (Cyclohexane/AcOEt: 94/6 ; dépôt solide). Le produit **(76)** est obtenu avec un rendement de 50% sous forme d'une poudre jaune. RMN ($^1$H, 400MHz, chloroforme-d) δ 8.74 (se, 1H), 8.17 (s, 1H), 7.31 (se, 1H). RMN ($^{13}$C, 101MHz, chloroforme-d) δ 156.07 (q, J = 38.4 Hz), 147.84, 142.55, 132.71, 127.55, 115,54 (q, J = 250 Hz). SM (IC$^+$) *m/z* 320 (MH$^+$).

***2-azido-5-bromo-3-chloropyrazine (77)***

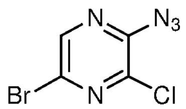

$C_4HBrClN_5$
MW: 234,44g.mol$^{-1}$

**(77)**

A une suspension de **(76)** (1g, 3.1mmol, 1éq) dans l'EtOH (18mL), l'acide chlorhydrique concentré est ajouté goutte à goutte. Le milieu réactionnel est laissé sous agitation à reflux durant la nuit (environ 18h). Le suivi est réalisé par CCM dans acétate d'éthyle/éther de pétrole (5:5). Une fois le produit de départ est consommé, le milieu réactionnel est laissé revenir à température ambiante et est ensuite refroidi à 0°C. Sous forte agitation, une solution de $NaNO_2$ (280mg, 4.1mmol, 1.3éq) dans l'eau (1.8mL), est ajoutée goutte à goutte. Après 30 min d'agitation à 0°C, les solvants volatiles sont éliminés par évaporation. Le brut réactionnel est repris à l'eau et est extrait à l'AcOEt. La phase organique est lavée avec une solution saturée de NaCl, séchée sur $MgSO_4$ puis concentré sous pression réduite. Le résidu brut est purifié par chromatographie sur colonne de gel de silice (Cyclohexane/AcOEt: 80/20 ; dépôt solide). Le produit **(77)** est obtenu avec un rendement de 76% sous forme d'un solide cristallin orange. RMN ($^1$H, 400MHz, chloroforme-d) δ 8.32 (s, 1H). RMN ($^{13}$C, 101MHz, chloroforme-d) δ 148.50, 143.51, 131.85. SM (IC$^+$) m/z 235 (MH$^+$), 207 (M-$N_2$+H$^+$).

***Synthèse de 2,3-dichloroquinoxaline (78)***

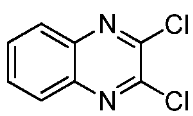

(78)

Dans 100 mL de dichloroéthane sont dissouts 10 g de quinoxaline-2,3-diol (61,67 mmol, 1 eq.) et 11,70 mL de $SOCl_2$ (160,35 mmol, 2,6 eq.). A cette solution sont ajoutés goutte-à-goutte 0,24 mL de DMF (3,08 mmol, 0,05 eq.) et le mélange est porté à reflux pendant 2 h. Ensuite, la solution est concentrée sous vide et le résidu est ensuite recristallisé dans un mélange ACN/eau pour obtenir 10,479 g (52,7 mmol, 85%) du composé attendu.

$^1$H NMR (250 MHz, Chloroform-*d*) δ 8.08 - 7.99 (m, 2H), 7.86 - 7.77 (m, 2H).

***Synthèse d'acide 5,6-dichloropyrazine-2,3-dicarboxylique (79)***

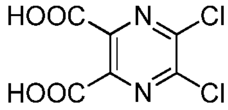

(79)

Dans 150 mL d'eau sont ajoutés 5 g de 2,3-dichloroquinoxaline **(78)** (25,12 mmol, 1 eq.) et 25,807 g de $KMnO_4$ (163,3 mmol, 6,5 eq.). Le mélange est porté à reflux pendant 2h. Après réaction, le mélange chaud est filtré pour ôter le $MnO_2$. La phase aqueuse est acidifiée avec du HCl aqueux. La solution est ensuite concentrée sous vide et séchée. Le résidu est extrait avec de l'acétone, la suspension est filtrée sur Büchner et le filtrat est concentré sous vide pour donner 2,005 g du composé attendu (8,46 mmol, 34%).
$^{13}C$ NMR (63 MHz, DMSO) $\delta$ 163.97, 147.06, 142.66.

***Synthèse de 2,3-dichlorofuro[3,4-b]pyrazine-5,7-dione (80)***

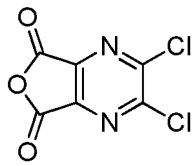

(80)

Dans 30 mL de $SOCl_2$ pur est ajouté 2 g d'acide 5,6-dichloropyrazine-2,3-dicarboxylique **(79)** (8,44 mmol). Le mélange est porté à reflux pendant 30 min. Le mélange est ensuite concentré sous vide pour donner le produit attendu avec un rendement quantitatif.
$^{13}C$ NMR (101 MHz, Acétone) $\delta$ 158.59, 155.69, 144.33.

***Synthèse de 2,3-dichloro-6-propyl-pyrrolo[3,4-b]pyrazine-5,7-dione (81)***

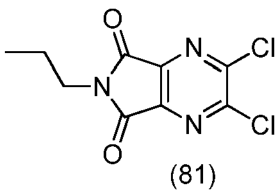

(81)

Dans 20 ml de THF anhydre sous argon sont ajoutés successivement 0,430 g de 2,3-dichlorofuro[3,4-b]pyrazine-5,7-dione **(80)** (1,96 mmol, 1 eq.) et 0,161 mL de propylamine (1,96 mmol, 1 eq.) à température ambiante. Le mélange est agité pendant une heure, puis 0,348 mL de pyridine (4,32 mmol, 2,2 eq.) et 0,185 mL (2,16 mmol, 1,1 eq.) de chlorure d'oxalyle sont ajoutés au mélange qui est ensuite porté à reflux une nuit. Le mélange est dissous dans de l'acétate d'éthyle et lavé avec NaCl sat. (2 fois). Les phases aqueuses sont réextraites à l'EA et les phases organiques combinées sont séchées sur $MgSO_4$, filtrées et concentrées sous vide. Le résidu est dissous dans un minimum de dichlorométhane et le produit est précipité par ajout d'éther de pétrole. Le précipité est filtré sur Büchner et lavé à l'éther de pétrole pour donner 0,293 g du composé attendu (1,13 mmol, 57%) sous forme d'un solide beige clair.
$^{1}H$ NMR (400 MHz, Chloroform-d) $\delta$ 3.77 (t, $J$ = 7.2 Hz, 2H), 1.75 (h, $J$ = 7.6 Hz, 4H), 0.97 (t, $J$ = 7.4 Hz, 2H).
$^{13}C$ NMR (101 MHz, $CDCl_3$) $\delta$ 162.35, 153.66, 143.78, 40.76, 21.94, 11.39.
HRMS (ESI): $[M+H]^+$ calculé pour $C_9H_8Cl_2N_3O_2$ 259.998808, mesuré 259.998893 (0.3 ppm).

***Synthèse de benzo[g]quinoxaline-2,3-diol (82)***

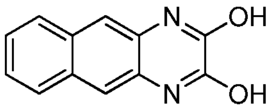

(82)

Dans 10 mL de diéthyl oxalate pur est ajouté 0,500 g de naphtalène 2,3-diamine (3,16 mmol). Le mélange est chauffé à 110°C une nuit. Après refroidissement, de l'éthanol est ajouté au mélange. Par filtration sur Büchner et lavage à l'éthanol, 0,545 g de solide ocre sont obtenus (2,57 mmol, 81%).

$^1$H NMR (250 MHz, DMSO-$d_6$) δ 12.08 (s, 2H), 7.85 - 7.78 (m, 2H), 7.54 (s, 2H), 7.42 - 7.35 (m, 2H).

***Synthèse de 2,3-dichlorobenzo[g]quinoxaline (83)***

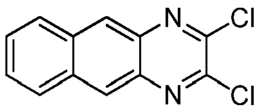

(83)

Dans un ballon est dissout 0,677 g de benzo[g]quinoxaline-2,3-diol **(82)** (3,19 mmol) dans 20 mL de $POCl_3$, la solution est portée à reflux une nuit. Le lendemain, le mélange est concentré sous pression réduite et le résidu dissout dans du dichlorométhane et lavé avec NaHCOs sat, puis NaCl sat. Après séchage sur $MgSO_4$, filtration et concentration sou vide, le résidu est purifié par flash filtration sur silice avec PE/DCM 1:1 comme éluant pour obtenir 0,229 g du produit dichloré (0,92 mmol, 29%) sous forme d'un solide ocre.

$^1$H NMR (250 MHz, Chloroform-d) δ 8.57 (s, 2H), 8.11 (m, 2H), 7.64 (m, 2H).

***Synthèse de N-prop-2-ynyl-1H-indazole-7-carboxamide (84)***

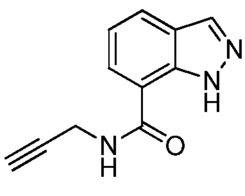

(84)

Dans de l'acétonitrile sont ajoutés successivement 0,200 g d'acide 7-carboxylique indazole (1,23 mmol, 1 eq.), 0,237 mL de propargylamine (3,70 mmol, 3 eq.) et 0,515 g d'HBTU (1,36 mmol, 1,1 eq.). Le mélange est agité une nuit à température ambiante puis la solution est diluée dans de l'acétate d'éthyle et lavée avec NaCl sat. Après séchage sur $MgSO_4$, filtration et concentration sous pression réduite, le résidu est repris avec du dichlorométhane et quelques gouttes d'éther de pétrole pour former un précipité blanc qui est filtré sur Büchner et lavé avec de l'éther de pétrole pour donner un solide blanc (0,150 g, 0,75 mmol, 61%).

$^1$H NMR (400 MHz, Acetone-d6) δ 12.38 (s, 1H), 8.38 (s, 1H), 8.13 (s, 1H), 8.01 (dd, $J$ = 8.0, 0.9 Hz, 1H), 7.97 (d, $J$ = 7.3 Hz, 1H), 7.22 (t, $J$ = 7.7 Hz, 1H), 4.29 (dd, $J$ = 5.6, 2.4 Hz, 2H), 2.70 (t, $J$ = 2.5 Hz, 1H).

$^{13}$C NMR (101 MHz, Acetone) δ 166.82, 134.62, 125.78, 125.26, 120.79, 100.91, 81.33, 72.07.

HRMS (ESI) : $[M+H]^+$ calculé pour $C_{11}H_{10}N_3O$ 200.081838, mesuré 200.081715 (0.6 ppm).

***Synthèse de 2-méthoxy-6-méthyl-benzènediazonium tétrafluoroborate* (85)**

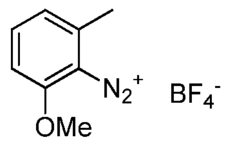

(85)

Dans un ballon à 0°C sont dissouts 1,009 g de 2-methoxy-5-methylaniline (7,35 mmol, 1 eq.) dans 8 mL de $HBF_4$ 50% aq. A cette solution est ajoutée goutte-à-goutte 0,608 g de $NaNO_2$ dissout dans 1 mL d'eau. La réaction est agitée vigoureusement 15 min. à 0°C et un précipité brun se forme. Ce précipité est filtré sur Büchner et lavé avec de l'éther diéthylique pour fournir 1,772 g du sel de diazonium attendu avec un rendement quantitatif.

$^1H$ NMR (250 MHz, Deuterium Oxide) δ 8.08 (dd, *J* = 8.8, 7.9 Hz, 1H), 7.36 (d, *J*= 8.9 Hz, 1H), 7.28 (d, *J*= 7.8 Hz, 1H), 4.21 (s, 3H), 2.70 (s, 3H).

***Synthèse de 7-méthoxyindazole (86)***

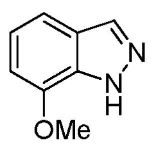

(86)

Dans un ballon sous argon sont dissous dans du chloroforme sec 1,750 g de 2-méthoxy-6-méthyl-benzènediazonium tétrafluoroborate (7,42 mmol, 1 eq.), 0,098 g d'éther couronne 18-6 (0,37 mmol, 0,05 eq.) préalablement activé et 1,456 g de KOAc (14,83 mmol, 2 eq.). Le mélange est agité une nuit à température ambiante puis la solution est extraite au dichlorométhane et lavée avec NaCl sat. Après séchage sur $MgSO_4$, filtration et concentration sous vide, le résidu est purifié par flash filtration sur silice pour fournir 0,623 g (4,20 mmol, 57%) sous forme d'un solide brun.

$^1H$ NMR (250 MHz, Chloroform-d) δ 10.52 (s, 1H), 8.05 (s, 0H), 7.34 (dd, *J* = 8.1, 0.7 Hz, 1H), 7.08 (dd, *J* = 8.2, 7.5 Hz, 0H), 6.75 (dd, *J* = 7.5, 0.7 Hz, 1H), 4.00 (s, 3H).

***Synthèse de 4-méthoxyindazole (87)***

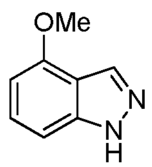

(87)

Dans 20 mL de dioxane sont ajoutés 1,008 g de 2-fluoro-5-méthoxy benzaldéhyde (6,54 mmol, 1 eq.) et 6,5 mL d'hydrazine monohydrate (1 mL/mmol aldéhyde). Le mélange est chauffé à reflux 20h. Après refroidissement, le mélange est dilué dans de l'acétate d'éthyle et lavé avec NaCl sat. La phase organique est séchée sur $MgSO_4$, filtrée et concentrée sous pression réduite. Le résidu est purifié par chromatographie sur silice avec DCM/MeOH pour obtenir 0,207 g (1,40 mmol, 21%) de l'indazole.

$^1H$ NMR (400 MHz, Chloroform-*d*) δ 9.00 (d, *J* = 0.9 Hz, 1H), 7.34 (td, *J* = 8.4, 6.2 Hz, 1H), 6.83 - 6.70 (m, 2H), 3.90 (s, 3H).

RMN en accord avec la littérature Lukin, K.; Hsu, M. C.; Fernando, D.; Leanna, M. R. *J. Org. Chem.* **2006,** *71* (21), 8166.

***Synthèse de 7-bromo-1H-pyrazolo[3,4-c]pyridine (88)***

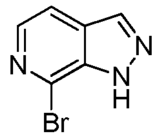

(88)

Dans 50 mL d'acide acétique sont ajoutés 1 g de 2-bromo-3-amino-4-methyl pyridine (5,35 mmol, 1 eq.) et 0,682 g d'acétate de potassium (6,95 mmol, 1,3 eq.). A cette solution est ajouté à température ambiante 0,443 g de nitrite de sodium (6,42 mmol, 1,2 eq.) dissouts dans 5 mL d'eau goutte-à-goutte. Après addition, le mélange est chauffé à 60°C une nuit. Une fois refroidie, le mélange est concentré sous vide et le résidu est dissous dans l'acétate d'éthyle, lavé avec $NaHCO_3$ sat. puis NaCl sat. La phase organique est séchée sur $MgSO_4$, filtrée et concentré sous vide. Le résidu est ensuite purifié par chromatographie flash avec PE/EA pour donner 0,419 g de l'indazole attendu (2,12 mmol, 40%).

$^1H$ NMR (250 MHz, Chloroform-d) $\delta$10.48 (bs, 1H), 8.23 (s, 1H), 8.12 (d, *J* = 5.5 Hz, 1H), 7.63 (d, *J* = 5.5 Hz, 1H).

***II.1. Synthèse des intermédiaires par substitution nucléophile aromatique***

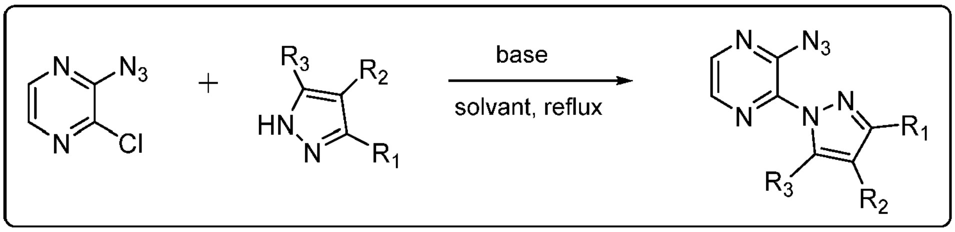


<u>Procédure générale A1</u>

Dans un ballon, placé sous atmosphère d'argon, le nucléophile (1éq) est solubilisé dans l'acétonitrile anhydre (concentration comprise entre 0.14-0.16M). Le carbonate de potassium (2éq) est ensuite introduit. Après une purge à l'argon et 10 min d'agitation à température ambiante, le 2-azido-3-chloropyrazine **(13)** (1éq) est introduit par des petites portions. La suspension obtenue est laissée sous agitation à reflux du solvant et sous atmosphère d'argon jusqu'à la consommation totale du 2-azido-3-chloropyrazine **(13).** Le traitement, ainsi que les méthodes de purification, ont été choisis en fonction des caractéristiques physico-chimiques (polarité, solubilité, stabilité, ...) de chaque azoture.

<u>Traitement général</u>: après retour à température ambiante, le milieu réactionnel est concentré, le résidu brut est repris à l'eau et est extrait à l'acétate d'éthyle. Les phases organiques sont rassemblées, lavées avec une solution saturée de chlorure de sodium, séchées sur $MgSO_4$, filtrées et concentrées sous pression réduite. Le résidu brut est purifié par chromatographie sur colonne de gel de silice.

<u>Procédure générale A2</u>

Dans un ballon sous argon est dissous 0.3 mmol (1 eq.) d'indazole dans 5 mL de DMF ou de THF anhydre. Le ballon est plongé dans un bain de glace et 1,1 eq. de NaH (60% mass.) (2.1 eq. si on utilise un indazole avec une fonction acide) sont ajoutés au mélange. La réaction est agitée à 0°C pendant 30 min. Ensuite, on ajoute 1,05 eq. d'azido-chloropyrazine dissoute dans 1 mL de solvant (DMF ou THF) anhydre goutte-à-goutte. On laisse réagir une nuit en laissant remonter la température jusqu'à l'ambiante.

***2-azido-3-(1H-pyrazol-1-yl)pyrazine (21)***

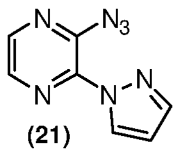

**(21)**

$C_7H_5N_7$
MW: 187,17g.mol$^{-1}$

Ce composé a été préparé selon la Procédure générale A1 avec le 1H-pyrazole comme nucléophile. Le temps de réaction est de 3h. Le suivi est réalisé par CCM dans dichlorométhane/acétate d'éthyle (7:3). Après extraction, le produit désiré **(21)** est obtenu avec un rendement de 81% (145mg) sous forme d'une poudre jaune. RMN ($^1$H, 250MHz, Chloroforme-d) $\delta$ 9.16 (dd, *J* = 2.8, 0.5 Hz, 1H), 8.68 (d, *J* = 4.5 Hz, 1H), 8.18 (d, *J* = 4.5 Hz, 1H), 8.07 (d, *J* = 1.2 Hz, 1H), 6.70 (dd, *J* = 2.8, 1.6 Hz, 1H). RMN ($^{13}$C, 250MHz, Chloroforme-d) $\delta$ 146.47, 132.41, 131.91, 116.77, 111.26. SM ($IC_+$) *m/z* 188($MH^+$), 160 ($M-N_2+H^+$).

***2-azido-3-(3-nitro-1H-pyrazol-1-yl)pyrazine (22)***

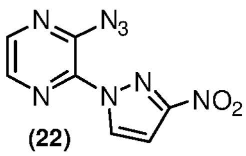

**(22)**

$C_7H_4N_8O_2$
MW: 232,16 g.mol$^{-1}$

Ce composé a été préparé selon la Procédure générale A1 avec le 3-nitro-1H-pyrazole comme nucléophile et le dichlorométhane anhydre comme solvant. Le temps de réaction est de 3h. Le suivi est réalisé par CCM dans dichlorométhane/méthanol (95/5, $R_f$=0.6). Après extraction et purification par chromatographie sur colonne de gel de silice (dichlorométhane/méthanol: 95/5; dépôt solide), le produit attendu **(22)** est obtenu avec un rendement de 27% (384mg) sous forme d'une poudre jaune. RMN ($^1$H, 400MHz, DMSO-$d_6$) $\delta$ 9.57 (d, *J* = 4.6 Hz, 1H), 8.23 (d, *J* = 2.9 Hz, 1H), 8.41 (d, *J* = 4.6 Hz, 1H), 7.51 (d, *J* = 2.9 Hz, 1H). RMN ($^{13}$C, 400MHz, DMSO-$d_6$) $\delta$ 158.5, 140.8, 134.7, 131.4, 120.1, 105.6. SM ($IC_+$) *m/z* 233($MH^+$), 205 ($MH^+-N_2$).

***2-azido-3-(4-nitro-1H-pyrazol-1-yl)pyrazine (23)***

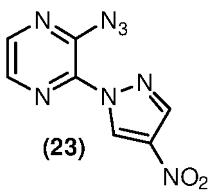

**(23)**

$C_7H_4N_8O_2$
MW: 232,16 g.mol$^{-1}$

Ce composé a été préparé selon la Procédure générale A1 avec le 4-nitro-1H-pyrazole comme nucléophile. Le temps de réaction est de 4h. Le suivi est réalisé par CCM dans dichlorométhane/méthanol (95/5, $R_f$=0.45). Après extraction, le produit attendu **(23)** est obtenu avec un rendement de 76% (840mg) sous forme d'une poudre jaune. RMN ($^1$H, 250MHz, DMSO-$d_6$) $\delta$ 9.81 (d, *J* = 0.7 Hz, 1H), 9.51 (d, *J* = 4.6 Hz, 1H), 8.82 (d, *J* = 0.7 Hz, 1H), 8.36 (d, *J* = 4.6 Hz, 1H). SM ($IC_+$) *m/z* 233($MH^+$), 205 ($MH^+-N_2$).

***2-azido-3-(3-methoxy-1H-pyrazol-1-yl)pyrazine (24)***

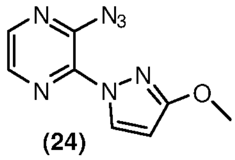

**(24)**

$C_8H_7N_7O$
MW: 217,19 g.mol$^{-1}$

Ce composé a été préparé selon la *Procédure générale A1* avec le 3-méthoxy-1H-pyrazole **(8)** comme nucléophile. Le temps de réaction est de 2h. Le suivi est réalisé par CCM dans l'acétate d'éthyle/éther de pétrole (5:5). Après extraction, le produit attendu **(24)** est obtenu avec un rendement de 82% (324mg) sous forme d'une poudre blanche. RMN ($^1$H, 400MHz, Chloroforme-de) δ 9.27 (d, *J* = 3.0 Hz, 1H), 8.54 (d, *J* = 4.5 Hz, 1H), 8.15 (d, *J* = 4.5 Hz, 1H), 6.21 (d, *J* = 3.0 Hz, 1H), 4.15 (s, 3H). RMN ($^{13}$C, 400MHz, Chloroforme-$d_6$) δ 135.33, 132.83, 114.90, 100.91, 57.44. SM (IC+) *m/z* 190(M-$N_2$+$H^+$), 218 (M+$H^+$), 240 (M+$Na^+$).

***2-azido-3-(3-nitro-1H-1,2,4-triazol-1-yl)pyrazine (25)***

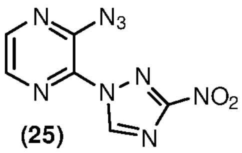

**(25)**

$C_6H_3N_9O_2$
MW: 233,15 g.mol$^{-1}$

Ce composé a été préparé selon la *Procédure générale A1* avec le 3-nitro-1H-1,2,4-triazole comme nucléophile. Le temps de réaction est de 1h30. Le suivi est réalisé par CCM dans dichlorométhane/acétate d'éthyle (7:3). Après extraction et purification par chromatographie sur colonne de gel de silice (dichlorométhane/ acétate d'éthyle : 7/3; dépôt solide), le produit attendu **(25)** est obtenu avec un rendement de 93% (140mg) sous forme d'une poudre jaune pâle. RMN ($^1$H, 400MHz, chloroforme-d) 9.98 (s, 1H), 8.98 (d, *J* = 4.5 Hz, 1H), 8.42 (d, *J* = 4.5 Hz, 1H). RMN ($^{13}$C, 400MHz, chloroforme-d) δ 146.86, 131.35, 118.93. SM (IC+) *m/z* 206(M-$N_2$+$H^+$), 234 (M+$H^+$), 256 (M+$Na^+$).

***2-azido-3-(1H-1,2,3-triazol-1-yl)pyrazine (26)***

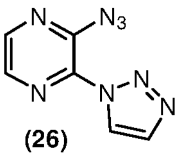

**(26)**

$C_6H_4N_8$
MW: 188,15g.mol$^{-1}$

Ce composé a été préparé selon la *Procédure générale A1* avec le (1H)-1,2,3-triazole comme nucléophile. Le temps de réaction est de 3h. Le suivi est réalisé par CCM dans dichlorométhane/acétate d'éthyle (7/3, $R_f$=0.59). Après extraction, le produit attendu **(26)** est obtenu avec un rendement de 95% (170mg) sous forme d'une poudre jaune pâle. RMN ($^1$H, 400MHz, chloroforme-d) δ 8.83 (d, *J* = 4.5 Hz, 1H), 8.31 (d, *J* = 4.5 Hz, 1H), 8.23 (s, 2H). RMN ($^{13}$C, 400MHz, chloroforme-d) 140.08 (2C), 131.96, 118.47. SM (IC+) *m/z* 161(M$H^+$-$N_2$), 189 (M$H^+$), 211 (M+$Na^+$).

*2-azido-3-(1H-1,2,4-triazol-1-yl)pyrazine (27)*

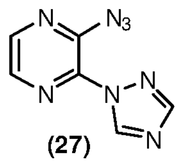

(27)

$C_6H_4N_8$
MW: 188,15 g.mol$^{-1}$

Ce composé a été préparé selon la *Procédure générale A1* avec le (1H)-1,2,4-triazole comme nucléophile. Le temps de réaction est de 3h. Le suivi est réalisé par CCM dans dichlorométhane/acétate d'éthyle (8:2). Après retour à température ambiante, le milieu réactionnel est filtré et est rincé au chloroforme. Le solide blanc est repris dans un mélange dichlorométhane/eau, est agité et est filtré pour donner le produit **(27)** avec un rendement de 81% (1.46g) sous forme d'une poudre blanche. RMN ($^1$H, 400MHz, chloroforme-d) δ 9.85 (s, 1H), 8.82 (d, *J* = 4.5 Hz, 1H), 8.39 (s, 1H), 8.28 (d, *J* = 4.5 Hz, 1H). RMN ($^{13}$C, 101MHz, chloroforme-d) δ 154.92, 145.66, 131.81, 117.96. SM (IC+) m/z 189(MH$^+$), 161(MH$^+$-$N_2$).

*1-(3-azidopyrazin-2-yl)-1H-benzo[d][1,2,3]triazole (28)*

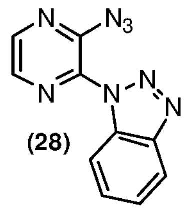

(28)

$C_{10}H_6N_8$
MW: 238,21g.mol$^{-1}$

Ce composé a été préparé selon la *Procédure générale A1* avec le (1H)-benzotriazole comme nucléophile. Le temps de réaction est de 2h30. Le suivi est réalisé par CCM dans acétate d'éthyle/éther de pétrole (5:5). Le mélange réactionnel est filtré à chaud (rincé avec de l'acétonitrile chaud). Le filtrat est concentré et le solide obtenu est trituré dans le mélange chloroforme/éther diéthylique (1/1: v/v). Le précipité est récupéré par filtration sur Milli-Pore. Le produit attendu **(28)** est obtenu avec un rendement de 95% (405mg) sous forme d'une poudre beige. IR (solide) : ν (cm$^{-1}$) 3091, 1522, 1481. RMN ($^1$H, 400MHz, DMSO-$d_6$) δ 9.51 (d, *J* = 4.6 Hz, 1H), 8.57 (dt, *J* = 8.4, 1.0 Hz, 1H), 8.49 (d, *J* = 4.6 Hz, 1H), 8.35 (dt, *J* = 8.3, 1.0 Hz, 1H), 7.85 (ddd, *J* = 8.3, 7.1, 1.1 Hz, 1H), 7.68 (ddd, *J* = 8.2, 7.1, 1.1 Hz, 1H). RMN ($^{13}$C, 101MHz, DMSO-$d_6$) δ 145.59, 141.86, 139.81, 131.29, 130.25, 126.45, 120.10, 118.90, 114.50. SM (IC+) *m/z* 211(MH$^+$-$N_2$), 239 (MH$^+$), 261 (M+Na$^+$);

*1-(3-azidopyrazin-2-yl)-1H-indazole (29)*

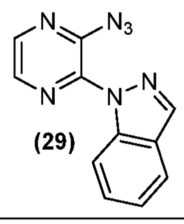

(29)

$C_{11}H_7N_7$
MW: 237,23 g.mol$^{-1}$

Ce composé a été préparé selon la *Procédure générale A1* avec le 1,2-benzodiazole comme nucléophile. Le

temps de réaction est de 6h. Après retour à température ambiante, le milieu réactionnel est filtré sur milli-pore. Le solide récupéré est repris dans un mélange eau/acétone (7/3 : v/v), est agité et le précipité formé est récupéré par filtration sur milli-pore. Le produit désiré **(29)** est obtenu avec un rendement de 80% (855mg) sous forme d'une poudre jaune pale. RMN ($^1$H, 400 MHz, DMSO-d6) $\delta$ 9.28 (d, *J* = 4.5 Hz, 1H), 8.75 (s, 1H), 8.75 (d, *J* = 8.9 Hz, 1H), 8.33 (d, *J* = 4.6 Hz, 1H), 8.03 (d, *J* = 7.9 Hz, 1H), 7.71 (t, *J* = 8.0 Hz, 1H), 7.49 (t, *J* = 7.6 Hz, 1H). RMN ($^{13}$C, 101 MHz, DMSO-d6) $\delta$ 131.24, 129.03, 121.83, 118.19, 118.17, 116.70, 115.17. SM (IC+) *m/z* 210(M-$N_2$+$H^+$), 238 (M+$H^+$), 260 (M+$Na^+$).

***2-azido-3-(4-(4,4,5,5-tétramethyl-1,3,2-dioxaborolan-2-yl)-1H pyrazol-1-yl)pyrazine (30)***

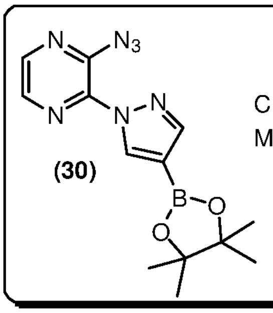

**(30)**

$C_{13}H_{16}BN_7O_2$
MW: 313,13 g.mol$^{-1}$

Ce composé a été préparé selon la Procédure générale A1 avec l'acide 4-pyrazoleboronique pinacol ester comme nucléophile. Le temps de réaction est de 3h. Le suivi est réalisé par CCM dans dichlorométhane/méthanol (98:2). Le milieu réactionnel est filtré à chaud et est rincé avec de l'acétonitrile. Le filtrat est concentré sous pression réduite et le solide obtenu est solubilisé dans quelques gouttes de chloroforme et est précipité dans le pentane. Le précipité est éliminé par filtration, le filtrat est concentré et est filtré sur silice (dichlorométhane/acétate d'éthyle : 8/2). Après quelques lavages au pentane le produit désiré **(30)** est obtenu avec un rendement de 87% (210mg) sous forme d'une poudre blanche. RMN ($^1$H, 400 MHz, chloroforme-d) $\delta$ 9.43 (s, 1H), 8.69 (d, *J* = 4.5 Hz, 1H), 8.25 (s, 1H), 8.17 (d, *J* = 4.5 Hz, 1H), 1.37 (s, 12H). RMN ($^{13}$C, 101 MHz, chloroforme-d) $\delta$ 150.31, 141.66, 138.52, 138.12, 131.62, 116.13, 83.90, 24.67. SM (IC+) *m/z* 314 (M$H^+$).

***2-azido-3-(3,5-diméthyl-4-(4,4,5,5-tétramethyl-1,3,2-dioxaborolan-2-yl)-1H-pyrazol-1-yl)pyrazine (31)***

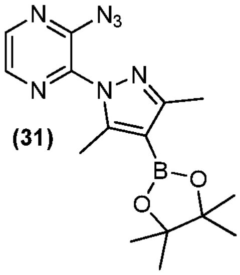

**(31)**

$C_{15}H_{20}BN_7O_2$
MW: 341,18g.mol$^{-1}$

Ce composé a été préparé selon la Procédure générale A1 avec l'acide 3,5-dimethyle-4-pyrazoleboronique pinacol ester comme nucléophile. Le temps de réaction est de 3h. Le suivi est réalisé par CCM dans dichlorométhane/méthanol (98:2). Après retour à température ambiante, le milieu réactionnel est filtré et le filtrat est concentré. Le résidu brut est filtré sur silice (dichlorométhane/acétate d'éthyle : 8/2). Après quelques lavages au pentane le produit désiré **(31)** est obtenu avec un rendement de 87% (210mg) sous forme d'une poudre blanche. RMN ($^1$H, 400 MHz, chloroforme-d) 8.64 (d, *J* = 4.2 Hz, 1H), 8.10 (d, *J* = 4.3 Hz, 1H), 2.83 (s, 3H), 2.53 (s, 3H), 1.34 (s, 12H). RMN ($^{13}$C, 101 MHz, chloroforme-d) $\delta$ 159.67 ($C_q$), 152.05 ($C_q$), 144.24 ($C_q$), 140.57 ($C_q$), 131.10, 116.26, 83.33 ($C_q$), 25.07, 14.78, 14.47. SM (IC+) m/z 341 (M$H^+$), 313 (M-$N_2$-$H^+$).

***2-azido-3-(4-bromo-1H-pyrazol-1-yl)pyrazine (32)***

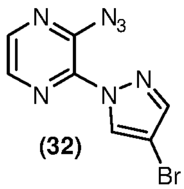

**(32)**

$C_7H_4BrN_7$
MW: 266,06 g.mol$^{-1}$

Ce composé a été préparé selon la *Procédure générale A1* avec le 4-bromo-1H-pyrazole comme nucléophile. Après 8h d'agitation à reflux du solvant dans le milieu est ajoutés une quantité supplémentaire de 4-bromo-1*H*-pyrazole (0.05éq). Le temps de réaction totale est de 9h. Le suivi est réalisé par CCM dans l'éther de pétrole/acétate d'éthyle (6:4). Après retour à température ambiante, le milieu réactionnel est concentré à sec, le résidu obtenu est repris à l'eau et est laissé sous agitation à 0°C pendant 20min. La suspension est filtrée pour donner le produit désiré **(32)** avec un rendement de 96% (3,3g) sous forme d'une poudre blanche. RMN ($^1H$, 250 MHz, chloroforme-d) $\delta$ 9.16 (s, 1H), 8.71 (d, *J* = 4.5 Hz, 1H), 8.17 (d, *J* = 4.5 Hz, 1H), 8.02 (s, 1H). RMN ($^{13}C$, 101 MHz, chloroforme-d) $\delta$ 146.47, 131.72, 130.93, 116.82, 100.19. SM (IC+) *m/z* 234 ($MH^+$-$N_2$), 266 ($MH^+$), 288 ($MNa^+$).

***2-azido-3-(4-iodo-1H-pyrazol-1-yl)pyrazine (33)***

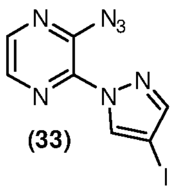

**(33)**

$C_7H_4IN_7$
MW: 313,06g.mol$^{-1}$

Ce composé a été préparé selon la *Procédure générale A1* avec le 4-bromo-1H-pyrazole comme nucléophile. Le temps de réaction est de 2h. Le suivi est réalisé par CCM dans dichlorométhane /acétate d'éthyle (8:2). Après retour à température ambiante, le milieu réactionnel est concentré à sec, le résidu est repris à l'eau distillée et est laissé sous agitation à 0°C pendant 20min. La suspension est filtrée et est rincée à l'acétonitrile pour obtenir le produit désiré **(33)** avec un rendement de 93% (2,8g) sous forme d'une poudre blanche. RMN ($^1H$, 250 MHz, DMSO-d6) $\delta$ 9.35 (d, *J* = 4.6 Hz, 1H), 9.13 (s, 1H), 8.26 (d, *J* = 4.6 Hz, 1H), 8.19 (s, 1H). RMN ($^{13}C$, 400 MHz, DMSO-d6) $\delta$ 149.21, 140.62, 139.20, 134.98, 131.50, 118.53, 64.59. SM (IC+) *m/z* 286 ($MH^+$-$N_2$), 314 ($MH^+$), 335 ($MNa^+$)

***1-(3-azidopyrazin-2-yl)-1H-pyrazolo[3,4-b]pyrazine et 2-(3-azido pyrazin-2-yl)-2H-pyrazolo[3,4-b]pyrazine (34.1 et 34.2)***

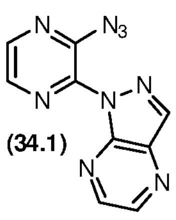

**(34.1)**

et

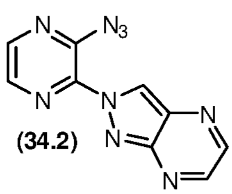

**(34.2)**

$C_9H_5N_9$
MW: 239,20g.mol$^{-1}$

Ces composés ont été préparés selon la *Procédure générale A1* avec le 1H-pyrazolo[3,4-b]pyrazine **(11)** comme nucléophile. Le temps de réaction est de 3h. Le suivi est réalisé par CCM dans dichlorométhane/ méthanol (97:3). Après retour à température ambiante, le milieu réactionnel est concentré à sec, le résidu est repris à l'eau distillée et est laissé sous agitation à 0°C pendant 20min. La suspension est filtrée pour donner le mélange de deux isomères **(34.1)** et **(34.2)** avec un rapport de 1:0.66 (obtenu par RMN). Le mélange de deux produits est engagé dans la réaction suivante sans purification supplémentaire. **(34.1)** : RMN ($^1$H, 250 MHz, DMSO-d6) $\delta$ 9.46 (d, *J* = 4.6 Hz, 1H), 9.06 (s, 1H), 8.88 (d, *J* = 2.3 Hz, 1H), 8.82 (d, *J* = 2.3 Hz, 1H), 8.41 (d, *J* = 4.6 Hz, 1H). **(34.2)** : RMN ($^1$H, 250 MHz, DMSO-d6) 9.91 (s, 1H), 9.57 (d, *J* = 4.6 Hz, 1H), 8.84 (d, *J* = 1.7 Hz, 1H), 8.76 (d, *J* = 1.4 Hz, 1H), 8.45 (d, *J* = 4.6 Hz, 1H). **(34.1)** et **(34.2) :** SM (IC+) *m/z* 212 (M-$N_2$+$H^+$), 240 ($MH^+$), 262 ($MNa^+$).

***2-azido-3-(1H-pyrazol-1-yl)quinoxaline (35)***

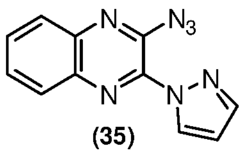

**(35)**

$C_{11}H_7N_7$
MW: 237,23 g.mol$^{-1}$

Dans un ballon placé sous atmosphère d'argon, le pyrazole (23mg, 0.34mmol, 1.1éq), le carbonate de césium (196mg, 0.6mmol, 2éq), et le dichlorométhane anhydre (5mL) sont introduits. Dans la suspension obtenue, une solution de 2-azido-3-chloroquinoxaline **(16)** (64mg, 0.3mmol, 1éq) dans le dichlorométhane anhydre (3mL) est ajoutée goutte à goutte. Le milieu réactionnel est laissé sous agitation à température ambiante pendant 5min et 6h à reflux du solvant. Le suivi est réalisé par CCM dans acétate d'éthyle/éther de pétrole (7:3). Après retour à température ambiante, le milieu est repris à l'eau et est extrait 3 fois au dichlorométhane. Les phases organiques sont rassemblées, séchées sur $MgSO_4$, filtrées et concentrées à sec. Le mélange brut est purifié par chromatographie sur colonne de gel de silice (éther de pétrole/acétate d'éthyle : 7/3; dépôt solide). Le produit attendu **(35)** est obtenu avec un rendement de 85% (61mg) sous forme d'une poudre jaune. RMN ($^1$H, 400 MHz, chloroforme-d) $\delta$ 9.30 (d, *J* = 2.8 Hz, 1H), 8.72 - 8.59 (m, 1H), 8.31 (dd, *J* = 6.4, 3.1 Hz, 1H), 8.08 (d, *J* = 2.8 Hz, 1H), 7.86 (dd, *J* = 6.2, 3.4 Hz, 2H), 6.72 (dd, *J* = 2.7, 1.6 Hz, 1H). RMN ($^{13}$C, 101 MHz, chloroforme-d) $\delta$ 145.63, 139.02, 138.30, 135.49, 131.69, 130.62, 130.43, 129.90, 124.37, 116.55, 110.43. SM (IC+) *m/z* 210 (M+$H^+$-$N_2$), 238 (M+$H^+$), 260 (M+$Na^+$), 497 (2M+$Na^+$).

***2-azido-3-(4-methoxy-1H-pyrazol-1-yl)pyrazine (89)***

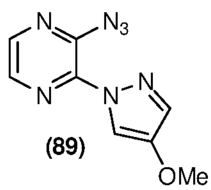

**(89)**

$C_8H_7N_7O$
MW: 217,19 g.mol$^{-1}$

Ce composé a été préparé selon la *Procédure générale A1* avec le 4-méthoxy-1H-pyrazole (préparé selon la méthode décrite dans WO2014/008197) comme nucléophile. Le temps de réaction est de 18h. Après retour à température ambiante, le milieu réactionnel est concentré à sec et est engagé sans purification dans l'étape suivante. SM (IC+) *m/z* 190 (M-$N_2$+$H^+$), 218 (M+$H^+$), 240 (M+$Na^+$).

***2-azido-3-(3-iodo-1H-pyrazol-1-yl)pyrazine (90)***

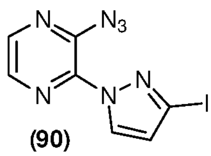

(90)

$C_7H_4IN_7$
MW: 313,06g.mol$^{-1}$

Ce composé a été préparé selon la <u>*Procédure générale A1*</u> avec le 3-iodo-1H-pyrazole (800mg, 4.1mmol, 1 éq) comme nucléophile. Le temps de réaction est de 1h45. Après retour à température ambiante, le milieu réactionnel est concentré à sec et est engagé sans purification dans l'étape suivante. RMN ($^1$H, 250 MHz, chloroforme-d) δ 9.17 (d, J = 2.8 Hz, 1H), 8.69 (d, J = 4.5 Hz, 1H), 8.21 (d, J = 4.6 Hz, 1H), 6.86 (d, J = 2.8 Hz, 1H).

***1-(3-azidopyrazin-2-yl)-4,5,6,7-tetrahydro-1H-indazole (91)***

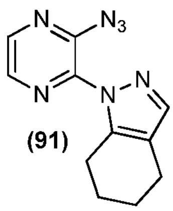

(91)

$C_{11}H_{11}N_7$
MW:241,26g.mol$^{-1}$

Ce composé a été préparé selon la <u>*Procédure générale A1*</u> avec le 4,5,6,7-tétrahydro-1H-indazole (230mg, 1.9mmol, 1éq) comme nucléophile. Le temps de réaction est de 4h. Après retour à température ambiante, le milieu réactionnel est concentré à sec et est engagé sans purification dans l'étape suivante. SM (IC+) m/z 214 ($M-N_2+H^+$), 242 ($M+H^+$).

***1-(3-azidopyrazin-2-yl)-4-nitro-1H-indazole (92)***

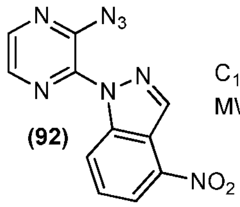

(92)

$C_{11}H_6N_8O_2$
MW: 282,22 g.mol$^{-1}$

Ce composé a été préparé selon la <u>*Procédure générale A1*</u> avec le 4-nitroindazole comme nucléophile. Le temps de réaction est de 3h. Après retour à température ambiante, le milieu réactionnel est concentré à sec et est engagé sans purification dans l'étape suivante.

***1-(3-azidopyrazin-2-yl)-5-nitro-1H-indazole (93)***

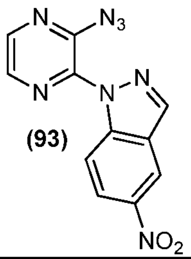

(93)

$C_{11}H_6N_8O_2$
MW: 282,22 g.mol$^{-1}$

Ce composé a été préparé selon la *Procédure générale A1* avec le 5-nitroindazole comme nucléophile. Le temps de réaction est de 20h. Après retour à température ambiante, le milieu réactionnel est concentré à sec et est engagé sans purification dans l'étape suivante.

***1-(3-azidopyrazin-2-yl)-6-nitro-1H-indazole (94)***

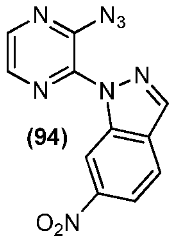

(94)

$C_{11}H_6N_8O_2$
MW: 282,22 g.mol$^{-1}$

Ce composé a été préparé selon la *Procédure générale A1* avec le 6-nitroindazole comme nucléophile. Le temps de réaction est de 22h. Après retour à température ambiante, le milieu réactionnel est concentré à sec et est engagé sans purification dans l'étape suivante.

***2-(3-azidopyrazin-2-yl)-7-nitro-2H-indazole (95)***

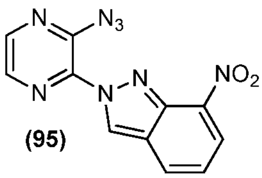

(95)

$C_{11}H_6N_8O_2$
MW: 282,22 g.mol$^{-1}$

Ce composé a été préparé selon la *Procédure générale A1* avec le 7-nitroindazole comme nucléophile. Le temps de réaction est de 18h. Après retour à température ambiante, le milieu réactionnel est concentré à sec et est engagé sans purification dans l'étape suivante.

***1-(3-azidopyrazin-2-yl)-1H-indazole (96)***

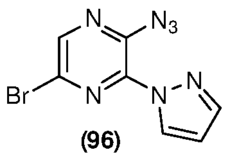

(96)

$C_7H_4BrN_7$
MW: 266,06 g.mol$^{-1}$

Ce composé a été préparé selon la <u>*Procédure générale A1*</u> avec le 1H-pyrazole comme nucléophile et le composé **(77)** comme dérivé halogéné. Le temps de réaction est de 12h. Après retour à température ambiante, le milieu réactionnel est concentré à sec et est engagé sans purification dans l'étape suivante. RMN ($^1$H, 400 MHz, chloroforme-d) δ 9.00 (d, J = 2.9 Hz, 1H), 8.79 (s, 1H), 8.07 (d, J = 1.6 Hz, 1H), 6.68 (dd, J = 2.9, 1.6 Hz, 1H). SM (IC+) *m/z* 239 (M-$N_2$+$H^+$), 267 ($MH^+$).

***2-azido-5-bromo-3-(1H-1,2,4-triazol-1-yl)pyrazine (97)***

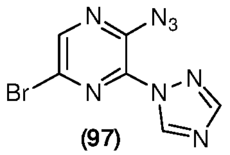

(97)

$C_6H_3BrN_8$
MW: 267,05 g.mol$^{-1}$

Ce composé a été préparé selon la <u>*Procédure générale A1*</u> avec le (1H)-1,2,4-triazole comme nucléophile et le composé **(77)** comme dérivé halogéné. Le temps de réaction est de 16h. Après retour à température ambiante, le milieu réactionnel est concentré à sec et est engagé sans purification dans l'étape suivante. RMN ($^1$H, 400 MHz, chloroforme-d) δ 9.76 (s, 1H), 8.97 (s, 1H), 8.47 (s, 1H). SM (IC+) *m/z* 240 (M-$N_2$+$H^+$), 268 ($MH^+$).

***2-azido-3-(4-bromo-3,5-diméthyl-pyrazol-1-yl)pyrazine (98)***

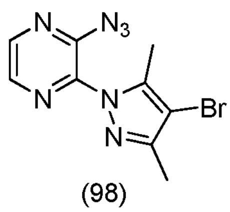

(98)

Synthétisé selon la méthode A1 à partir de 0,200 g de 2-azido-3-chloro-pyrazine (1,29 mmol, 1 eq.), 0,248 g de 4-bromo-3,5-diméthyl-1H-pyrazole (1,41 mmol, 1,1 eq.) et 0,355 g de $K_2CO_3$ (2,57 mmol, 2 eq.). Le résidu est ensuite dissous dans un minimum de dichlorométhane et le produit désiré est précipité par ajout d'éther de pétrole. Le précipité est ensuite filtré sur Büchner et lavé à l'éther de pétrole pour donner 0,286 g (0,97 mmol, 76%) du produit attendu sous forme d'une poudre jaune pâle.

$^1$H NMR (250 MHz, Chloroform-d) δ 8.67 (d, *J* = 4.5 Hz, 1H), 8.09 (d, *J* = 4.5 Hz, 1H), 2.70 (s, 3H), 2.44 (s, 3H). $^{13}$C NMR (63 MHz, $CDCl_3$) δ 152.67, 143.82, 141.36, 140.09, 130.94, 116.50, 102.75, 13.88, 13.16. HRMS (ESI) : $[M+H]^+$ calculé pour $C_9H_9BrN_7$ 294.009732, mesuré 294.009714 (0.1 ppm), $[M-N_2+H]^+$ $C_9H_9BrN_5$ calculated 266.003584, mesuré 266.003493 (-0.6 ppm).

***3-chloro-6-propyl-2-pyrazol-1-yl-pyrrolo[3,4-b]pyrazine-5,7-dione (99)***

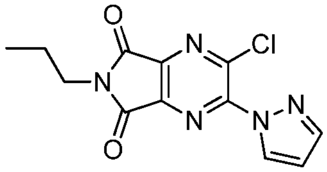

**(99)**

Synthétisé selon la méthode A1 à partir de 0,206 g de 2,3-dichloro-6-propyl-pyrrolo[3,4-b]pyrazine-5,7-dione (0,79 mmol, 1 eq.), 0,070 g de pyrazole (1,03 mmol, 1,3 eq.) et 0,142 g de $K_2CO_3$ (1,03 mmol, 1,3 eq.). Le résidu est ensuite purifié par chromatographie sur gel de silice pour donner 0,080 g du produit désiré (0,27 mmol, 35%).
$^1$H NMR (250 MHz, Chloroform-d) δ 8.49 (dd, *J* = 2.8, 0.7 Hz, 1H), 7.94 (dd, *J* = 1.7, 0.7 Hz, 1H), 6.62 (dd, *J* = 2.8, 1.7 Hz, 1H), 3.83 - 3.73 (m, 2H), 1.86 - 1.68 (m, 2H), 0.98 (t, *J* = 7.4 Hz, 3H).$^{13}$C NMR (101 MHz, $CDCl_3$) δ 162.93, 162.55, 148.43, 146.12, 144.57, 142.95, 142.73, 131.82, 109.93, 40.67, 21.98, 11.40.HRMS (ESI) : $[M+H]^+$ calculé pour $C_{12}H_{11}ClN_5O_2$ 292.059579, mesuré 292.059488 (0.3 ppm).

***3-chloro-2-pyrazol-1-yl-benzo[g]quinoxaline (100)***

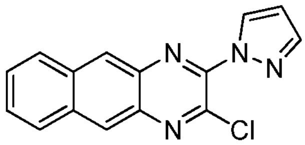

**(100)**

Préparé selon la méthode A1 à partir de 0,118 g de 2,3-dichlorobenzo[g]quinoxaline (0,47 mmol, 1 eq.), 0,042 g de pyrazole (0,62 mmol, 1,3 eq.) et 0,131 g de $K_2COs$ (0,95 mmol, 2 eq.). Le mélange est porté à reflux au micro-ondes 2 h et extrait avec de l'acétate d'éthyle. Le résidu est purifié par chromatographie sur silice PE/EA pour donner 0,069 g du produit désiré (0,25 mmol, 52%).
$^1$H NMR (400 MHz, Chloroform-d) δ 8.66 (s, 1H), 8.64 (s, 1H), 8.41 (d, *J* = 2.6 Hz, 1H), 8.12 (ddd, *J* = 9.3, 6.0, 2.9 Hz, 2H), 7.94 (d, *J* = 1.7 Hz, 1H), 7.64 (dd, *J* = 6.5, 3.2 Hz, 2H), 6.61 (dd, *J* = 2.7, 1.7 Hz, 1H).
$^{13}$C NMR (101 MHz, $CDCl_3$) δ 143.13, 142.58, 141.18, 137.37, 135.71, 134.51, 134.35, 131.27, 128.65, 128.54, 127.66, 127.40, 126.76, 108.38. HRMS (ESI) : $[M+H]^+$ calculé pour $C_{15}H_{10}ClN_4$ 281.058850, mesuré 281.058480 (1.3 ppm).

***2-(3-azidopyrazin-2-yl)indazole-7-carboxylate de sodium (101)***

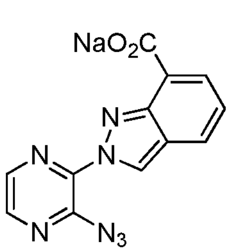

**(101)**

Synthétisé selon la méthode A2 à partir de 0,100 g d'acide indazole-7-carboxylique (0,62 mmol, 1 eq), 0,101 g d'azido-chloropyrazine (0,65 mmol, 1,05 eq.), 0,052 g de NaH (60% mass.) (1,30 mmol, 2,1 eq.). Précipitation directe par ajout d'acétate d'éthyle et filtration sur Büchner. Masse obtenue : 0,202 g, quantitatif.
$^1$H NMR (400 MHz, DMSO-d6) δ 13.05 (s, 1H), 9.92 (s, 1H), 9.56 (d, *J* = 4.4 Hz, 1H), 8.47 (d, *J* = 4.5 Hz, 1H), 8.22 (d, *J* = 8.5 Hz, 1H), 8.09 (dd, *J* = 6.9, 1.1 Hz, 1H), 7.31 (dd, *J* = 8.5, 6.9 Hz, 1H). HRMS (ESI) : $[M+H]^+$ calculé pour

$C_{12}H_8N_7O_2$ 282.073399, mesuré 282.073194 (0.7 ppm).

*1-(3-azidopyrazin-2-yl)indazole-5-sulfonate de sodium (102)*

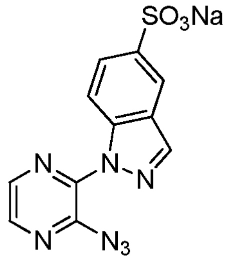

**(102)**

Synthétisé selon la méthode A2 à partir de 0,127 g d'acide indazole-sulfonique (0,64 mmol, 1 eq.), 0,100 g de pyrazine (0,64 mmol, 1 eq.), 0,054 g de NaH, 1,35 mmol, 2,1 eq.). Précipitation directe par ajout d'acétate d'éthyle et filtration sur Büchner. Quantitatif, utilisé tel quel pour réaction suivante.

$^1$H NMR (250 MHz, DMSO-d6) $\delta$ 9.27 (d, J= 4.6 Hz, 1H), 8.78 (d, J= 0.8 Hz, 1H), 8.67 (dt, J= 8.8, 0.8 Hz, 1H), 8.33 (d, J= 4.6 Hz, 1H), 8.21 (dd, J= 1.6, 0.8 Hz, 1H), 7.94 (dd, J= 8.8, 1.6 Hz, 1H). HRMS (ESI) : $[M]^-$ calculé pour $C_{11}H_6N_7O_3S$ 316.025832, mesuré 316.025876 (-0.5 ppm).

*2-(3-azidopyrazin-2-yl)-7-bromo-indazole (103)*

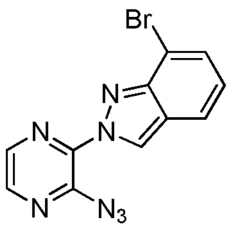

**(103)**

Synthétisé selon la méthode A2 à partir de ,0905 g (0,46 mmol, 1 eq.) de 7-bromoindazole, 0,020 g (0,51 mmol, 1,1 eq.) de NaH (60% mass.), 0,075 g (0,48 mmol, 1,05 eq.) d'azido-chloropyrazine. Produit précipité par ajout d'1 mL d'eau et d'acétate d'éthyle au mélange, filtré sur Büchner. Masse obtenue 0,133 g (0,42 mmol, 92%).

$^1$H NMR (400 MHz, DMSO-d6) $\delta$ 9.88 (s, 1H), 9.54 (d, *J* = 4.5 Hz, 1H), 8.46 (d, *J* = 4.6 Hz, 1H), 7.96 (dd, *J* = 8.6, 0.9 Hz, 1H), 7.73 (dd, *J* = 7.1, 0.8 Hz, 1H), 7.11 (dd, *J* = 8.5, 7.1 Hz, 1H). $^{13}$C NMR (101 MHz, DMSO) $\delta$ 148.91, 141.90, 139.92, 131.69, 131.62, 127.59, 124.43, 123.20, 121.78, 119.72, 111.03.

HRMS (ESI) : $[M+H]^+$ calculé pour $C_{11}H_7BrN_7$ 315.994082, mesuré 315.994348 (0.8 ppm).

*2-(3-azidopyrazin-2-yl)-N-prop-2-ynyl-indazole-7-carboxamide (104)*

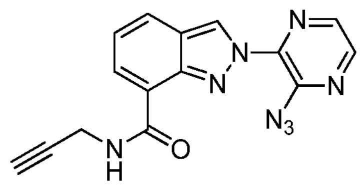

**(104)**

Synthétisé selon la méthode A2 à partir de 0,060 g (0,30 mmol, 1 eq.) de N-prop-2-ynyl-1H-indazole-7-carboxamide, 0,25 g (0,63 mmol, 2,1 eq.) de NaH (60% mass.), 0,049 g (0,0,32 mmol, 1,05 eq.) d'azido-chloropyrazine. Le produit est précipité par ajout d'eau et d'acétate d'éthyle et est ensuite filtré sur Büchner et lavé à l'acétate d'éthyle pour obtenir 0,033 g (0,104 mmol, 35%) d'indazolo-azido pyrazine.

$^1H$ NMR (250 MHz, DMSO-d6) δ 9.82 (s, 1H), 9.76 (t, *J* = 5.5 Hz, 1H), 9.55 (d, *J* = 4.6 Hz, 1H), 8.46 (d, *J* = 4.6 Hz, 1H), 8.23 - 8.14 (m, 2H), 7.39 (dd, *J* = 8.5, 6.9 Hz, 1H), 4.35 (dd, *J* = 5.6, 2.5 Hz, 2H), 3.20 (t, J = 2.55 Hz, 1H). HRMS (ESI): $[M+H]^+$ calculé pour $C_{15}H_{11}N_8O$ 319.105033, mesuré 319.105287 (0.8 ppm).

***2-(3-azidopyrazin-2-yl)-7-methoxy-indazole (105)***

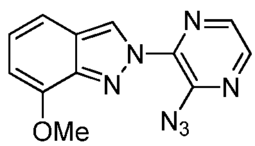

**(105)**

Synthétisé selon la méthode A2 à partir de 0,100 g (0,67 mmol, 1 eq.) de 7-methoxyindazole **(86),** 0,030 g (0,74 mmol, 1,1 eq.) de NaH (60% mass.), 0,110 g (0,71 mmol, 1,05 eq.) d'azido-chloropyrazine Le produit est ensuite précipité par ajout d'eau et d'acétate d'éthyle et est ensuite filtré sur Büchner et lavé à l'acétate d'éthyle pour obtenir 0,126 g (0,47 mmol, 70%) d'indazolo-azido pyrazine.

$^1H$ NMR (250 MHz, Chloroform-*d*) δ 9.74 (s, 1H), 8.76 (d, *J* = 4.5 Hz, 1H), 8.31 (d, *J* = 4.5 Hz, 1H), 7.34 - 7.27 (m, 1H), 7.08 (dd, *J* = 8.6, 7.2 Hz, 1H), 6.60 (d, *J* = 7.2 Hz, 1H), 4.04 (s, 3H). HRMS (ESI) : $[M+H]^+$ calculé pour $C_{12}H_{10}N_7O$ 268.094134, mesuré 268.093999 (-0.5 ppm).

***1-(3-azidopyrazin-2-yl)-4-méthoxy-indazole (106)***

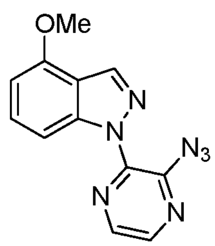

**(106)**

Synthétisé selon la méthode A2 à partir de 0,098 g (0,66 mmol, 1 eq.) de 4-methoxyindazole **(87),** 0,029 g (0,74 mmol, 1,1 eq.) de NaH (60% mass.), 0,103 g (0,66 mmol, 1 eq.) d'azido-chloropyrazine. Le produit est ensuite précipité par ajout d'eau et d'acétate d'éthyle et est ensuite filtré sur Büchner et lavé à l'acétate d'éthyle pour obtenir 0,143 g (0,54 mmol, 81%).

$^1H$ NMR (400 MHz, DMSO-d6) δ 9.26 (d, *J* = 4.5 Hz, 1H), 8.70 (s, 1H), 8.33 - 8.25 (m, 2H), 7.62 (t, *J* = 8.2 Hz, 1H), 6.97 (d, *J* = 7.8 Hz, 1H), 4.02 (s, 3H). HRMS (ESI) : $[M+H]^+$ calculé pour $C_{12}H_{10}N_7O$ 268.094134, mesuré 268.094006

(-0.5 ppm), $[M-N_2+H]^+$ $C_{12}H_{10}N_5O$ calculé pour 240.087638, mesuré 240.087986 (-0.6 ppm).

***2-(3-azidopyrazin-2-yl)-7-bromo-pyrazolo[3,4-c]pyridine (107)***

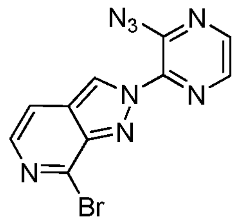

**(107)**

Synthétisé selon la méthode A2 à partir de 0,150 g (0,76 mmol, 1 eq.) de 7-bromo-1H-pyrazolo[3,4-c]pyridine **(88),** 0,033 g (0,83 mmol, 1,1 eq.) de NaH (60% mass), 0,124 g (0,80 mmol, 1,05 eq.) d'azido-chloropyrazine. Le produit est ensuite précipité par ajout d'eau et d'acétate d'éthyle et est ensuite filtré sur Büchner et lavé à l'éther de pétrole pour obtenir 0,185 g (0,58 mmol, 77%) d'indazolo-azido pyrazine.

$^1$H NMR (250 MHz, DMSO-d6) $\delta$ 9.94 (s, 1H), 9.64 (d, $J$ = 4.6 Hz, 1H), 8.51 (d, $J$ = 4.6 Hz, 1H), 8.00 (d, $J$ = 5.9 Hz, 1H), 7.91 (d, $J$ = 5.9 Hz, 1H). HRMS (ESI) : $[M+H]^+$ calculé pour $C_{10}H_6BrN_8$ 316.989331, mesuré 316.989092 (0.8 ppm).

***1-(3-azidopyrazin-2-yl)-1H-indazole-5-carboxylate de sodium (108)***

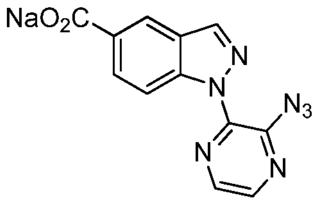

**(108)**

Synthétisé selon la méthode A2 à partir de 0,100 g d'acide 5-indazole carboxylique (0.617 mmol, 1éq), 0.051 g de NaH (60% mass) (1.296 mmol, 2.1 éq), 0,100 g d'azidopyrazine (0.648 mmol, 1.05 éq). Précipitation directe par ajout d'acétate d'éthyle et filtration sur Büchner. Rendement quantitatif.

$^1$H NMR (250 MHz, DMSO-d6) $\delta$ 9.61 (s, 1H), 9.48 (d, $J$ = 4.6 Hz, 1H), 9.30 - 9.25 (m, 9H), 9.18 (d, $J$ = 1.3 Hz, 9H), 8.69 (d, $J$ = 0.9 Hz, 9H), 8.41 (d, $J$ = 4.4 Hz, 1H), 8.39 - 8.35 (m, 9H). HRMS (ESI) : $[M]^-$ calculé pour $C_{12}H_6N_7O_2$ 280.058846, mesuré 280.059302 (1.6 ppm).

***1-(3-azidopyrazin-2-yl)-1H-indazole-4-carboxylate de sodium (109)***

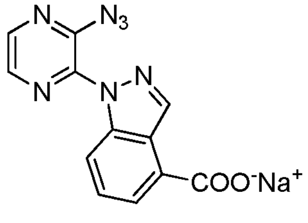

**(109)**

Synthétisé selon la méthode A2 à partir de 0,100 g d'acide 4-indazole carboxylique (0.617 mmol, 1éq), 0.051 g de NaH (60% mass) (1.296 mmol, 2.1 éq), 0,096 g d'azidopyrazine (0.648 mmol, 1.05 éq). Précipitation directe par ajout d'acétate d'éthyle et filtration sur Büchner. Masse obtenue 0,169 g (90%).

$^1$H NMR (250 MHz, DMSO-d6) δ 9.30 - 9.19 (m, 1H), 8.72 (d, *J* = 8.5 Hz, 1H), 8.30 (d, *J* = 4.6 Hz, 0H), 7.96 - 7.88 (m, 1H), 7.69 - 7.55 (m, 1H). HRMS (ESI) : $[C_{12}H_6N_7O_2]^-$calculé pour $C_{12}H_6N_7O_2$ 280.058846, mesuré 280.059302 (1.6 ppm).

***1-(3-azidopyrazin-2-yl)-1H-indazole-6-carboxylate de sodium (110)***

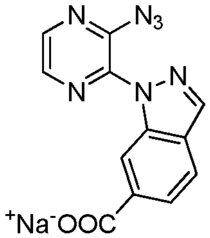

**(110)**

Synthétisé selon la méthode A2 à partir de 0,100 g d'acide 6-indazole carboxylique (0.617 mmol, 1éq), 0.051 g de NaH (60% mass) (1.296 mmol, 2.1 éq), 0,100 g d'azidopyrazine (0.648 mmol, 1.05 éq). Précipitation directe par ajout d'acétate d'éthyle et filtration sur Büchner. Rendement quantitatif.

$^1$H NMR (400 MHz, DMSO-d6) δ 9.62 (d, *J* = 1.1 Hz, 1H), 8.41 (d, *J* = 4.6 Hz, 1H), 7.96 (s, 3H), 7.77 - 7.75 (m, 2H). HRMS (ESI) : $[C_{12}H_6N_7O_2]^-$ calculé pour $C_{12}H_6N_7O_2$ 280.058846, mesuré 280.059264 (-1.5 ppm).

***1-(3-azidopyrazin-2-yl)-5-méthyl-1H-indazole (111)***

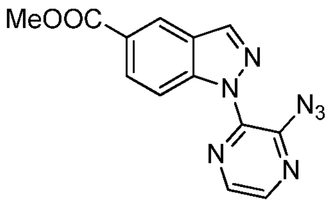

**(111)**

Synthétisé selon la méthode A2 à partir de 0,100 g d'ester méthylique de 5-indazole (0.568 mmol, 1éq), 0.025 g de NaH (60% mass) (0,625 mmol, 1.1 éq), 0,088 g d'azidopyrazine (0.568 mmol, 1 éq). Précipitation directe par ajout d'eau et d'acétate d'éthyle et filtration sur Büchner. Masse obtenue 0,128 g (76%).

$^1$H NMR (250 MHz, DMSO-d6) δ 9.34 (d, *J* = 4.6 Hz, 1H), 8.89 (d, *J* = 0.8 Hz, 1H), 8.79 (d, *J* = 8.8 Hz, 1H), 8.68 (dd, *J* = 1.5, 0.7 Hz, 2H), 8.36 (d, *J* = 4.6 Hz, 1H), 8.26 (dd, *J* = 8.8, 1.7 Hz, 1H).

HRMS (ESI): $[M+Na]^+$ calculé pour $C_{13}H_9N_7NaO_2$ 318.070993, mesuré 318.070841 (0.5 ppm).

**1-(3-azidopyrazin-2-yl)-6-methyl-1H-indazole (112)**

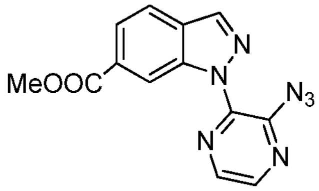

**(112)**

Synthétisé selon la méthode A2 à partir de 0,100 g d'ester méthylique de 6-indazole (0.568 mmol, 1éq), 0.025 g de NaH (60% mass) (0,625 mmol, 1.1 éq), 0,088 g d'azidopyrazine (0.568 mmol, 1 éq). Précipitation directe par ajout d'eau et d'acétate d'éthyle et filtration sur Büchner. Masse obtenue 0,090 g (53%).

$^1$H NMR (400 MHz, DMSO-d6) δ 9.34 (d, *J* = 4.6 Hz, 1H), 9.31 - 9.28 (m, 1H), 8.87 (d, *J* = 0.9 Hz, 1H), 8.41 (d, *J* = 4.6 Hz, 1H), 8.17 - 8.10 (m, 1H), 8.03 (dd, *J* = 8.3, 1.4 Hz, 1H), 3.96 (s, 3H). HRMS (ESI) : $[M+Na]^+$ calculé pour $C_{13}H_9N_7NaO_2$ 318.070993, mesuré 318.071239 (-0.3 ppm). $[M+H]^+$ calculé pour $C_{13}H_{10}N_7O_2$ 296.089049, mesuré 296.089172 (-0.4ppm).

*2-(3-azidopyrazin-2-yl)indazole-7-carboxylate de méthyle (113)*

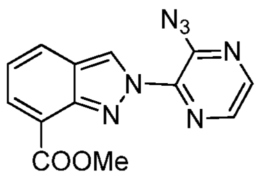

**(113)**

Synthétisé selon la méthode A2 à partir de 0,100 g d'ester méthylique de 7-indazole (0.568 mmol, 1éq), 0.025 g de NaH (60% mass) (0,625 mmol, 1.1 éq), 0,088 g d'azidopyrazine (0.568 mmol, 1 éq). Précipitation directe par ajout d'eau et d'acétate d'éthyle et filtration sur Büchner. Masse obtenue 0,051 g (30%).

$^1$H NMR (250 MHz, DMSO-d6) δ 9.90 (s, 1H), 9.54 (s, 1H), 8.47 (s, 1H), 8.33 - 8.05 (m, 3H), 7.32 (s, 1H), 3.96 (s, 2H). $^1$H NMR (400 MHz, DMSO-d6) δ 9.90 (s, 1H), 9.54 (d, *J* = 4.6 Hz, 1H), 8.47 (d, *J* = 4.5 Hz, 1H), 8.25 (dd, *J* = 8.5, 1.1 Hz, 1H), 8.10 (dd, *J* = 6.9, 1.1 Hz, 1H), 7.31 (d, *J* = 1.5 Hz, 1H), 3.95 (s, 3H). HRMS (ESI) : $[M-N_2+Na]^+$ calculé pour $C_{13}H_9N_5NaO_2$ 290.064845, mesuré 290.064815 (0.1 ppm).

*1-(3-azidopyrazin-2-yl)-1H-indazole-3-carboxylate de méthyle (114)*

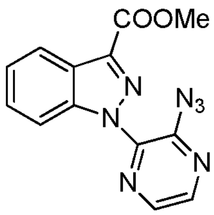

**(114)**

Synthétisé selon la méthode A2 à partir de 0,100 g d'ester méthylique de 3-indazole (0.568 mmol, 1éq), 0.025 g de NaH (60% mass) (0,625 mmol, 1.1 éq), 0,088 g d'azidopyrazine (0.568 mmol, 1 éq). Précipitation directe par ajout d'eau et d'acétate d'éthyle et filtration sur Büchner. Masse obtenue 0,125 g (74%).

$^1$H NMR (400 MHz, DMSO-d6) δ 9.43 (d, *J* = 4.7 Hz, 1H), 8.72 (d, *J* = 8.6 Hz, 1H), 8.40 (d, *J* = 4.6 Hz, 1H), 8.30 (d, *J* = 8.0 Hz, 1H), 7.81 - 7.71 (m, 1H), 7.62 (s, 0H), 4.06 (s, 3H). $^{13}$C NMR (101 MHz, DMSO) δ 161.70, 143.61, 140.31, 140.04, 139.70, 131.11, 129.51, 125.88, 124.19, 121.94, 118.26, 115.36, 52.49. HRMS (ESI) : $[M+H]^+$ calculé

pour $C_{13}H_{10}N_7O_2$ 296.089049, mesuré 296.088940 (0.4 ppm).

*1-(3-azidopyrazin-2-yl)-1H-indazole-4-carboxylate de méthyle (115)*

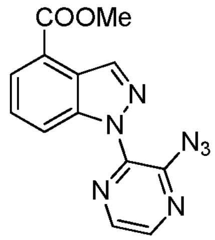

**(115)**

Synthétisé selon la méthode A2 à partir de 0,100 g d'ester méthylique de 3-indazole (0.568 mmol, 1éq), 0.025 g de NaH (60% mass) (0,625 mmol, 1.1 éq), 0,088 g d'azidopyrazine (0.568 mmol, 1 éq). Précipitation directe par ajout d'eau et d'acétate d'éthyle et filtration sur Büchner. Masse obtenue 0,121 g (72%).

$^1H$ NMR (400 MHz, DMSO-d6) $\delta$ 9.34 (s, 1H), 9.02 (s, 1H), 8.35 (s, 1H), 8.23 - 8.07 (m, 2H), 7.84 (s, 1H), 4.02 (s, 3H). $^{13}C$ NMR (101 MHz, DMSO) $\delta$ 165.40, 143.81, 139.75, 139.55, 139.53, 131.14, 128.78, 126.79, 124.38, 122.75, 120.03, 117.35, 67.00, 59.73, 52.52, 25.11, 20.75, 14.07. HRMS (ESI) : $[M+H]^+$ calculé pour $C_{13}H_{10}N_7O_2$ 296.089049, mesuré 296.089090 (-0.1 ppm).

## III - PREPARATION DES COMPOSES SELON L'INVENTION

### *III.1. Cyclisation via la thermolyse*

La procédure générale B, décrite ci-après, est mise en oeuvre pour préparer les composés selon l'invention.

Procédure générale B

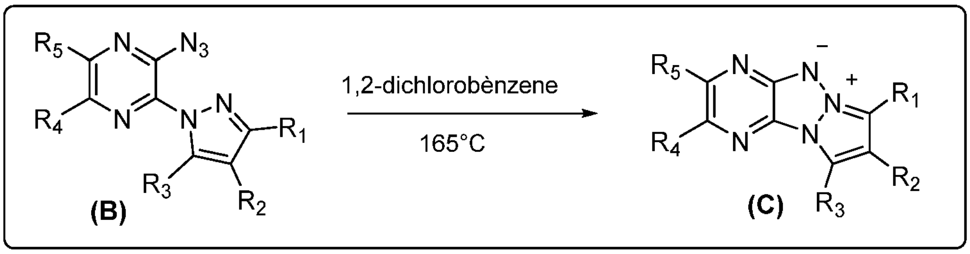


Une solution de l'intermédiaire **(B)** dans le 1,2-dichlorobenzène (concentration comprise entre 0.06 et 0.1M) placé sous atmosphère d'argon est chauffé à 165°C jusqu'à la consommation totale du réactif de départ. Après retour à température ambiante le solvant est éliminé soit par distillation sous vide soit par filtration sur gel de silice en utilisant un solvant apolaire (ex : éther de pétrole). Le produit brut est purifié par chromatographie sur colonne de gel de silice et est ensuite précipité dans un mélange chloroforme/pentane.

***Exemple* 1** *: **Pyrazolo[1 ',2':1,2][1,2,3]triazolo[4,5-b]pyrazin-6-ium-5-ide*** (36)

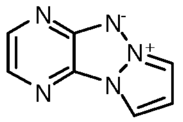

$C_7H_5N_5$
MW: 159,15 g.mol$^{-1}$

(36)

Ce composé a été préparé selon la *procédure générale* ***B*** à partir du 2-azido-3-(1H-pyrazol-1-yl)pyrazine **(21)** (100mg, 0.54mmol, 1éq) dans 1,2-dichlorobenzène (5mL). Le temps de réaction est de 1h30. Le solvant est éliminé par distillation sous vide. Le produit brut est purifié par chromatographie sur colonne de gel de silice (dichlorométhane/méthanol: 98/2 ; dépôt liquide) pour obtenir le produit désiré **(36** : **exemple 1)** avec un rendement de 79% (67mg) sous forme d'une poudre jaune. Rf (DCM/AcOEt/MeOH: 80/19/1): 0.35. RMN ($^1$H, 400MHz, chloroforme-d) $\delta$ 8.44 (d, *J* = 2.6 Hz, 1H), 8.08 (d, *J* = 3.3 Hz, 1H), 7.90 (d, *J* = 2.6 Hz, 1H), 7.84 (d, *J* = 2.5 Hz, 1H), 6.93 (t, *J* = 2.9 Hz, 1H). RMN ($^{13}$C, 101MHz, chloroforme-d) $\delta$ 151.64, 142.43, 129.02, 109.32, 108.80, 108.13. SM (IC+) *m/z* 159 (M+H$^+$).

***Exemple 2 : 8-nitropyrazolo[1',2':1,2][1,2,3]triazolo[4,5-b]pyrazln-6-ium-5-ide (37)***

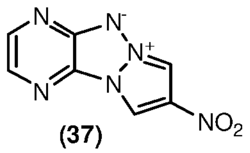

$C_7H_4N_6O_2$
MW: 204,15 g.mol$^{-1}$

**(37)**

Ce composé a été préparé selon la *procédure générale* ***B*** à partir du 2-azido-3-(4-nitro-1H-pyrazol-1-yl)pyrazine **(23)** (840mg, 3.63mmol, 1éq) dans 1,2-dichlorobenzène (37mL). Le temps de réaction est de 4h. Le solvant est éliminé par distillation sous vide. Le mélange brut est purifié par chromatographie sur colonne de gel de silice (3 paliers: dichlorométhane/acétate d'éthyle: 9/1, dichlorométhane/méthanol : 98/2 et 95/5). Afin d'isoler le produit pur plusieurs purifications successives ont été réalisées. Le produit désiré **(37 : exemple 2)** est obtenu avec un rendement de 69% (513mg) sous forme d'une poudre jaune. RMN (1H, 400MHz, chloroforme-d) $\delta$ 8.71 (d, J = 0.7 Hz, 1H), 8.58 (d, J = 2.6 Hz, 1H), 8.38 (d, J = 0.5 Hz, 1H), 8.08 (d, J = 2.6 Hz, 1H). RMN ($^{13}$C, 101MHz, chloroforme-d) 145.43, 133.09, 106.77, 103.91. SM ($IC_+$) m/z 205 ($MH_+$).

***Exemple 3 : 7-nitropyrazolo[1;2':1,2][1,2,3]triazolo[4,5-b]pyrazin-6-ium-5-ide (38)***

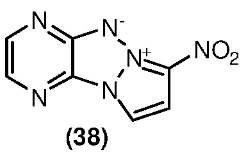

$C_7H_4N_6O_2$
MW: 204,15 g.mol$^{-1}$

**(38)**

Ce composé a été préparé selon la *procédure générale* ***B*** à partir du 2-azido-3-(3-nitro-1H-pyrazol-1-yl)pyrazine **(22)** (380mg, 1.64mmol, 1éq) dans 1,2-dichlorobenzène (15mL). Le temps de réaction est de 1h. Après retour à température ambiante, le solvant est éliminé par distillation sous vide. Le solide marron obtenu est lavé plusieurs fois au pentane pour éliminer les traces du solvant. Le produit désiré **(38 : exemple 3)** est obtenu avec un rendement de 93% (513mg) sous forme d'une poudre marron. RMN ($^1$H, 250 MHz, chloroforme-d) $\delta$ 8.86 (d, *J* = 2.5 Hz, 1H), 8.42 (d, *J* = 2.4 Hz, 1H), 8.06 (d, *J* = 3.9 Hz, 1H), 7.67 (d, *J* = 4.0 Hz, 1H). SM (IC+) *m/z* 205 (M+H$^+$).

***Exemple** 4 : 7-nitro-[1,2,4]triazolo[1;2':1,2][1,2,3]triazolo[4,5-b]pyrazin-6-ium-5-ide (39)*

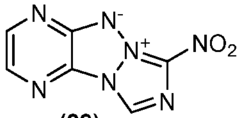

$C_6H_3N_7O_2$
MW: 205,14 g.mol$^{-1}$

**(39)**

Ce composé a été préparé selon la *procédure générale* ***B*** à partir du 2-azido-3-(3-nitro-1H-1,2,4-triazol-1-yl)pyrazine **(25)** (130mg, 0.56mmol, 1éq) dans le 1,2-dichlorobenzène (7.5mL). Le temps de réaction est de 3h. Après retour à température ambiante, le mélange brut est purifié par chromatographie sur colonne de gel de silice (4 paliers: éther de pétrole: 100% ; dichlorométhane/acétate d'éthyle : 100/0, 98/2 et 95/5; dépôt liquide). Le produit désiré **(39 : exemple 4)** est obtenu avec un rendement de 14% (16mg). RMN ($^1$H, 400 MHz, chloroforme-d) δ 8.98 (d, *J*= 2.4 Hz, 1H), 8.80 (s, 1H), 8.54 (d, *J*= 2.4 Hz, 1H). RMN ($^{13}$C, 101 MHz, chloroforme-d) δ 147.70, 138.71, 120.76. SM (IC+) *m/z* 206(MH$^+$)

***Exemple** 5 : 7-methoxypyrazolo[1',2':1,2][1,2,3]triazolo[4,5-b]pyrazin-6-ium-5-ide (40)*

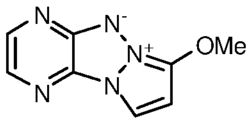

$C_8H_7N_5O$
MW: 189,18 g.mol$^{-1}$

**(40)**

Ce composé a été préparé selon la *procédure générale* ***B*** à partir du composé **(24)** (320mg, 1.47mmol, 1éq) dans le 1,2-dichlorobenzène (15mL). Le temps de réaction est de 2h. Après retour à température ambiante, le solvant est éliminé par distillation sous vide et le résidu brut est purifié par chromatographie sur colonne de gel de silice (dichlorométhane/méthanol: 97/3; dépôt solide). Le produit désiré **(40** : **exemple 5)** est obtenu avec un rendement de 51% (142mg) sous forme d'une poudre marron. RMN ($^1$H, 400 MHz, Chloroforme-d) δ 8.32 (d, *J* = 2.8 Hz, 1H), 7.96 (d, *J* = 3.5 Hz, 1H), 7.70 (d, *J* = 2.8 Hz, 1H), 6.48 (d, *J* = 3.5 Hz, 1H), 4.24 (s, 3H). RMN ($^{13}$C, 101 MHz, Chloroforme-d) δ 143.10, 142.65, 128.35, 110.29, 95.74, 60.15. SM ($IC_+$) *m/z* 190(MH$^+$).

***Exemple 6:** [1,2,3]triazolo[2',1.1,2][1,2,3]triazolo[4,5-b]pyrazin-9-ium-10-ide (41)*

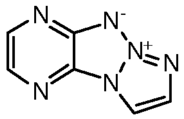

$C_6H_4N_6$
MW: 160,14 g.mol$^{-1}$

**(41)**

Ce composé a été préparé selon la *procédure générale* ***B*** à partir du composé **(26)** (30mg, 0.19mmol, 1éq) dans le 1,2-dichlorobenzène (1mL). Le temps de réaction est de 3h. Après retour à température ambiante, le solvant est éliminé par distillation sous vide et le résidu brut est purifié par chromatographie sur colonne de gel de silice (dichlorométhane / méthanol: 97/3; ou dichlorométhane /acétate d'éthyle: 7/3; dépôt solide). Le produit désiré **(41** : **exemple 6)** est obtenu avec un rendement de 80% (26mg) sous forme d'une poudre jaune pâle. RMN ($^1$H, 400 MHz, Chloroforme-d) δ 8.72 (d, *J* = 2.4 Hz, 1H), 8.35 (d, *J* = 2.4 Hz, 1H), 8.22 (d, *J* = 1.1 Hz, 1H), 8.02 (d, *J* = 1.2 Hz, 1H). RMN ($^{13}$C, 101

MHz, Chloroforme-d) δ 150.92, 144.68, 138.32, 135.51, 127.36, 104.83. SM (IC+) *m/z* 161($MH^+$)

***Exemple 7** : [1,2,4]triazolo[1',2':1,2][1,2,3]triazolo[4,5-b]pyrazin-6-ium-5-ide (42)*

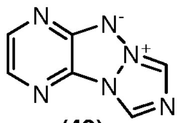

(42)

$C_6H_4N_6$
MW: 160,14 g.mol-1

Ce composé a été préparé selon la *procédure générale* ***B*** à partir du composé **(27)** (470mg, 2.50mmol, 1éq) dans le 1,2-dichlorobenzène (37mL). Le temps de réaction est de 3h. Après retour à température ambiante, le mélange brut est purifié par chromatographie sur colonne de gel de silice (2 paliers: éther de pétrole: 100% ; dichlorométhane/méthanol : 97/3). Le produit désiré **(42** : **exemple 7)** est obtenu avec un rendement de 60% (238mg) sous forme d'une poudre jaune. RMN ($^1H$, 400 MHz, Chloroforme-d) δ 8.80 (s, 1H), 8.58 (d, *J* = 2.4 Hz, 1H), 8.46 (s, 1H), 8.03 (d, *J* = 2.4 Hz, 1H). RMN ($^{13}C$, 101 MHz, Chloroforme-d) δ 153.79, 145.45, 131.68, 123.44, 119.38. SM($IC^+$) *m/z* 161($M+H^+$).

***Exemple 8*** : ***Benzo[4',5][1,2,3]triazolo[1;2':1,2][1,2,3]triazolo[4,5-b]pyrazin-6-ium-5-ide (43)***

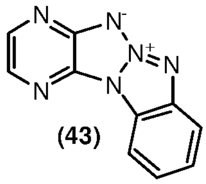

(43)

$C_{10}H_6N_6$
MW: 210,20 g.mol-1

Ce composé a été préparé selon la *procédure générale* ***B*** à partir du composé **(28)** (1.49mmol) dans le 1,2-dichlorobenzène (20mL). Le temps de réaction est de 1h45. Après retour à température ambiante, le mélange brut est purifié par chromatographie sur colonne de gel de silice (3 paliers: éther de pétrole: 100% ; dichlorométhane/acétate d'éthyle: 10/0, 9/1 ; dépôt liquide). Le produit **(43 : exemple 8)** est obtenu avec un rendement de 73% (229mg) sous forme d'une poudre jaune-verte. RMN ($^1H$, 400 MHz, Chloroforme-d) δ 8.74 (d, *J* = 2.6 Hz, 1H), 8.42 (d, *J* = 2.6 Hz, 1H), 8.30 (d, *J* = 8.2 Hz, 1H), 8.06 (d, *J* = 8.1 Hz, 1H), 7.67 (td, *J* = 7.4, 1.1 Hz, 2H). RMN ($^{13}C$, 101MHz, Chloroforme-d) δ 143.60, 143.10, 136.05, 130.77, 127.54, 125.25, 122.42, 117.98, 110.80. SM ($IC^+$) *m/z* 211($MH^+$).

***Exemple 9*** : ***Pyrazino[2',3':4,5][1,2,3]triazolo[1,2-a]indazol-6-ium-5-ide*** **(44)**

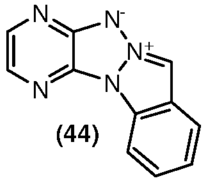

(44)

$C_{11}H_7N_5$
MW: 209,21 g.mol-1

Ce composé a été préparé selon la *procédure générale* ***B*** à partir du composé **(29)** (2.11mmol) dans le 1,2-dichlorobenzène (35mL). Le temps de réaction est de 1h. Après retour à température ambiante, le mélange brut est purifié par chromatographie sur colonne de gel de silice (3 paliers: éther de pétrole: 100% ; dichlorométhane/acétate

d'éthyle: 10/0, 9/1 et 8/2 ; dépôt solide). Le produit **(44 : exemple 9)** est obtenu avec un rendement quantitatif (450mg) sous forme d'une solide orange. RMN ($^1$H, 400 MHz, Chloroforme-d) δ 8.39 (d, *J* = 2.8 Hz, 1H), 8.31 (d, *J* = 8.3 Hz, 1H), 8.16 (s, 1H), 7.99 (d, *J* = 2.8 Hz, 1H), 7.86 (d, *J* = 8.2 Hz, 1H), 7.61 (t, *J* = 7.7 Hz, 1H), 7.54 (t, *J* = 7.6 Hz, 1H). RMN ($^{13}$C, 101 MHz, Chloroforme-d) δ 152.98, 141.28, 132.42, 130.84, 125.91, 125.72, 125.52, 123.79, 119.95, 111.26, 102.35. SM(IC$^+$) *m/z* 210 (MH$^+$).

***Exemple 10 :** **8,9,10,11-tetrahydropyrazino[2',3':4,5][1,2,3]triazolo[1,2-a]indazol-6-ium-5-ide (45)***

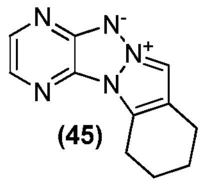

**(45)**

$C_{11}H_{11}N_5$
MW: 213,24 g.mol$^{-1}$

Ce composé a été préparé selon la *procédure générale* ***B*** à partir du composé **(91)** (1.9mmol) dans le 1,2-dichlorobenzène (25mL). Le temps de réaction est de 2h. Après retour à température ambiante, le mélange brut est purifié par chromatographie sur colonne de gel de silice (3 paliers: éther de pétrole: 100% ; dichlorométhane/acétate d'éthyle/ méthanol: 80/20/0, 80/18/2; dépôt solide). Le produit **(45 : exemple 10)** est obtenu avec un rendement de 79% (332mg) sous forme d'une poudre jaune. RMN ($^1$H, 400 MHz, Chloroforme-d) δ 8.33 (d, J = 2.8 Hz, 1H), 7.81 (s, 1H), 7.77 (d, J = 2.8 Hz, 1H), 3.00 (t, J = 6.5 Hz, 2H), 2.83 (t, J = 6.5 Hz, 2H), 2.06-1.97 (m, 2H), 1.96-1.84 (m, 2H). RMN (13C, 101 MHz, Chloroforme-d) δ 142.26, 128.57, 107.06, 22.71, 21.79, 21.43, 20.58. SM ($IC_+$) m/z 214 ($MH_+$).

***Exemple 11** : **pyrazolo[1',2':1,2][1,2,3]triazolo[4,5-b]quinoxalin-4-ium-5-ide(46)***

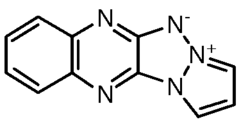

**(46)**

$C_{11}H_7N_5$
MW: 209,21g. mol$^{-1}$

Ce composé a été préparé selon la *procédure générale* ***B*** à partir du composé **(35)** (0.23mmol) dans le 1,2-dichlorobenzène (2mL). Le temps de réaction est de 29h. Le solvant est éliminé par distillation sous vide. Après retour à température ambiante, le résidu brut est purifié par chromatographie sur colonne de gel de silice (dichlorométhane/méthanol: 10/0, 98/2 et 95/5 ; dépôt solide). Le produit **(46 : exemple 11)** est obtenu avec un rendement de 53% (26mg) sous forme d'une poudre marron foncé. RMN ($^1$H, 400 MHz, Chloroforme-d) δ 8.24 (d, *J* = 3.4 Hz, 1H), 8.04 (d, *J* = 8.5 Hz, 1H), 8.01 (d, *J* = 8.4 Hz, 1H), 7.93 (d, *J* = 2.4 Hz, 1H), 7.7 (t, *J* = 7.6 Hz, 1H), 7.53 (t, *J* = 7.6 Hz, 1H), 6.95 (t, *J* = 2.4 Hz, 1H). RMN ($^{13}$C, 101 MHz, Chloroforme-d) δ 152.66, 143.53, 135.23, 132.08, 129.62, 128.41, 127.91, 125.43, 113.51, 112.18, 110.18. SM (IC+) *m/z* 210(MH$^+$).

***Exemple 12:** **8-(4,4,5,5-tetramethyl-1,3,2-dioxaborolan-2-yl)pyrazolo [1',2':1,2][1,2,3]triazolo[4,5-b]pyrazin-6-ium-5-ide (47)***

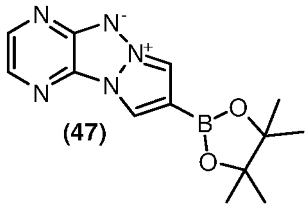

(47)

$C_{13}H_{16}BN_5O_2$
MW: 285,11g.mol$^{-1}$

Ce composé a été préparé selon la *procédure générale* ***B*** à partir du composé **(30)** (0.36mmol) dans le 1,2-dichlorobenzène (5mL). Le temps de réaction est de 45min. Le solvant est éliminé par distillation sous vide. Après retour à température ambiante, le milieu réactionnel est concentré. Le résidu brut obtenu est solubilisé dans un minimum de chloroforme et est dilué dans le pentane (précipitation d'impuretés). Le filtrat est concentré et est coévaporé au chloroforme. Le produit **(47** : **exemple 12)** est obtenu avec un rendement de 65% (67mg) sous forme d'une poudre jaune. RMN ($^1$H, 400MHz, Chloroforme-d) $\delta$ 8.38 (d, *J* = 2.6 Hz, 1H), 8.28 (s, 1H), 7.95 (s, 1H), 7.84 (d, *J* = 2.5 Hz, 1H), 1.34 (s, 12H). RMN ($^{13}$C, 400MHz, Chloroforme-d) $\delta$ 153.31, 143.53, 129.86, 129.12, 116.15, 113.31, 84.71, 24.90. SM(IC$^+$) *m/z* 286 (MH$^+$), 204(MH$^+$-$C_6H_{12}$).

***Exemple 13** : 7,9-diméthyl-8-( 4,4,5,5-tétraméthyl- 1,3,2-dioxaborolan-2-yl) pyrazolo[1',2':1,2][1,2,3] triazolo[4,5-b]pyrazin-6-ium-5-ide (48)*

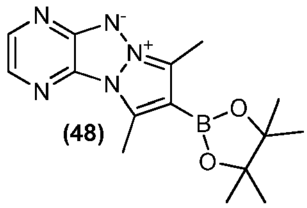

(48)

$C_{15}H_{20}BN_5O_2$
MW: 313,17 g.mol$^{-1}$

Ce composé a été préparé selon la *procédure générale* ***B*** à partir du composé **(31)** (0.59mmol) dans le 1,2-dichlorobenzène (10mL). Le temps de réaction est de 30min. Le solvant est éliminé par évaporation sous pression réduite. Le résidu brut obtenu est solubilisé dans un minimum de chloroforme et est dilué dans le pentane (précipitation d'impuretés). Le filtrat est concentré et est coévaporé au chloroforme. Le produit **(48** : **exemple 13)** est obtenu avec un rendement de 69% (127mg) sous forme d'une poudre jaune. RMN ($^1$H, 400MHz, chloroforme-d) $\delta$ 8.25 (d, J = 2.9 Hz, 1H), 7.69 (d, J = 2.9 Hz, 1H), 2.99 (s, 3H), 2.72 (s, 3H), 1.37 (s, 12H). RMN ($^{13}$C, 400MHz, Chloroforme-d) $\delta$ 154.26 ($C_q$), 142.43, 131.99 ($C_q$), 131.53 ($C_q$), 127.94, 125.26 ($C_q$), 83.96 ($C_q$), 25.08, 11.51, 10.68. SM (IC+) m/z 314 (MH$^+$).

***Exemple 14** : 8-bromopyrazolo[1',2':1,2][1,2,3]triazolo[4,5-b]pyrazin-6-ium-5-ide (49)*

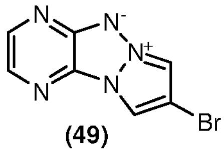

(49)

$C_7H_4BrN_5$
MW: 238,05 g.mol$^{-1}$

Ce composé a été préparé selon la *procédure générale* ***B*** à partir du composé **(32)** (5.6mmol) dans le 1,2-dichlorobenzène (90mL). Le temps de réaction est de 20min. Après retour à température ambiante, le mélange brut est purifié par chromatographie sur colonne de gel de silice (éther de pétrole: 100% ; dichlorométhane/acétate d'éthyle: 10/0 et 9/1; dépôt solide). Le produit **(49 : exemple 14)** est obtenu avec un rendement de 97% (1.34g) sous forme d'une

poudre jaune. RMN ($^1$H, 400MHz, Chloroforme-d) δ 8.50 (d, *J* = 2.8 Hz, 1H, H), 8.11 (s, 1H), 7.97 (d, *J*= 2.4 Hz, 1H, H), 7.87 (s, 1H). RMN ($^{13}$C, 101MHz, Chloroforme-d) δ 151.48 ($C_q$), 143.86, 131.27, 128.87($C_q$), 110.12, 109.50, 98.49. SM ($IC^+$) *m/z* 239 ($MH^+$).

***Exemple 15** : 8-iodopyrazolo[1',2':1,2][1,2,3]triazolo[4,5-b]pyrazin-6-ium-5-ide(50)*

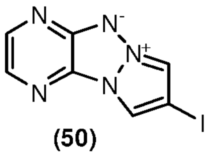

**(50)**

$C_7H_4IN_5$
MW: 285,05g.mol$^{-1}$

Ce composé a été préparé selon la *procédure générale **B*** à partir du composé **(33)** (7.67mmol) dans le 1,2-dichlorobenzène (120mL). Le temps de réaction est de 20min. Après retour à température ambiante, le mélange brut est purifié par chromatographie sur colonne de gel de silice (éther de pétrole: 100% ; dichlorométhane/acétate d'éthyle: 10/0, 8/2; dépôt solide). Le produit **(50 : exemple 15)** est obtenu avec un rendement de 93% (2.03g) sous forme d'une poudre jaune-orange. $R_f$ (DCM/AcOEt/MeOH : 80/19/1) : 0.32. RMN ($^1$H, 400MHz, Chloroforme-d) δ 8.50 (d, *J* = 2.6 Hz, 1H), 8.14 (s, 1H), 7.96 (d, *J* = 2.6 Hz, 1H), 7.87 (s, 1H). RMN ($^{13}$C, 101MHz, Chloroforme-d) δ 150.91, 142.91, 130.07, 127.73, 113.37, 112.02. SM ($IC^+$) *m/z* 286 ($MH^+$).

***Exemple 16** : 7-iodopyrazolo[1',2':1,2][1,2,3]triazolo[4,5-b]pyrazin-6-ium-5-ide (51)*

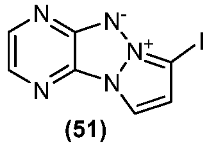

**(51)**

$C_7H_4IN_5$
MW: 285,05g.mol$^{-1}$

Ce composé a été préparé selon la *procédure générale **B*** à partir du composé **(90)** (4.1mmol) dans le 1,2-dichlorobenzène (30mL). Le temps de réaction est de 2h. Après retour à température ambiante, le mélange brut est purifié par chromatographie sur colonne de gel de silice (2 paliers: éther de pétrole: 100% ; dichlorométhane/acétate d'éthyle : 8/1; dépôt solide). Le produit **(51 : exemple 16)** est obtenu avec un rendement quantitatif (888mg) sous forme d'une poudre jaune-orange. RMN ($^1$H, 400 MHz, Chloroforme-d) δ 8.49 (d, J = 2.7 Hz, 1H), 8.08 (d, J = 3.4 Hz, 1H), 7.93 (d, J = 2.7 Hz, 1H), 7.02 (d, J = 3.4 Hz, 1H). RMN ($^{13}$C, 101 MHz, Chloroforme-d) δ 152.22, 143.82, 130.99, 130.67, 117.52, 111.95, 56.64. SM (IC+) m/z 286 ($MH^+$).

***Exemple 17** : **Composé (52.1) et composé (52.2)***

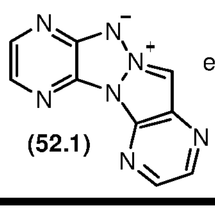

**(52.1)**

et

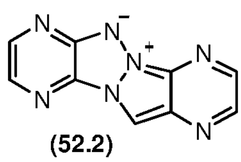

**(52.2)**

$C_9H_5N_7$
MW: 211,19 g.mol$^{-1}$

Ce composé a été préparé selon la *procédure générale **B*** à partir du mélange de deux isomères **(34.1 et 34.2)**

(0.84mmol) dans le 1,2-dichlorobenzène (12mL). Le temps de réaction est de 1h. Après retour à température ambiante, le mélange brut est purifié par chromatographie sur colonne de gel de silice (2 paliers: éther de pétrole: 100% ; acétate d'éthyle/cyclohexane: 4/6). Après deux purifications successives, les produits **(52.1)** et **(52.2)** sont isolés sous forme d'une poudre orange et une poudre rouge, avec des rendements de 20% (35mg) et de 4% (7mg). **(52.1):** RMN ($^1$H, 400MHz, Chloroforme-d) δ 8.87 (d, *J* = 2.4 Hz, 1H), 8.64 (d, *J* = 2.4 Hz, 1H), 8.60 (d, *J* = 2.8 Hz, 1H), 8.38 (s, 1H), 8.27 (d, *J* = 2.9 Hz, 1H). RMN ($^{13}$C 101 MHz, Chloroforme-d) 144.51, 142.81, 139.03, 134.12, 100.92. SM (IC$^+$) *m/z* 212 (MH$^+$). **(52.2):** RMN ($^1$H, 400MHz, Chloroforme-d) δ 8.93 (d, *J* = 1.9 Hz, 1H), 8.82 (d, *J* = 2.4 Hz, 1H), 8.77 (s, 1H), 8.67 (d, *J* = 1.9 Hz, 1H), 8.29 (d, *J* = 2.3 Hz, 1H). RMN ($^{13}$C, 101 MHz, Chloroforme-d) δ 147.07, 146.88, 141.21, 134.45, 100.14, 99.98. SM (IC$^+$) *m/z* 212 (MH$^+$).

***Exemple 18* : Benzo[d][1,2,3]triazolo[1,2-a][1,2,3]triazol-10-ium-9-ide (53)**

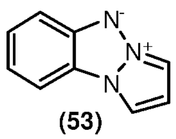

**(53)**

$C_9H_7N_3$
MW: 157,18g.mol$^{-1}$

Dans un tube scellé sec et placé sous argon, le 1-(2-nitrophényl)-1H-pyrazole **(1)** (760mg, 4.02mmol, 1éq) et la triéthyle phosphite (15mL, 87mmol, 22éq) sont introduits. Le milieu réactionnel est chauffé au micro-ondes à 176°C pendant 1h30. Après retour à température ambiante, le solvant est éliminé par distillation sous vide. Le mélange brut est purifié par chromatographie sur colonne de gel de silice (2 paliers : éther de pétrole/acétate d'éthyle : 8/2 et 5/5; dépôt solide). Après deux purifications successives le composé **(53 : exemple 18)** est obtenu avec un rendement de 24% (151mg) sous forme d'une poudre jaune claire. RMN ($^1$H, 400 MHz, Chloroforme-d) δ 7.67 - 7.62 (m, 1H), 7.60 (d, *J* = 2.6 Hz, 0H), 7.49 (d, *J* = 8.4 Hz, 1H), 7.36 (ddd, *J* = 8.3, 7.1, 1.2 Hz, 1H), 6.96 (ddd, *J* = 8.1, 7.0, 1.0 Hz, 1H), 6.79 (t, *J* = 2.9 Hz, 1H). RMN ($^{13}$C, 400 MHz, Chloroforme-d) δ 126.26, 116.17, 112.93, 109.74, 108.91, 104.57, 103.77. SM (IC+) *m/z* 190 (M+H$^+$), 212 (M+Na$^+$).

***Exemple 19* : Pyrazolo[1',2':1,2][1,2,3]triazolo[4,5-d]pyrimidin-9-ium-10-ide (54)**

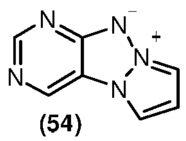

**(54)**

$C_7H_5N_5$
MW: 159,15g.mol$^{-1}$

Ce composé a été préparé selon la *procédure générale **B*** à partir du composé **(20)** (0.096mmol) dans le 1,2-dichlorobenzène (0.75mL). Le temps de réaction est de 20min. Après retour à température ambiante, le mélange brut est purifié par chromatographie sur colonne de gel de silice (3 paliers: éther de pétrole: 100% ; dichlorométhane/méthanol: 10/0 et 95/5; dépôt solide). Le produit **(54 : exemple 19)** est obtenu avec un rendement de 66% (15mg) sous forme d'une poudre beige. RMN ($^1$H, 400 MHz, Chloroforme-d) δ 8.91 (s, 1H), 8.88 (s, 1H), 7.96 (d, *J* = 3.2 Hz, 1H), 7.89 (d, *J* = 2.4 Hz, 1H), 6.93 (se, 1H). RMN ($^{13}$C, 101 MHz, Chloroforme-d) δ 160.88, 156.43, 136.24, 113.86, 111.40, 109.73, 109.40. SM (IC$^+$) *m/z* 160 (MH$^+$).

***Exemple 38* : 8-méthoxypyrazolo[1',2':1,2][1,2,3]triazolo[4,5-b]pyrazln-6-ium-5-ide (116)**

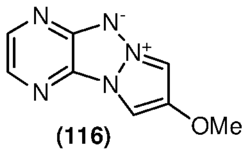

(116)

$C_8H_7N_5O$
MW: 189,18 g.mol$^{-1}$

Ce composé a été préparé selon la *procédure générale B* à partir du composé **(89)** (0.52mmol) dans le 1,2-dichlorobenzène (12mL). Le temps de réaction est de 2h. Après retour à température ambiante, le mélange brut est purifié par chromatographie sur colonne de gel de silice (2 paliers: éther de pétrole: 100% ; dichlorométhane/acétate d'éthyle : 8/2). Le produit **(116 : exemple 38)** est obtenu avec un rendement de 40% (39mg) sous forme d'une poudre jaune-marron. RMN ($^1$H, 400 MHz, Chloroforme-d) δ 8.40 (d, J = 2.7 Hz, 1H), 7.89 (d, J = 2.7 Hz, 1H), 7.70 (s, 1H), 7.66 (s, 1H). RMN ($^{13}$C, 101 MHz, Chloroforme-d) δ 142.33, 130.28, 99.58, 94.93, 58.98, les carbones quaternaires ne sont pas visibles. SM (IC+) m/z 190 (MH$^+$).

***Exemple 39: 8-nitropyrazino[2',3':4,5][1,2,3]triazolo[1,2-a]indazol-6-ium-5-ide (117)***

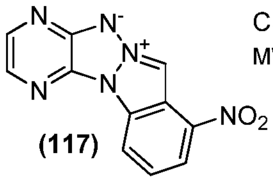

(117)

$C_{11}H_6N_6O_2$
MW: 254,21g.mol$^{-1}$

Ce composé a été préparé selon la *procédure générale B* à partir du composé **(92)** (1.2 mmol) dans le 1,2-dichlorobenzène (11mL). Le temps de réaction est de 2h30. Après retour à température ambiante, le mélange brut est purifié par chromatographie sur colonne de gel de silice (2 paliers: éther de pétrole: 100% ; dichlorométhane/ méthanol : 99/1). Le produit **(117 : exemple 39)** est obtenu avec un rendement de 88% sous forme d'une poudre orange. RMN ($^1$H, 400 MHz, DMSO-d6) δ 9.21 (s, 1H), 8.71 (d, J = 8.2 Hz, 1H), 8.59 - 8.55 (m, 1H), 8.27 (d, J = 2.8 Hz, 1H), 7.80 (t, J = 8.1 Hz, 1H). RMN ($^{13}$C, 101 MHz, DMSO-d6) δ 142.0, 132.7, 126.5, 123.59, 122.4, 117.8, 102.3. SM (IC$^+$) *m/z* 255 (MH$^+$).

***Exemple 40 : 9-nitropyrazino[2',3':4,5][1,2,3]triazolo[1,2-a]indazol-6-ium-5-ide (118)***

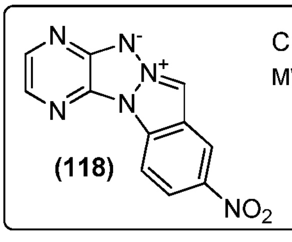

(118)

$C_{11}H_6N_6O_2$
MW: 254,21g.mol$^{-1}$

Ce composé a été préparé selon la *procédure générale B* à partir du composé **(93)** (0.64mmol) dans le 1,2-dichlorobenzène (6mL). Le temps de réaction est de 1h30. Après retour à température ambiante, le mélange brut est purifié par chromatographie sur colonne de gel de silice (4 paliers: éther de pétrole: 100% ; dichlorométhane/ méthanol : 99/1, 98/2, 97/3). Le produit **(118 : exemple 40)** est obtenu avec un rendement de 70% sous forme d'une poudre orange. RMN ($^1$H, 400 MHz, DMSO-d6) δ 8.83 (d, J = 1.3 Hz, 1H), 8.51 (d, J = 2.9 Hz, 1H), 8.45 - 8.43 (m, 1H), 8.31 (s, 1H), 8.12 (d, J = 2.8 Hz, 1H). RMN ($^{13}$C, 101 MHz, DMSO-d6) δ 142.45, 132.38, 126.94, 123.19, 119.58, 116.35, 111.74, 102.26, certains carbones quaternaires notamment ceux de la pyrazine, ne sont pas visibles. SM (IC$^+$) *m/z* 255 (MH$^+$).

***Exemple 41** : 10-nitropyrazino[2',3':4,5][1,2,3]triazolo[1,2-a]indazol-6-ium-5-ide (119)*

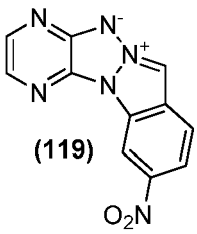

**(119)**

$C_{11}H_6N_6O_2$
MW: 254,21g.mol$^{-1}$

Ce composé a été préparé selon la *procédure générale **B*** à partir du composé **(94)** (0.64 mmol) dans le 1,2-dichlorobenzène (6mL). Le temps de réaction est de 1h. Après retour à température ambiante, le mélange brut est purifié par chromatographie sur colonne de gel de silice (4 paliers: éther de pétrole: 100% ; dichlorométhane/ méthanol : 99/1, 98/2, 97/3). Le produit **(119 : exemple 41)** est obtenu avec un rendement de 92% sous forme d'une poudre rouge. RMN ($^1$H, 400 MHz, DMSO-d6) 8.98 (s, 1H), 8.92 (s, 1H), 8.55 (s, 1H), 8.36 (d, J = 9.0 Hz, 1H), 8.26 (s, 1H), 8.15 (d, J = 9.0 Hz, 1H). SM (IC$^+$) *m*/*z* 255 (MH$^+$).

***Exemple 42** : 7-nitropyrazino[2',3':4,5][1,2,3]triazolo[2,1-a]indazol-6-ium-5-ide (120)*

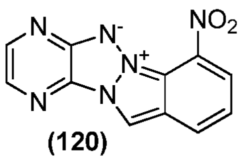

**(120)**

$C_{11}H_6N_6O_2$
MW: 254,21g.mol$^{-1}$

Ce composé a été préparé selon la *procédure générale B* à partir du composé **(95)** (2.2mmol) dans le 1,2-dichlorobenzène (21mL). Le temps de réaction est de 2h. Après retour à température ambiante, le mélange brut est purifié par chromatographie sur colonne de gel de silice (4 paliers: éther de pétrole: 100% ; dichlorométhane/ méthanol : 99/1, 98/2, 96/4). Le produit **(120 : exemple 42)** est obtenu avec un rendement de 67% sous forme d'une poudre rouge foncé. RMN ($^1$H, 400 MHz, DMSO-d6) $\delta$ 9.49 (s, 1H), 8.73 (d, J = 2.5 Hz, 1H), 8.42 (d, 1H), 8.30 (dd, J = 7.5, 0.8 Hz, 1H), 8.23 (d, J = 2.5 Hz, 1H), 7.62 (t, J = 8.0 Hz, 1H). RMN ($^{13}$C, 101 MHz, DMSO-d6) $\delta$ 150.7, 145.98, 136.1, 132.22, 127.0, 127.99, 124.92, 123.90, 122.91, 112.0, 104.31. SM (IC$^+$) *m*/*z* 255 (MH$^+$).

***Exemple 43** : 2-bromopyrazolo[1',2':1,2][1,2,3]triazolo[4,5-b] pyrazin-6-ium-5-ide (121)*

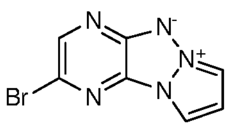

**(121)**

$C_7H_4BrN_5$
MW: 238,05g.mol$^{-1}$

Ce composé a été préparé selon la *procédure générale B* à partir du composé **(96)** (1.9mmol) dans le 1,2-dichlorobenzène (8,5mL). Le temps de réaction est de 40min. Après retour à température ambiante, le mélange brut est purifié par chromatographie sur colonne de gel de silice (3 paliers: éther de pétrole: 100% ; dichlorométhane/ acétate d'éthyle: 95/5, 9/1). Le produit **(121 : exemple 43)** est obtenu avec un rendement de 46% sous forme des paillètes dorées. RMN ($^1$H, 400MHz, chloroforme-d) $\delta$ 8.51 (s, 1H), 8.07 (dd, J = 3.3, 0.5 Hz, 1H), 7.85 (dd, J = 2.7, 0.5 Hz, 1H),

6.95 (dd, J = 3.4, 2.6 Hz, 1H). RMN ($^{13}C$, 101MHz, chloroforme-d) $\delta$ 150.89 (Cq), 144.90, 127.97 (Cq), 122.41 (Cq), 110.52, 110.14, 109.37. SM (IC+) m/z 239 ($MH^+$).

***Exemple 44 : 2-bromo-[1,2,4]trlazolo[1',2':1,2][1,2,3]triazolo[4,5-b]pyrazln-6-ium-5-ide (122)***

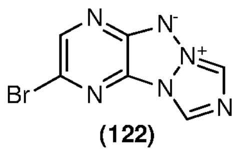

**(122)**

$C_6H_3BrN_6$
MW: 239,04g.mol$^{-1}$

Ce composé a été préparé selon la *procédure générale B* à partir du composé **(97)** (0.6mmol) dans le 1,2-dichlorobenzène (3mL). Le temps de réaction est de 90min. Après retour à température ambiante, le mélange brut est purifié par chromatographie sur colonne de gel de silice (4 paliers: éther de pétrole: 100% ; dichlorométhane/ méthanol: 99/1, 98/2, 97/3). le produit **(122 : exemple 44)** est obtenu avec un rendement de 15% sous forme d'un solide doré. RMN ($^1H$, 400MHz, chloroforme-d) $\delta$ 8.78 (s, 1H), 8.66 (s, 1H), 8.47 (s, 1H). RMN ($^{13}C$, 101MHz, chloroforme-d) $\delta$ . SM (IC+) m/z 240 ($MH^+$).

***Exemple 45 : Synthèse du composé (123)***

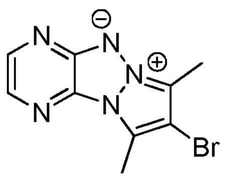

**(123)**

Synthétisé selon la méthode de thermolyse à partir de 0,150 g de 2-azido-3-(4-bromo-3,5-dimethyl-pyrazol-1-yl)pyrazine **(98)** (0,51 mmol) à 160°C pendant une heure. Masse obtenue : 0,122 g (0,46 mmol, 90%), poudre jaune.

$^1H$ NMR (400 MHz, Chloroform-d) $\delta$ 8.33 (s, 0H), 7.78 (d, *J*= 2.4 Hz, 1H), 2.84 (d, *J* = 1.2 Hz, 2H), 2.62 (s, 3H). $^{13}C$ NMR (101 MHz, $CDCl_3$) $\delta$ 152.24, 142.63, 131.08, 129.13, 122.36, 118.84, 98.73, 10.28, 9.83. HRMS (ESI) : $[M+H]^+$ calculé pour $C_9H_9BrN_5$ 266.003584, mesuré 266.003483 (0.4 ppm).

***Exemple 46 : propyl-phtalimide triazapentalène (124)***

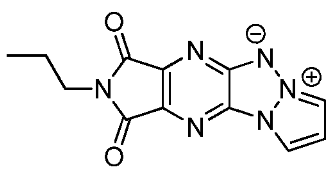

**(124)**

Dans 10 mL d'acétonitrile sec sont dissous 0,080 g de 3-chloro-6-propyl-2-pyrazol-1-yl-pyrrolo[3,4-b]pyrazine-5,7-dione (**99**) (0,27 mmol, 1 eq.) et 0,020 g d'azoture de sodium (0,30 mmol, 1,1 eq.). Le mélange est chauffé 1 h à 70°C et la solution devient orange. Le mélange est ensuite dissout dans de l'acétate d'éthyle et est lavé avec NaCl sat. La phase organique est séchée sur $MgSO_4$, filtrée et purifiée par chromatographie sur silice avec un mélange DCM/EA comme éluant fournissant 0,029 g du produit attendu (0,107 mmol, 40%) sous forme d'un solide jaune doré.

$^1$H NMR (400 MHz, DMSO-d6) δ 9.05 - 9.00 (m, 1H), 8.76 (d, *J* = 1.9 Hz, 1H), 7.33 (t, *J* = 2.5 Hz, 1H), 3.58 (t, *J* = 6.8 Hz, 2H), 1.63 (h, *J* = 6.7 Hz, 3H), 0.93 - 0.85 (m, 3H). $^{13}$C NMR (101 MHz, DMSO) δ 165.26, 165.18, 154.55, 145.95, 132.57, 129.00, 116.30, 114.94, 111.61, 21.56, 11.26. HRMS (ESI) : $[M+H]^+$ calculé pour $C_{12}H_{11}N_6O_2$ 271.093800, mesuré 271.093924 (0.5 ppm).

***Example 47** : 2-azido-3-pyrazol-1-yl-benzo[g]quinoxaline (125)*

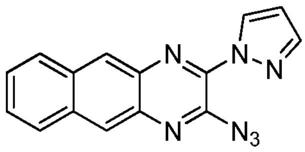

**(125)**

Dans 5 mL d'acétonitrile sont ajoutés 0,065 g (0,23 mmol, 1 eq.) de 3-chloro-2-pyrazol-1-yl-benzo[g]quinoxaline (**100**) et 0,075 g d'azoture de sodium (1,16 mmol, 5 eq.). Le mélange est chauffé à reflux une nuit. Après refroidissement, le mélange est extrait à l'acétate d'éthyle et lavé avec NaCl sat. Après séchage sur MgSOa, filtration et concentration sous vide, le résidu est purifié par chromatographie sur silice PE/EA pour obtenir 0,025 g (0,087 mmol, 38%) de l'azoture attendu.

$^1$H NMR (250 MHz, Chloroform-d) δ 9.31 (d, *J* = 2.8 Hz, 1H), 9.05 (s, 2H), 8.79 (s, 2H), 8.21 - 8.06 (m, 6H), 7.80 - 7.64 (m, 4H), 6.72 (ddd, *J* = 2.8, 1.6, 0.4 Hz, 2H). $^{13}$C NMR (101 MHz, $CDCl_3$) δ 145.43, 138.54, 138.44, 133.50, 132.79, 132.69, 131.47, 129.13, 128.87, 128.85, 128.19, 127.95, 122.41, 114.53, 110.24. HRMS (ESI) : $[M+H]^+$ calculé pour $C_{15}H_{10}N_7$ 288.099220, mesuré 288.099782 (1.9 ppm), $[M-N_2+H]^+$ calculé pour $C_{15}H_{10}N_5$ 260.093072, mesuré 260.093339 (1 ppm).

***Exemple 48** : Synthèse du composé (126)*

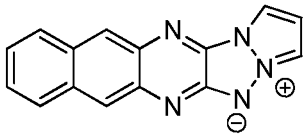

**(126)**

Synthétisé selon la méthode de thermolyse à partir de 0,025 g (0,087 mmol) de 2-azido-3-pyrazol-1-yl-benzo[g]quinoxaline (**125**) à 180°C pendant 24h. Masse obtenue : 0,017 g (0,066 mmol, 75%), poudre rouge.

$^1$H NMR (400 MHz, DMSO-d6) δ 8.96 (d, *J* = 3.5 Hz, 1H), 8.64 (d, *J* = 2.5 Hz, 1H), 8.61 (s, 1H), 8.38 (s, 1H), 8.14 (d, *J* = 8.4 Hz, 1H), 8.09 (d, *J* = 8.4 Hz, 1H), 7.54 (ddd, *J* = 8.2, 6.5, 1.2 Hz, 1H), 7.51 - 7.45 (m, 1H), 7.21 (dd, *J* = 3.6, 2.5 Hz, 1H). $^{13}$C NMR (101 MHz, DMSO) δ 153.15, 140.09, 135.10, 133.25, 133.13, 129.79, 127.89, 127.14, 126.17, 125.89, 124.37, 122.60, 117.85, 115.79, 110.99, 40.15, 39.94, 39.73, 39.52, 39.31, 39.10, 38.89. HRMS (ESI) : $[M+H]^+$ calculé pour $C_{15}H_{10}N_5$ 260.093072, mesuré 260.092575 (-1.9 ppm).

***Exemple 49** : Triazapentalène 5-sulfonate (127)*

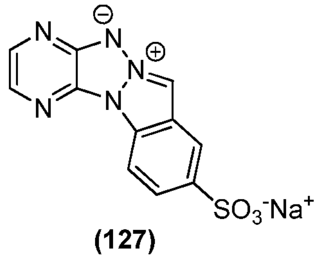

**(127)**

Synthétisé selon la méthode de thermolyse à partir du brut de réaction de (**102**) à 180°C pendant 62 h. Précipitation par ajout d'acétate d'éthyle. Masse obtenue : 0,140 g (0,45 mmol, 70% sur 2 étapes), poudre ocre. Echantillon analytique obtenu par purification sur silice C18 avec eau/MeOH comme éluant.

$^1$H NMR (400 MHz, Methanol-d4) δ 8.65 (s, 1H), 8.48 - 8.46 (m, 1H), 8.38 (d, *J* = 8.7 Hz, 1H), 8.31 (d, *J* = 3.0 Hz, 1H), 8.13 (dd, *J* = 8.6, 1.5 Hz, 1H), 8.04 (d, *J* = 3.0 Hz, 1H). $^{13}$C NMR (101 MHz, MeOD) δ 153.86, 144.13, 141.31, 131.89, 127.54, 125.07, 124.76, 119.53, 111.94, 105.72. HRMS (ESI) : $[M+H]^+$ calculé pour $C_{11}H_8N_5O_3S$ 290.034237, mesuré 290.034255 (-0.1 ppm).

***<u>Exemple 50</u> : Triazapentalène 7-carboxylate (128)***

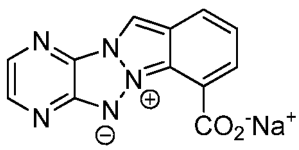

**(128)**

Synthétisé selon la méthode de thermolyse à partir du brut de réaction de **(101)** à 170°C pendant 24 h. Précipitation par ajout d'acétate d'éthyle. Masse obtenue : 0,120 g (0,44 mmol, 70% sur 2 étapes), poudre rouge.Echantillon analytique purifié par chromatographie DCM/EtOH + 1% $NH_3$. 9 mg obtenus.

$^1$H NMR (250 MHz, DMSO-d6) δ 9.21 (s, 1H), 8.62 (d, *J* = 2.8 Hz, 1H), 8.09 (d, *J* = 2.6 Hz, 1H), 8.02 - 7.91 (m, 1H), 7.58 m, 1H), 7.50 - 7.38 (m, 1H). HRMS (ESI) : $[M+H]^+$ calculé pour $C_{12}H_8N_5O_2$ 254.067251, mesuré 254.067243 (0 ppm).

***<u>Exemple 51</u> : 7-bromotriazapentalène (129)***

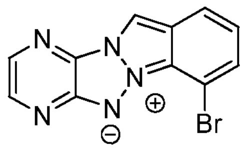

**(129)**

Synthétisé selon la méthode de thermolyse à partir de 0,095 g (0,30 mmol) de 2-(3-azidopyrazin-2-yl)-7-bromoindazole (**103**) à 140°C pendant 2 h.

Chromatographie sur colonne de silice avec un gradient PE/EA pour fournir 0,055 g du produit attendu (0,19 mmol, 64%).Poudre rouge.

$^1$H NMR (400 MHz, Chloroform-d) δ 8.68 (d, *J* = 2.5 Hz, 1H), 8.53 (s, 1H), 8.10 (d, *J* = 2.4 Hz, 1H), 7.81 (d, *J* = 8.6 Hz, 1H), 7.66 (dd, *J* = 7.3, 0.8 Hz, 1H), 7.31 - 7.26 (m, 1H). $^{13}$C NMR (101 MHz, $CDCl_3$) δ 145.84, 132.01, 129.13, 127.11, 126.11, 124.50, 118.96, 105.53, 100.61. HRMS (ESI) : $[M+H]^+$ calculé pour $C_{11}H_7BrN_5$ 287.987934, mesuré 287.988055 (0.4 ppm).

***Exemple 52 : Synthèse de 7-propargylamide triazapentalène (130)***

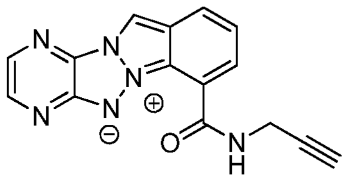

**(130)**

Synthétisé selon la méthode de thermolyse à partir de 0,029 g (0,09 mmol) de 2-(3-azidopyrazin-2-yl)-N-prop-2-ynyl-indazole-7-carboxamide (**104**) pendant 1 h à 150°C. Chromatographie sur colonne de silice avec un gradient PE/EA pour fournir 0,003 g du produit attendu (0,010 mmol, 11%).Poudre orange.

$^1H$ NMR (250 MHz, Chloroform-d) δ 11.42 (s, 1H), 8.72 - 8.64 (m, 3H), 8.14 (d, *J* = 2.5 Hz, 1H), 8.05 (d, *J* = 8.5 Hz, 1H), 7.60 (dd, *J* = 8.6, 7.3 Hz, 1H), 4.49 (dd, *J* = 4.9, 2.5 Hz, 2H), 2.34 (t, *J* = 2.5 Hz, 1H). HRMS (ESI) : $[M+H]^+$ calculé pour $C_{15}H_{11}N_6O$ 291.098885, mesuré 291.099003 (0.4 ppm).

***Exemple 53 : 7-méthoxy triazapentalène (131)***

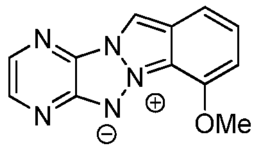

**(131)**

Synthétisé selon la méthode de thermolyse à partir de 0,068 g (0,25 mmol) de 2-(3-azidopyrazin-2-yl)-7-methoxy-indazole (105) pendant 2 h à 140°C.

Chromatographie sur colonne de silice avec un gradient PE/EA pour fournir 0,009 g du produit attendu (0,038 mmol, 15%).Poudre rouge.

$^1H$ NMR (400 MHz, Chloroform-d) δ 8.63 (d, *J* = 2.5 Hz, 1H), 8.44 (s, 1H), 8.04 (d, *J* = 2.5 Hz, 1H), 7.37 (d, *J* = 8.6 Hz, 1H), 7.30 (dd, *J* = 8.7, 7.4 Hz, 1H), 6.76 (d, *J* = 7.4 Hz, 1H), 4.17 (s, 3H). $^{13}C$ NMR (101 MHz, $CDCl_3$) δ 151.74, 147.85, 145.19, 131.27, 127.43, 126.23, 124.80, 115.42, 111.63, 103.57, 100.68, 56.44. HRMS (ESI) : $[M+H]^+$ calculé pour $C_{12}H_{10}N_5O$ 240.087986, mesuré 240.087862 (0.5 ppm).

***Exemple 54 : 4-méthoxytriazapentalène (132)***

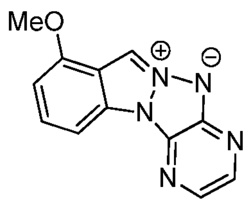

**(132)**

Synthétisé selon la méthode de thermolyse à partir de 0,054 g (0,20 mmol) de 1-(3-azidopyrazin-2-yl)-4-methoxy-indazole (**106**) pendant 3h30 à 145°C. Chromatographie sur colonne de silice avec un gradient PE/EA pour fournir 0,048 g du produit attendu (quantitatif). Poudre jaune.

$^1H$ NMR (250 MHz, Chloroform-d) δ 8.37 (d, *J* = 2.8 Hz, 1H), 8.19 (s, 1H), 7.95 (d, *J* = 2.9 Hz, 1H), 7.86 (d, *J*

= 8.3 Hz, 1H), 7.52 (t, *J* = 8.2 Hz, 1H), 6.87 (d, *J* = 8.0 Hz, 1H), 4.05 (s, 3H). $^{13}C$ NMR (101 MHz, $CDCl_3$) δ 153.20, 152.53, 141.36, 130.51, 127.20, 126.90, 115.71, 104.37, 103.81, 101.19, 55.95. HRMS (ESI) : $[M+H]^+$ calculé pour $C_{12}H_{10}N_5O$ 240.087638, mesuré 240.087986 (-1.5 ppm).

***Exemple 55: 9-(méthoxycarbonyl)pyrazino[2',3':4,5][1,2,3]triazolo[1,2-a]indazol-6-ium-5-ide (133)***

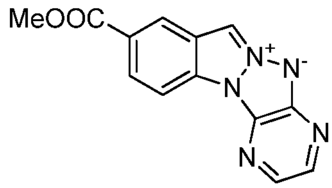

**(133)**

Synthétisé selon la méthode de thermolyse à partir de 0,029 g (0,098 mmol) de 1-(3-azidopyrazin-2-yl)-5-methyl-1H-indazole (**111**) pendant 2 h à 130°C.

Chromatographie sur colonne de silice avec un gradient PE/EA pour fournir 0,013 g du produit attendu (49%).Poudre jaune.

$^1H$ NMR (250 MHz, Chloroform-d) δ 8.64 (d, *J* = 1.2 Hz, 1H), 8.45 (s, 1H), 8.35 (s, 1H), 8.25 (d, *J* = 0.8 Hz, 1H), 8.06 (s, 1H), 4.01 (s, 3H). $^{13}C$ NMR (101 MHz, $CDCl_3$) δ 166.47, 152.97, 141.93, 132.19, 131.54, 127.68, 127.37, 126.20, 123.52, 122.61, 111.13, 102.57, 52.74, 0.14. HRMS (ESI) : $[M+H]^+$ calculé pour $C_{13}H_{10}N_5O_2$ 268.082901, mesuré 268.082845 (0.2 ppm).

***Exemple 56: 8-(méthoxycarbonyl)pyrazino[2',3':4,5][1,2,3]triazolo[1,2-a]indazol-6-ium-5-ide (134)***

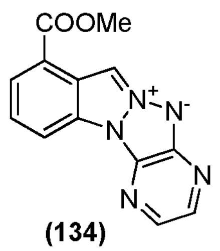

**(134)**

Synthétisé selon la méthode de thermolyse à partir de 0,083 g (0,281 mmol) de methyl 1-(3-azidopyrazin-2-yl)-1H-indazole-4-carboxylate (**115**) pendant 2 h à 130°C. Chromatographie sur colonne de silice avec un gradient PE/EA pour fournir 0,056 g du produit attendu (74%).Poudre jaune.

$^1H$ NMR (250 MHz, Chloroform-d) δ 8.72 (d, *J* = 0.9 Hz, 1H), 8.52 (s, 1H), 8.49 (s, 1H), 8.46 (d, *J* = 2.9 Hz, 1H), 8.06 (d, *J* = 2.9 Hz, 1H), 7.64 (dd, *J* = 8.3, 7.6 Hz, 1H), 4.07 (s, 3H).

$^{13}C$ NMR (63 MHz, $CDCl_3$) δ 165.93, 152.77, 141.90, 131.76, 128.40, 126.18, 124.44, 123.12, 121.34, 115.58, 103.74, 52.71, 0.15.

HRMS (ESI) : $[M+H]^+$ calculé pour $C_{13}H_{10}N_5O_2$ 268.082901, mesuré 268.082705 (0.7 ppm).

***Exemple 57 : 10-(méthoxycarbonyl)pyrazlno[2',3':4,5][1,2,3]triazolo[1,2-a]indazol-6-ium-5-ide (135)***

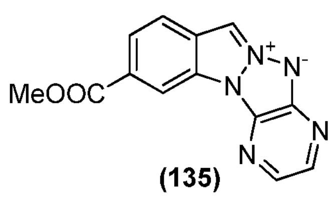

**(135)**

Synthétisé selon la méthode de thermolyse à partir de 0,071 g (0,24 mmol) de 1-(3-azidopyrazin-2-yl)-6-methyl-1H-indazole (**112**) pendant 2 h à 130°C. Chromatographie sur colonne de silice avec un gradient PE/EA pour fournir 0,047 g du produit attendu (72%).Poudre jaune.

$^1H$ NMR (250 MHz, Chloroform-d) δ 9.01 (s, 1H), 8.56 - 8.44 (m, 1H), 8.19 (s, 2H), 4.16 - 3.87 (m, 3H). $^{13}C$ NMR (101 MHz, $CDCl_3$) δ 166.51, 152.53, 142.02, 132.32, 126.73, 126.60, 126.28, 125.01, 119.40, 113.24, 101.94, 52.72, 31.08. HRMS (ESI): $[M+H]^+$ calculé pour $C_{13}H_{10}N_5O_2$ 268.082901 mesuré 268.083053 (-0.6ppm) IR: 3084.54, 2951.99, 1712.19, 1221.02, 1163.67, 1022.85, 960.77, 763.66, 749.73, 718.82 $cm^{-1}$

***<u>Exemple 58</u>: 7-(méthoxycarbonyl)pyrazino[2',3':4,5][1,2,3]triazolo[1,2-a]indazol-6-ium-5-ide (136)***

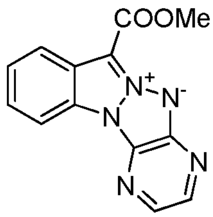

**(136)**

Synthétisé selon la méthode de thermolyse à partir de 0,100 g (0,34 mmol) de methyl 1-(3-azidopyrazin-2-yl)-1H-indazole-3-carboxylate (**114**) pendant 2 h à 130°C. Chromatographie sur colonne de silice avec un gradient PE/EA pour fournir 0,074 g du produit attendu (81%).Poudre jaune.

$^1H$ NMR (400 MHz, Chloroform-d) δ 8.67 (d, *J* = 2.7 Hz, 1H), 8.31 (d, *J* = 2.7 Hz, 1H), 7.75 - 7.55 (m, 1H), 4.18 (s, 3H). $^{13}C$ NMR (101 MHz, $CDCl_3$) δ 143.17, 135.37, 127.13, 125.95, 124.99, 124.07, 121.49, 111.10, 52.62.

HRMS (ESI): $[M+H]^+$ calculé pour $C_{13}H_{10}N_5O_2$ 268.082901, mesuré 268.082915 (-0.1 ppm) IR : 3072.28, 2952.75, 1690.06, 1462.51, 1296.16, 1241.92, 1019.41, 915.05, 866.28, 759.74, 740.15 $cm^{-1}$ Point de fusion : 248.7°C

***<u>Exemple 59</u> : Synthèse du composé (137)***

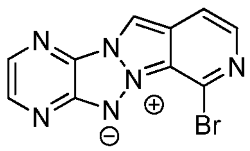

**(137)**

Synthétisé selon la méthode de thermolyse à partir de 0,101 g (0,32 mmol) de 2-(3-azidopyrazin-2-yl)-7-bromo-pyrazolo[3,4-c]pyridine (**107**) pendant 2 h à 140°C. Chromatographie sur colonne de silice avec un gradient PE/EA pour fournir 30 mg du produit attendu (poudre orange).

$^1H$ NMR (250 MHz, Chloroform-d) δ 8.85 (d, *J* = 2.3 Hz, 1H), 8.50 (s, 1H), 8.33 (d, *J* = 2.4 Hz, 1H), 8.21 (d, *J* = 6.1 Hz, 1H), 7.63 (d, *J* = 6.2 Hz, 1H). HRMS (ESI) : $[M+H]^+$ calculé pour $C_{10}H_6BrN_6$ 288.983183, mesuré 288.982549 (2.2 ppm).

***<u>Exemple 60</u> : Synthèse du composé (138)***

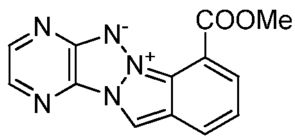

**(138)**

Synthétisé selon la méthode de thermolyse à partir de 0,026 g (0,088 mmol) de 1-(3-azidopyrazin-2-yl)-1H-indazole-7-carboxylate de méthyle (**113**) pendant 1 journée à 130°C. Chromatographie sur colonne de silice avec un gradient PE/EA pour fournir 0,005 g du produit attendu (21%). Poudre rouge.

$^1$H NMR (250 MHz, Chloroform-d) δ 8.66 (d, *J* = 2.5 Hz, 0H), 8.64 (s, 0H), 8.09 - 8.02 (m, 1H), 8.00 (dd, *J* = 7.2, 1.0 Hz, 0H), 7.49 (dd, *J* = 8.6, 7.1 Hz, 0H), 4.18 (s, 1H). $^{13}$C NMR (63 MHz, $CDCl_3$) δ 165.55, 151.53, 145.85, 131.71, 128.58, 124.67, 124.44, 124.03, 120.15, 119.15, 118.91, 101.19, 53.56, 53.08, 31.06. HRMS (ESI): $[M+H]^+$ calculé pour $C_{13}H_{10}N_5O_2$ 268.082901 mesuré 268.082803 (-0.4ppm)

***III.2. Couplage de Suzuki***

La procédure générale C, décrite ci-après, est mise en oeuvre pour préparer les composés selon l'invention.

Procédure générale C

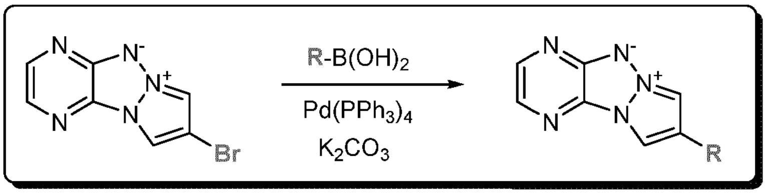


Dans un ballon *(ou tube scellé)* sec placé sous atmosphère d'argon, le 8-bromopyrazolo[1',2':1,2][1,2,3]triazolo[4,5-b]pyrazin-6-ium-5-ide **(49)** (1éq) est solubilisé dans le mélange 1,4-dioxane/$H_2O$ *(ou 1,4-dioxane/EtOH)* (2.5/1 : v/v ; 0.3M). Après l'introduction de l'acide boronique (1.5éq) et du carbonate de potassium (6éq), le milieu réactionnel est dégazé pendant 5min. Le $Pd(PPh_3)_4$ (5mol%) est introduit en dernier. La suspension obtenue est laissée sous agitation à reflux du solvant *(ou au micro-ondes à 150°C)* jusqu'à la consommation totale du dérivé bromé. Après retour à température ambiante le milieu réactionnel est concentré à sec et le résidu brut est purifié par chromatographie sur colonne de gel de silice et est ensuite précipité dans mélange chloroforme/pentane.

***Exemple 20: 8-phénylpyrazolo[1',2':1,2][1,2,3]triazolo[4,5-b]pyrazin-6-ium-5-ide (55)***

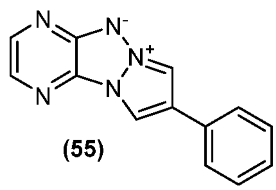

**(55)**

$C_{13}H_9N_5$
MW: 235,25g.mol$^{-1}$

Ce composé a été préparé selon la procédure générale C dans un ballon, en utilisant l'acide phénylboronique (28mg, 0.22mmol, 1.5éq) dans le 1,4-dioxane/$H_2O$ (3.5mL/1.5mL). Le mélange réactionnel est laissé sous agitation à reflux du solvant pendant 45min. Le résidu brut est purifié par chromatographie sur colonne de gel de silice (dichlorométhane/acétate d'éthyle : 8/2 ; dépôt solide). Après une précipitation dans le chloroforme/pentane, le produit désiré **(55 : exemple 20)** est obtenu avec un rendement de 88% (29mg) sous forme d'une poudre jaune. RMN ($^1$H, 250MHz,

chloroforme-d) δ 8.45 (d, *J* = 2.7 Hz, 1H), 8.28 (s, 1H), 8.09 (s, 1H), 7.93 (d, *J* = 2.7 Hz, 1H), 7.62 (dd, *J* = 8.2, 1.4 Hz, 2H), 7.55 - 7.36 (m, 3H). RMN ($^{13}C$, 400MHz, chloroforme-d) δ 143.41, 130.46, 130.13, 129.55, 128.91, 127.05, 126.59, 106.72, 106.21, 77.67, 77.16, 76.65. SM (IC+) *m/z* 236 ($MH^+$).

***Exemple 21**: 8-(4-methoxyphenyl)pyrazolo[1',2':1,2][1,2,3]triazolo[4,5-b]pyrazin-6-ium-5-ide (56)*

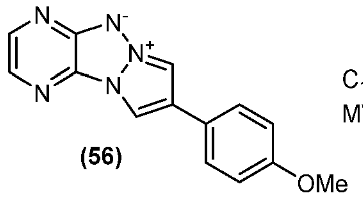

**(56)**

$C_{14}H_{11}N_5O$
MW: 265,28g.$mol^{-1}$

Ce composé a été préparé selon la *procédure générale C* dans un ballon, en utilisant l'acide 4-méthoxy phénylboronique (74mg, 0.47mmol, 1.5éq) dans le 1,4-dioxane/$H_2O$ (7.5mL/3mL). Le mélange réactionnel est laissé sous agitation à reflux du solvant pendant 5h. Le résidu brut est purifié par chromatographie sur colonne de gel de silice (dichlorométhane/ acétate d'éthyle : 8/2 et dichlorométhane/ méthanol 97/3 ; dépôt solide). Le produit **(56 : exemple 21)** est obtenu avec un rendement de 70% (97mg) sous forme d'une poudre jaune. RMN ($^{1}H$, 400MHz, chloroforme-d) δ 8.43 (d, *J* = 2.7 Hz, 1H), 8.19 (s, 1H), 8.03 (s, 1H), 7.91 (d, *J* = 2.7 Hz, 1H), 7.54 (d, *J* = 8.7 Hz, 2H), 7.02 (d, *J* = 8.7 Hz, 2H). RMN ($^{13}C$, 101MHz, chloroforme-d) δ 160.42, 143.34, 130.50, 127.84, 126.93, 122.76, 114.97, 106.58, 105.66, 55.60. SM (IC+) *m/z* 266 ($MH^+$).

***Exemple 22** : 8-(4-(diméthylamino)phényl)pyrazolo[1',2':1,2][1,2,3]triazolo [4,5-b]pyrazin-6-ium-5-ide (57)*

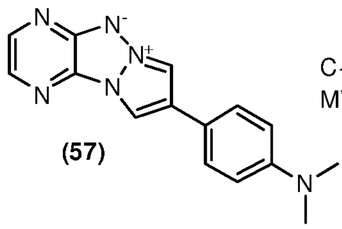

**(57)**

$C_{15}H_{14}N_6$
MW: 278,32g.$mol^{-1}$

Ce composé a été préparé selon la *procédure générale C* dans un ballon, en utilisant l'acide 4-(N,N-diméthylamino)phénylboronique (72mg, 0.47mmol, 1.5éq) dans le 1,4-dioxane/$H_2O$ (7.5mL/3mL). Le mélange réactionnel est laissé sous agitation à reflux du solvant pendant 18h. Le résidu brut est purifié par chromatographie sur colonne de gel de silice (dichlorométhane/acétate d'éthyle : 9/1 et 7/3 ; dépôt solide). Après une précipitation dans le mélange chloroforme/pentane et des lavages au pentane, le produit désiré **(57 : exemple 22)** est obtenu avec un rendement de 82% (73mg) sous forme d'une poudre jaune. RMN ($^{1}H$, 400 MHz, DMSO-d6) δ 8.97 (s, 1H), 8.85 (s, 1H), 8.33 (d, *J* = 2.8 Hz, 1H), 7.88 (d, *J* = 2.8 Hz, 1H), 7.69 (d, *J* = 8.8 Hz, 2H), 6.80 (d, *J* = 8.8 Hz, 2H), 2.95 (s, 6H). SM (IC+) *m/z* 279 ($MH^+$).

***Exemple 23** : 8-(pyrimidin-5-yl)pyrazolo[1',2':1,2][1,2,3]triazolo[4,5-b]pyrazin-6-ium-5-ide (58)*

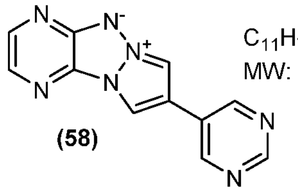

(58)

$C_{11}H_7N_7$
MW: 237,23g.mol$^{-1}$

Ce composé a été préparé selon la *procédure générale C* dans un tube scellé, en utilisant l'acide pyrimidine-5-boronique (93mg, 0.47mmol, 1.5éq) dans le 1,4-dioxane/$H_2O$ (7.5mL/3mL). Le mélange réactionnel est chauffé au micro-ondes à 150°C pendant 20min (changement de couleur de jaune vers marron). Le résidu brut est purifié par chromatographie sur colonne de gel de silice (dichlorométhane/méthanol : 97/3 et 95/5 ; dépôt solide). Après une précipitation dans le mélange chloroforme/pentane et des lavages au pentane, le produit désiré **(58 : exemple 23)** est obtenu avec un rendement de 74% (56mg) sous forme d'une poudre jaune. RMN ($^1H$, 250MHz, DMSO-d6) $\delta$ 9.35 (s, 1H), 9.31 (s, 1H), 9.15 (s, 1H), 9.08 (s, 1H), 8.40 (d, *J* = 2.7 Hz, 1H), 7.94 (d, *J* = 2.7 Hz, 1H). RMN ($^{13}C$, 101MHz, DMSO-d6) $\delta$ 158.09, 154.54, 152.44, 143.37, 130.34, 119.63, 109.28, 107.67. SM (IC+) *m/z* 238 ($MH^+$).

***Exemple 24:** 8-(4-(éthoxycarbonyl)phényl)pyrazolo[1',2':1,2][1,2,3] triazolo[4,5-b]pyrazin-6-ium-5-ide (59)*

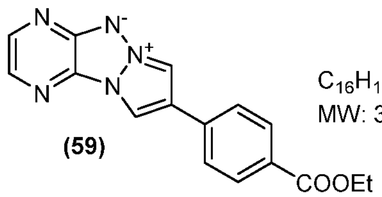

(59)

$C_{16}H_{13}N_5O_2$
MW: 307,31g.mol$^{-1}$

Ce composé a été préparé selon la *procédure générale C* dans un tube scellé, en utilisant l'acide 4-(éthoxycarbonyl)phénylboronique (93mg, 0.48mmol, 1.6éq) dans le 1,4-dioxane/EtOH (7.5mL/3mL). Le mélange réactionnel est chauffé au micro-ondes à 150°C pendant 20min (changement de couleur de jaune vers marron). Le résidu brut est purifié par chromatographie sur colonne de gel de silice (dichlorométhane/méthanol : 100/0 et 97/3; dépôt solide). Après une précipitation dans le mélange chloroforme/pentane et des lavages au pentane, le produit désiré **(59 : exemple 24)** est obtenu avec un rendement de 85% (84mg) sous forme d'une poudre jaune. RMN ($^1H$, 400MHz, chloroforme-d) $\delta$ 8.48 (d, *J* = 2.3 Hz, 1H), 8.35 (s, 1H), 8.15 (d, *J* = 3.3 Hz, 2H), 7.95 (d, *J* = 2.4 Hz, 1H), 7.69 (d, *J* = 8.2 Hz, 2H), 4.42 (q, *J* = 7.1 Hz, 2H), 1.43 (t, *J* = 7.1 Hz, 3H). RMN ($^{13}C$, 400MHz, chloroforme-d) $\delta$ 165.86, 152.07, 143.35, 134.32, 130.46, 126.00, 125.62, 106.39, 61.08, 14.30. SM (IC+) *m/z* 308 ($MH^+$).

***Exemple 25:** 8-(4-cyanophényl)pyrazolo[1',2':1,2][1,2,3]triazolo[4,5-b]pyrazin-6-ium-5-ide (60)*

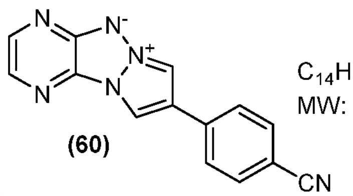

(60)

$C_{14}H_8N_6$
MW: 260,26g.mol$^{-1}$

Ce composé a été préparé selon la *procédure générale C* dans un tube scellé, en utilisant l'acide 4-cyanophénylboronique (48mg, 0.32mmol, 1.5éq) dans le 1,4-dioxane/ $H_2O$ (7.5mL/3mL). Le mélange réactionnel est chauffé au micro-ondes à 150°C pendant 30min (changement de couleur de jaune vers vert-marron). Après retour à température

ambiante le milieu réactionnel est filtré sur millipore et est rincé au 1,4-dioxane. Après des lavages au pentane, le produit désiré **(60 : exemple 25)** est obtenu avec un rendement quantitatif (58mg) sous forme d'une poudre verdâtre. RMN ($^1H$, 250MHz, DMSO-d6) δ 9.32 (s, 1H), 9.08 (s, 1H), 8.37 (d, *J* = 2.7 Hz, 1H), 8.07 (d, *J* = 8.7 Hz, 2H), 7.97 (d, *J* = 8.7 Hz, 2H), 7.96 (d, *J* = 2.7 Hz, 1H). SM (IC+) *m*/*z* 261 ($MH^+$).

***Exemple 26** : 8-(4-carboxyphényl)pyrazolo[1',2':1,2][1,2,3]triazolo[4,5-b]pyrazin-6-ium-5-ide (61)*

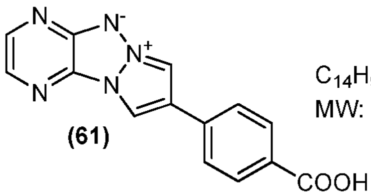

**(61)**

$C_{14}H_9N_5O_2$
MW: 279,26g.mol$^{-1}$

Ce composé a été préparé selon la *procédure générale C* dans un tube scellé, en utilisant l'acide 4-éthoxycarbonyl)phényl-boronique (93mg, 0.48mmol, 1.6éq) dans le 1,4-dioxane/$H_2O$ (7.5mL/3mL). Le mélange réactionnel est chauffé au micro-ondes à 150°C pendant 20min (changement de couleur de jaune vers marron). Le résidu brut est purifié par chromatographie sur colonne de gel de silice (dichlorométhane/méthanol : 100/0, 97/3, 90/10 et 70/30; dépôt solide). Après des lavages au pentane, le produit **(61 : exemple 26)** est obtenu avec un rendement de 85% (84mg) sous forme d'une poudre jaune. SM (IC+) *m*/*z* 280 ($MH^+$)

***Exemple 27:** 8-(thiophén-2-yl)pyrazolo[1',2':1,2][1,2,3]triazolo[4,5-b]pyrazin-6-ium-5-ide (62)*

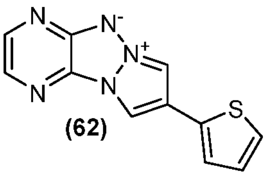

**(62)**

$C_{11}H_7N_5S$
MW: 241,27g.mol$^{-1}$

Ce composé a été préparé selon la *procédure générale C* dans un tube scellé, en utilisant l'acide 2-thienylboronique (62mg, 0.48mmol, 1.5éq) dans le 1,4-dioxane/$H_2O$ (7.5mL/3mL). Le mélange réactionnel est chauffé au micro-ondes à 150°C pendant 1h (changement de couleur de jaune vers marron). Le résidu brut est purifié par chromatographie sur colonne de gel de silice (dichlorométhane/acétate d'éthyle : 100/0 et 90/10; dépôt solide). Le produit désiré **(62 : exemple 27)** est obtenu avec un rendement de 86% (67mg) sous forme d'une poudre jaune. RMN ($^1H$, 400MHz, chloroforme-d) δ 8.45 (d, *J* = 2.0 Hz, 1H), 8.20 (s, 1H), 8.01 (s, 1H), 7.93 (d, *J* = 2.4 Hz, 1H), 7.37 (d, *J* = 5.1 Hz, 1H), 7.31 (d, *J* = 3.5 Hz, 1H), 7.13 (t, *J* = 3.65 Hz, 1H). RMN ($^{13}C$, 101MHz, chloroforme-d) δ 152.31, 143.48, 132.06, 130.72, 129.35, 128.31, 126.07, 125.56, 120.91, 106.49, 105.95. SM (IC+) *m*/*z* 242 ($MH^+$).

***Exemple 28** : 7-phénylpyrazolo[1',2':1,2][1,2,3]triazolo[4,5-b]pyrazin-6-ium-5-ide (63)*

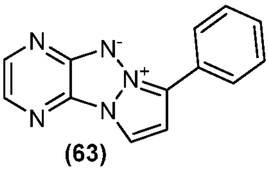

**(63)**

$C_{13}H_9N_5$
MW: 235,25 g.mol$^{-1}$

Ce composé a été préparé selon la *procédure générale C* dans un ballon sec, en utilisant le 7-iodopyrazolo[1',2':1,2][1,2,3]triazolo[4,5-b]pyrazin-6-ium-5-ide **(51)** (100mg, 0.35mmol, 1éq) et l'acide phénylboronique (65mg, 0.53mmol, 1.5éq) dans le 1,4-dioxane/$H_2O$ (5.6mL/2.2mL). Le mélange réactionnel est laissé sous agitation à reflux du solvant pendant 1h30. Après retour à température ambiante le milieu réactionnel est concentré à sec et le résidu brut est purifié par chromatographie sur colonne de gel de silice (dichlorométhane/méthanol 100/0, 95/5 ; dépôt solide). Après une précipitation dans le mélange chloroforme/pentane et des lavages au pentane, le produit désiré **(63 : exemple 28)** est obtenu avec un rendement de 28% (80mg) sous forme d'une poudre orange. RMN ($^1H$, 250MHz, chloroforme-d) $\delta$ 8.47 (d, $J$ = 2.9 Hz, 1H), 8.38 (d, $J$ = 7.3 Hz, 2H), 8.15 (d, $J$ = 3.4 Hz, 1H), 7.92 (d, $J$ = 2.7 Hz, 2H), 7.61 - 7.52 (m, 1H), 7.44 (d, $J$ = 7.2 Hz, 1H). RMN ($^{13}C$, 101MHz, chloroforme-d) $\delta$ 142.34, 129.42, 128.22, 128.00, 125.97, 125.14, 109.91, 106.54.

***Exemple 62**: 8-(5-acétylthiophén-2-yl)pyrazolo[1',2':1,2][1,2,3]triazolo [4,5-b]pyrazin-6-ium-5-ide (139)*

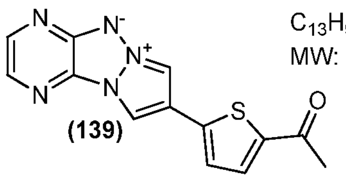

**(139)**

$C_{13}H_9N_5OS$
MW: 283,31g.mol$^{-1}$

Ce composé a été préparé selon la *procédure générale C* dans un ballon, en utilisant l'acide 5-acétyl-2-thienylboronique (55mg, 0.32mmol, 1.5éq) dans le 1,4-dioxane/$H_2O$ (4.25mL/1.75mL). Le mélange réactionnel est laissé sous agitation à reflux du solvant pendant 48h. Le résidu brut est purifié par chromatographie sur colonne de gel de silice (dichlorométhane/acétate d'éthyle : 100/0 et 90/10; dépôt solide). Après une précipitation dans le mélange chloroforme/pentane et des lavages au pentane, le produit désiré **(139 : exemple 62)** est obtenu avec un rendement de 8% sous forme d'une poudre jaune. RMN ($^1H$, 400MHz, chloroforme-d) $\delta$ 8.49 (d, J = 2.6 Hz, 1H), 8.29 (s, 1H), 8.07 (s, 1H), 7.98 (d, J = 2.6 Hz, 1H), 7.69 (d, J = 4.2 Hz, 2H), 7.33 (d, J = 3.9 Hz, 1H), 2.60 (s, 3H). RMN ($^{13}C$, 101MHz, chloroforme-d, 3K scans) $\delta$ aucun carbone n'est visible. SM (IC+) m/z 284 (MH+).

***Exemple 63** : 8-(2,6-dlméthoxyphényl)pyrazolo[1',2':1,2][1,2,3]triazolo[4,5-b]pyrazin-6-ium-5-ide (140)*

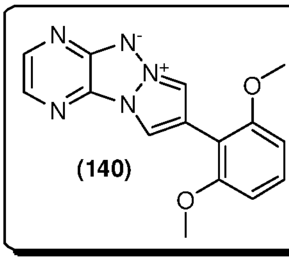

**(140)**

$C_{15}H_{13}N_5O_2$
MW: 295,30g.mol$^{-1}$

Ce composé a été préparé selon la *procédure générale C* dans un tube scellé, en utilisant l'acide 2,6-diméthoxyphenylboronique (49mg, 0.26mmol, 1.5éq) dans le 1,4-dioxane/$H_2O$ (4.25mL/1.75mL). Le mélange réactionnel est chauffé au micro-ondes à 150°C pendant 20min. Le résidu brut est purifié par chromatographie sur colonne de gel de silice (dichlorométhane/acétate d'éthyle : 90/10 et 70/30 ; dépôt solide) ; Le produit **(140 : exemple 63)** est obtenu avec un rendement de 80% (41mg) sous forme d'une poudre jaune. RMN ($^1H$, 400MHz, chloroforme-d) $\delta$ 8.61 (s, 1H), 8.44 (s, 1H), 8.37 (d, J = 2.8 Hz, 1H), 7.83 (d, J = 2.8 Hz, 1H), 7.32 (t, J = 8.4 Hz, 1H), 6.71 (d, J = 8.4 Hz, 2H), 3.94 (s, 6H). RMN ($^{13}C$, 101MHz, chloroforme-d) $\delta$ 157.76, 152.73, 142.63, 129.55, 128.91, 117.40, 111.39, 111.17, 107.52, 104.30, 55.90. SM (IC+) *m/z* 296 ($MH^+$).

***Exemple 64** : 8-(furan-2-yl)pyrazolo[1',2':1,2][1,2,3]triazolo[4,5-b]pyrazin-6-ium-5-ide (141)*

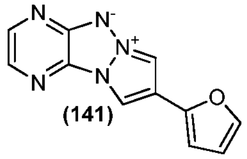

(141)

$C_{11}H_7N_5O$
MW: 225,21g.mol$^{-1}$

Ce composé a été préparé selon la *procédure générale C* dans un tube scellé, en utilisant l'acide 2-furanylboronique (30mg, 0.26mmol, 1.5éq) dans le 1,4-dioxane/$H_2O$ (4.75mL/1.75mL). Le mélange réactionnel est chauffé au micro-ondes à 150°C pendant 18min. Le résidu brut est purifié par chromatographie sur colonne de gel de silice (dichlorométhane/acétate d'éthyle : 100/0 et 90/10; dépôt solide). Le produit **(141 : exemple 64)** est obtenu avec un rendement de 83% sous forme d'une poudre jaune-verte. RMN ($^1$H, 400MHz, chloroforme-d) δ 8.45 (d, J = 2.7 Hz, 1H), 8.23 (s, 1H), 8.03 (s, 1H), 7.93 (d, J = 2.7 Hz, 1H), 7.52 (dd, J = 1.9, 0.8 Hz, 1H), 6.66 (dd, J = 3.4, 0.8 Hz, 1H), 6.53 (dd, J = 3.4, 1.8 Hz, 1H). RMN ($^{13}$C, 101MHz, chloroforme-d) δ 152.36 (Cq), 144.99 (Cq), 143.30, 142.82, 130.51, 129.24 (Cq), 117.98 (Cq), 111.74, 107.32, 105.32, 104.97. SM (IC+) m/z 226 (MH+).

***Exemple 65** : (E)-B-styrylpyrazolo[1',2':1,2][1,2,3]triazolo[4,5-b]pyrazin-6-ium-5-ide (142)*

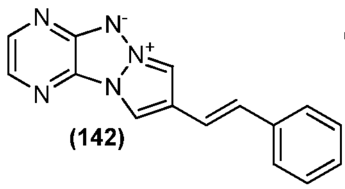

(142)

$C_{15}H_{11}N_5$
MW: 261,29g.mol$^{-1}$

Ce composé a été préparé selon la *procédure générale C* dans un tube scellé, en utilisant l'acide E-2-phenylvinylboronique (120mg, 0.79mmol, 1.5éq) dans le 1,4-dioxane/$H_2O$ (7.5mL/3mL). Le mélange réactionnel est chauffé au micro-ondes à 150°C pendant 30min. Le résidu brut est purifié par chromatographie sur colonne de gel de silice (dichlorométhane/acétate d'éthyle : 100/0, 95/5 et 90/10; dépôt solide). Le produit désiré **(142 : exemple 65)** est obtenu avec un rendement de 62% sous forme d'une poudre jaune. RMN ($^1$H, 400MHz, chloroforme-d) δ 8.43 (d, J = 2.7 Hz, 1H), 8.12 (s, 1H), 8.00 (s, 1H), 7.91 (d, J = 2.7 Hz, 1H), 7.52 (d, J = 7.3 Hz, 3H), 7.40 (t, J = 7.4 Hz, 3H), 7.32 (t, J = 7.3 Hz, 1H), 7.10 (d, J = 16.4 Hz, 1H), 7.02 (d, J = 16.4 Hz, 1H). RMN ($^{13}$C, 101MHz, chloroforme-d) δ 135.98, 132.19, 130.32, 129.26, 128.87, 128.49, 126.54, 124.57, 116.53, 107.09, 106.32, 33.41. SM (IC+) m/z 262 (MH+).

***Exemple 66**: 2-(phényl)pyrazolo[1',2':1,2][1,2,3]triazolo[4,5-b]pyrazin-6-ium-5-ide (143)*

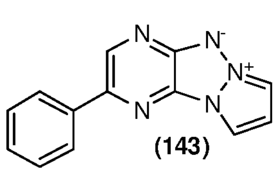

(143)

$C_{13}H_9N_5$
MW:235,25g.mol$^{-1}$

Ce composé a été préparé selon la *procédure générale C* dans un ballon, en utilisant l'acide phénylboronique (11mg, 0.09mmol, 1.5éq) dans le 1,4-dioxane/$H_2O$ (3.5mL/1.5mL). Le mélange réactionnel est laissé sous agitation à reflux du solvant pendant 1h30. Le résidu brut est purifié par chromatographie sur colonne de gel de silice (ether de pétrole/acétate d'éthyle : 7/3, 6/4 et 5/5 ; dépôt solide). Après une précipitation dans le chloroforme/pentane, le produit désiré **(143 : exemple 66)** est obtenu avec un rendement de quantitatif sous forme d'une poudre jaune. RMN ($^1$H, 400MHz, chloroforme-d) δ 8.95 (s, 1H), 8.13 (d, J = 3.3 Hz, 1H), 8.01 (d, J = 8.4 Hz, 2H), 7.85 (d, J = 2.6 Hz, 1H), 7.51

(t, J = 7.6 Hz, 2H), 7.41 (t, J = 7.4 Hz, 1H), 6.94 (t, J = 3.0 Hz, 1H). RMN ($^{13}C$, 400MHz, chloroforme-d) δ 151.42, 149.39, 141.13, 139.44, 136.93, 129.04, 128.40, 126.14, 110.18, 109.68, 109.10. SM (IC+) *m/z* 236 ($MH^+$).

***Exemple 67* : 2-(pyrimidin-5-yl)pyrazolo[1',2':1,2][1,2,3]triazolo[4,5-b]pyrazin-6-ium-5-ide (144)**

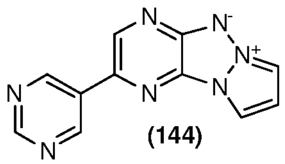

**(144)**

$C_{11}H_7N_7$
MW:237,23g.mol$^{-1}$

Ce composé a été préparé selon la *procédure générale C* dans un ballon sec et sous argon, en utilisant l'acide pyrimidine-5-boronique (39mg, 0.32mmol, 1.5éq) dans le 1,4-dioxane/$H_2O$ (5mL/2mL). Le mélange réactionnel est laissé sous agitation à reflux du solvant pendant 4h. Le résidu brut est purifié par chromatographie sur colonne de gel de silice (dichlorométhane/acétate d'éthyle : 8/2 ; dépôt solide). Le produit désiré **(144 : exemple 67)** est obtenu avec un rendement quantitatif (53mg) sous forme d'une poudre orange. RMN ($^1H$, 400MHz, chloroforme-d) δ 9.36 (s, 2H), 9.23 (s, 1H), 8.95 (s, 1H), 8.19 (dd, J = 3.4, 0.5 Hz, 1H), 7.92 (dd, J = 2.7, 0.5 Hz, 1H), 7.00 (dd, J = 3.4, 2.7 Hz, 1H). RMN ($^{13}C$, 101MHz, chloroforme-d) δ 157.79, 153.89, 140.66, 132.94, 130.59, 111.40, 110.37, 110.14. SM (IC+) m/z 238 (MH+).

***Exemple 68* : 2-(4-nitrophényl)pyrazolo[1',2':1,2][1,2,3]triazolo[4,5-b]pyrazin-6-ium-5-ide (145)**

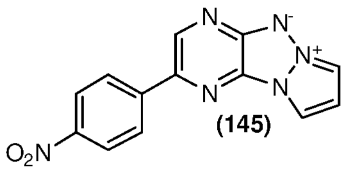

**(145)**

$C_{13}H_8N_6O_2$
MW:280,25g.mol$^{-1}$

Ce composé a été préparé selon la *procédure générale C* dans un ballon sec et sous argon, en utilisant l'acide 4-nitrophenylboronique (275mg, 1mmol, 1.5éq) dans le 1,4-dioxane/$H_2O$. Le mélange réactionnel est laissé sous agitation à reflux du solvant pendant 6h. Le résidu brut est purifié par chromatographie sur colonne de gel de silice (dichlorométhane/méthanol : 99/1,98/2, 97/3 ; dépôt solide). Le produit désiré **(145 : exemple 68)** est obtenu avec un rendement de 79% sous forme d'une poudre rouge. RMN ($^1H$, 400MHz, chloroforme-d) 9.2 (s, 1H), 8.8 (m, 1H), 8.5 (m, 1H), 8.36 (qd, J=9.1, 2.3 Hz, 4H), 7.23 (t, J=3.0 Hz, 1H). RMN ($^{13}C$, 101MHz, chloroforme-d) δ 152.4, 146.9, 143.5, 141.8, 134.2, 129.8, 126.4, 124.7, 114.1, 112.5, 111.2. SM (IC+) m/z 281(MH+).

***Exemple 69* : Synthèse du composé (146)**

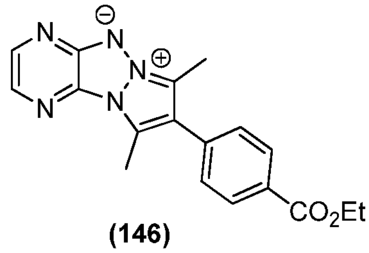

**(146)**

Dans un mélange toluène/THF/eau 1 :1 :1 (15 mL) sont dissouts 0,050 g de 4-bromo-3,5-diméthyl-pyrazolo-pyrazino triazapentalène (**123**) (0,19 mmol, 1 eq.), 0,109 g de d'acide para-éthylcarboxyphényl boronique (0,56 mmol, 3 eq.), et 0,080 g de carbonate de sodium (0,75 mmol, 4 eq.). Le mélange est dégazé par bullage d'argon 5 minutes puis 0,022 g de $Pd(PPh_3)_4$ (0,019 mmol, 0,1 eq.) sont ajoutés. La solution est chauffée au micro-ondes à reflux pendant 2 h (600W, 85°C). Après refroidissement, la solution est diluée dans de l'acétate d'éthyle et lavée avec NaCl sat.La phase organique est séchée sur $MgSO_4$, filtrée et purifiée par 4 chromatographies sur silice successives en employant différents éluants (PE/EA, DCM/MeOH, DCM/Et2O puis PE/EA). Après purification, 21 mg du produit attendu sont obtenus sous forme d'une poudre jaune (0,063 mmol, 33%).

$^1H$ NMR (250 MHz, Chloroform-d) δ 8.33 (d, *J* = 2.8 Hz, 1H), 8.22 - 8.17 (m, 2H), 7.78 (d, *J* = 2.8 Hz, 1H), 7.50 - 7.43 (m, 2H), 4.44 (q, *J* = 7.1 Hz, 2H), 2.88 (s, 3H), 2.64 (s, 3H), 1.43 (t, *J* = 7.1 Hz, 3H).

$^{13}C$ NMR (101 MHz, $CDCl_3$) δ 166.22, 153.00, 142.49, 135.92, 135.05, 131.50, 130.37, 130.30, 129.89, 128.74, 128.39, 125.65, 121.87, 121.22, 118.00, 61.42, 30.47, 14.50, 10.12, 9.79.

HRMS (ESI) : $[M+H]^+$ calculé pour $C_{18}H_{18}N_5O_2$ 336.145501, mesuré 336.145456 (-0.1 ppm).

***Exemple 70 : Synthèse de 7-(2-thiophène)-triazapentalène (147)***

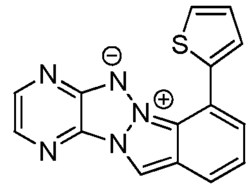

**(147)**

Dans un ballon sont dissous 0,055 g de 7-bromotriazapentalène (**129**) (0,19 mmol, 1 eq.), 0,049 g d'acide thiophène-2-boronique (0,38 mmol, 2 eq.) et 0,079 g de $K_2CO_3$ (0,57 mmol, 3 eq.) dans un mélange THF/eau 1 :1. Le mélange est dégazé par bullage d'argon et on ajoute ensuite 0,011 g de $Pd(PPh_3)_4$ (0,01 mmol, 0,05 eq.). La solution est chauffée à reflux au micro-ondes 2 h à une puissance de 680 W, le produit de départ n'étant pas totalement consommé, on ajoute 1 eq. d'acide boronique et 0,05 eq. de catalyseur au Pd et la réaction est chauffée 2h50 de plus. Le mélange est ensuite dilué dans de l'acétate d'éthyle et lavée avec NaCl sat. Après séchage sur $MgSO_4$, filtration et concentration sous pression réduite, le résidu est purifié par chromatographie avec PE/EA comme éluant pour donner 20 mg du produit attendu (0,069 mmol, 36%).

$^1H$ NMR (250 MHz, Chloroform-d) δ 8.60 (d, *J* = 2.3 Hz, 2H), 8.12 (dd, *J* = 3.7, 1.1 Hz, 1H), 8.04 (d, *J* = 2.5 Hz, 1H), 7.82 (dd, *J* = 8.5, 1.0 Hz, 1H), 7.60 (dd, *J* = 7.1, 0.9 Hz, 1H), 7.50 - 7.40 (m, 2H), 7.32 - 7.26 (m, 1H).

HRMS (ESI) : $[M+H]^+$ calculé pour $C_{15}H_{10}N_5S$ 292.065143, mesuré 292.065085 (-0.2 ppm).

***III.3. Couplage de Stille***

La procédure générale D, décrite ci-après, est mise en oeuvre pour préparer les composés selon l'invention.

Procédure générale D

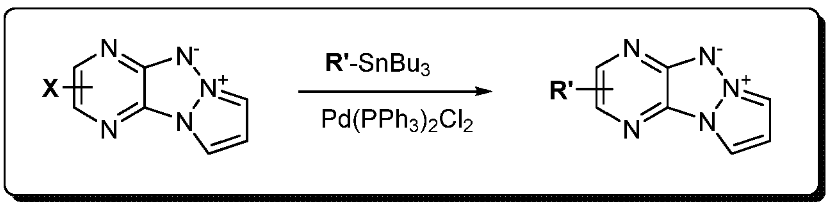


Dans un ballon *(ou tube scellé)* sec placé sous atmosphère d'argon, contenant une suspension de l'intermédiaire halogéné (par exemple composé **121**) (1éq) dans le toluène (0.25M), le dérivé stannylé (1éq) est introduit goutte à goutte. Le milieu réactionnel est dégagé à l'argon pendant 10min, ensuite, le $Pd(PPh_3)_2Cl_2$ (10mol%) est introduit. La

suspension obtenue est laissée sous agitation à reflux du solvant jusqu'à la consommation totale du dérivé halogéné. Après retour à température ambiante le milieu réactionnel est concentré à sec et le résidu brut est purifié par chromatographie sur colonne de gel de silice et est ensuite précipité dans mélange chloroforme/pentane.

***Exemple 71** : 2-(pyridin-2-yl)pyrazolo[1',2:1,2][1,2,3]triazolo[4,5-b]pyrazin-6-ium-5-ide (148)*

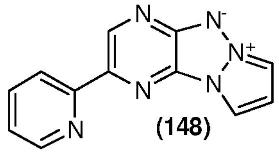

**(148)**

$C_{12}H_8N_6$
MW:236,24g.mol$^{-1}$

Ce composé a été préparé selon la *procédure générale D* en utilisant le 2-(tributylstannyl)pyridine (1mmol). Le mélange réactionnel est laissé sous agitation à reflux du solvant pendant 30h. Le résidu brut est purifié par chromatographie sur colonne de gel de silice (DCM/ MeOH : 100/0, 99/1, 98/2, 97/3 ; dépôt solide). Le produit **(148 : exemple 71)** est obtenu avec un rendement de 87% sous forme d'une poudre orange. RMN ($^1$H, 400MHz, chloroforme-d) δ 9.51 (t, 1H), 8.65 (d, J=4.2 Hz, 1H), 8.11 (d, J=3.0 Hz, 1H), 7.84 (m, 1H), 7.77 (t, J=7.7 Hz, 1H), 7.23 (t, 1H), 6.91 (t, 1H). RMN (13C, 101MHz, chloroforme-d) δ 154.8, 152.4, 149.5, 142.7, 137.7, 137.0, 128.4, 122.8, 120.0, 110.7, 109.7. SM (IC+) m/z 237 (MH+).

***Exemple 72** : 2-(pyrimidin-2-yl)pyrazolo[1,2':1,2][1,2,3]triazolo[4,5-b]pyrazin-6-ium-5-ide (149)*

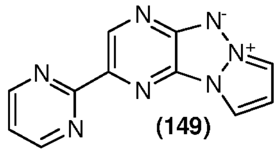

**(149)**

$C_{12}H_8N_6$
MW:236,24g.mol$^{-1}$

Ce composé a été préparé selon la *procédure générale D* en utilisant le 2-(tributylstannyl)pyrimidine (0.2mmol). Le mélange réactionnel est laissé sous agitation à reflux du solvant pendant 24h. Le résidu brut est purifié par chromatographie sur colonne de gel de silice (DCM/AcOEt /MeOH : 85/10/5 ; dépôt solide). Le produit désiré **(149 : exemple 72)** est obtenu avec un rendement de 60% sous forme d'une poudre orange. RMN ($^1$H, 400MHz, chloroforme-d) δ 9.71 (s, 1H), 8.87 (d, J=4.8 Hz, 2H), 8.31 (d, J=3.4 Hz 1H); 7.88 (d, J=2.7 Hz, 1H), 7.23 (t, 1H), 6.92 (t, J=3.0 Hz, 1H).. RMN ($^{13}$C, 101MHz, chloroforme-d) δ 162.8, 157.7 (2C), 153.0, 145.4, 135.7, 129.2, 119.4, 112.4, 110.7, 109.8. SM (IC+) m/z 238 (MH+).

***III.4. Couplage de Sonogashira***

La procédure générale E, décrite ci-après, est mise en oeuvre pour préparer les composés selon l'invention.

Procédure générale E

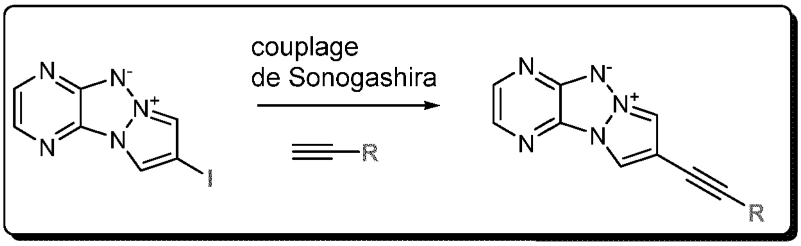


Dans un tube scellé sec sous argon, le dérivé iodé (**50 : exemple 15**) (1éq) est solubilisé dans un mélange ACN/DMF (4/1 : v/v, C=0.1M). La triéthyle amine distillée (2éq) et l'alcyne (2éq) sont ensuite ajoutés. Le mélange réactionnel est dégazé puis le $Pd(Ph_3)_2Cl_2$ (0.1 éq) et le CuI (0.1 éq) sont introduits. Le milieu réactionnel est laissé sous agitation à température ambiante jusqu'à la consommation totale du dérivé iodé. Le solvant est ensuite éliminé par évaporation sous vide et le résidu brut est purifié par chromatographie sur colonne de gel de silice.

***Ethyl 2-(2-naphthamido)pent-4-ynoate (64.2)***

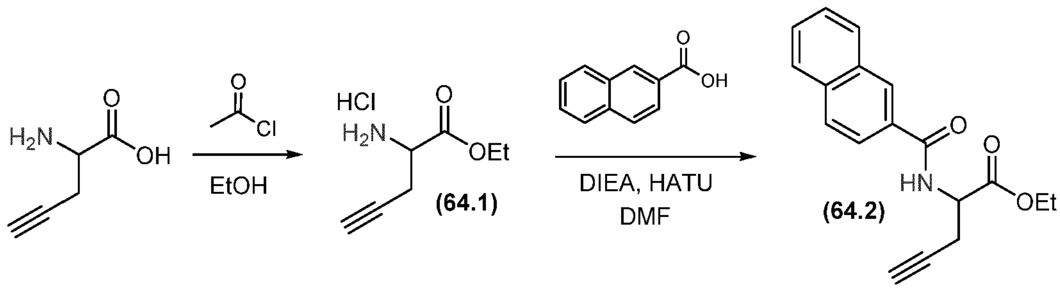


Le chlorure d'acétyle (580µL, 8.22mmol, 3.1éq) est introduit dans un ballon contenant l'éthanol (12mL) refroidi à 0°C. Après 30min d'agitation à 0°C le L,D-propargylglycine (300mg, 2.65mmol, 1éq) est introduit par des petites portions. La solution est laissée revenir à température ambiante, puis est portée à reflux pendant 4h. Le solvant est évaporé pour obtenir le produit **(64.1)** salifié sous la forme d'un solide blanc avec un rendement quantitatif (478mg).

L'intermédiaire (**64.1**) et le 2-naphtalènecarboxilique acide (456.3 mg, 2.65mmol, 1éq) sont solubilisés dans le DMF anhydre (9mL). Après l'ajout du DIEA (1.4mL, 7.95mmol, 3éq), le milieu réactionnel est refroidi à 0°C. Le HATU (1g, 2.65mmol, 1éq) est introduit en dernier par petites portions. Le mélange réactionnel est laissé sous agitation à 0°C pendant 2h. Après retour à température ambiante le solvant est évaporé et le solide brut est solubilisé dans l'AcOEt. La phase organique est lavée successivement avec une solution saturée de NaHCOs, puis à l'eau et avec une solution saturée en NaCl. La phase organique est séchée sur $MgSO_4$, filtrée et concentrée à sec pour donner une huile marronâtre (847mg). Le produit brut est purifié par chromatographie sur colonne de gel de silice (dichlorométhane: 100%; dépôt solide). Le produit désiré **(64.2)** est obtenu avec un rendement de 85% (683mg : sur les deux étapes) sous forme d'un solide jaune pâle. RMN ($^1H$, 250MHz, chloroforme-d) δ 8.35 (dd, $J$ = 1.7, 0.9 Hz, 1H), 7.99 - 7.78 (m, 4H), 7.64 - 7.47 (m, 2H), 7.15 (d, $J$ = 7.6 Hz, 1H), 4.99 (dt, $J$ = 7.6, 4.6 Hz, 1H), 4.32 (qd, $J$ = 7.1, 4.5 Hz, 2H), 2.07 (t, $J$ = 2.6 Hz, 1H). RMN ($^{13}C$, 400MHz, chloroforme-d) δ 170.68, 167.14, 135.08, 132.73, 131.11, 129.16, 128.69, 127.97, 126.96, 123.71, 78.67, 71.84, 62.28, 51.28, 22.81, 14.34.

***Ethyl 2-((tert-butoxycarbonyl)amino)pent-4-ynoate (65.2)***

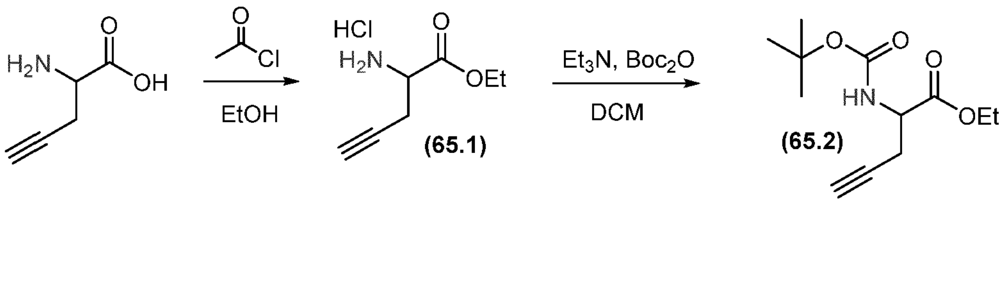

Le chlorure d'acétyle (2mL, 27.40mmol, 6.2éq) est introduit dans un ballon contenant l'Ethanol (40mL) refroidi à 0°C. Après 30min d'agitation à 0°C le L,D-propargylglycine (500mg, 4.42mmol, 1éq) est introduit par des petites portions. La solution est laissée revenir à température ambiante, puis est laissée sous agitation à reflux du solvant pendant 4h. Le solvant est évaporé pour obtenir le produit **(65.1)** salifié sous la forme d'un solide blanc avec un rendement quantitatif.

La triéthyl amine (3.1mL, 22.10mmol, 5éq) est ajouté à une solution contenant l'ester **(65.1)** dans le dichlorométhane distillé (11mL). Après 10min d'agitation à température ambiante, le dicarbonate de di-tert-butyle (1.45g, 6.64mmol, 1.5éq) est introduit. Le mélange réactionnel est laissé sous agitation à température ambiante pendant 18h. Le milieu est ensuite lavé avec une solution de $K_2CO_3$ à 5%, eau, une solution de NaHCOs saturée, séché sur $MgSO_4$, filtré et concentré à sec. Le produit brut est purifié par chromatographie sur colonne de gel de silice (Cyclohexane/AcOEt: 8/2 ; dépôt liquide). Le produit désiré **(65.2)** sous sa forme racémique est obtenu avec un rendement de 97% (1.03g : sur 2 étapes) sous forme d'une huile incolore. $R_f$ (Cyclohexane/ AcOEt : 8/2): 0.44. RMN ($^1H$, 250MHz, acétonitrile-$d_3$) $\delta$ 5.34 (d, *J* = 7.1 Hz, 1H, NH), 4.52 - 4.40 (m, 1H), 4.24 (p, *J* = 7.0 Hz, 2H), 2.73 (s, 1H), 1.45 (s, 9H), 1.29 (t, *J* = 7.1 Hz, 3H). RMN ($^{13}C$, 400MHz, chloroforme-d) $\delta$ 170.73, 155.25, 80.27, 78.72, 71.64, 61.90, 52.08, 28.44, 27.06, 23.02, 14.31. SM(IC+) *m/z* 142($M-C_5H_7O_2+Na^+$), 164 ($M-C_5H_7O_2+H^+$), 186($M-C_4H_8+H^+$), 208($M-C_4H_7+Na^+$), 242($M+H^+$), 264($M+Na^+$).

***Exemple 29 : 8-(4-(2-naphthamido)-5-ethoxy-5-oxopent-1-yn-1-yl)pyrazolo[1',2':1,2][1,2,3]triazolo[4,5-b]pyrazin-6-ium-5-ide (66)***

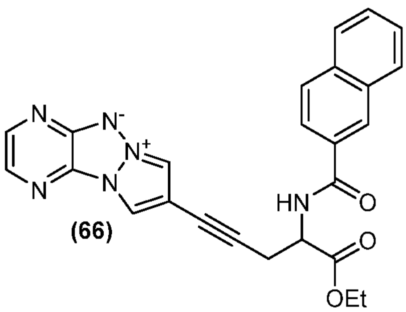

(66)

$C_{25}H_{20}N_6O_3$
MW: 452,47g.mol$^{-1}$

Ce composé a été préparé selon la *procédure générale E* à partir du éthyl 2-(2-naphthamido)pent-4-ynoate **(64.2)** (1.2mmol). Le milieu réactionnel est laissé sous agitation à température ambiante pendant 4h jusqu'à la solubilisation totale. Le résidu brut est purifié par chromatographie sur colonne de gel de silice (dépôt solide; DCM/AcOEt: gradient de 100/0 à 7/3). Le produit désiré **(66 : exemple 29)** est obtenu avec un rendement de 79% (477mg) sous forme d'une poudre orange. $R_f$ (DCM/ AcOEt : 8 /2) : 0.25. RMN ($^1H$, 400 MHz, Acétone-d6) $\delta$ 8.53 (d, *J* = 6.9 Hz, 2H), 8.39 (s, 1H), 8.22 (s, 1H), 8.02 (d, *J* = 4.3 Hz, 3H), 7.97 (d, *J* = 8.3 Hz, 1H), 7.91 (s, 1H), 7.60 (p, *J* = 7.0 Hz, 2H), 5.00 (t, *J* = 5.9 Hz, 1H), 4.28 (p, *J* = 6.4, 5.9 Hz, 2H), 3.21 (d, *J* = 6.7 Hz, 2H), 2.96 (s, 1H) 1.30 (t, *J* = 6.8 Hz, 3H). RMN ($^{13}C$, 101 MHz, Acétone-d6) $\delta$ 171.21, 167.57, 153.41, 143.83, 135.80, 133.67, 132.43, 130.90, 129.84, 129.07, 128.63, 128.60, 127.63, 125.01, 113.72, 111.50, 108.10, 90.01, 72.91, 62.12, 52.93, 23.34, 14.58. SM (IC+) *m/z* 453 ($M+H^+$).

***Exemple 30: 8-(4-((tert-butoxycarbonyl)amino)-5-ethoxy-5-oxopent-1-yn-1-yl)pyrazolo[1 ',2':1,2] [1,2,3]triazolo[4,5-b]pyrazin-6-ium-5-ide (67)***

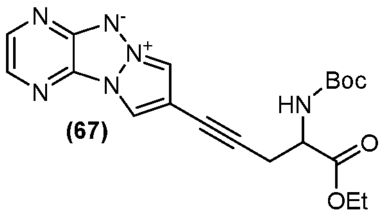

(67)

$C_{19}H_{22}N_6O_4$
MW: 398,42g.mol$^{-1}$

Ce composé a été préparé selon la <u>*procédure générale E*</u> à partir du éthyl 2-(2-Boc)pent-4-ynoate **(65.2)** (1.26mmol). Le milieu réactionnel est laissé sous agitation à température ambiante pendant 4h (solubilisation partielle). Le résidu brut est purifié par chromatographie sur colonne de gel de silice (dépôt solide; DCM/AcOEt de 100/0 à 9/1, puis DCM/MeOH 98/2). Le produit désiré **(67 : exemple 30)** est obtenu avec un rendement de 87% (420mg) sous forme d'une poudre orange. $R_f$ (DCM/ AcOEt : 8 /2) : 0.39. RMN ($^1$H, 400MHz, acetonitrile-$d_3$) δ 8.32 (s, 1H), 8.24 (s, 1H), 7.93 (s, 1H), 7.86 (s, 1H), 5.87 (d, *J* = 7.0 Hz, 1H), 4.46 - 4.32 (m, 1H), 4.17 (p, *J* = 6.7 Hz, 2H), 2.92 (d, *J* = 6.0 Hz, 2H), 1.39 (s, 9H), 1.23 (t, *J* = 7.1 Hz, 3H). RMN ($^{13}$C, 101MHz, acétonitrile-$d_3$) 142.95, 130.16, 112.84, 110.62, 61.45, 59.98, 52.44, 27.54, 22.81, 20.17, 13.59. (les carbones quaternaires ne sont pas visibles). SM (IC+) *m/z* 399 (MH$^+$).

***<u>Exemple</u> 31 : 8-(3-((tert-butoxycarbonyl)amino)prop-1-yn-1-yl)pyrazolo [1',2':1,2][1,2,3]triazolo[4,5-b]pyrazin-6-ium-5-ide (68)***

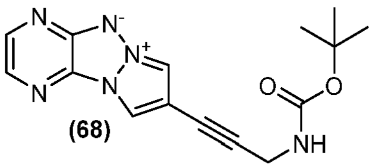

(68)

$C_{15}H_{16}N_6O_2$
MW: 312,33g.mol$^{-1}$

Ce composé a été préparé selon la <u>*procédure générale E*</u> à partir du tert-butyl prop-2-ynylcarbamate (112mg, 0.70mmol, 2éq). Le milieu réactionnel est laissé sous agitation à température ambiante pendant 2h (solubilisation totale). Le résidu brut est purifié par chromatographie sur colonne de gel de silice (DCM/AcOEt/MeOH : 10/0/0, 8/2/0 puis 8/1.8/0.2 ; dépôt solide). Le produit désiré **(68 : exemple 31)** est obtenu avec un rendement de 78% (85mg) sous forme d'une poudre jaune. $R_f$ (DCM/AcOEt : 8 /2) : 0.2. RMN ($^1$H, 400MHz, chloroforme-d) δ 8.47 (s, 1H, $H_1$ ou $H_2$), 8.11 (s, 1H, $H_3$ ou $H_4$), 7.95 (s, 1H, $H_1$ ou $H_2$), 7.86 (s, 1H, $H_3$ ou $H_4$), 4.81 (se, 1H, $H_6$), 4.19 (d, *J* = 5.1 Hz, 2H, $H_5$), 1.48 (s, 9H, $H_7$). RMN ($^{13}$C, 400MHz, chloroforme-d) δ 155.43, 152.80, 143.78, 132.29, 130.91, 128.69, 112.22, 110.69, 107.17, 90.09, 80.30, 72.07, 28.51. SM (IC+) *m/z* 313 (M+H$^+$), 257 (M-tBu+H$^+$).

***<u>Exemple 32</u> : 7-(3-((tert-butoxycarbonyl)amino)prop-1-yn-1-yl)pyrazolo [1',2':1,2] [1,2,3] triazolo[4,5-b]pyrazin-6-ium-5-ide (69)***

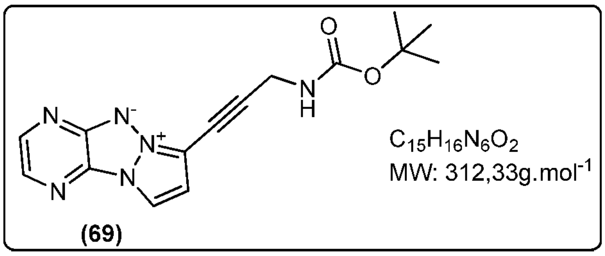

$C_{15}H_{16}N_6O_2$
MW: 312,33g.mol$^{-1}$

(69)

Ce composé a été préparé selon la <u>*procédure générale E*</u> à partir du tert-butyl prop-2-ynylcarbamate (2.1mmol).

Le milieu réactionnel est laissé sous agitation à reflux du solvant pendant 18h (solubilisation totale). Le résidu brut est purifié par chromatographie sur colonne de gel de silice (DCM/AcOEt: 10/0 et 8/2; dépôt solide). Le produit désiré **(69: exemple 32)** est obtenu avec un rendement de 37% (123mg) sous forme d'une poudre jaune. RMN ($^1$H, 250MHz, chloroforme-d) δ 8.52 (d, J = 2.7 Hz, 1H), 8.02 - 7.92 (m, 2H), 6.97 (d, J = 3.5 Hz, 1H), 4.34 (s, 2H), 1.55 (s, 9H). SM (IC+) m/z 313 ($MH^+$), 257 (M-tBu+H+), 184 ($M-C_6H_{10}N+H^+$).

***Exemple 33:** 8-(phényléthynyl)pyrazolo[1',2':1,2][1,2,3]triazolo[4,5-b]pyrazin-6-ium-5-ide (70)*

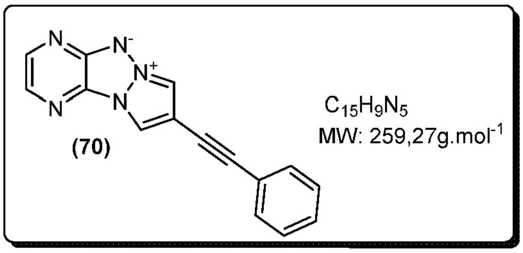

(70)

$C_{15}H_9N_5$
MW: 259,27g.mol$^{-1}$

Ce composé a été préparé selon la *procédure générale E* à partir du phényl-acétylène (1.1mmol). Le milieu réactionnel est laissé sous agitation à température ambiante pendant 1h30. Le résidu brut est purifié par chromatographie sur colonne de gel de silice (DCM/AcOEt: 10/0 ,9/1 ; dépôt solide). Le produit désiré **(70 : exemple 33)** est obtenu avec un rendement de 51% (41mg) sous forme d'une poudre jaune. $R_f$ (DCM/AcOEt/MeOH: 80/19/1): 0.85. RMN ($^1$H, 250MHz, chloroforme-d) δ 8.48 (d, *J* = 2.7 Hz, 1H), 8.21 (s, 1H), 7.98 (s, 1H), 7.95 (d, *J* = 2.7 Hz, 1H), 7.60 - 7.52 (m, 2H), 7.43 - 7.35 (m, 3H). RMN ($^{13}$C, 400MHz, chloroforme-d) δ 152.66, 143.75, 131.83, 130.87, 129.34, 128.69, 122.01, 111.91, 110.51, 107.86, 93.27, 78.13. SM (IC+) *m/z* 260 ($MH^+$).

***Exemple 34 :** 8-((4-méthoxyphényl)éthynyl)pyrazolo[1',2':1,2][1,2,3] triazolo[4,5-b]pyrazin-6-ium-5-ide (71)*

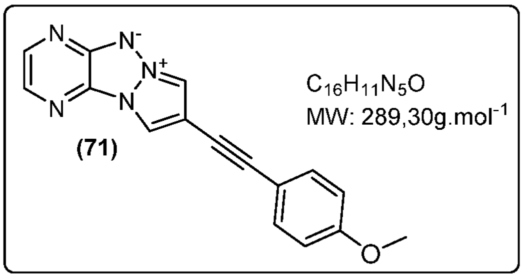

(71)

$C_{16}H_{11}N_5O$
MW: 289,30g.mol$^{-1}$

Ce composé a été préparé selon la *procédure générale E* à partir du 1-éthynyl-4-méthoxybenzène (0.73mmol). Le milieu réactionnel est laissé sous agitation à température ambiante pendant 6h (changement de couleur de jaune vers vert). Le résidu brut est purifié par chromatographie sur colonne de gel de silice (DCM/AcOEt: 10/0, 9/1 ; dépôt solide dans DCM. Le produit désiré **(71 : exemple 34)** est obtenu avec un rendement de 44% (41mg) sous forme d'une poudre jaune. $R_f$ (DCM/AcOEt/MeOH : 80/19/1): 0.41. RMN ($^1$H, 400MHz, chloroforme-d) δ 8.46 (d, *J* = 2.7 Hz, 1H), 8.18 (s, 1H), 7.94 (d, *J* = 1.6 Hz, 3H), 7.55 - 7.46 (m, 2H), 6.95 - 6.87 (m, 2H). RMN ($^{13}$C, 400MHz, chloroforme-d) δ 160.59, 152.64, 143.66, 133.38, 130.77, 129.16, 114.36, 114.01, 111.69, 110.54, 108.35, 93.36, 55.51. SM ($IC^+$) *m/z* 290 ($MH^+$).

***Exemple 73 :** 8-((4-(trifluorométhyl)phényl)éthynyl)pyrazolo[1',2':1,2] [1,2,3]triazolo[4,5-b]pyrazin-6-ium-5-ide (150)*

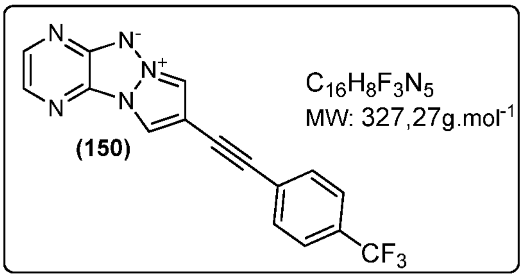

(150)

$C_{16}H_8F_3N_5$
MW: 327,27g.mol$^{-1}$

Ce composé a été préparé selon la <u>*procédure générale E*</u> à partir du 4-trifluorophénylacétylène (1.05mmol). Le milieu réactionnel est laissé sous agitation à température ambiante pendant 24h. Le résidu brut est purifié par chromatographie sur colonne de gel de silice (DCM/AcOEt: 100/0, 95/5 ; dépôt solide dans DCM. Le produit désiré **(150 : exemple 73)** est obtenu avec un rendement de 58% (41mg) sous forme d'une poudre jaune. RMN (1H, 400MHz, chloroforme-d) δ 8.49 (d, J = 2.7 Hz, 1H), 8.25 (s, 1H), 7.99 (s, 1H), 7.97 (d, J = 2.7 Hz, 1H), 7.66 (s, 4H). RMN (13C, 101MHz, chloroforme-d) δ 152.53, 143.79, 131.91, 130.99, 130.95, 125.67, 125.50, 125.46, 111.89, 110.18, 106.95, 91.63, 80.36. SM (IC+) m/z 328 (MH+).

***<u>Exemple 35</u> : 8-((triéthylsilyl)éthynyl)pyrazolo[1;2':1,2][1,2,3]triazolo[4,5-b]pyrazin-6-ium-5-ide (72)***

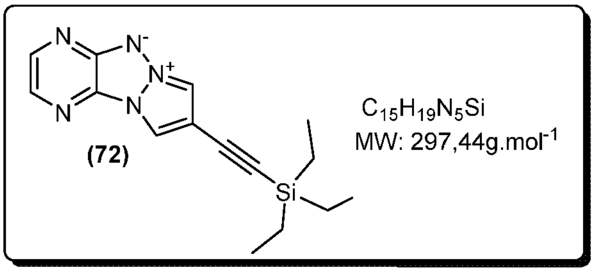

(72)

$C_{15}H_{19}N_5Si$
MW: 297,44g.mol$^{-1}$

Ce composé a été préparé selon la <u>*procédure générale E*</u> à partir du triéthyl(ethynyl)silane (0.73mmol). Le milieu réactionnel est laissé sous à température ambiante pendant 7h (changement de couleur de jaune vers vert). Le résidu brut est purifié par chromatographie sur colonne de gel de silice (DCM/Cyclohexane: 10/0, 8/2 ; dépôt solide dans DCM). Le produit désiré **(72 : exemple 35)** est obtenu avec un rendement de 62% (65mg) sous forme d'une poudre jaune. $R_f$ (cyclohexane/AcOEt : 5/5): 0.71. RMN ($^1$H, 250MHz, chloroforme-d) δ 8.45 (d, *J* = 2.7 Hz, 1H), 8.15 (s, 1H), 7.92 (d, *J* = 2.7 Hz, 1H), 7.88 (s, 1H), 1.06 (t, *J* = 7.8 Hz, 9H), 0.71 (q, *J* = 7.8 Hz, 6H). SM (IC$^+$) *m/z* 298 (MH$^+$).

***<u>Exemple 36</u>: 8-((triméthylsilyl)éthynyl)pyrazolo[1',2':1,2][1,2,3]triazolo [4,5-b]pyrazin-6-ium-5-ide (73)***

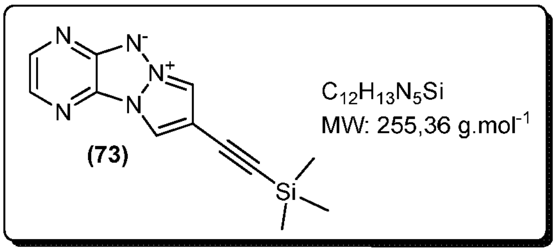

(73)

$C_{12}H_{13}N_5Si$
MW: 255,36 g.mol$^{-1}$

Ce composé a été préparé selon la <u>*procédure générale E*</u> à partir du triméthyl(éthynyl)silane (500μL, 3.7mmol, 2éq) sont ajoutés. Le milieu réactionnel est laissé sous agitation à température ambiante pendant 2h (changement de couleur de jaune vers vert-marron). Le résidu brut est purifié par chromatographie sur colonne de gel de silice (Cyclohexane/AcOEt: 6/4 ; dépôt solide). Le produit désiré **(73 : exemple 36)** est obtenu avec un rendement de 68% (304mg) sous forme d'une poudre jaune. RMN ($^1$H, 400MHz, chloroforme-d) δ 8.46 (d, J = 2.7 Hz, 1H), 8.14 (s, 1H), 7.94 (d, J

= 2.6 Hz, 1H), 7.88 (s, 1H), 0.29 (s, 9H).

***Exemple** 37:* **8-éthynylpyrazolo[1',2':1,2][1,2,3]triazolo[4,5-b]pyrazin-6-ium-5-ide (74)**

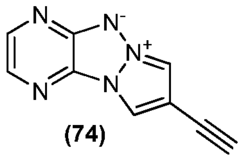

**(74)**

$C_9H_5N_5$
MW: 183,17g.mol$^{-1}$

Le composé **(73 : exemple 36)** (230mg, 0.90mmol, 1éq) est solubilisé dans une solution anhydre d'ammoniac dans le méthanol à 7N (28mL). Le mélange réactionnel est laissé sous agitation à température ambiante pendant 25min. Le solvant est évaporé sous pression réduite et le résidu brut est filtré sur gel de silice (DCM/AcOEt : 7/3) pour donner le produit désiré **(74 : exemple 34)** avec un rendement de 89% (147mg) sous forme d'une poudre jaune-verte claire. RMN ($^1$H, 400MHz, chloroforme-d) δ 8.47 (d, J = 2.7 Hz, 1H), 8.19 (s, 1H), 7.95 (d, J = 2.7 Hz, 1H), 7.92 (s, 1H), 3.23 (d, J = 2.6 Hz, 1H). RMN ($^{13}$C, 101MHz, chloroforme-d) δ 152.56, 143.77, 130.89, 128.85, 112.62, 110.67, 106.36, 81.58, 72.58. SM (IC+) m/z 184 (MH$^+$).

***Exemple 74 : Synthèse du composé (151)***

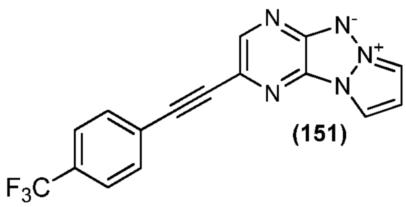

**(151)**

$C_{16}H_8F_3N_5$
MW: 327,27g.mol$^{-1}$

Ce composé a été préparé selon la *procédure générale E* à partir du 4-trifluorophénylacétylène (0.12mmol). Le milieu réactionnel est laissé sous agitation à température ambiante pendant une nuit. Après extraction à l'AcOEt et concentration, le résidu brut est purifié par chromatographie sur colonne de gel de silice (éther de pétrole/AcOEt : 70/30 ; dépôt solide dans DCM). Le produit désiré **(151 : exemple 74)** est obtenu avec un rendement de sous forme d'une poudre jaune. RMN ($^1$H, 400MHz, chloroforme-d) δ 8.69 (s, 1H), 8.14 (d, J = 3.4 Hz, 1H), 7.89 (d, J = 2.6 Hz, 1H), 7.72 (d, J = 8.1 Hz, 2H), 7.64 (d, J = 8.2 Hz, 2H), 6.97 (t, J = 3.0 Hz, 1H). RMN ($^{13}$C, 101MHz, chloroforme-d) δ 151.20, 147.49, 132.03, 125.54, 123.53, 111.42, 110.44, 110.08, 89.72, 89.07. SM (IC+) m/z 328 (MH$^+$).

***Exemple 75 : Synthèse de 7-(3-((tert-butoxycarbonyl)amino)prop-1-yn-1-yl)pyrazino[2',3':4,5][1,2,3]triazolo[2,1-a]indazol-6-ium-5-ide (152)***

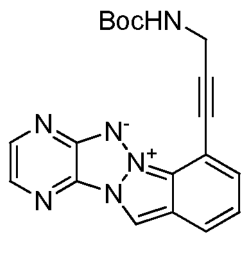

**(152)**

Dans un tricol de 100mL, sont introduits 110mg d'alcyne (0.7mmol-2éq) ; 100mg de 7-bromo indazole (**129**) (0.35momol - 1 éq) et 18 mg de triphénylphosphine (0.07mmol- 0.2éq), dissouts dans 10 mL de triéthylamine anhydre et 5mL de THF anhydre. Le milieu est alors dégazé à l'argon pendant quinze minutes. Puis 14mg de $Pd(dba)_2$ (0.02mmol-0.05éq) et 4 mg de CuI (0.02mmol - 0.05éq) sont ajoutés. Le milieu réactionnel est finalement porté à reflux du solvant (80°C). Après retour à température ambiante, le milieu réactionnel est filtré sur célite, et extrait à l'acétate d'éthyle (x2). Les phases organiques sont rassemblées et lavées avec une solution saturée en NaHCOs, séchées sur $MgSO_4$ et concentrées à sec. Le résidu brut est ensuite purifié par chromatographie sur colonne de gel de silice (dichlorométhane : 100% puis DCM/MeOH - gradient : 1%) pour donner 0,044 g de solide rouge (35%).

$^1$H NMR (250 MHz, Chloroform-d) δ 8.64 (d, *J* = 2.5 Hz, 1H), 8.53 (s, 1H), 8.07 (d, *J* = 2.6 Hz, 1H), 7.81 (d, *J* = 8.7 Hz, 1H), 7.39 (d, *J* = 8.6 Hz, 1H), 4.39 (s, 1H). $^{13}$C NMR (101 MHz, $CDCl_3$) δ 151.44, 145.41, 131.51, 129.78, 128.69, 128.57, 127.36, 125.30, 123.14, 121.84, 120.16, 108.83, 101.05, 93.57, 78.34, 31.91, 28.56. HRMS (ESI) : $[M+H]^+$ calculé pour $C_{19}H_{19}N_6O_2$ 363.156400, mesuré 363.156550(-0.4 ppm).

### *III.5. Fonctionalisation de la position 4 des structures de type [pyrazino]-1,3a,6a-triazapentalène*

***1-morpholinobutane-1,3-dione (153)***

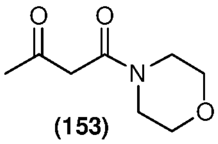

**(153)**

$C_8H_{13}NO_3$
MW: 171,20g.mol$^{-1}$

A une solution d'éthyle acétoacétate (1mL, 7.8mmol, 1éq) dans le toluène anhydre (80mL), le 4-DMAP (95mg, 0.78mmol,0.1éq) et la morpholine (2.4mL, 15.5mL, 2éq) sont introduits. Le mélange réactionnel est placé sous agitation à reflux pendant la nuit (environ 18h). Après retour à température, le solvant est évaporé sous pression réduite et le résidu brut est purifié par chromatographie sur colonne de gel de silice (Cyclohexane/ AcOEt: 9/1, 7/3, 5/5, 3/7 ; dépôt solide). Le produit désiré (153) est obtenu avec un rendement de 95% sous forme d'un solide cristallin jaune pale. RMN ($^1$H, 400MHz, chloroforme-d) δ 3.70 - 3.61 (m, 6H), 3.56 (s, 2H), 3.48 - 3.34 (m, 2H), 2.28 (s, 3H).

RMN en accord avec les données bibliographiques *(*Eur.J.O.Chem. 2013,19, 4131-4145).

***4-morpholino-4-thioxobutan-2-one (154)***

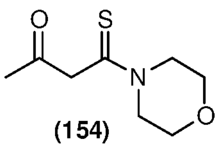

**(154)**

$C_8H_{13}NO_2S$
MW: 187,26g.mol$^{-1}$

Dans un tube scellé, contenant une solution de 1-morpholinobutane-1,3-dione (100mg, 0.58mmol, 1éq) dans le toluène anhydre (5mL), le réactif de Lawesson (122mg, 0.29mmol, 0.5éq) est introduit. La suspension obtenue est chauffée au micro-ondes à 140°C pendant 5min. Après retour à température, le solvant est évaporé sous pression réduite et le résidu brut est purifié par chromatographie sur colonne de gel de silice (Ether de pétrole/ AcOEt: 7/3, 5/5 ; dépôt solide). Le produit désiré **(154)** est obtenu avec un rendement de 79% sous forme d'un solide cristallin marron. RMN ($^1$H, 400MHz, chloroforme-d) δ 4.33 (t, J = 4.9 Hz, 2H), 4.20 (s, 2H), 3.83 - 3.63 (m, 8H), 2.31 (d, J = 1.2 Hz, 3H). RMN ($^{13}$C, 101 MHz, chloroforme-d) δ 201.57, 192.96, 66.41, 66.36, 58.79, 51.29, 49.99, 30.26. SM ($IC^+$) m/z 188 ($MH^+$).

***4-(1-(3-chloropyrazin-2-yl)-3-methyl-1H-pyrazol-5-yl)morpholine (155)***

(155)

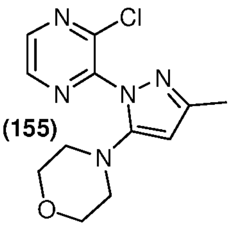

$C_{12}H_{14}ClN_5O$
MW: 279,73g.mol$^{-1}$

Dans un tube scellé, sec et placé sous argon, contenant le 4-morpholino-4-thioxobutan-2-one (50mg, 0.27mmol, 1éq) et le (3-chloropyrazin-2-yl)hydrazine **(12)** (48mg, 0.32mmol, 1.2éq) dans le toluène (2.4mL), la pyridine (4.5éq) est introduite. La suspension obtenue est chauffée au micro-ondes à 140°C pendant 45min. Après retour à température, le mélange brut est repris au toluène (20mL) et est lavé avec ne solution de HCl (0.5M). La phase aqueuse est lavée avec une solution saturée en NaCl, séchée sur $MgSO_4$et concentré à sec. Le résidu brut est purifié par chromatographie sur colonne de gel de silice (Cyclohexane/ AcOEt: 6/4 et 5/5 ; dépôt solide). Le produit désiré **(155)** est obtenu avec un rendement de 59% sous forme d'un solide marron claire. RMN ($^1H$, 400MHz, chloroforme-d) δ 8.51 (d, J = 2.4 Hz, 1H), 8.46 (d, J = 2.4 Hz, 1H), 5.77 (d, J = 0.5 Hz, 1H), 3.64 - 3.55 (m, 4H), 2.90 - 2.81 (m, 4H), 2.30 (d, J = 0.4 Hz, 3H).

***4-(1-(3-azidopyrazin-2-yl)-3-methyl-1H-pyrazol-5-yl)morpholine (156)***

(156)

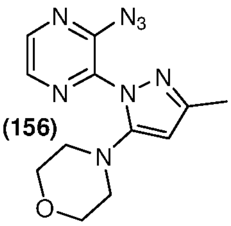

$C_{12}H_{14}N_8O$
MW: 286,30g.mol$^{-1}$

A une solution de (**155**) (150mg, 0.54mmol, 1éq) dans le DMF (5mL), le $NaN_3$ (70mg, 1.1mmol, 2éq) est introduit. La solution est chauffée à 100°C pendant 9h. Après retour à température ambiante, le mélange réactionnel est concentré et est purifié par chromatographie sur colonne de gel de silice (DCM/ AcOEt: 9/1 ; dépôt solide). Le produit désiré **(156)** est obtenu avec un rendement de 27% sous forme des cristaux jaunes. RMN ($^1H$, 400MHz, chloroforme-d) δ 8.64 (d, J = 4.5 Hz, 1H), 8.11 (d, J = 4.5 Hz, 1H), 5.89 (s, 1H), 3.79 (dd, J = 5.6, 3.7 Hz, 4H), 3.11 (dd, J = 5.6, 3.7 Hz, 4H), 2.41 (s,3H). RMN ($^{13}C$, 101MHz, chloroforme-d) δ 155.43, 154.33, 143.54, 130.98, 116.07, 97.66, 66.33, 52.54, 14.68, un carbone quaternaire n'est pas visible. SM (IC$^+$) m/z 287 (MH$^+$), 252 (MH$^+$-$N_2$) , 309 (MNa$^+$).

***<u>Exemple</u> 77: 7-méthyl-9-morpholinopyrazolo[1',2:1,2][1,2,3]triazolo[4,5-b]pyrazin-6-ium-5-ide (157)***

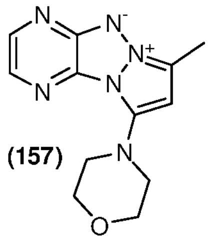

(157)

$C_{12}H_{14}N_6O$
MW: 258,29g.mol$^{-1}$

Ce composé a été préparé selon la *<u>procédure générale B</u>* à partir du composé **(156)** (0.9mmol) dans le 1,2-dichlorobenzène (2,5mL). Le temps de réaction est de 2h. Après retour à température ambiante, le mélange brut est purifié par chromatographie sur colonne de gel de silice (3 paliers: éther de pétrole: 100% ; cyclohexane/ acétate d'éthyle: 6/4, puis dichlorométhane/acétate d'éthyle 9/1). Le produit **(157 : exemple 77)** est obtenu avec un rendement de 85% sous forme d'une poudre orange. RMN ($^1H$, 400MHz, chloroforme-d) δ 8.16 (d, J = 2.9 Hz, 1H), 7.59 (d, J = 2.9 Hz, 1H),

5.89 (s, 1H), 4.06 - 3.93 (m, 4H), 3.51 - 3.34 (m, 4H), 2.59 (s, 3H). RMN ($^{13}C$, 101MHz, chloroforme-d) δ 141.47, 139.04, 132.20, 127.11, 120.60, 100.09, 93.20, 66.28, 49.74, 10.78. SM ($IC^+$) m/z 259 ($MH^+$).

***Exemple* 78 : *Synthèse du composé (158)***

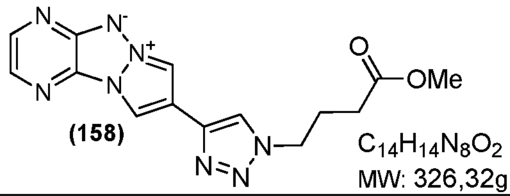

**(158)**

$C_{14}H_{14}N_8O_2$
MW: 326,32g.mol$^{-1}$

Le composé **(73 : exemple 36)** (20mg, 0.11 mmol, 1éq) est solubilisé dans un mélange tBuOH/$H_2O$ (1.4mL/0.4mL). Le 4-azidobutanoate de méthyle (préparé selon WO2010/082050) (19mg, 0.13mmol, 1.2éq), l'ascorbate de sodium (8.4mg) et le sulfate de cuivre pentahydraté (7.3mg) sont introduits. La suspension obtenue est laissée sous agitation à température ambiante pendant 24h puis 5h à 50°C. Après retour à température ambiante, le mélange réactionnel est concentré et est purifié par chromatographie sur colonne de gel de silice ( DCM/MeOH : 100/0, 95/5 et 9/1). Le produit **(158 : exemple 78)** est obtenu avec un rendement de 86% sous forme d'une poudre jaune. RMN ($^1H$, 400MHz, méthanol-d4) δ 8.75 (s, 1H), 8.50 (s, 1H), 8.39 (s, 1H), 8.33 (d, J = 2.9 Hz, 1H), 7.94 (d, J = 3.6 Hz, 1H), 4.53 (t, J = 6.7 Hz, 3H), 3.64 (s, 4H), 2.46 - 2.36 (m, 1H), 2.31 - 2.19 (m, 1H). SM ($IC^+$) m/z 327 ($MH^+$).

***III.6. Fonctionalisation des tétracyclique polyazotés***

***Exemple* 79 : *Synthèse du composé (159)***

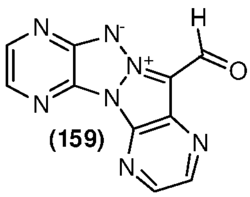

**(159)**

$C_{10}H_5N_7O$
MW:239,20g.mol$^{-1}$

Dans un ballon contenant le DMF anhydre (0.2mL), placé sous atmosphère d'argon et à 0°C, l'oxychlorure de phosphore (54µL, 0.56mmol, 4éq) est introduit goutte à goutte. Après 30 min d'agitation à cette température, une solution de (52.1) (30mg, 0.14mmol, 1éq) dans le DMF (0.2mL) est ajoutée. La solution jaune obtenue est chauffée à 80°C pendant 1h. Après retour à température ambiante, le milieu réactionnel est concentré et est purifié par chromatographie sur colonne de gel de silice (dichlorométhane/acétate d'éthyle : 95/5; dépôt solide). Le produit désiré **(159 : exemple 79)** est obtenu avec un rendement de 54% (34mg) sous forme d'une poudre jaune. RMN ($^1H$, 400MHz, chloroforme-d) δ 10.68 (s, 1H), 9.01 (d, J = 2.5 Hz, 1H), 8.90 (d, J = 2.5 Hz, 1H), 8.71 (d, J = 2.5 Hz, 1H), 8.64 (d, J = 2.5 Hz, 1H). SM ($IC^+$) m/z 240 ($MH^+$).

***Exemple 80* : *Synthèse du composé (160)***

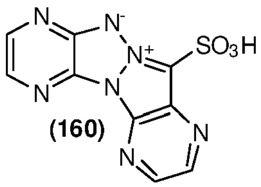

(160)

$C_9H_5N_7O_3S$
MW:291,25g.mol$^{-1}$

A une solution de **(52.1)** dans le $CHCl_3$ (2mL) est ajouté l'acide chlorosulfonique (20μL, 0.3mmol, 2.5éq). La suspension orange obtenue est laissée sous agitation à 80°C pendant 2h (un précipité orange se forme et se cumule sur les parois). Après retour à température ambiante, le milieu est concentré et le résidu est purifié par chromatographie en phase inverse ($H_2O$/ MeOH : 80/20 ; dépôt liquide avec $H_2O$). Le produit désiré **(160** : **exemple 80)** est obtenu avec un rendement de 15% (5mg) sous forme d'une poudre jaune. RMN (1H, 400MHz, D2O) δ 8.99 (d, J = 2.4 Hz, 1H), 8.79 (d, J = 2.4 Hz, 1H), 8.70 (d, J = 2.8 Hz, 1H), (d, J = 2.8 Hz, 1H). RMN (13C, 101MHz, D2O) δ 150.32(Cq), 144.74, 142.31, 139.73, 135.51, 132.26(Cq), 132.04(Cq), 131.70(Cq), 130.37(Cq). SM ($IC^+$) m/z 292 ($MH^+$), 314 ($MNa^+$).

***Exemple 81** : Synthèse du composé (161)*

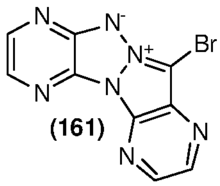

(161)

$C_9H_4BrN_7$
MW:239,20g.mol$^{-1}$

A une solution du composé (**52.1**)(50mg, 0.24mmol, 1éq) dans l'ACN (4mL), placée à 0°C et sous atmosphère d'argon, le NBS (46mg, 0.26mmol, 1.1 éq) est ajouté par petites portions. Le mélange réactionnel est laissé sous agitation en laissant la température revenir doucement à la température ambiante. Après 3h de réaction, le mélange réactionnel est repris au DCM. La phase organique est lavée avec une solution de NasSsOs, puis à l'eau, est séchée sur $MgSO_4$et est concentrée à sec. Le résidu brut est filtré sur un lit de silice (dichlorométhane/ acétate d'éthyle : 10/0 et 95/5 ; dépôt solide). Le produit désiré **(161 : exemple 81)** est obtenu avec un rendement de 83% (55mg) sous forme d'une poudre orange. RMN ($^{1}H$, 400MHz, chloroforme-d) δ 8.92 (d, J = 2.4 Hz, 1H), 8.68 (d, J = 2.4 Hz, 1H), 8.64 (d, J = 2.8 Hz, 1H), 8.30 (d, J = 2.7 Hz, 1H). RMN ($^{13}C$, 101MHz, chloroforme-d, 10K scans) δ 144.47, 143.01, 139.53, 134.36, les carbones quaternaires ne sont pas visibles. SM (IC+) m/z 240 (MH+).

***Exemple 82** : Synthèse du composé (162)*

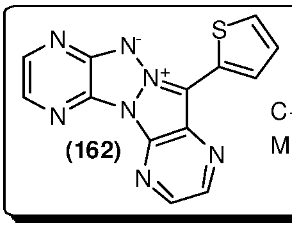

(162)

$C_{13}H_7N_7S$
MW:293,31g.mol$^{-1}$

Ce composé a été préparé selon la *procédure générale C :* en utilisant l'acide 2-thiénylboronique (12mg, 0.09mmol, 1.5éq) dans le 1,4-dioxane/ $H_2O$ (1.2mL/0.4mL). Le mélange réactionnel est laissé sous agitation à 50°C pendant 1h et à 70°C pendant 3h. Après retour à température ambiante le milieu réactionnel est concentré à sec et le résidu brut est purifié par chromatographie sur colonne de gel de silice (dichlorométhane/acétate d'éthyle : 10/0 et 90/10 ; dépôt solide). Le produit désiré **(162 : exemple 82)** est obtenu avec un rendement de 86% (15mg) sous forme d'une

poudre orange. RMN ($^1$H, 400MHz, chloroforme-d) δ 8.98 (d, J = 2.4 Hz, 1H), 8.71 (d, J = 2.4 Hz, 1H), 8.66 (dd, J = 3.8, 1.1 Hz, 1H), 8.57 (d, J = 2.8 Hz, 1H), 8.24 (d, J = 2.8 Hz, 1H), 7.65 (dd, J = 5.0, 1.0 Hz, 1H), 7.36 (dd, J = 5.0, 3.8 Hz, 1H). RMN ($^{13}$C, 101MHz, chloroforme-d, 10K scans) δ 143.62, 142.27, 139.45, 133.87, 128.03, 128.00, 128.00, les carbones quaternaires ne sont pas visibles. SM (IC+) m/z 294 ($MH^+$).

***Exemple 83 : Synthèse du composé (163)***

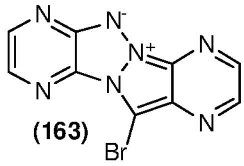

**(163)**

$C_9H_4BrN_7$
MW:239,20g.mol$^{-1}$

A une solution du composé (**52.2**)(30mg, 1éq) dans l'ACN (2.5mL), placée à 0°C et sous atmosphère d'argon, le NBS (27.5mg, 1.1éq) est ajouté par petites portions. Le mélange réactionnel est laissé sous agitation en laissant la température revenir doucement à la température ambiante. Après 3h de réaction le mélange réactionnel est repris au DCM. La phase organique est lavée avec une solution de NasSsOs, puis à l'eau, est séchée sur $MgSO_4$et est concentrée à sec. Le résidu brut est filtré sur un lit de silice (dichlorométhane/ acétate d'éthyle : 10/0 et 95/5 ; dépôt solide). Le produit désiré **(163 : exemple 83)** est obtenu avec un rendement de 26% sous forme d'une poudre violette. RMN ($^1$H, 400MHz, chloroforme-d) δ 8.97 (d, J = 1.9 Hz, 1H), 8.84 (d, J = 2.3 Hz, 1H), 8.69 (d, J = 1.9 Hz, 1H), 8.39 (d, J = 2.3 Hz, 1H). RMN (13C, 101MHz, chloroforme-d, 10K scans) δ 147.03, 146.71, 141.59, 134.70, les carbones quaternaires ne sont pas visibles. SM (IC+) m/z 240 (MH+).

***Exemple 84 : triazapentalène 7-bromo-3-sulfonate de sodium (164)***

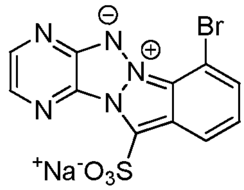

**(164)**

Dans un ballon sous argon est versé 0,231 mL d'acide chlorosulfonique (3,47 mmol, 10 eq.). Le ballon est plongé dans un bain de glace et on ajoute goutte-à-goutte 0,100 g de (**129**) (0,35 mmol, 1 eq.) dissout dans 5 mL de dichlorométhane anhydre. La réaction est laissée sous agitation une nuit et la température remonter à température ambiante. Le lendemain, la réaction est stoppée par ajout de galce et de quelques mL de NaOH 1M. La reaction est extraite à l'eau et lavée à l'acétate d'éthyle. La phase aqueuse est collectée et concentrée sous pression réduite. Le résidu est ensuite purifié par chromatographie en phase inverse avec un gradient eau/MeOH pour fournir 24 mg de (**164**) (0,062 mmol, 18%) après lyophilisation.

$^1$H NMR (250 MHz, Deuterium Oxide) δ 8.68 (d, *J* = 2.5 Hz, 1H), 8.39 (d, *J* = 2.5 Hz, 1H), 8.06 (d, *J* = 8.7 Hz, 1H), 7.41 (d, *J* = 7.3 Hz, 1H), 7.27 - 7.17 (m, 1H). $^{13}$C NMR (63 MHz, $D_2O$) δ 149.08, 145.12, 134.44, 129.63, 128.14, 127.10, 122.67, 119.88, 118.77, 116.19, 103.09. HRMS (ESI) : $[M+H]^+$ calculé pour $C_{11}H_7BrN_5O_3S$ 367.944749, mesuré 367.944495 (0.7 ppm).

**IV - CARACTERISATION PHOTOPHYSIQUE DES COMPOSES DE L'INVENTION**

**a. Détermination du rendement quantique**

Le rendement quantique est une propriété intrinsèque des fluorophores et est indispensable pour la caractérisation de nouvelles molécules possédant des propriétés photophysiques intéressantes. Selon la définition, le rendement quantique ($\Phi_F$) est le rapport entre la quantité de photons émis sur le nombre de photons absorbés par une unité de temps. Ici, nous allons utiliser le cas le plus simple de cette définition, à savoir, la forte dilution des échantillons à analyser.

Pour le calcul du rendement quantique nous avons utilisé la méthode comparative. Le principe consiste à comparer les spectres d'émission de la molécule d'intérêt avec une référence dont le rendement quantique est connu. On calcule le rendement quantique de fluorescence en utilisant l'équation suivante :

$$\Phi(x) = \Phi(ref)\,\frac{K(x)}{K(ref)}\,\frac{n(x)^2}{n(ref)^2}$$

Le $\Phi$**(x)** correspond au rendement quantique du composé à analyser et le $\Phi$**(réf)** est le rendement quantique de la référence. Les valeurs des **K(x)** et **K(réf)** représentent les valeurs des pentes des droites obtenues en traçant les surfaces de fluorescence en fonction des l'intensité d'absorption. Les **n(x)** et **n(réf)** représentent les indices de réfraction des solvants dans lesquels l'analyse à été réalisée.

On choisit comme fluorophore de référence une molécule dont les maxima d'absorption et d'émission sont proches de celles du produit à analyser. Le référence qui a un profil qui s'approche le plus de nos composés est la *COUMARINE 153* (qui a un rendement de fluorescence de 0,38 calculé dans l'éthanol). On ajuste ensuite les concentrations des deux chromophores afin d'avoir des absorptions maximales comprises entre 0,1 et 0,01 permettant d'éviter les effets de filtre interne.

**b. Evolution des propriétés photophysiques des chromophores fluorescents en fonction du solvant**

| Référence | Structure | Solvant | $\lambda_{Abs}$(nm) | $\lambda_{exc}$(nm) | $\lambda_{Em}$(nm) | Intensité relative de fluorescence |
|---|---|---|---|---|---|---|
| Exemple 7 | 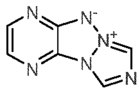 | DMSO | 414 | 414 | 501 | 3403 |
| | | MeOH | 407 | 407 | 510 | 734 |
| | | $CHCl_3$ | 409 | 409 | 468 | 7389 |
| | | ACN | 409 | 409 | 488 | 5665 |
| | | DCM | 410 | 410 | 468 | 8117 |
| | | Acétone | 410 | 410 | 480 | 5009 |
| | | H2O | 404 | 404 | 513 | 334 |

**c. Analyses photophysiques dans le DMSO**

| Réf. | Structure | $\lambda_{Abs}$ (nm) | $\lambda_{exc}$ (nm) | $\lambda_{Em}$ (nm) | $\varepsilon$ (L .mol$^{-1}$.cm$^{-1}$) | $\Phi_F$ (%) | $\varepsilon\ \Phi_F$ |
|---|---|---|---|---|---|---|---|
| DS102 | 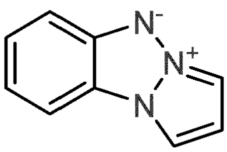 | 370 | 370 | Non fluorescent | 12700 | Ø | Ø |
| FST-L-14H04 | 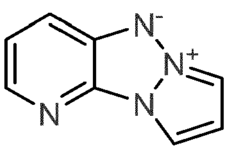 | 387 | 387 | 446 | 18700 | 1,8 | 340 |

(suite)

| Réf. | Structure | $\lambda_{Abs}$ (nm) | $\lambda_{exc}$ (nm) | $\lambda_{Em}$ (nm) | ε (L .mol$^{-1}$.cm$^{-1}$) | $\Phi_F$ (%) | ε $\Phi_F$ |
|---|---|---|---|---|---|---|---|
| DS234 | 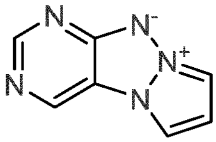 | 362 | 399 | 447 | 7107 (à 362 nm)<br>1174 (à 399 nm) | 15,4 | 181 |
| DS136 | 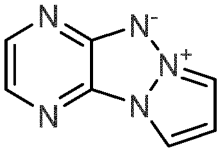 | 418 | 418 | 516 | 15500 | 15 | 2330 |
| FST-L-14H06 | 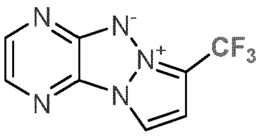 | 373 | 373 | Non fluorescent | 12600 | Ø | Ø |
| DS54 | 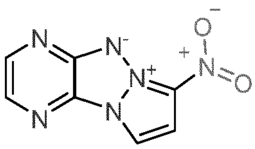 | 461 | 411 | 508 | 2400 (λ = 461) | <0,1 | <2 |
| DS28 DS104 | 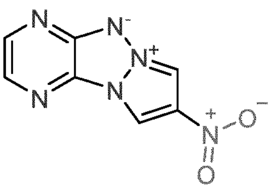 | 384 | 420 | 516 | 4300 (λ = 420) | <0,1 | <4 |
| DS75 | 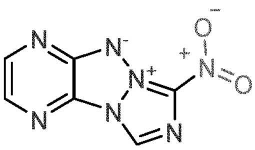 | 445 | 360 | 494 | 30000 (λ = 445) | <0,1 | <30 |
| DS55 | 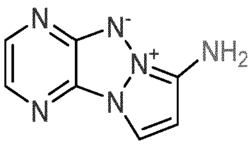 | 458 | 458 | 550 | 9300 | 0,6 | 60 |
| FST-L-14H05 | 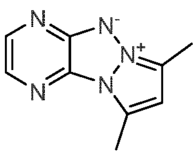 | 397 | 397 | 465 | 9770 | 3,3 | 322 |
| DS100 | 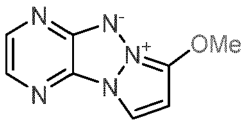 | 437 | 437 | 526 | 13200 | 3,6 | 475 |

(suite)

| Réf. | Structure | $\lambda_{Abs}$ (nm) | $\lambda_{exc}$ (nm) | $\lambda_{Em}$ (nm) | $\varepsilon$ ($L.mol^{-1}.cm^{-1}$) | $\Phi_F$ (%) | $\varepsilon\ \Phi_F$ |
|---|---|---|---|---|---|---|---|
| DS142 | 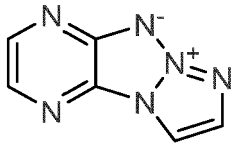 | 394 | 394 | 444 | 12000 | 7,2 | 860 |
| DS143 | 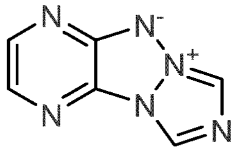 | 414 | 414 | 499 | 5700 | 44,9 | 2559 |
| DS323 | 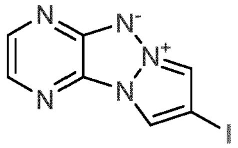 | 419 | 419 | 509 | 7521 | 12,5 | 940 |
| DS303 | 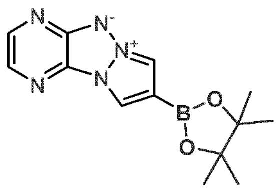 | 422 | 422 | 508 | 15900 | 18,8 | 2980 |
| DS293 | 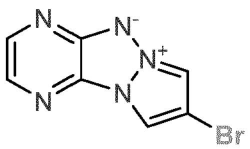 | 416 | 416 | 503 | 15500 | 24,8 | 3830 |
| DS285 | 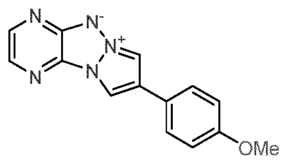 | 421 | 421 | 517 | 13200 | 8,7 | 1150 |
| DS289 | 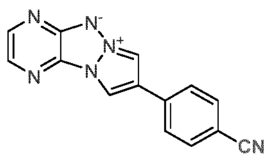 | 425 | 425 | 506 | 4285 | 25,8 | 1180 |
| DS333 | 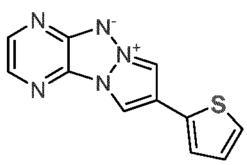 | 420 | 420 | 515 | 10800 | 12,3 | 1330 |
| DS261 | 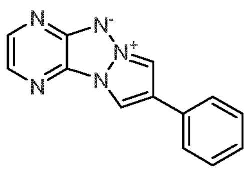 | 422 | 422 | 514 | 14300 | 14,2 | 2030 |

(suite)

| Réf. | Structure | $\lambda_{Abs}$ (nm) | $\lambda_{exc}$ (nm) | $\lambda_{Em}$ (nm) | ε (L .mol$^{-1}$.cm$^{-1}$) | $\Phi_F$ (%) | ε $\Phi_F$ |
|---|---|---|---|---|---|---|---|
| DS319 | 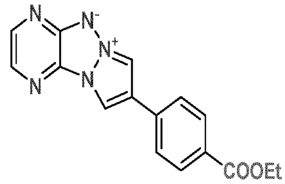 | 424 | 424 | 509 | 14662 | 22,3 | 3270 |
| DS314 | 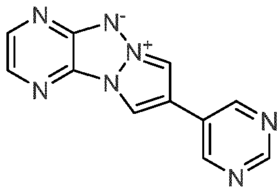 | 424 | 424 | 504 | 14200 | 28,6 | 4050 |
| DS126 | 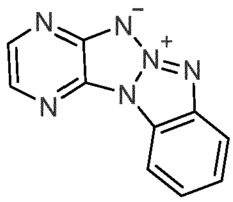 | 393 | 460 | 512 | 215 (λ= 460)<br>22200 (λ= 393) | 55 | 118 |
| DS59 | 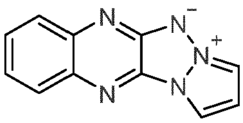 | 468 | 468 | 535 | 12700 | 3,7 | 470 |
| DS129, 213 | 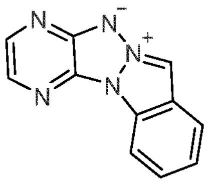 | 425 | 425 | 495 | 20574 | 8,2 | 1687 |
| DS367 | 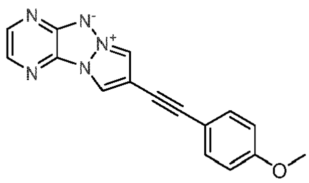 | 420 | 420 | 506 | 13500 | 19,8 | 2670 |
| DS366 | 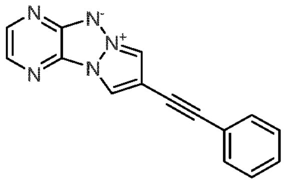 | 421 | 421 | 503 | 10790 | 29,6 | 3190 |
| DS368 | 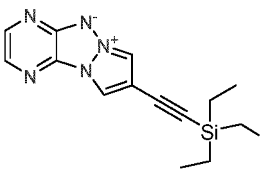 | 420 | 420 | 504 | 15400 | 21,1 | 3240 |

(suite)

| Réf. | Structure | $\lambda_{Abs}$ (nm) | $\lambda_{exc}$ (nm) | $\lambda_{Em}$ (nm) | ε (L .mol$^{-1}$.cm$^{-1}$) | $\Phi_F$ (%) | ε $\Phi_F$ |
|---|---|---|---|---|---|---|---|
| DS335 | 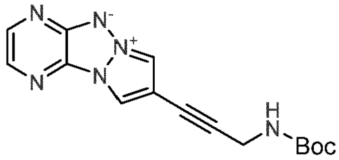 | 419 | 419 | 504 | 14400 | 22,7 | 3273 |
| DS365 | 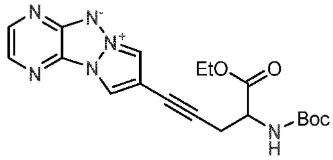 | 419 | 419 | 506 | 14350 | 25,5 | 3660 |

| structure | λexc | λem | ε | ϕ | ε*ϕ |
|---|---|---|---|---|---|
| 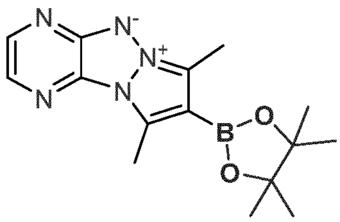 | 432 | 526 | 5729 | 5,4 | 309 |
| 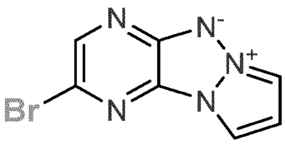 | 437 | 527 | 11180 | 2 | 223 |
| 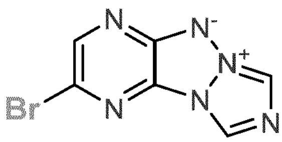 | 433 | 523 | 17400 | 24,8 | 4315 |
| 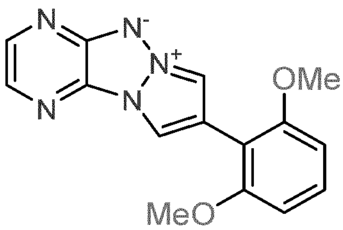 | 421 | 516 | 8899 | 6,2 | 551 |
| 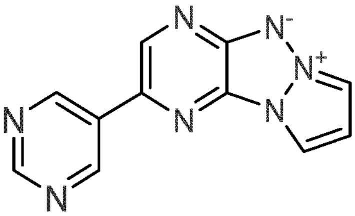 | 444 | 518 | 17500 | 17,8 | 3115 |

(suite)

| structure | λexc | λem | ε | ϕ | ε*ϕ |
|---|---|---|---|---|---|
| 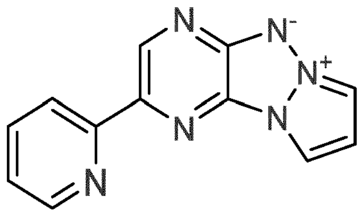 | 447 | 514 | 17560 | 30,4 | 5338 |
| 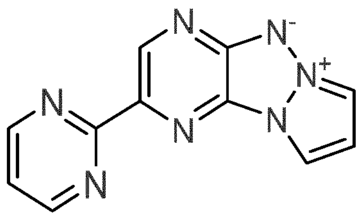 | 439 | 523 | 10221 | 30,2 | 3086 |
| 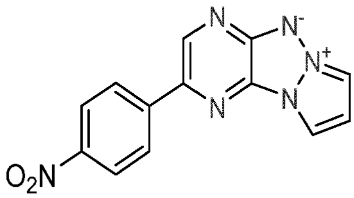 | 468 | 510 | 26164 | 0,1 | 26 |
| 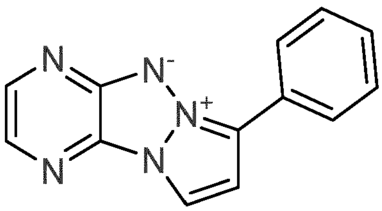 | 438 | 515 | 17014 | 19,5 | 3317 |
| 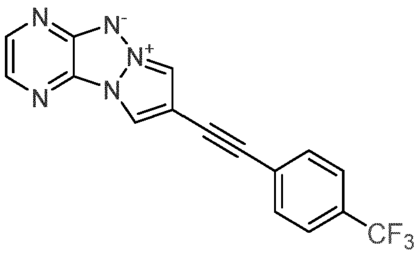 | 422 | 503 | 10731 | 31,9 | 3423 |
| 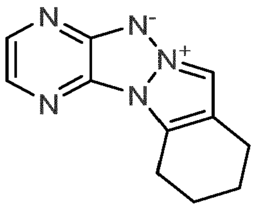 | 426 | 482 | 8779 | 6,3 | 553 |
| 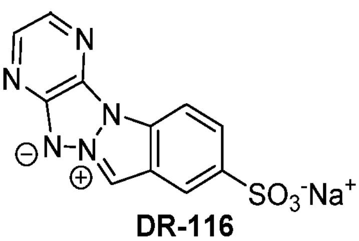 DR-116 | 426 | 496 | 14926 | 13,60 | 2029 |

(suite)

| structure | λexc | λem | ε | ϕ | ε*ϕ |
|---|---|---|---|---|---|
| 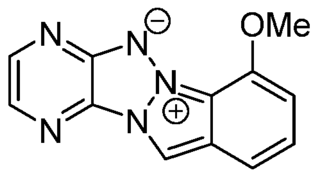 DR-143 | 492 | 585 | 2612,27 | 0,30 | 7 |
| 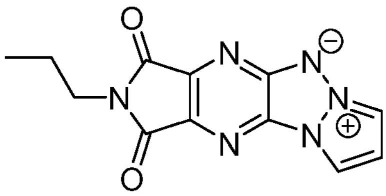 **DR-031** | 427 | 567 | 10011 | <0,1 | <10 |
| 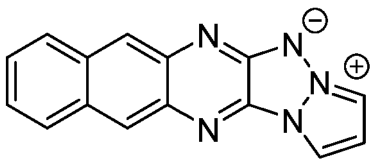 **DR-082** | 398/420/522/556 | 580 | 8700 | 8,70 | 756 |
| 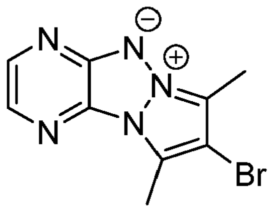 **DR-007** | 424 | 525 | 11456,74 | 5,7 | 653 |
| 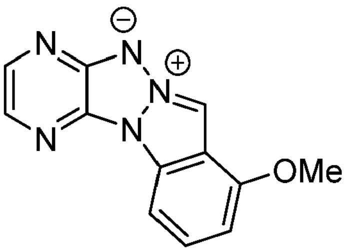 DR-SATT-148 | 433 | 504 | 5993,4 | 25,12 | 1505 |
| 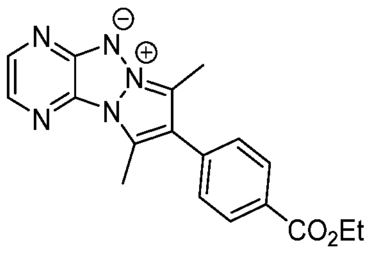 **DR-034** | 430 | 527 | 10377,36 | 5,1 | 529 |

(suite)

| structure | λexc | λem | ε | ϕ | ε*ϕ |
|---|---|---|---|---|---|
| 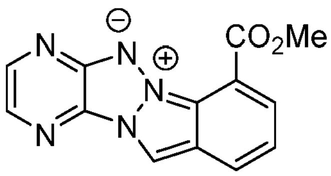 DR-EN-48 | 502 | 568 | 9658,2 | 5,8 | 560 |
| 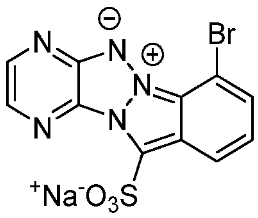 DR-SATT-186 | 496 | 565 | 12884 | 19,5 | 2512 |
| 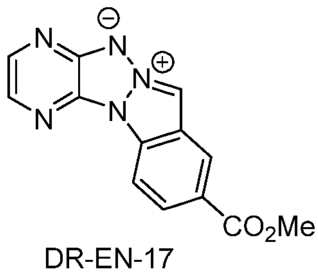 DR-EN-17 | 427 | 481 | 15579,877 | 10,2 | 1589 |
| 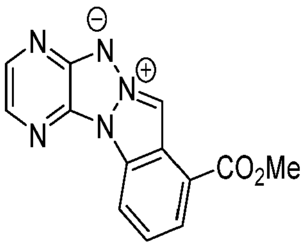 DR-EN-35 | 446 | 486 | 9461,7 | 1,2 | 113 |
| 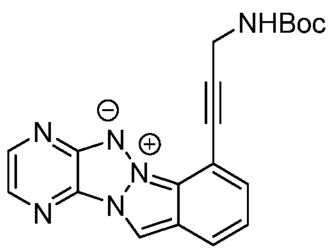 DR-EN-41 | 503 | 575 | 8653,4 | 2,7 | 233 |

(suite)

| structure | λexc | λem | ε | ϕ | ε*ϕ |
|---|---|---|---|---|---|
| 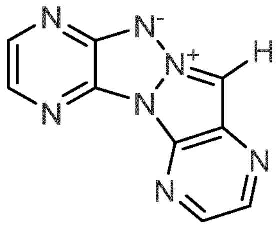 | 446 | 525 | 19153 | 1,1 | 210 |
| 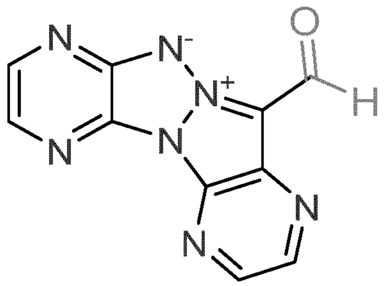 | 449 | 493 | 27197 | | |
| 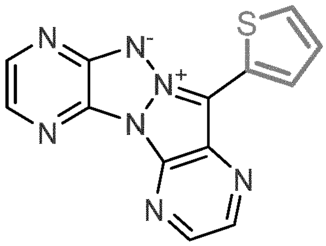 | 484 | 580 | 22396 | | |
| 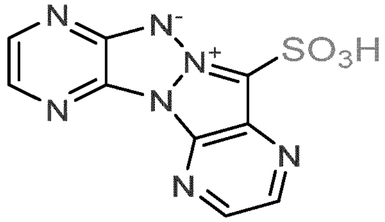 | 452 | 521 | 16413 | 0,4 | 65 |
| 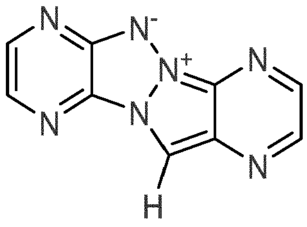 | 532 | 603 | 12147 | 10,3 | 1251 |

**d. Analyses photophysiques dans le dichlorométhane**

| Réf. | Structure | $\lambda_{Abs}$ (nm) | $\lambda_{exc}$ (nm) | $\lambda_{Em}$ (nm) | ε (L .mol$^{-1}$.cm$^{-1}$) | $\Phi_F$ (%) | ε $\Phi_F$ |
|---|---|---|---|---|---|---|---|
| DS129 | 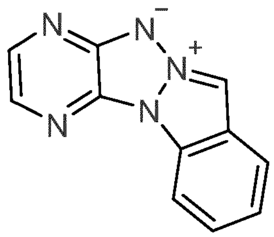 | 421 | 421 | 477 | 34300 | 3,1 | 1060 |
| DS323 | 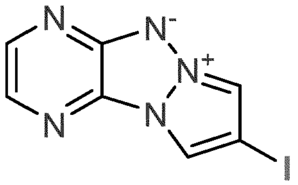 | 414 | 414 | 482 | 16400 | 27,3 | 4490 |
| DS303 | 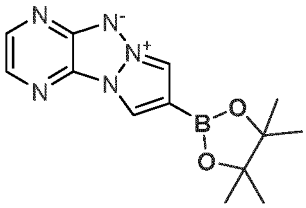 | 420 | 420 | 495 | 9300 | 50,5 | 4700 |
| DS293 | 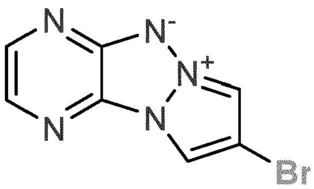 | 412 | 412 | 487 | 19300 | 27,4 | 5280 |
| DS136 | 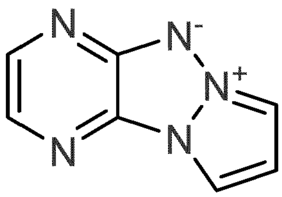 | 415 | 415 | 494 | 13000 | 42,4 | 5500 |
| DS261 | 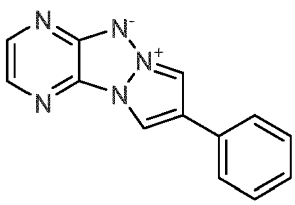 | 418 | 418 | 500 | 13800 | 44,6 | 6200 |
| DS314 | 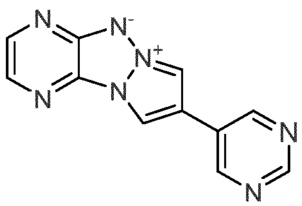 | 418 | 418 | 477 | 11500 | 50,7 | 5800 |
| DS319 | 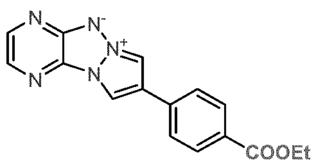 | 419 | 419 | 485 | 14200 | 47,7 | 6760 |

| structure | $\lambda_{exc}$ | $\lambda_{em}$ | ε | ϕ | ε*ϕ |
|---|---|---|---|---|---|
| 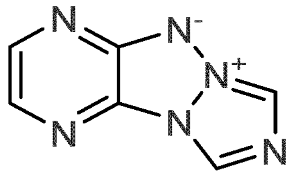 | 410 | 470 | 12133 | 36,6 | 4440 |
| 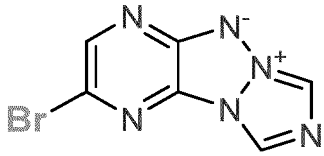 | 428 | 493 | 10500 | **63,5** | 6667 |
| 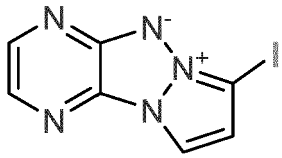 | 420 | 495 | 17394 | 5,4 | 939 |
| 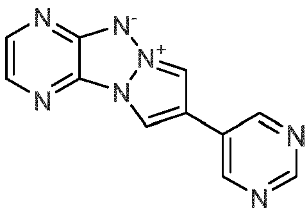 | 418 | 477 | 11500 | **50,7** | 5830 |
| 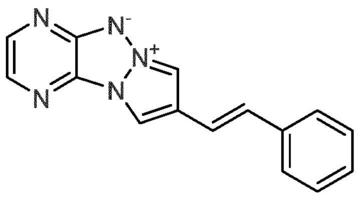 | 417 | 497 | 16515 | 21,5 | 3550 |
| 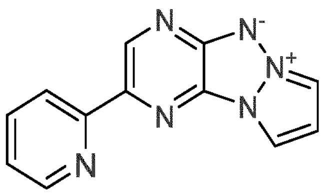 | 438 | 493 | 17560 | **45,3** | 7954 |
| 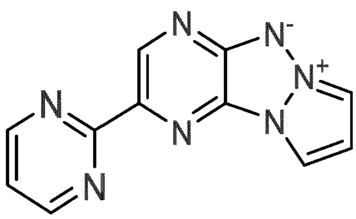 | 437 | 498 | 9590 | **37,8** | 3625 |
| 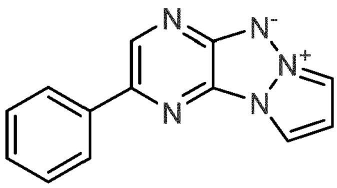 | 438 | 498 | 13973 | 48,2 | 6734 |

(suite)

| structure | $\lambda_{exc}$ | $\lambda_{em}$ | ε | ϕ | ε*ϕ |
|---|---|---|---|---|---|
| 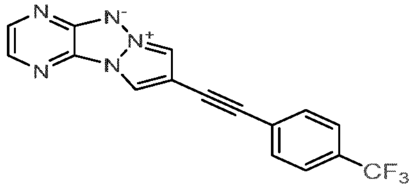 | 417 | 479 | 14897 | 43,5 | 6480 |
| 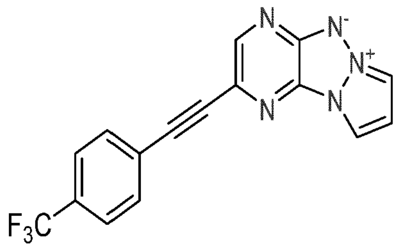 | 446 | 503 | 21957 | 48,8 | 10715 |
| 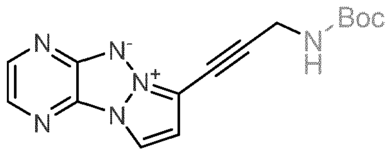 | 426 | 500 | 22366 | 14 | 3131 |
| 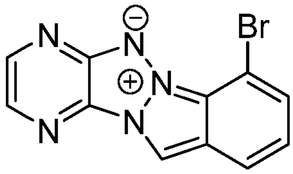 **DR-122** | 491 | 557 | 7000 | 10,80 | 756 |
| 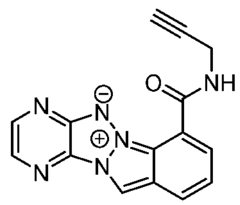 **DR-126** | 470 | 547 | | 12,00 | 0 |
| 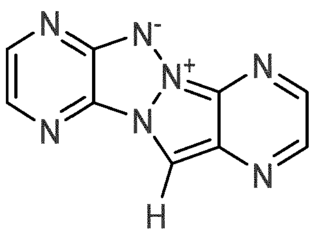 | 522 | 576 | 8600 | **67,2** | 5779 |
| 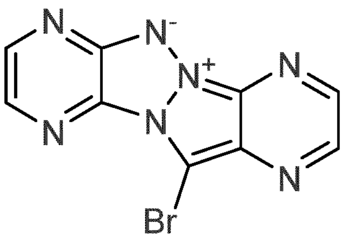 | 530 | 604 | 3267 | **25,3** | 826 |

## Revendications

**1.** Utilisation comme chromophore fluorescent d'un composé de formule (I) :

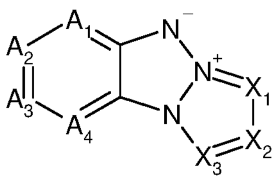

(I)

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, ($C_1$-$C_6$)alkyle, ($C_6$-$C_{10}$)aryle, hétéroaryle comprenant de 5 à 10 atomes, ($C_2$-$C_6$)alcényle, ($C_2$-$C_6$)alcynyle, CN, hydroxy, ($C_1$-$C_6$)alcoxy, thiol, ($C_1$-$C_6$)alkylthio, $(CH_2)_nSO_2$-$OR_a$ où n = 1-6, $CH_2SO_2$-$NR_aR_b$, $NO_2$, $SO_3R_a$, $NR_aR_b$, C(O)$OR_a$, C(O)$R_a$, et C(O)$NR_aR_b$, $R_a$ et $R_b$ représentant H ou un groupe ($C_1$-$C_6$)alkyle,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$, représentent, indépendamment les uns des autres, un atome d'hydrogène ou un atome d'halogène, ou sont choisis dans le groupe constitué de :

. un groupe ($C_1$-$C_6$)alkyle, ($C_3$-$C_6$)cycloalkyle ou ($C_6$-$C_{10}$)aryle,
ledit groupe ($C_6$-$C_{10}$)aryle étant éventuellement substitué par au moins un substituant choisi parmi : halogène, CN, ($C_1$-$C_6$)alkyle, OH, ($C_1$-$C_6$)alcoxy, C(O)$OR_a$ et $NR_aR_b$, $R_a$ et $R_b$ représentant H ou un groupe ($C_1$-$C_6$)alkyle,
. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, ($C_1$-$C_6$)alkyle, OH, ($C_1$-$C_6$)alcoxy, C(O)$OR_a$, C(O)$R_a$ et $NR_aR_b$, $R_a$ et $R_b$ représentant H ou un groupe ($C_1$-$C_6$)alkyle,
. un groupe hétérocycloalkyle comprenant de 4 à 10 chaînons et contenant de un à trois hétéroatomes choisis parmi O, S ou N,
. $NO_2$,
. CHO,
. un groupe C(O)$OR_a$, $R_a$ étant tel que défini ci-dessus,
. un groupe -HC=CH-Ar, Ar représentant un groupe ($C_6$-$C_{10}$)aryle,
. $SO_3H$,
. un groupe $NR_cR_d$, $R_c$ et $R_d$ représentant H ou un groupe ($C_1$-$C_6$)alkyle, ou pouvant former un groupe hétéro($C_2$-$C_5$)cycloalkyle avec l'atome d'azote qui les porte ;
. un groupe $OR_e$, $R_e$ représentant H, un groupe ($C_1$-$C_6$)alkyle ou un groupe ($C_6$-$C_{10}$)aryle ; et
. un groupe de formule (A) suivante :

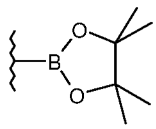

(A)

ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,
ledit composé de formule (I) pouvant être sous forme de sel ou de stéréoisomère pur ou sous forme de mélange d'énantiomères et/ou de diastéréoisomères, y compris de mélanges racémiques, ou sous forme de tautomères, à l'exception du composé de formule :

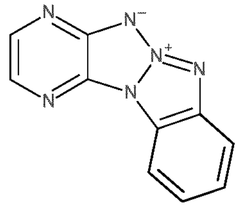

2. Utilisation selon la revendication 1, dans laquelle $A_1$ et $A_4$ sont -N-, $A_2$ est $-C(Y_2)-$ et $A_3$ est $-C(Y_3)-$.

3. Composé de formule (I) suivante :

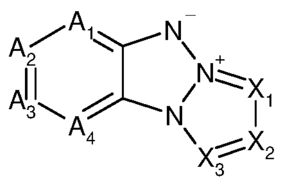

(I)

dans laquelle :

- $A_1$ est -N- ou $-C(Y_1)-$ ;
- $A_2$ est -N- ou $-C(Y_2)-$ ;
- $A_3$ est -N- ou $-C(Y_3)-$ ;
- $A_4$ est -N- ou $-C(Y_4)-$ ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, $(C_1\text{-}C_6)$alkyle, $(C_6\text{-}C_{10})$aryle, hétéroaryle comprenant de 5 à 10 atomes, $(C_2\text{-}C_6)$alcényle, $(C_2\text{-}C_6)$alcynyle, CN, hydroxy, $(C_1\text{-}C_6)$alcoxy, thiol, $(C_1\text{-}C_6)$alkylthio, $(CH_2)_nSO_2\text{-}OR_a$ où n = 1-6, $CH_2SO_2\text{-}NR_aR_b$, $NO_2$, $SO_3R_a$, $NR_aR_b$, $C(O)OR_a$, $C(O)R_a$, et $C(O)NR_aR_b$, $R_a$ et $R_b$ représentant H ou un groupe $(C_1\text{-}C_6)$alkyle,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou $-C(Y_5)-$ ;
- $X_2$ est -N- ou $-C(Y_6)-$ ;
- $X_3$ est -N- ou $-C(Y_7)-$ ;
- $Y_5$, $Y_6$ et $Y_7$, représentent, indépendamment les uns des autres, un atome d'hydrogène ou un atome d'halogène, ou sont choisis dans le groupe constitué de :

. un groupe $(C_1\text{-}C_6)$alkyle, $(C_3\text{-}C_6)$cycloalkyle ou $(C_6\text{-}C_{10})$aryle, ledit groupe $(C_6\text{-}C_{10})$aryle étant éventuellement substitué par au moins un substituant choisi parmi : halogène, CN, $(C_1\text{-}C_6)$alkyle, OH, $(C_1\text{-}C_6)$alcoxy, $C(O)OR_a$ et $NR_aR_b$, $R_a$ et $R_b$ représentant H ou un groupe $(C_1\text{-}C_6)$alkyle,

. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, $(C_1\text{-}C_6)$alkyle, OH, $(C_1\text{-}C_6)$alcoxy, $C(O)OR_a$, $C(O)R_a$ et $NR_aR_b$, $R_a$ et $R_b$ représentant H ou un groupe $(C_1\text{-}C_6)$alkyle,

. un groupe hétérocycloalkyle comprenant de 4 à 10 chaînons et contenant de un à trois hétéroatomes choisis parmi O, S ou N,

. $NO_2$,

. CHO,

. un groupe $C(O)OR_a$, $R_a$ étant tel que défini ci-dessus,

. un groupe -HC=CH-Ar, Ar représentant un groupe $(C_6\text{-}C_{10})$aryle,

. $SO_3H$,

. un groupe $NR_cR_d$, $R_c$ et $R_d$ représentant H ou un groupe $(C_1\text{-}C_6)$alkyle, ou pouvant former un groupe hétéro$(C_2\text{-}C_5)$cycloalkyle avec l'atome d'azote qui les porte ;

. un groupe $OR_e$, $R_e$ représentant H, un groupe $(C_1\text{-}C_6)$alkyle ou un groupe $(C_6\text{-}C_{10})$aryle ; et

. un groupe de formule (A) suivante :

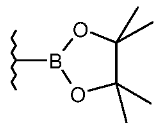

(A)

ledit composé de formule (I) pouvant être sous forme de sel ou de stéréoisomère pur ou sous forme de mélange d'énantiomères et/ou de diastéréoisomères, y compris de mélanges racémiques, ou sous forme de tautomères, à l'exception des composés suivants :

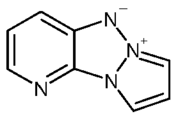
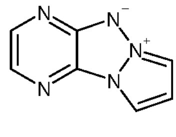
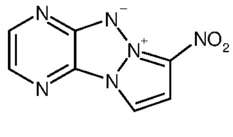
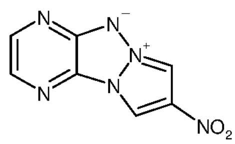
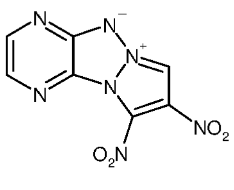
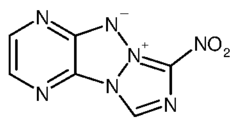
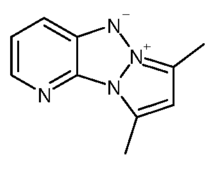
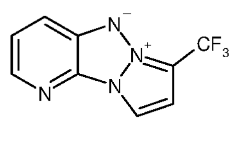
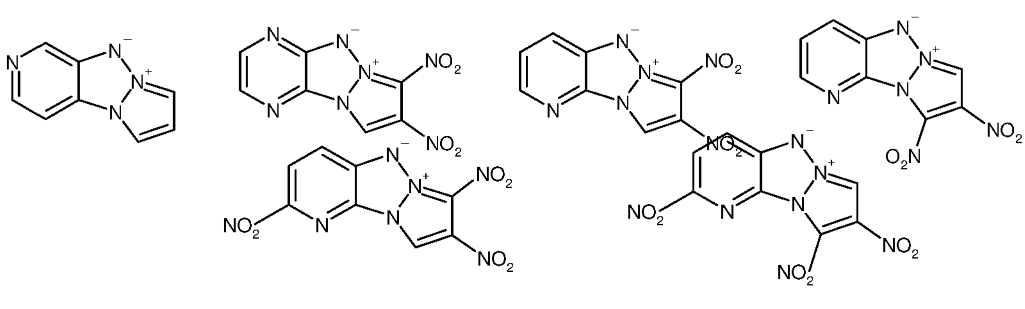

**4.** Composé selon la revendication 3, répondant à la formule (III) suivante :

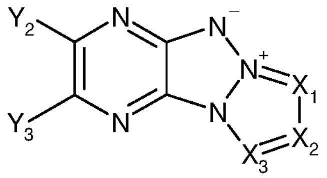

(III)

$Y_2$, $Y_3$, $X_1$, $X_2$ et $X_3$ étant tels que définis dans la revendication 3.

**5.** Composé selon la revendication 3, répondant à la formule (III-1) suivante :

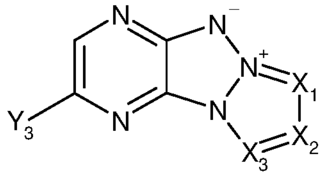

(III-1)

$Y_3$, $X_1$, $X_2$ et $X_3$ étant tels que définis dans la revendication 3.

**6.** Composé selon la revendication 3, répondant à la formule (V-1) suivante :

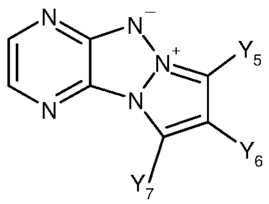

(V-1)

$Y_5$, $Y_6$ et $Y_7$ étant tels que définis dans la revendication 3.

**7.** Composé selon la revendication 3, répondant à la formule (VI) suivante :

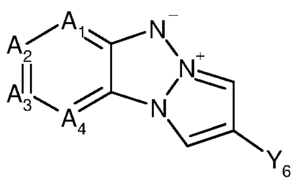

(VI)

$A_1$, $A_2$, $A_3$, $A_4$ et $Y_6$ étant tels que définis dans la revendication 3.

**8.** Composé selon la revendication 3, répondant à la formule (VII) suivante :

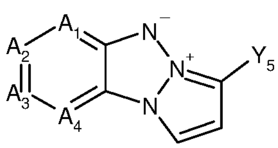

(VII)

$A_1$, $A_2$, $A_3$, $A_4$ et $Y_5$ étant tels que définis dans la revendication 3.

**9.** Composition aqueuse comprenant au moins un composé de formule (I) selon la revendication 3.

**10.** Composé de formule (I') suivante :

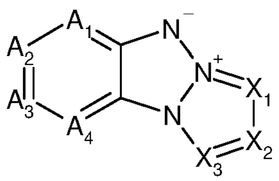

(I')

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, ($C_1$-$C_6$)alkyle, ($C_6$-$C_{10}$)aryle, hétéroaryle comprenant de 5 à 10 atomes, ($C_2$-$C_6$)alcényle, ($C_2$-$C_6$)alcynyle, CN, hydroxy, ($C_1$-$C_6$)alcoxy, thiol, ($C_1$-$C_6$)alkylthio, $(CH_2)_nSO_2$-$OR_a$ où n = 1-6, $CH_2SO_2$-$NR_aR_b$, $NO_2$, $SO_3R_a$, $NR_aR_b$, C(O)$OR_a$, C(O)$R_a$, et C(O)$NR_aR_b$, $R_a$ et $R_b$ représentant H ou un groupe ($C_1$-$C_6$)alkyle, ou peuvent représenter un groupe de formule -L-Z, L représentant un bras espaceur comprenant de 1 à 10 atomes de carbone et Z représentant un groupe réactif susceptible de se lier à une molécule biologique, choisi dans le groupe constitué des halogènes, des acides carboxyliques, des esters de succinimide, des esters de tétrafluorophényle, des azotures d'acyle, des anhydrides, des halogénures d'acide, des acrylamides, des alcools, des amines, des alcynes, des aminooxyacétamides, des azotures, des imidoesters, des esters de sulfonate, des halogéno-acétamides, des halogénures d'alkyle, des halogénures de sulfonyle, des hydrazines, des hydrazides, des isocyanates, des isothiocyanates, des tétrazines et des maléimides,

ou $Y_1$ et $Y_2$ forment ensemble, avec les atomes de carbone qui les portent, un groupe (hétéro)cycloalkyle ou un groupe (hétéro)aryle comprenant de 5 à 30 atomes,
et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$, représentent, indépendamment les uns des autres, un atome d'hydrogène ou un atome d'halogène, ou sont choisis dans le groupe constitué de :

. un groupe ($C_1$-$C_6$)alkyle, ($C_3$-$C_6$)cycloalkyle ou ($C_6$-$C_{10}$)aryle,
ledit groupe ($C_6$-$C_{10}$)aryle étant éventuellement substitué par au moins un substituant choisi parmi : halogène, CN, ($C_1$-$C_6$)alkyle, OH, ($C_1$-$C_6$)alcoxy, C(O)$OR_a$ et $NR_aR_b$, $R_a$ et $R_b$ représentant H ou un groupe ($C_1$-$C_6$)alkyle,
. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, ($C_1$-$C_6$)alkyle, OH, ($C_1$-$C_6$)alcoxy, C(O)$OR_a$, C(O)$R_a$ et $NR_aR_b$, $R_a$ et $R_b$ représentant H ou un groupe ($C_1$-$C_6$)alkyle,
. un groupe hétérocycloalkyle comprenant de 4 à 10 chaînons et contenant de un à trois hétéroatomes choisis parmi O, S ou N,
. $NO_2$,
. CHO,
. un groupe C(O)$OR_a$, $R_a$ étant tel que défini ci-dessus,
. un groupe -HC=CH-Ar, Ar représentant un groupe ($C_6$-$C_{10}$)aryle,
. $SO_3H$,
. un groupe $NR_cR_d$, $R_c$ et $R_d$ représentant H ou un groupe ($C_1$-$C_6$)alkyle, ou pouvant former un groupe hétéro($C_2$-$C_5$)cycloalkyle avec l'atome d'azote qui les porte ;
. un groupe $OR_e$, $R_e$ représentant H, un groupe ($C_1$-$C_6$)alkyle ou un groupe ($C_6$-$C_{10}$)aryle ; et

. un groupe de formule (A) suivante :

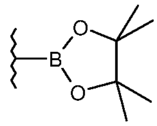

(A)

ou $Y_5$, $Y_6$ et $Y_7$ représentent un groupe de formule -L-Z, L et Z étant tels que définis ci-dessus, ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,
ledit composé de formule (I') pouvant être sous forme de sel ou de stéréoisomère pur ou sous forme de mélange d'énantiomères et/ou de diastéréoisomères, y compris de mélanges racémiques, ou sous forme de tautomères, dans laquelle l'un au moins des $Y_1$, $Y_2$, $Y_3$, $Y_4$, $Y_5$, $Y_6$ et $Y_7$ est un groupe de formule -L-Z,
à l'exception du composé de formule suivante :

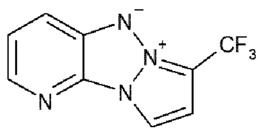

.

**11.** Conjugué comprenant une molécule biologique et un composé de formule (I) selon l'une quelconque des revendications 1 à 3, dans lequel ledit composé de formule (I) est lié à la molécule biologique par l'intermédiaire d'un linker, ladite molécule biologique étant choisie dans le groupe constitué des anticorps, des protéines, des peptides, des glucides, des lipides, des polysaccharides, des acides gras, des acides aminés, des acides désoxyribonucléiques, des acides ribonucléiques, des oligonucléotides, des médicaments et des ligands.

**12.** Conjugué selon la revendication 11, de formule (I") suivante :

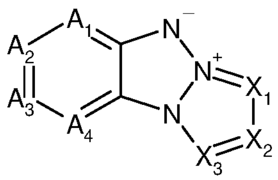

(I'')

dans laquelle :

- $A_1$ est -N- ou -C($Y_1$)- ;
- $A_2$ est -N- ou -C($Y_2$)- ;
- $A_3$ est -N- ou -C($Y_3$)- ;
- $A_4$ est -N- ou -C($Y_4$)- ;

l'un au moins des $A_1$, $A_2$, $A_3$ et $A_4$ représentant -N- ;

- $Y_1$, $Y_2$, $Y_3$ et $Y_4$ sont choisis indépendamment les uns des autres dans le groupe constitué de : H, halogène, ($C_1$-$C_6$)alkyle, ($C_6$-$C_{10}$)aryle, hétéroaryle comprenant de 5 à 10 atomes, ($C_2$-$C_6$)alcényle, ($C_2$-$C_6$)alcynyle, CN, hydroxy, ($C_1$-$C_6$)alcoxy, thiol, ($C_1$-$C_6$)alkylthio, $(CH_2)_nSO_2$-$OR_a$ où n = 1-6, $CH_2SO_2$-$NR_aR_b$, $NO_2$, $SO_3R_a$, $NR_aR_b$, $C(O)OR_a$, $C(O)R_a$, et $C(O)NR_aR_b$, $R_a$ et $R_b$ représentant H ou un groupe ($C_1$-$C_6$)alkyle,

ou peuvent représenter un groupe de formule -L'-Z', L' représentant un linker et Z' représentant une molécule biologique choisie dans le groupe constitué des anticorps, des protéines, des peptides, des glucides, des lipides, des polysaccharides, des acides gras, des acides aminés, des acides désoxyribonucléiques, des acides ribonucléiques et des oligonucléotides,
ou $Y_1$ et $Y_2$ forment ensemble, avec les atomes de carbone qui les portent, un groupe (hétéro)cycloalkyle ou un groupe (hétéro)aryle comprenant de 5 à 30 atomes,

et/ou $Y_2$ et $Y_3$ forment ensemble, avec les atomes de carbone qui les portent, un groupe (hétéro)cycloalkyle ou un groupe (hétéro)aryle comprenant de 5 à 30 atomes,
et/ou $Y_3$ et $Y_4$ forment ensemble, avec les atomes de carbone qui les portent, un groupe (hétéro)cycloalkyle ou un groupe (hétéro)aryle comprenant de 5 à 30 atomes,

- $X_1$ est -N- ou -C($Y_5$)- ;
- $X_2$ est -N- ou -C($Y_6$)- ;
- $X_3$ est -N- ou -C($Y_7$)- ;
- $Y_5$, $Y_6$ et $Y_7$, représentent, indépendamment les uns des autres, un atome d'hydrogène ou un atome d'halogène, ou sont choisis dans le groupe constitué de :

. un groupe ($C_1$-$C_6$)alkyle, ($C_3$-$C_6$)cycloalkyle ou ($C_6$-$C_{10}$)aryle,
ledit groupe ($C_6$-$C_{10}$)aryle étant éventuellement substitué par au moins un substituant choisi parmi : halogène, CN, ($C_1$-$C_6$)alkyle, OH, ($C_1$-$C_6$)alcoxy, C(O)$OR_a$ et $NR_aR_b$, $R_a$ et $R_b$ représentant H ou un groupe ($C_1$-$C_6$)alkyle,
. un groupe hétéroaryle comprenant de 5 à 10 chaînons et contenant de 1 à 4 hétéroatomes choisis parmi O, S ou N, ledit groupe hétéroaryle étant éventuellement substitué par au moins un substituant choisi parmi : halogène, CN, ($C_1$-$C_6$)alkyle, OH, ($C_1$-$C_6$)alcoxy, C(O)$OR_a$, C(O)$R_a$ et $NR_aR_b$, $R_a$ et $R_b$ représentant H ou un groupe ($C_1$-$C_6$)alkyle,
. un groupe hétérocycloalkyle comprenant de 4 à 10 chaînons et contenant de un à trois hétéroatomes choisis parmi O, S ou N,
. $NO_2$,
. CHO,
. un groupe C(O)$OR_a$, $R_a$ étant tel que défini ci-dessus,
. un groupe -HC=CH-Ar, Ar représentant un groupe ($C_6$-$C_{10}$)aryle,
. $SO_3H$,
. un groupe $NR_cR_d$, $R_c$ et $R_d$ représentant H ou un groupe ($C_1$-$C_6$)alkyle, ou pouvant former un groupe hétéro($C_2$-$C_5$)cycloalkyle avec l'atome d'azote qui les porte ;
. un groupe $OR_e$, $R_e$ représentant H, un groupe ($C_1$-$C_6$)alkyle ou un groupe ($C_6$-$C_{10}$)aryle ; et
. un groupe de formule (A) suivante :

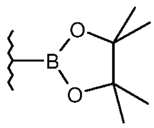

(A)

ou $Y_5$, $Y_6$ et $Y_7$ représentent un groupe de formule -L'-Z', L' et Z' étant tels que définis ci-dessus,
ou $Y_5$ et $Y_6$ forment ensemble, avec les atomes de carbone qui les portent, un groupe (hétéro)cycloalkyle ou un groupe (hétéro)aryle comprenant de 5 à 10 atomes,
et/ou $Y_6$ et $Y_7$ forment ensemble, avec les atomes de carbone qui les portent, un groupe (hétéro)cycloalkyle ou un groupe (hétéro)aryle comprenant de 5 à 10 atomes,
ledit composé de formule (I") pouvant être sous forme de sel ou de stéréoisomère pur ou sous forme de mélange d'énantiomères et/ou de diastéréoisomères, y compris de mélanges racémiques, ou sous forme de tautomères, dans laquelle l'un au moins des $Y_1$, $Y_2$, $Y_3$, $Y_4$, $Y_5$, $Y_6$ et $Y_7$ est un groupe de formule -L'-Z'.

**13.** Utilisation d'un conjugué selon la revendication 11 ou 12, pour la détection par fluorescence d'une molécule biologique.

**Patentansprüche**

**1.** Verwendung einer Verbindung von Formel (I) als fluoreszierendes Chromophor:

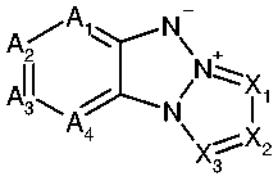

(I)

wobei:

- $A_1$ -N- oder -C($Y_1$)- ist;
- $A_2$ -N- oder -C($Y_2$)- ist;
- $A_3$ -N- oder -C($Y_3$)- ist;
- $A_4$ -N- oder -C($Y_4$)- ist;

wobei mindestens eines von $A_1$, $A_2$, $A_3$ und $A_4$ -N- darstellt;

- $Y_1$, $Y_2$, $Y_3$ und $Y_4$ unabhängig voneinander ausgewählt sind aus der Gruppe bestehend aus: H, Halogen, ($C_1$-$C_6$)-Alkyl, ($C_6$-$C_{10}$)-Aryl, Heteroaryl umfassend 5 bis 10 Atome, ($C_2$-$C_6$)-Alkenyl, ($C_2$-$C_6$)-Alkinyl, CN, Hydroxy, ($C_1$-$C_6$)-Alcoxy, Thiol, ($C_1$-$C_6$)-Alkylthio, $(CH_2)_nSO_2$-$OR_a$ wobei n = 1-6, $CH_2SO_2$-$NR_aR_b$, $NO_2$, $SO_3Ra$, $NR_aR_b$, $C(O)OR_a$, $C(O)R_a$ und $C(O)NR_aR_b$, wobei $R_a$ und $R_b$ H oder eine ($C_1$-$C_6$)-Alkylgruppe darstellen,

wobei $Y_1$ und $Y_2$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_2$ und $Y_3$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_3$ und $Y_4$ zusammen mit den Kohlenstoffatomen, die sie tragen, eine (Hetero)cycloalkylgruppe oder eine (Hetero)arylgruppe umfassend 5 bis 30 Atome bilden,

- $X_1$ -N- oder -C($Y_5$)- ist;
- $X_2$ -N- oder -C($Y_6$)- ist;
- $X_3$ -N- oder -C($Y_7$)- ist;
- $Y_5$, $Y_6$ und $Y_7$ unabhängig ein Wasserstoffatom oder ein Halogenatom darstellen oder ausgewählt sind aus der Gruppe, bestehend aus:

. einer ($C_1$-$C_6$)-Alkyl-, ($C_3$-$C_6$)-Cycloalkyl- oder ($C_6$-$C_{10}$)-Arylgruppe,
wobei die ($C_6$-$C_{10}$)-Arylgruppe optional mit mindestens einem Substituenten substituiert ist, der ausgewählt ist aus: Halogen, CN, ($C_1$-$C_6$)-Alkyl, OH, ($C_1$-$C_6$)-Alcoxy, $C(O)OR_a$ und $NR_aR_b$, wobei $R_a$ und $R_b$ H oder eine ($C_1$-$C_6$)-Alkylgruppe darstellen,
. einer Heteroarylgruppe, umfassend 5 bis 10 Glieder und die 1 bis 4 Heteroatome enthält, ausgewählt aus O, S oder N, wobei die Heteroarylgruppe optional mit mindestens einem Substituenten substituiert ist, ausgewählt aus: Halogen, CN, ($C_1$-$C_6$)alkyle, OH, ($C_1$-$C_6$)alcoxy, $C(O)OR_a$, $C(O)R_a$ und $NR_aR_b$, wobei $R_a$ und $R_b$ H oder eine ($C_1$-$C_6$)-Alkylgruppe darstellen,
. einer Heterocycloalkylgruppe, umfassend 4 bis 10 Glieder und die ein bis drei Heteroatome enthält, ausgewählt aus O, S oder N,
. $NO_2$,
. CHO,
. einer $C(O)OR_a$-Gruppe, wobei $R_a$ wie oben definiert ist,
. einer -HC=CH-Ar-Gruppe, wobei Ar eine ($C_6$-$C_{10}$)-Arylgruppe darstellt,
. $SO_3H$,
. einer $NR_cR_d$-Gruppe, wobei $R_e$ und $R_d$ H oder eine ($C_1$-$C_6$)-Alkylgruppe darstellen oder mit dem Stickstoffatom, das sie trägt, eine ($C_2$-$C_5$)-Heterocycloalkylgruppe bilden können;
. einer $OR_e$-Gruppe, wobei $R_e$ H, eine ($C_1$-$C_6$)-Alkylgruppe oder eine ($C_6$-$C_{10}$)-Arylgruppe darstellt; und
. einer Gruppe folgender Formel (A):

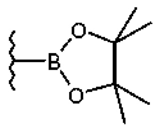

(A)

wobei $Y_5$ und $Y_6$ zusammen mit den Kohlenstoffatomen, die sie tragen, eine (Hetero)cycloalkylgruppe oder eine (Hetero)arylgruppe umfassend 5 bis 10 Atome bilden,
und/oder $Y_6$ und $Y_7$ zusammen mit den Kohlenstoffatomen, die sie tragen, eine (Hetero)cycloalkylgruppe oder eine (Hetero)arylgruppe umfassend 5 bis 10 Atome bilden,
wobei die Verbindung von Formel (I) in Form eines reinen Salzes oder Stereoisomers oder in Form eines Gemischs von Enantiomeren und/oder Diastereomeren, einschließlich racemischer Gemische, oder in Form von Tautomeren sein kann,
mit Ausnahme der Verbindung folgender Formel:

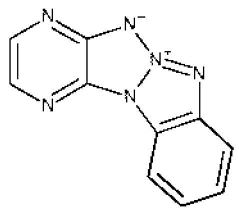

**2.** Verwendung nach Anspruch 1, wobei $A_1$ und $A_4$ -N- sind, $A_2$ -C($Y_2$)- ist und $A_3$ -C($Y_3$)- ist.

**3.** Verbindung von folgender Formel (I):

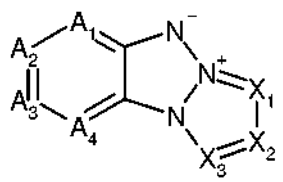

(I)

wobei:

- $A_1$ -N- oder -C($Y_1$)- ist;
- $A_2$ -N- oder -C($Y_2$)- ist;
- $A_3$ -N- oder -C($Y_3$)- ist;
- $A_4$ -N- oder -C($Y_4$)- ist;

wobei mindestens eines von $A_1$, $A_2$, $A_3$ und $A_4$ -N- darstellt;

- $Y_1$, $Y_2$, $Y_3$ und $Y_4$ unabhängig voneinander ausgewählt sind aus der Gruppe bestehend aus: H, Halogen, ($C_1$-$C_6$)-Alkyl, ($C_6$-$C_{10}$)-Aryl, Heteroaryl umfassend 5 bis 10 Atome, ($C_2$-$C_6$)-Alkenyl, ($C_2$-$C_6$)-Alkinyl, CN, Hydroxy, ($C_1$-$C_6$)-Alcoxy, Thiol, ($C_1$-$C_6$)-Alkylthio, $(CH_2)_nSO_2$-$OR_a$ wobei n = 1-6, $CH_2SO_2$-$NR_aR_b$, $NO_2$, $SO_3R_a$, $NR_aR_b$, C(O)$OR_a$, C(O)$R_a$ und C(O)$NR_aR_b$, wobei $R_a$ und $R_b$ H oder eine ($C_1$-$C_6$)-Alkylgruppe darstellen,

wobei $Y_1$ und $Y_2$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_2$ und $Y_3$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_3$ und $Y_4$ zusammen mit den Kohlenstoffatomen, die sie tragen, eine (Hetero)cycloalkylgruppe oder eine (Hetero)arylgruppe umfassend 5 bis 30 Atome bilden,

- $X_1$ -N- oder -C($Y_5$)- ist;
- $X_2$ -N- oder -C($Y_6$)- ist;
- $X_3$ -N- oder -C($Y_7$)- ist;

- $Y_5$, $Y_6$ und $Y_7$ unabhängig ein Wasserstoffatom oder ein Halogenatom darstellen oder ausgewählt sind aus der Gruppe, bestehend aus:

. einer ($C_1$-$C_6$)-Alkyl-, ($C_3$-$C_6$)-Cycloalkyl- oder ($C_6$-$C_{10}$)-Arylgruppe, wobei die ($C_6$-$C_{10}$)-Arylgruppe optional mit mindestens einem Substituenten substituiert ist, der ausgewählt ist aus: Halogen, CN, ($C_1$-$C_6$)-Alkyl, OH, ($C_1$-$C_6$)-Alcoxy, C(O)$OR_a$ und $NR_aR_b$, wobei $R_a$ und $R_b$ H oder eine ($C_1$-$C_6$)-Alkylgruppe darstellen,
. einer Heteroarylgruppe, umfassend 5 bis 10 Glieder und die 1 bis 4 Heteroatome enthält, ausgewählt aus O, S oder N, wobei die Heteroarylgruppe optional mit mindestens einem Substituenten substituiert ist, ausgewählt aus: Halogen, CN, ($C_1$-$C_6$)alkyle, OH, ($C_1$-$C_6$)alcoxy, C(O)$OR_a$, C(O)$R_a$ und $NR_aR_b$, wobei $R_a$ und $R_b$ H oder eine ($C_1$-$C_6$)-Alkylgruppe darstellen,
. einer Heterocycloalkylgruppe, umfassend 4 bis 10 Glieder und die ein bis drei Heteroatome enthält, ausgewählt aus O, S oder N,
. $NO_2$,
. CHO,
. einer C(O)$OR_a$-Gruppe, wobei $R_a$ wie oben definiert ist,
. einer -HC=CH-Ar-Gruppe, wobei Ar eine ($C_6$-$C_{10}$)-Arylgruppe darstellt,
. $SO_3H$,
. einer $NR_cR_d$-Gruppe, wobei $R_c$ und $R_d$ H oder eine ($C_1$-$C_6$)-Alkylgruppe darstellen oder mit dem Stickstoffatom, das sie trägt, eine ($C_2$-$C_5$)-Heterocycloalkylgruppe bilden können;
. einer $OR_e$-Gruppe, wobei $R_e$ H, eine ($C_1$-$C_6$)-Alkylgruppe oder eine ($C_6$-$C_{10}$)-Arylgruppe darstellt; und
. einer Gruppe folgender Formel (A):

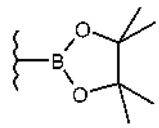

(A)

wobei die Verbindung von Formel (I) in Form eines reinen Salzes oder Stereoisomers oder in Form eines Gemischs von Enantiomeren und/oder Diastereomeren, einschließlich racemischer Gemische, oder in Form von Tautomeren sein kann,
mit Ausnahme der folgenden Verbindungen:

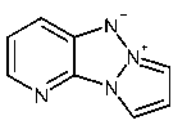
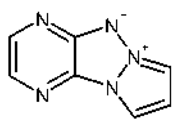
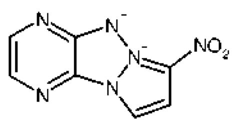
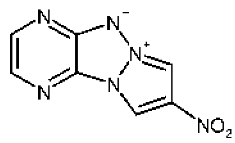

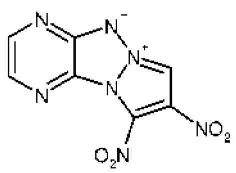
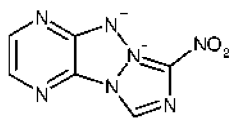
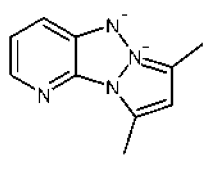
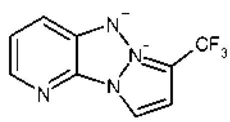

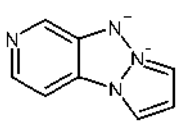
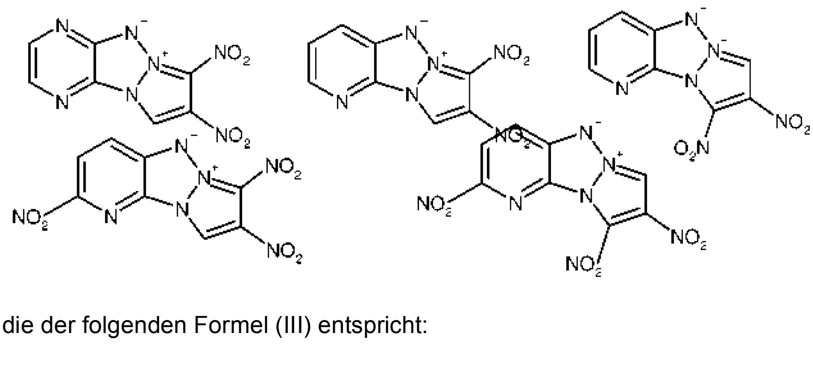

**4.** Verbindung nach Anspruch 3, die der folgenden Formel (III) entspricht:

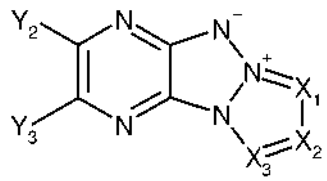

(III)

wobei $Y_2$, $Y_3$, $X_1$, $X_2$ und $X_3$ wie definiert in Anspruch 3 sind.

**5.** Verbindung nach Anspruch 3, die der folgenden Formel (III-1) entspricht:

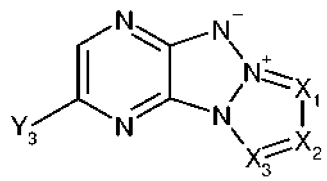

(III-1)

wobei $Y_3$, $X_1$, $X_2$ und $X_3$ wie definiert in Anspruch 3 sind.

**6.** Verbindung nach Anspruch 3, die der folqenden Formel (V-1) entspricht:

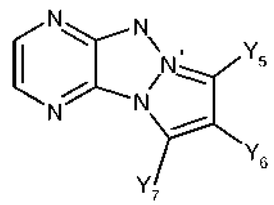

(V-1)

wobei $Y_5$, $Y_6$ und $Y_7$ wie definiert in Anspruch 3 sind.

**7.** Verbindung nach Anspruch 3, die der folgenden Formel (VI) entspricht:

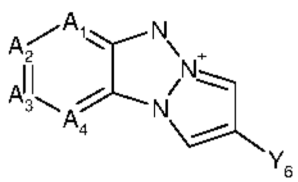

(VI)

wobei $A_1$, $A_2$, $A_3$, $A_4$ und $Y_6$ wie definiert in Anspruch 3 sind.

**8.** Verbindung nach Anspruch 3, die der folgenden Formel (VII) entspricht:

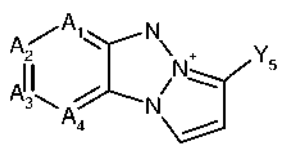

(VII)

wobei $A_1$, $A_2$, $A_3$, $A_4$ und $Y_5$ wie definiert in Anspruch 3 sind.

**9.** Wässrige Zusammensetzung, umfassend mindestens eine Verbindung von Formel (I) nach Anspruch 3.

**10.** Verbindung von folgender Formel (I):

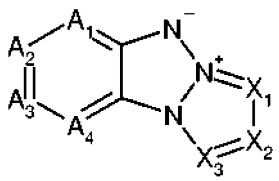

(I')

wobei:

- $A_1$ -N- oder -C($Y_1$)- ist;
- $A_2$ -N- oder -C($Y_2$)- ist;
- $A_3$ -N- oder -C($Y_3$)- ist;
- $A_4$ -N- oder -C($Y_4$)- ist;

wobei mindestens eines von $A_1$, $A_2$, $A_3$ und $A_4$ -N- darstellt;

- $Y_1$, $Y_2$, $Y_3$ und $Y_4$ unabhängig voneinander ausgewählt sind aus der Gruppe bestehend aus: H, Halogen, ($C_1$-$C_6$)-Alkyl, ($C_6$-$C_{10}$)-Aryl, Heteroaryl umfassend 5 bis 10 Atome, ($C_2$-$C_6$)-Alkenyl, ($C_2$-$C_6$)-Alkinyl, CN, Hydroxy, ($C_1$-$C_6$)-Alcoxy, Thiol, ($C_1$-$C_6$)-Alkylthio, $(CH_2)_nSO_2$-$OR_a$ wobei n = 1-6, $CH_2SO_2$-$NR_aR_b$, $NO_2$, $SO_3R_a$, $NR_aR_b$, C(O)$OR_a$, C(O)$R_a$ und C(O)$NR_aR_b$, wobei $R_a$ und $R_b$ H oder eine ($C_1$-$C_6$)-Alkylgruppe darstellen, oder eine Gruppe von Formel -L-Z darstellen können,

L einen Spacerarm umfassend 1 bis 10 Kohlenstoffatome darstellt und
Z eine reaktive Gruppe darstellt, die an ein biologisches Molekül binden kann, ausgewählt aus der Gruppe bestehend aus Halogenen, Carbonsäuren, Succinimid-Estern, Tetrafluorphenyl-Estern, Acyl-Aziden, Anhydriden, Säurehalogeniden, Acrylamiden, Alkoholen, Aminen, Alkinen, Aminooxyacetamiden, Aziden, Imidoestern, Sulfonatestern, Halogenacetamiden, Alkylhalogeniden, Sulfonylhalogeniden, Hydrazinen, Hydraziden, Isocyanaten, Isothiocyanaten, Tetrazinen und Maleimiden,

wobei $Y_1$ und $Y_2$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_2$ und $Y_3$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_3$ und $Y_4$ zusammen mit den Kohlenstoffatomen, die sie tragen, eine (Hetero)cycloalkylgruppe oder eine (Hetero)arylgruppe umfassend 5 bis 30 Atome bilden,

- $X_1$ -N- oder -C($Y_5$)- ist;
- $X_2$ -N- oder -C($Y_6$)- ist;
- $X_3$ -N- oder -C($Y_7$)- ist;
- $Y_5$, $Y_6$ und $Y_7$ unabhängig ein Wasserstoffatom oder ein Halogenatom darstellen oder ausgewählt sind aus der Gruppe, bestehend aus:

. einer ($C_1$-$C_6$)-Alkyl-, ($C_3$-$C_6$)-Cycloalkyl- oder ($C_6$-$C_{10}$)-Arylgruppe,
wobei die ($C_6$-$C_{10}$)-Arylgruppe optional mit mindestens einem Substituenten substituiert ist, der ausgewählt ist aus: Halogen, CN, ($C_1$-$C_6$)-Alkyl, OH, ($C_1$-$C_6$)-Alcoxy, C(O)$OR_a$ und $NR_aR_b$, wobei $R_a$ und $R_b$ H oder eine ($C_1$-$C_6$)-Alkylgruppe darstellen,
. einer Heteroarylgruppe, umfassend 5 bis 10 Glieder und die 1 bis 4 Heteroatome enthält, ausgewählt aus O, S oder N, wobei die Heteroarylgruppe optional mit mindestens einem Substituenten substituiert ist, ausgewählt aus: Halogen, CN, ($C_1$-$C_6$)alkyle, OH, ($C_1$-$C_6$)alcoxy, C(O)$OR_a$, C(O)$R_a$ und $NR_aR_b$, wobei $R_a$ und $R_b$ H oder eine ($C_1$-$C_6$)-Alkylgruppe darstellen,
. einer Heterocycloalkylgruppe, umfassend 4 bis 10 Glieder und die ein bis drei Heteroatome enthält, ausgewählt aus O, S oder N,
. $NO_2$,
. CHO,
. einer C(O)$OR_a$-Gruppe, wobei $R_a$ wie oben definiert ist,
. einer -HC=CH-Ar-Gruppe, wobei Ar eine ($C_6$-$C_{10}$)-Arylgruppe darstellt,
. $SO_3H$,
. einer $NR_cR_d$-Gruppe, wobei $R_c$ und $R_d$ H oder eine ($C_1$-$C_6$)-Alkylgruppe darstellen oder mit dem Stickstoffatom, das sie trägt, eine ($C_2$-$C_5$)-Heterocycloalkylgruppe bilden können;

. einer $OR_e$-Gruppe, wobei $R_e$ H, eine $(C_1\text{-}C_6)$-Alkylgruppe oder eine $(C_6\text{-}C_{10})$-Arylgruppe darstellt; und
. einer Gruppe folgender Formel (A):

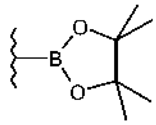

(A)

wobei $Y_5$, $Y_6$ und $Y_7$ eine Gruppe von Formel -L-Z darstellen, wobei L und Z wie oben definiert sind, wobei $Y_5$ und $Y_6$ zusammen mit den Kohlenstoffatomen, die sie tragen, eine (Hetero)cycloalkylgruppe oder eine (Hetero)arylgruppe umfassend 5 bis 10 Atome bilden,
und/oder $Y_6$ und $Y_7$ zusammen mit den Kohlenstoffatomen, die sie tragen, eine (Hetero)cycloalkylgruppe oder eine (Hetero)arylgruppe umfassend 5 bis 10 Atome bilden,
wobei die Verbindung von Formel (I') in Form eines reinen Salzes oder Stereoisomers oder in Form eines Gemischs von Enantiomeren und/oder Diastereomeren, einschließlich racemischer Gemische, oder in Form von Tautomeren sein kann,
wobei mindestens eines von $Y_1$, $Y_2$, $Y_3$, $Y_4$, $Y_5$, $Y_6$ und $Y_7$ eine Gruppe von Formel -L-Z ist,
mit Ausnahme der Verbindung folgender Formel:

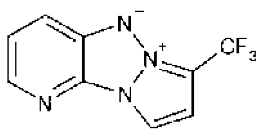

.

**11.** Konjugat, umfassend ein biologisches Molekül und eine Verbindung von Formel (I) nach einem der Ansprüche 1 bis 3, wobei die Verbindung von Formel (I) über einen Linker an das biologische Molekül gebunden ist, wobei das biologische Molekül ausgewählt ist aus der Gruppe, bestehend aus Antikörpern, Proteinen, Peptiden, Kohlenhydraten, Lipiden, Polysacchariden, Fettsäuren, Aminosäuren, Desoxyribonukleinsäuren, Ribonukleinsäuren, Oligonukleotiden, Arzneimitteln und Liganden.

**12.** Konjugat nach Anspruch 11 von folgender Formel (I"):

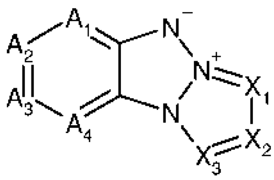

(I")

wobei:

- $A_1$ -N- oder -C($Y_1$)- ist;
- $A_2$ -N- oder -C($Y_2$)- ist;
- $A_3$ -N- oder -C($Y_3$)- ist;
- $A_4$ -N- oder -C($Y_4$)- ist;

wobei mindestens eines von $A_1$, $A_2$, $A_3$ und $A_4$ -N- darstellt;

- $Y_1$, $Y_2$, $Y_3$ und $Y_4$ unabhängig voneinander ausgewählt sind aus der Gruppe bestehend aus: H, Halogen, $(C_1\text{-}C_6)$-Alkyl, $(C_6\text{-}C_{10})$-Aryl, Heteroaryl umfassend 5 bis 10 Atome, $(C_2\text{-}C_6)$-Alkenyl, $(C_2\text{-}C_6)$-Alkinyl, CN, Hydroxy, $(C_1\text{-}C_6)$-Alcoxy, Thiol, $(C_1\text{-}C_6)$-Alkylthio, $(CH_2)_nSO_2\text{-}OR_a$ wobei n = 1-6, $CH_2SO_2\text{-}NR_aR_b$, $NO_2$, SOsRa, $NR_aR_b$, $C(O)OR_a$, $C(O)R_a$ und $C(O)NR_aR_b$, wobei $R_a$ und $R_b$ H oder eine $(C_1\text{-}C_6)$-Alkylgruppe darstellen,

oder eine Gruppe der Formel -L'-Z' darstellen können, wobei L' einen Linker darstellt und Z' ein biologisches Molekül darstellt, das ausgewählt ist aus der Gruppe, bestehend aus Antikörpern, Proteinen, Peptiden, Kohlenhydraten, Lipiden, Polysacchariden, Fettsäuren, Aminosäuren, Desoxyribonukleinsäuren, Ribonukleinsäu-

ren und Oligonukleotiden,
wobei $Y_1$ und $Y_2$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_2$ und $Y_3$ zusammen mit den Kohlenstoffatomen, die sie tragen, eine (Hetero)cycloalkylgruppe oder eine (Hetero)arylgruppe umfassend 5 bis 30 Atome bilden,
und/oder $Y_3$ und $Y_4$ zusammen mit den Kohlenstoffatomen, die sie tragen, eine (Hetero)cycloalkylgruppe oder eine (Hetero)arylgruppe umfassend 5 bis 30 Atome bilden,

- $X_1$ -N- oder -C($Y_5$)- ist;
- $X_2$ -N- oder -C($Y_6$)- ist;
- $X_3$ -N- oder -C($Y_7$)- ist;
- $Y_5$, $Y_6$ und $Y_7$ unabhängig ein Wasserstoffatom oder ein Halogenatom darstellen oder ausgewählt sind aus der Gruppe, bestehend aus:

. einer ($C_1$-$C_6$)-Alkyl-, ($C_3$-$C_6$)-Cycloalkyl- oder ($C_6$-$C_{10}$)-Arylgruppe,
wobei die ($C_6$-$C_{10}$)-Arylgruppe optional mit mindestens einem Substituenten substituiert ist, der ausgewählt ist aus: Halogen, CN, ($C_1$-$C_6$)-Alkyl, OH, ($C_1$-$C_6$)-Alcoxy, C(O)OR$_a$ und NR$_a$R$_b$, wobei $R_a$ und $R_b$ H oder eine ($C_1$-$C_6$)-Alkylgruppe darstellen,
. einer Heteroarylgruppe, umfassend 5 bis 10 Glieder und die 1 bis 4 Heteroatome enthält, ausgewählt aus O, S oder N, wobei die Heteroarylgruppe optional mit mindestens einem Substituenten substituiert ist, ausgewählt aus: Halogen, CN, ($C_1$-$C_6$)alkyle, OH, ($C_1$-$C_6$)alcoxy, C(O)OR$_a$, C(O)R$_a$ und NR$_a$R$_b$, wobei $R_a$ und $R_b$ H oder eine ($C_1$-$C_6$)-Alkylgruppe darstellen,
. einer Heterocycloalkylgruppe, umfassend 4 bis 10 Glieder und die ein bis drei Heteroatome enthält, ausgewählt aus O, S oder N,
. $NO_2$,
. CHO,
. einer C(O)OR$_a$-Gruppe, wobei $R_a$ wie oben definiert ist,
. einer -HC=CH-Ar-Gruppe, wobei Ar eine ($C_6$-$C_{10}$)-Arylgruppe darstellt,
. $SO_3H$,
. einer NR$_c$R$_d$-Gruppe, wobei $R_e$ und $R_d$ H oder eine ($C_1$-$C_6$)-Alkylgruppe darstellen oder mit dem Stickstoffatom, das sie trägt, eine ($C_2$-$C_5$)-Heterocycloalkylgruppe bilden können;
. einer OR$_e$-Gruppe, wobei $R_e$ H, eine ($C_1$-$C_6$)-Alkylgruppe oder eine ($C_6$-$C_{10}$)-Arylgruppe darstellt; und
. einer Gruppe folgender Formel (A):

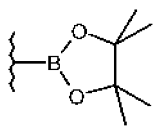
(A)

wobei $Y_5$, $Y_6$ und $Y_7$ eine Gruppe von Formel -L'-Z' darstellen, wobei L' und Z' wie oben definiert sind,
wobei $Y_5$ und $Y_6$ zusammen mit den Kohlenstoffatomen, die sie tragen, eine (Hetero)cycloalkylgruppe oder eine (Hetero)arylgruppe umfassend 5 bis 10 Atome bilden,
und/oder $Y_6$ und $Y_7$ zusammen mit den Kohlenstoffatomen, die sie tragen, eine (Hetero)cycloalkylgruppe oder eine (Hetero)arylgruppe umfassend 5 bis 10 Atome bilden,
wobei die Verbindung von Formel (I") in Form eines reinen Salzes oder Stereoisomers oder in Form eines Gemischs von Enantiomeren und/oder Diastereomeren, einschließlich racemischer Gemische, oder in Form von Tautomeren sein kann,
wobei mindestens eines von $Y_1$, $Y_2$, $Y_3$, $Y_4$, $Y_5$, $Y_6$ und $Y_7$ eine Gruppe von Formel -L'-Z' ist.

**13.** Verwendung eines Konjugats nach Anspruch 11 oder 12 zur Fluoreszenzdetektion eines biologischen Moleküls.

## Claims

**1.** A use, as a fluorescent chromophore, of a compound with formula (I): wherein:

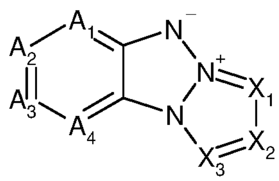

(I)

- $A_1$ is -N- or -C($Y_1$)-;
- $A_2$ is -N- or -C($Y_2$)-;
- $A_3$ is -N- or -C($Y_3$)-;
- $A_4$ is -N- or -C($Y_4$)-;

at least one of $A_1$, $A_2$, $A_3$ and $A_4$ representing -N-;

- $Y_1$, $Y_2$, $Y_3$ and $Y_4$ are chosen independently of one another in the group consisting of: H, halogen, ($C_1$-$C_6$)alkyl, ($C_6$-$C_{10}$)aryl, heteroaryl comprising from 5 to 10 atoms, ($C_2$-$C_6$)alkenyl, ($C_2$-$C_6$)alkynyl, CN, hydroxy, ($C_1$-$C_6$)alcoxy, thiol, ($C_1$-$C_6$)alkylthio, $(CH_2)_nSO_2$-$OR_a$ where n = 1-6, $CH_2SO_2$-$NR_aR_b$, $NO_2$, SOs-Ra, $NR_aR_b$, C(O)$OR_a$, C(O)$R_a$, and C(O)$NR_aR_b$, $R_a$ and $R_b$ representing H or a ($C_1$-$C_6$)alkyl group,

where $Y_1$ and $Y_2$ together form, with the carbon atoms that carry them, a (hetero)cycloalkyl group or a (hetero)aryl group comprising from 5 to 30 atoms,
and/or $Y_2$ and $Y_3$ together form, with the carbon atoms that carry them, a (hetero)cycloalkyl group or a (hetero)aryl group comprising from 5 to 30 atoms,
and/or $Y_3$ and $Y_4$ together form, with the carbon atoms that carry them, a (hetero)cycloalkyl group or a (hetero)aryl group comprising from 5 to 30 atoms,

- $X_1$ is -N- or -C($Y_5$)-;
- $X_2$ is -N- or -C($Y_6$)-;
- $X_3$ is -N- or -C($Y_7$)-;
- $Y_5$, $Y_6$ and $Y_7$ represent, independently of one another, a hydrogen atom or a halogen atom, or are chosen from the group consisting of:

. a ($C_1$-$C_6$)alkyl, ($C_3$-$C_6$)cycloalkyl or ($C_6$-$C_{10}$)aryl group,
said ($C_6$-$C_{10}$)aryl group optionally being substituted by at least one substituent chosen from among: halogen, CN, ($C_1$-$C_6$)alkyl, OH, ($C_1$-$C_6$)alkoxy, C(O)$OR_a$ and $NR_aR_b$, $R_a$ and $R_b$ representing H or a ($C_1$-$C_6$)alkyl group,
. a heteroaryl group comprising from 5 to 10 chain links and containing from 1 to 4 heteroatoms chosen from among O, S or N, said heteroaryl group optionally being substituted by at least one substituent chosen from among: halogen, CN, ($C_1$-$C_6$)alkyl, OH, ($C_1$-$C_6$)alkoxy, C(O)$OR_a$ and $NR_aR_b$, $R_a$ and $R_b$ representing H or a ($C_1$-$C_6$)alkyl group,
. a heterocycloalkyl group comprising from 4 to 10 chain links and containing from one to three heteroatoms chosen from among O, S or N,
. $NO_2$,
. CHO,
. a C(O)$OR_a$ group, $R_a$ being as defined above,
. a -HC=CH-Ar group, Ar representing a ($C_6$-$C_{10}$)aryl group,
. $SO_3H$,
. a $NR_cR_d$ group, $R_c$ and $R_d$ representing H or a ($C_1$-$C_6$)alkyl group, or being able to form a hetero($C_2$-$C_5$)cycloalkyl group with the nitrogen atom that carries them;
. an $OR_e$ group, $R_e$ representing H, a ($C_1$-$C_6$)alkyl group or a ($C_6$-$C_{10}$)aryl group; and
. a group with the following formula (A):

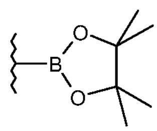

(A)

where $Y_5$ and $Y_6$ together form, with the carbon atoms that carry them, a (hetero)cycloalkyl group or a (hetero)aryl

group comprising from 5 to 10 atoms,

and/or $Y_6$ and $Y_7$ together form, with the carbon atoms that carry them, a (hetero)cycloalkyl group or a (hetero)aryl group comprising from 5 to 10 atoms,

said compound with formula (I) being able to be in salt or pure stereoisomer form or in the form of a mixture of enantiomers and/or diastereoisomers, including racemic mixtures, or in the form of tautomers,

with the exception of the compound with formula:

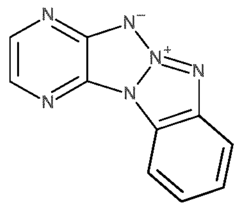

2. The use according to claim 1, wherein $A_1$ and $A_4$ are -N-, $A_2$ is -C($Y_2$)- and $A_3$ is -C($Y_3$)-.

3. A compound with the following formula (I):

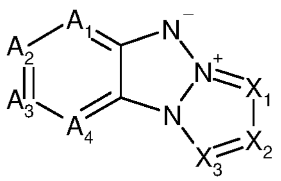

(I)

wherein:

- $A_1$ is -N- or -C($Y_1$)-;
- $A_2$ is -N- or -C($Y_2$)-;
- $A_3$ is -N- or -C($Y_3$)-;
- $A_4$ is -N- or -C($Y_4$)-;

at least one of $A_1$, $A_2$, $A_3$ and $A_4$ representing -N-;

- $Y_1$, $Y_2$, $Y_3$ and $Y_4$ are chosen independently of one another in the group consisting of: H, halogen, ($C_1$-$C_6$)alkyl, ($C_6$-$C_{10}$)aryl, heteroaryl comprising from 5 to 10 atoms, ($C_2$-$C_6$)alkenyl, ($C_2$-$C_6$)alkynyl, CN, hydroxy, ($C_1$-$C_6$)alcoxy, thiol, ($C_1$-$C_6$)alkylthio, $(CH_2)_nSO_2$-$OR_a$ where n = 1-6, $CH_2SO_2$-$NR_aR_b$, $NO_2$, SOs-Ra, $NR_aR_b$, C(O)$OR_a$, C(O)$R_a$, and C(O)$NR_aR_b$, $R_a$ and $R_b$ representing H or a ($C_1$-$C_6$)alkyl group,

where $Y_1$ and $Y_2$ together form, with the carbon atoms that carry them, a (hetero)cycloalkyl group or a (hetero)aryl group comprising from 5 to 30 atoms,

and/or $Y_2$ and $Y_3$ together form, with the carbon atoms that carry them, a (hetero)cycloalkyl group or a (hetero)aryl group comprising from 5 to 30 atoms,

and/or $Y_3$ and $Y_4$ together form, with the carbon atoms that carry them, a (hetero)cycloalkyl group or a (hetero)aryl group comprising from 5 to 30 atoms,

- $X_1$ is -N- or -C($Y_5$)-;
- $X_2$ is -N- or -C($Y_6$)-;
- $X_3$ is -N- or -C($Y_7$)-;
- $Y_5$, $Y_6$ and $Y_7$ represent, independently of one another, a hydrogen atom or a halogen atom, or are chosen from the group consisting of:

. a ($C_1$-$C_6$)alkyl, ($C_3$-$C_6$)cycloalkyl or ($C_6$-$C_{10}$)aryl group,

said ($C_6$-$C_{10}$)aryl group optionally being substituted by at least one substituent chosen from among: halogen, CN, ($C_1$-$C_6$)alkyl, OH, ($C_1$-$C_6$)alkoxy, C(O)$OR_a$ and $NR_aR_b$, $R_a$ and $R_b$ representing H or a ($C_1$-$C_6$)alkyl group,

. a heteroaryl group comprising from 5 to 10 chain links and containing from 1 to 4 heteroatoms chosen from among O, S or N, said heteroaryl group optionally being substituted by at least one substituent

chosen from among: halogen, CN, $(C_1-C_6)$alkyl, OH, $(C_1-C_6)$alkoxy, $C(O)OR_a$ and $NR_aR_b$, $R_a$ and $R_b$ representing H or a $(C_1-C_6)$alkyl group,

. a heterocycloalkyl group comprising from 4 to 10 chain links and containing from one to three heteroatoms chosen from among O, S or N,

. $NO_2$,

. CHO,

. a $C(O)OR_a$ group, $R_a$ being as defined above,

. a -HC=CH-Ar group, Ar representing a $(C_6-C_{10})$aryl group,

. $SO_3H$,

. a $NR_cR_d$ group, $R_c$ and $R_d$ representing H or a $(C_1-C_6)$alkyl group, or being able to form a hetero$(C_2-C_5)$cycloalkyl group with the nitrogen atom that carries them;

. an $OR_e$ group, $R_e$ representing H, a $(C_1-C_6)$alkyl group or a $(C_6-C_{10})$aryl group; and

. a group with the following formula (A):

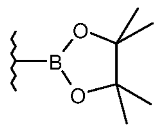

(A)

said compound with formula (I) being able to be in salt or pure stereoisomer form or in the form of a mixture of enantiomers and/or diastereoisomers, including racemic mixtures, or in the form of tautomers,
with the exception of the following compounds:

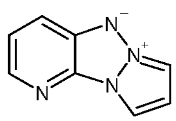
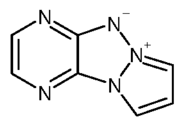
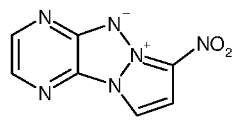
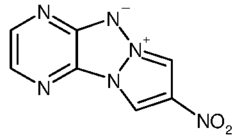
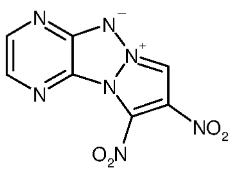
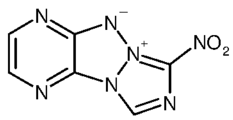
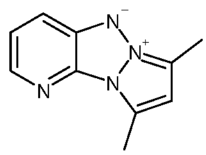
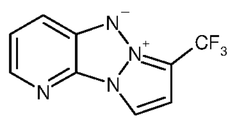
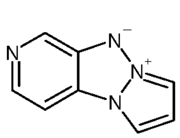
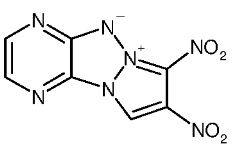
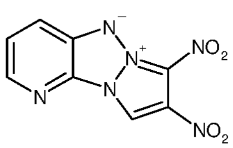
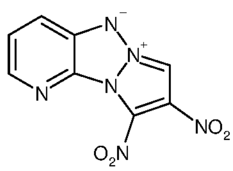
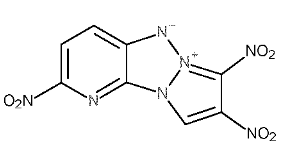
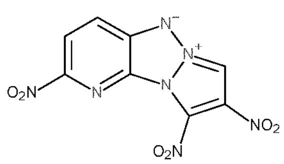

**4.** The compound of claim 3, having the following formula (III):

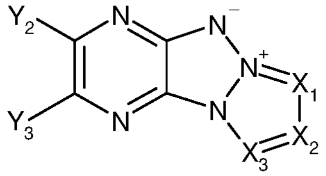

(III)

$Y_2$, $Y_3$, $X_1$, $X_2$ and $X_3$ being as defined in claim 3.

**5.** The compound of claim 3, having the following formula (III-1):

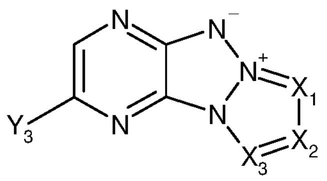

(III-1)

$Y_3$, $X_1$, $X_2$ and $X_3$ being as defined in claim 3.

**6.** The compound of claim 3, having the following formula (V-1):

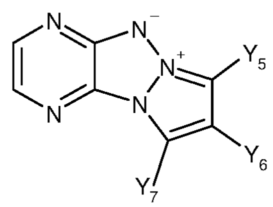

(V-1)

$Y_5$, $Y_6$ and $Y_7$ being as defined in claim 3.

**7.** The compound of claim 3, having the following formula (VI):

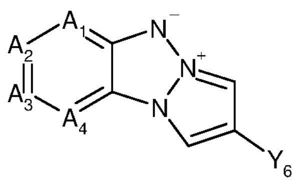

(VI)

$A_1$, $A_2$, $A_3$, $A_4$ and $Y_6$ being as defined in claim 3.

**8.** The compound of claim 3, having the following formula (VII):

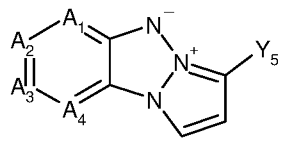

(VII)

$A_1$, $A_2$, $A_3$, $A_4$ and $Y_5$ being as defined in claim 3.

**9.** An aqueous composition comprising at least one compound with formula (I) according to claim 3.

**10.** A compound with the following formula (I'):

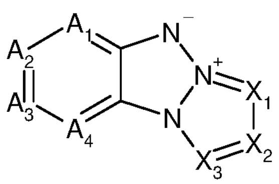

(I')

wherein:

- $A_1$ is -N- or -C($Y_1$)-;
- $A_2$ is -N- or -C($Y_2$)-;
- $A_3$ is -N- or -C($Y_3$)-;
- $A_4$ is -N- or -C($Y_4$)-;

at least one of $A_1$, $A_2$, $A_3$ and $A_4$ representing -N-;

- $Y_1$, $Y_2$, $Y_3$ and $Y_4$ are chosen independently of one another in the group consisting of: H, halogen, ($C_1$-$C_6$)alkyl, ($C_6$-$C_{10}$)aryl, heteroaryl comprising from 5 to 10 atoms, ($C_2$-$C_6$)alkenyl, ($C_2$-$C_6$)alkynyl, CN, hydroxy, ($C_1$-$C_6$)alcoxy, thiol, ($C_1$-$C_6$)alkylthio, $(CH_2)_nSO_2$-$OR_a$ where n = 1-6, $CH_2SO_2$-$NR_aR_b$, $NO_2$, SOs-Ra, $NR_aR_b$, C(O)$OR_a$, C(O)$R_a$, and C(O)$NR_aR_b$, $R_a$ and $R_b$ representing H or a ($C_1$-$C_6$)alkyl group, or may represent a group of formula -L-Z,

  L representing a spacer link comprising from 1 to 10 carbon atoms and
  Z representing a reactive group able to link to a biologic molecule, chosen from the group consisting of: halogens, carboxylic acids, succinimide esters, tetrafluorophenyl esters, acyl azides, anhydrides, acid halogenides, acrylamides, alcohols, amines, alkynes, aminooxyacetamides, azides, imidoesters, sulfonate esters, halogeno-acetamides, alkyl halogenides, sulfonyl halogenides, hydrazines, hydrazides, isocyanates, isothiocyanates, tetrazines and maleimides,

where $Y_1$ and $Y_2$ together form, with the carbon atoms that carry them, a (hetero)cycloalkyl group or a (hetero)aryl group comprising from 5 to 30 atoms,
and/or $Y_2$ and $Y_3$ together form, with the carbon atoms that carry them, a (hetero)cycloalkyl group or a (hetero)aryl group comprising from 5 to 30 atoms,
and/or $Y_3$ and $Y_4$ together form, with the carbon atoms that carry them, a (hetero)cycloalkyl group or a (hetero)aryl group comprising from 5 to 30 atoms,

- $X_1$ is -N- or -C($Y_5$)-;
- $X_2$ is -N- or -C($Y_6$)-;
- $X_3$ is -N- or -C($Y_7$)-;
- $Y_5$, $Y_6$ and $Y_7$ represent, independently of one another, a hydrogen atom or a halogen atom, or are chosen from the group consisting of:

  . a ($C_1$-$C_6$)alkyl, ($C_3$-$C_6$)cycloalkyl or ($C_6$-$C_{10}$)aryl group,
  said ($C_6$-$C_{10}$)aryl group optionally being substituted by at least one substituent chosen from among: halogen, CN, ($C_1$-$C_6$)alkyl, OH, ($C_1$-$C_6$)alkoxy, C(O)$OR_a$ and $NR_aR_b$, $R_a$ and $R_b$ representing H or a ($C_1$-$C_6$)alkyl group,
  . a heteroaryl group comprising from 5 to 10 chain links and containing from 1 to 4 heteroatoms chosen from among O, S or N, said heteroaryl group optionally being substituted by at least one substituent chosen from among: halogen, CN, ($C_1$-$C_6$)alkyl, OH, ($C_1$-$C_6$)alkoxy, C(O)$OR_a$ and $NR_aR_b$, $R_a$ and $R_b$ representing H or a ($C_1$-$C_6$)alkyl group,
  . a heterocycloalkyl group comprising from 4 to 10 chain links and containing from one to three heteroatoms chosen from among O, S or N,
  . $NO_2$,
  . CHO,
  . a C(O)$OR_a$ group, $R_a$ being as defined above,
  . a -HC=CH-Ar group, Ar representing a ($C_6$-$C_{10}$)aryl group,
  . $SO_3H$,
  . a $NR_cR_d$ group, $R_c$ and $R_d$ representing H or a ($C_1$-$C_6$)alkyl group, or being able to form a hetero($C_2$-$C_5$)cycloalkyl group with the nitrogen atom that carries them;
  . an $OR_e$ group, $R_e$ representing H, a ($C_1$-$C_6$)alkyl group or a ($C_6$-$C_{10}$)aryl group; and
  . a group with the following formula (A):

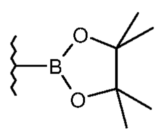

(A)

or $Y_5$, $Y_6$ and $Y_7$ represent a group of formula -L-Z, L and Z being as defined above,
or $Y_5$ and $Y_6$ together form, with the carbon atoms that carry them, a (hetero)cycloalkyl group or a (hetero)aryl group comprising from 5 to 10 atoms,
and/or $Y_6$ and $Y_7$ together form, with the carbon atoms that carry them, a (hetero)cycloalkyl group or a (hetero)aryl group comprising from 5 to 10 atoms,
said compound with formula (I') being able to be in salt or pure stereoisomer form or in the form of a mixture of enantiomers and/or diastereoisomers, including racemic mixtures, or in the form of tautomers,
wherein at least one of $Y_1$, $Y_2$, $Y_3$, $Y_4$, $Y_5$, $Y_6$ and $Y_7$ is a group with formula -L-Z,
with the exception of the following compound:

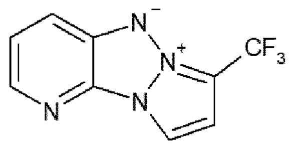

**11.** A conjugate comprising a biological molecule and a compound with formula (I) according to any one of claims 1 to 3, in which said compound with formula (I) is linked to the biological molecule via a linker, said biological molecule being chosen from the group consisting of antibodies, proteins, peptides, carbohydrates, lipids, polysaccharides, fatty acids, amino acids, deoxyribonucleic acids, ribonucleic acids, oligonucleotides, medicinal drugs and ligands.

**12.** The conjugate according to claim 11, with the following formula (I"):

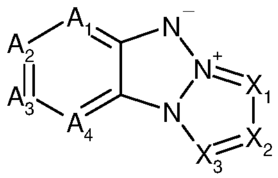

(I'')

wherein:

- $A_1$ is -N- or -C($Y_1$)-;
- $A_2$ is -N- or -C($Y_2$)-;
- $A_3$ is -N- or -C($Y_3$)-;
- $A_4$ is -N- or -C($Y_4$)-;

at least one of $A_1$, $A_2$, $A_3$ and $A_4$ representing -N-;

- $Y_1$, $Y_2$, $Y_3$ and $Y_4$ are chosen independently of one another in the group consisting of: H, halogen, ($C_1$-$C_6$)alkyl, ($C_6$-$C_{10}$)aryl, heteroaryl comprising from 5 to 10 atoms, ($C_2$-$C_6$)alkenyl, ($C_2$-$C_6$)alkynyl, CN, hydroxy, ($C_1$-$C_6$)alcoxy, thiol, ($C_1$-$C_6$)alkylthio, $(CH_2)_nSO_2$-$OR_a$ where n = 1-6, $CH_2SO_2$-$NR_aR_b$, $NO_2$, $SO_3R_a$, $NR_aR_b$, C(O)$OR_a$, C(O)$R_a$, and C(O)$NR_aR_b$, $R_a$ and $R_b$ representing H or a ($C_1$-$C_6$)alkyl group,

or can represent a group with formula -L'-Z', L' representing a linker and Z' representing a biological molecule chosen from the group consisting of antibodies, proteins, peptides, carbohydrates, lipids, polysaccharides, fatty acids, amino acids, deoxyribonucleic acids, ribonucleic acids, oligonucleotides, medicinal drugs and ligands,
where $Y_1$ and $Y_2$ together form, with the carbon atoms that carry them, a (hetero)cycloalkyl group or a (hetero)aryl group comprising from 5 to 30 atoms,
and/or $Y_2$ and $Y_3$ together form, with the carbon atoms that carry them, a (hetero)cycloalkyl group or a (hetero)aryl group comprising from 5 to 30 atoms,
and/or $Y_3$ and $Y_4$ together form, with the carbon atoms that carry them, a (hetero)cycloalkyl group or a (hetero)aryl group comprising from 5 to 30 atoms,

- $X_1$ is -N- or -C($Y_5$)-;
- $X_2$ is -N- or -C($Y_6$)-;
- $X_3$ is -N- or -C($Y_7$)-;
- $Y_5$, $Y_6$ and $Y_7$ represent, independently of one another, a hydrogen atom or a halogen atom, or are chosen from the group consisting of:

. a $(C_1-C_6)$alkyl, $(C_3-C_6)$cycloalkyl or $(C_6-C_{10})$aryl group,
said $(C_6-C_{10})$aryl group optionally being substituted by at least one substituent chosen from among: halogen, CN, $(C_1-C_6)$alkyl, OH, $(C_1-C_6)$alkoxy, $C(O)OR_a$ and $NR_aR_b$, $R_a$ and $R_b$ representing H or a $(C_1-C_6)$alkyl group,
. a heteroaryl group comprising from 5 to 10 chain links and containing from 1 to 4 heteroatoms chosen from among O, S or N, said heteroaryl group optionally being substituted by at least one substituent chosen from among: halogen, CN, $(C_1-C_6)$alkyl, OH, $(C_1-C_6)$alkoxy, $C(O)OR_a$ and $NR_aR_b$, $R_a$ and $R_b$ representing H or a $(C_1-C_6)$alkyl group,
. a heterocycloalkyl group comprising from 4 to 10 chain links and containing from one to three heteroatoms chosen from among O, S or N,
. $NO_2$,
. CHO,
. a $C(O)OR_a$ group, $R_a$ being as defined above,
. a -HC=CH-Ar group, Ar representing a $(C_6-C_{10})$aryl group,
. $SO_3H$,
. a $NR_cR_d$ group, $R_c$ and $R_d$ representing H or a $(C_1-C_6)$alkyl group, or being able to form a hetero$(C_2-C_5)$cycloalkyl group with the nitrogen atom that carries them;
. an $OR_e$ group, $R_e$ representing H, a $(C_1-C_6)$alkyl group or a $(C_6-C_{10})$aryl group; and
. a group with the following formula (A):

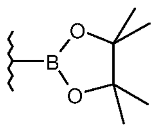

(A)

or $Y_5$, $Y_6$ and $Y_7$ represent a group with formula -L'-Z', L' and Z' being as defined above,
or $Y_5$ and $Y_6$ together form, with the carbon atoms that carry them, a (hetero)cycloalkyl group or a (hetero)aryl group comprising from 5 to 10 atoms,
and/or $Y_6$ and $Y_7$ together form, with the carbon atoms that carry them, a (hetero)cycloalkyl group or a (hetero)aryl group comprising from 5 to 10 atoms,
said compound with formula (I") being able to be in salt or pure stereoisomer form or in the form of a mixture of enantiomers and/or diastereoisomers, including racemic mixtures, or in the form of tautomers,
wherein at least one of $Y_1$, $Y_2$, $Y_3$, $Y_4$, $Y_5$, $Y_6$ and $Y_7$ is a group with formula -L'-Z'.

**13.** A use of a conjugate according to claim 11 or 12, for detecting a biological molecule by fluorescence.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014008197 A
- WO 2010082050 A


### Littérature non-brevet citée dans la description


- **QI LU et al.** *Heteroatom Chemistry,* 1992
- *Eur.J.O.Chem,* 2013, vol. 19, 4131-4145